(19) Europäisches Patentamt
European Patent Office
Office européen des brevets

(11) EP 2 402 758 B1

(12) **EUROPEAN PATENT SPECIFICATION**

(45) Date of publication and mention of the grant of the patent:
**10.09.2014 Bulletin 2014/37**

(51) Int Cl.:
***G01N 33/574*** *(2006.01)* ***G01N 33/48*** *(2006.01)*
***C12Q 1/68*** *(2006.01)*

(21) Application number: **11163930.8**

(22) Date of filing: **19.09.2006**

(54) **Methods and uses for identifying the origin of a carcinoma of unknown primary origin**

Verfahren und Verwendungen zum Identifizieren des Ursprungs eines Karzinoms mit unbekanntem primären Ursprung

Procédés et utilisations pour identifier l'origine d'un carcinome d'origine primaire inconnue

(84) Designated Contracting States:
**AT BE BG CH CY CZ DE DK EE ES FI FR GB GR HU IE IS IT LI LT LU LV MC NL PL PT RO SE SI SK TR**

(30) Priority: **19.09.2005 US 718501 P**
**12.10.2005 US 725680 P**

(43) Date of publication of application:
**04.01.2012 Bulletin 2012/01**

(62) Document number(s) of the earlier application(s) in accordance with Art. 76 EPC:
**06814892.3 / 1 934 615**

(73) Proprietor: **Janssen Diagnostics, LLC**
**Raritan, NJ 08869 (US)**

(72) Inventors:
- **Wang, Yixin**
**Basking Ridge, NJ 07920 (US)**
- **Mazumder, Abhijit**
**Basking Ridge, NJ 07970 (US)**
- **Talantov, Dmitri**
**San Diego, CA 92130 (US)**
- **Jatkoe, Timothy**
**Bedminster, NJ 07921 (US)**
- **Baden, Jonathan**
**Bridgewater, NJ 08807 (US)**

(74) Representative: **Goodfellow, Hugh Robin**
**Carpmaels & Ransford LLP**
**One Southampton Row**
**London WC1B 5HA (GB)**

(56) References cited:
**WO-A-2004/081564 WO-A-2006/002433**
**WO-A-2008/095152**

- **TOTHILL RICHARD W ET AL: "An expression-based site of origin diagnostic method designed for clinical application to cancer of unknown origin", CANCER RESEARCH, vol. 65, no. 10, May 2005 (2005-05), pages 4031-4040, XP002508605, ISSN: 0008-5472**
- **PHILLIP BUCKHAULTS ET AL: "Identifying tumor origin using a gene expression-based classification map.", CANCER RESEARCH, vol. 63, no. 14, 1 July 2003 (2003-07-01), pages 4144-4149, XP055027317, ISSN: 0008-5472**
- **DENNIS JAYNE L ET AL: "Identification from public data of molecular markers of adenocarcinoma characteristic of the site of origin", CANCER RESEARCH, AMERICAN ASSOCIATION FOR CANCER RESEARCH, BALTIMORE, MD.; US, vol. 62, no. 21, 1 November 2002 (2002-11-01), pages 5999-6005, XP002494617,**
- **DENNIS JAYNE L ET AL: "Hunting the primary: novel strategies for defining the origin of tumours", JOURNAL OF PATHOLOGY, vol. 205, no. 2, January 2005 (2005-01), pages 236-247, XP002508604, ISSN: 0022-3417**
- **DENNIS JAYNE L ET AL: "Markers of adenocarcinoma characteristic of the site of origin: development of a diagnostic algorithm", CLINICAL CANCER RESEARCH, THE AMERICAN ASSOCIATION FOR CANCER RESEARCH, US, vol. 11, no. 10, 15 May 2005 (2005-05-15), pages 3766-3772, XP002494618, ISSN: 1078-0432, DOI: 10.1158/1078-0432.CCR-04-2236**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **BLOOM G ET AL: "Multi-platform, multi-site, microarray-based human tumor classification", AMERICAN JOURNAL OF PATHOLOGY, AMERICAN SOCIETY FOR INVESTIGATIVE PATHOLOGY, US, vol. 164, no. 1, 1 January 2004 (2004-01-01), pages 9-16, XP002365381, ISSN: 0002-9440**
- **TALANTOV DIMITRI ET AL: "A quantitative reverse transcriptase-polymerase chain reaction assay to identify metastatic carcinoma tissue of origin", JOURNAL OF MOLECULAR DIAGNOSTICS, AMERICAN SOCIETY FOR INVESTIGATIVE PATHOLOGY, BETHESDA, MD, US, vol. 8, no. 3, 1 July 2006 (2006-07-01), pages 320-329, XP002494616, ISSN: 1525-1578**
- **VARADHACHARY GAURI R ET AL: "Molecular profiling of carcinoma of unknown primary and correlation with clinical evaluation", JOURNAL OF CLINICAL ONCOLOGY, AMERICAN SOCIETY OF CLINICAL ONCOLOGY, US, vol. 26, no. 27, 20 September 2008 (2008-09-20), pages 4442-4448, XP009109641, ISSN: 0732-183X**
- **IBRAHIM C K ET AL: "Validation of a 10 gene multiplex quantitative reverse transcriptase-polymerase chain reaction (qRT-PCR) assay to detect the primary site of metastatic carcinoma of unknown origin (CUP)", MODERN PATHOLOGY, vol. 20, no. Suppl. 2, March 2007 (2007-03), page 350A, XP002508606, & 96TH ANNUAL MEETING OF THE UNITED-STATES-AND-CANADIAN-ACADEMY-OF-PATH OLOGY; SAN DIEGO, CA, USA; MARCH 24 -30, 2007 ISSN: 0893-3952**

SEQUENCE LISTING

<110> Veridex, LLC

<120> Methods and Materials for Identifying the origin of a Carcinoma of Unknown Primary origin

<130> P054566EP

<150> US60/718,501
<151> 2005-09-19

<150> US60/725,680
<151> 2005-10-12

<160> 107

<170> PatentIn version 3.3

<210> 1
<211> 476
<212> DNA
<213> Homo sapiens

<400> 1

```
gaaaaaccag ccactgcttt acaggacagg gggttgaagc tgagccccgc ctcacaccca      60
cccccatgca ctcaaagatt ggattttaca gctacttgca attcaaaatt cagaagaata     120
aaaaatggga acatacagaa ctctaaaaga tagacatcag aaattgttaa gttaagcttt     180
ttcaaaaaat cagcaattcc ccagcgtagt caagggtgga cactgcacgc tctggcatga     240
tgggatggcg accgggcaag ctttcttcct cgagatgctc tgctgcttga gagctattgc     300
tttgttaaga tataaaaagg ggtttctttt tgtctttctg taaggtggac ttccagattt     360
tgattgaaag tcctagggtg attctatttc tgctgtgatt tatctgctga aagctcagct     420
ggggttgtgc aagctaggga cccattcctg tgtaatacaa tgtctgcacc aatgct         476
```

<210> 2
<211> 493
<212> DNA
<213> Homo sapiens

<400> 2

gtgattcaaa tgggttttcc acgctagggc ggggcacaga ttggagaggg ctctgtgctg 60
acatggctct ggactctaaa gaccaaactt cactctgggc acactctgcc agcaaagagg 120
actcgcttgt aaataccagg attttttttt ttttttgaag ggaggacggg agctggggag 180
aggaaagagt cttcaacata acccacttgt cactgacaca aaggaagtgc cccctccccg 240
gcaccctctg gccgcctagg ctcagcggcg accgccctcc gcgaaaatag tttgtttaat 300
gtgaacttgt agctgtaaaa cgctgtcaaa agttggacta aatgcctagt ttttagtaat 360
ctgtacattt tgttgtaaaa agaaaaacca ctcccagtcc ccagcccttc acatttttta 420
tgggcattga caaatctgtg tatattattt ggcagtttgg tatttgcggc gtcagtcttt 480
ttctgttgta act 493

<210> 3
<211> 545
<212> DNA
<213> Homo sapiens

<400> 3

ccatcccata gaagtccagc agacaggatt tgttaagtgc cagactttgt caggaagtca 60
aggagcttct gctttgtccg cctctgggtc tgtccagcca gctgtttcca tccctgaccc 120
tctgcagcat ggtaactatt tagtaacgga gacttactcg gcttctggtt ccctcgtgca 180
accttccact gcaggctttg atccacttct cacacaaaat gtgatagtga cagaaagggt 240
gatctgtccc atttccagtg ttcctggcaa cctagctggc ccaacgcagc tacgagggtc 300
acatactatg ctctgtacag aggatccttg ctcccgtcta atatgaccag aatgagctgg 360
aataccacac tgaccaaatc tggatctttg gactaaagta ttcaaaatag catagcaaag 420
ctcactgtat tgggctaata atttggcact tattagcttc tctcataaac tgatcacgat 480
tataaattaa atgtttgggt tcataccccca aaagcaatat gttgtcactc ctaattctca 540
agtac 545

<210> 4
<211> 284
<212> DNA
<213> Homo sapiens

<400> 4

ctgcacccac ctacttagat atttcatgtg ctatagacat tagagagatt tttcattttt 60
ccatgacatt tttcctctct gcaaatggct tagctacttg tgtttttccc ttttggggca 120
agacagactc attaaatatt ctgtacattt tttctttatc aaggagatat atcagtgttg 180
tctcatagaa ctgcctggat tccatttatg ttttttctga ttccatcctg tgtccccttc 240
atccttgact cctttggtat ttcactgaat ttcaaacatt tgtc 284

<210> 5

<211> 394
<212> DNA
<213> Homo sapiens

<220>
<221> misc_feature
<222> (58)..(58)
<223> n is a, c, g, or t

<220>
<221> misc_feature
<222> (95)..(95)
<223> n is a, c, g, or t

<220>
<221> misc_feature
<222> (99)..(99)
<223> n is a, c, g, or t

<220>
<221> misc_feature
<222> (119)..(119)
<223> n is a, c, g, or t

<220>
<221> misc_feature
<222> (123)..(123)
<223> n is a, c, g, or t

<220>
<221> misc_feature
<222> (130)..(130)
<223> n is a, c, g, or t

<220>
<221> misc_feature
<222> (151)..(151)
<223> n is a, c, g, or t

<220>
<221> misc_feature
<222> (155)..(155)
<223> n is a, c, g, or t

<220>
<221> misc_feature
<222> (161)..(161)
<223> n is a, c, g, or t

<220>
<221> misc_feature
<222> (212)..(212)
<223> n is a, c, g, or t

<400> 5

```
ttcctgaggc acatcctaac gcaagtttga ccatgtatgt ttgcacccct tttccccnaa      60
ccctgacctt cccatgggcc ttttccagga ttccnaccng gcagatcagt tttagtgana     120
canatccgcn tgcagatggc ccctccaacc ntttntgttg ntgtttccat ggcccagcat     180
tttccaccct taaccctgtg ttcaggcact tnttccccca ggaagccttc cctgcccacc     240
ccatttatga attgagccag gtttggtccg tggtgtcccc cgcacccagc aggggacagg     300
caatcaggag ggcccagtaa aggctgagat gaagtggact gagtagaact ggaggacaag     360
agttgacgtg agttcctggg agtttccaga gatg                                 394
```

<210> 6
<211> 470
<212> DNA
<213> Homo sapiens

<220>
<221> misc_feature
<222> (61)..(61)
<223> n is a, c, g, or t

<220>
<221> misc_feature
<222> (82)..(82)
<223> n is a, c, g, or t

<400> 6

```
atcctctaca gccagatgtc acagggatac gtctactttc acttggtgct ggagaattca      60
naagtcaaga acatgctaag cntaagggac ccaaggtaga aagagatcaa gcagcaaagc     120
acaggttctc ctggatgaaa ttactagcac ataaagttgg gagacaccta agccaagaca     180
ctggttctcc ttccggaatg aggccctggg aggaccttcc tagccaagac actggttctc     240

cttccagaat gaggccctgg aaggaccctc ctagtgatct gttactctta aaacaaagta     300
actcatctaa gattttggtt gggagatggc atttggcttc tgagaaaggt agctatgaaa     360
taatccaaga tactgatgaa gacacagctg ttaacaattg gctgatcagc ccccagaatg     420
cctcacgtgc ttggggagaa agcacccctc ttgccaacaa gcctggaaag                470
```

<210> 7
<211> 396
<212> DNA
<213> Homo sapiens

<400> 7

```
gcagcagcct caccatgaag ttgctgatgg tcctcatgct ggcggccctc tcccagcact     60
gctacgcagg ctctggctgc cccttattgg agaatgtgat ttccaagaca atcaatccac    120
aagtgtctaa gactgaatac aaagaacttc ttcaagagtt catagacgac aatgccacta    180
caaatgccat agatgaattg aaggaatgtt ttcttaacca aacggatgaa actctgagca    240
atgttgaggt gtttatgcaa ttaatatatg acagcagtct ttgtgattta ttttaacttt    300
ctgcaagacc tttggctcac agaactgcag ggtatggtga gaaaccaact acggattgct    360
gcaaaccaca ccttctcttt cttatgtctt tttact                              396
```

<210> 8
<211> 491
<212> DNA
<213> Homo sapiens

<400> 8

```
gagtggggcc cttaaactgg attcaaaaaa tgctctaaac ataggaatgg ttgaagaggt     60
cttgcagtct tcagatgaaa ctaaatctct agaagaggca caagaatggc taaagcaatt    120
catccaaggg ccaccggaag taattagagc tttgaaaaaa tctgtttgtt caggcagaga    180
gctatatttg gaggaagcat tacagaacga aagagatctt ttaggaacag tttggggtgg    240
gcctgcaaat ttagaggcta ttgctaagaa aggaaaattt aataaataat tggtttttcg    300
tgtggatgta ctccaagtaa agctccagtg actaatatgt ataaatgtta aatgatatta    360
aatatgaaca tcagttaaaa aaaaaattct ttaaggctac tattaatatg cagacttact    420
tttaatcatt tgaaatctga actcatttac ctcatttctt gccaattact cccttgggta    480
tttactgcgt a                                                         491
```

<210> 9
<211> 265
<212> DNA
<213> Homo sapiens

<400> 9

```
tggtgtaatt ttgtcctctc tgtgtcctgg ggaatactgg ccatgcctgg agacatatca     60
ctcaatttct ctgaggacac agataggatg ggtgtctgt gttatttgtg gggtacagag     120
atgaaagagg ggtgggatcc acactgagag agtggagagt gacatgtgct ggacactgtc    180
catgaagcac tgagcagaag ctggaggcac aacgcaccag acactcacag caaggatgga    240

gctgaaaaca taacccactc tgtcc                                          265
```

<210> 10
<211> 441
<212> DNA
<213> Homo sapiens

<400> 10

```
atagatgtac atacctcctt gcacaaatgg aggggaattc attttcatca ctgggagtgt      60
ccttagtgta taaaaaccat gctggtatat ggcttcaagt tgtaaaaatg aaagtgactt     120
taaaagaaaa taggggatgg tccaggatct ccactgataa gactgttttt aagtaactta     180
aggacctttg ggtctacaag tatatgtgaa aaaaatgaga cttactgggt gaggaaatcc     240
attgtttaaa gatggtcgtg tgtgtgtgtg tgtgtgtgtg tgtgttgtgt tgtgttttgt     300
tttttaaggg agggaattta ttatttaccg ttgcttgaaa ttactgtgta aatatatgtc     360
tgataatgat ttgctctttg acaactaaaa ttaggactgt ataagtacta gatgcatcac     420
tgggtgttga tcttacaaga t                                               441
```

<210> 11
<211> 21
<212> DNA
<213> Homo sapiens

<400> 11
cacagccccg acctttgatg a 21

<210> 12
<211> 19
<212> DNA
<213> Homo sapiens

<400> 12
ggtcccagag cccgtctca 19

<210> 13
<211> 26
<212> DNA
<213> Homo sapiens

<400> 13
agctgtccag ctgcaaagga aaagcc 26

<210> 14
<211> 75
<212> DNA
<213> Homo sapiens

<400> 14

```
cacagccccg acctttgatg agaactcagc tgtccagctg caaaggaaaa gccaagtgag      60
acgggctctg ggacc                                                       75
```

<210> 15
<211> 17
<212> DNA
<213> Homo sapiens

<400> 15
ccaacccaga cccgcgc 17

<210> 16
<211> 21
<212> DNA
<213> Homo sapiens

<400> 16
cgcccatgcc gctcatgttc a 21

<210> 17
<211> 21
<212> DNA
<213> Homo sapiens

<400> 17
cccgccatct cccgcttcat g 21

<210> 18
<211> 78
<212> DNA
<213> Homo sapiens

<400> 18

ccaacccaga cccgcgcttc cccgccatct cccgcttcat gggcccggcg agcggcatga 60

acatgagcgg catgggcg 78

<210> 19
<211> 23
<212> DNA
<213> Homo sapiens

<400> 19
gagagaagga gaagataact caa 23

<210> 20
<211> 22
<212> DNA
<213> Homo sapiens

<400> 20
actccagaga ttcggtaggt ga 22

<210> 21
<211> 26
<212> DNA
<213> Homo sapiens

<400> 21
attgccaaga ttacttcaga ttacca 26

<210> 22
<211> 97
<212> DNA
<213> Homo sapiens

<400> 22

gcagagaagg agaagataac tcaaaaagaa acccaattgc caagattact tcagattacc 60

aagcaaccca gaaaatcacc taccgaatct ctggagt 97

<210> 23
<211> 21
<212> DNA
<213> Homo sapiens

<400> 23
tccctcggca gtggaagctt a 21

<210> 24
<211> 24
<212> DNA
<213> Homo sapiens

<400> 24
tcctcaaact ctgtgtgcct ggta 24

<210> 25
<211> 29
<212> DNA
<213> Homo sapiens

<400> 25
ccaaaatcaa tggtactcat gcccgactg 29

<210> 26
<211> 95
<212> DNA
<213> Homo sapiens

<400> 26

tccctcggca gtggaagctt acaaaacgac tgggaagttt ccaaaatcaa tggtactcat 60

gcccgactgt ctaccaggca cacagagttt gagga 95

<210> 27
<211> 21
<212> DNA
<213> Homo sapiens

<400> 27
agttgctgat ggtcctcatg c 21

<210> 28
<211> 24
<212> DNA
<213> Homo sapiens

<400> 28
cacttgtgga ttgattgtct tgga 24

<210> 29

<211> 23
<212> DNA
<213> Homo sapiens

<400> 29
ccctctccca gcactgctac gca 23

<210> 30
<211> 107
<212> DNA
<213> Homo sapiens

<400> 30

agttgctgat ggtcctcatg ctggcggccc tctcccagca ctgctacgca ggctctggct 60
gccccttatt ggagaatgtg atttccaaga caatcaatcc acaagtg 107

<210> 31
<211> 20
<212> DNA
<213> Homo sapiens

<400> 31
cgcccacctg gacatctgga 20

<210> 32
<211> 23
<212> DNA
<213> Homo sapiens

<400> 32
cactggtcga ggcacagtag tga 23

<210> 33
<211> 25
<212> DNA
<213> Homo sapiens

<400> 33
gtcagcggcc tggatgaaag agcgg 25

<210> 34
<211> 86
<212> DNA
<213> Homo sapiens

<400> 34

cgcccacctg gacatctgga agtcagcggc ctggatgaaa gagcggactt cacctggggc 60
gattcactac tgtgcctcga ccagtg 86

<210> 35
<211> 23
<212> DNA
<213> Homo sapiens

<400> 35
gcggagccca atacagaata cac 23

<210> 36
<211> 19
<212> DNA
<213> Homo sapiens

<400> 36
cggggctact ccaggcaca 19

<210> 37
<211> 25
<212> DNA
<213> Homo sapiens

<400> 37
tcagaggcat tcaggatgtg cgacg 25

<210> 38
<211> 80
<212> DNA
<213> Homo sapiens

<400> 38

gcggagccca atacagaata cacacgcacg gtgtcttcag aggcattcag gatgtgcgac 60

gtgtgcctgg agtagccccg 80

<210> 39
<211> 20
<212> DNA
<213> Homo sapiens

<400> 39
ctgttgatgg caggcttggc 20

<210> 40
<211> 20
<212> DNA
<213> Homo sapiens

<400> 40
ttgctcacct gggctttgca 20

<210> 41
<211> 21
<212> DNA
<213> Homo sapiens

<400> 41
gcagccaggc actgccctgc t 21

<210> 42
<211> 74
<212> DNA
<213> Homo sapiens

<400> 42

ctgttgatgg caggcttggc cctgcagcca ggcactgccc tgctgtgcta ctcctgcaaa 60

gcccaggtga gcaa 74

<210> 43
<211> 25
<212> DNA
<213> Homo sapiens

<400> 43
tgaagaaata tcctgggatt attca 25

<210> 44
<211> 27
<212> DNA
<213> Homo sapiens

<400> 44
tatgtggtat cttctggaat atcatca 27

<210> 45
<211> 27
<212> DNA
<213> Homo sapiens

<400> 45
acaaagggaa acagatattg aagactc 27

<210> 46
<211> 87
<212> DNA
<213> Homo sapiens

<400> 46

tgaagaaata tcctgggatt attcagaatt tgtacaaagg gaaacagata ttgaagactc 60

tgatgatatt ccagaagata ccacata 87

<210> 47
<211> 19
<212> DNA
<213> Homo sapiens

<400> 47
cccccagtgg gtcctcaca 19

<210> 48
<211> 22
<212> DNA
<213> Homo sapiens

<400> 48
aggatgaaac aagctgtgcc ga 22

<210> 49

<211> 26
<212> DNA
<213> Homo sapiens

<400> 49
caggaacaaa agcgtgatct tgctgg 26

<210> 50
<211> 82
<212> DNA
<213> Homo sapiens

<400> 50

cccccagtgg gtcctcacag ctgcccactg catcaggaac aaaagcgtga tcttgctggg 60

tcggcacagc ttgtttcatc ct 82

<210> 51
<211> 19
<212> DNA
<213> Homo sapiens

<400> 51
gccctgaggc actcttcca 19

<210> 52
<211> 22
<212> DNA
<213> Homo sapiens

<400> 52
cggatgtcca cgtcacactt ca 22

<210> 53
<211> 25
<212> DNA
<213> Homo sapiens

<400> 53
cttccttcct gggcatggag tcctg 25

<210> 54
<211> 100
<212> DNA
<213> Homo sapiens

<400> 54

gccctgaggc actcttccag ccttccttcc tgggcatgga gtcctgtggc atccacgaaa 60

ctaccttcaa ctccatcatg aagtgtgacg tggacatccg 100

<210> 55
<211> 22
<212> DNA
<213> Homo sapiens

<400> 55
ccacacacag cctactttcc aa 22

<210> 56
<211> 21
<212> DNA
<213> Homo sapiens

<400> 56
tacccacgcg aatcactctc a 21

<210> 57
<211> 27
<212> DNA
<213> Homo sapiens

<400> 57
aacggcaatg cggctgcaac ggcggaa 27

<210> 58
<211> 103
<212> DNA
<213> Homo sapiens

<400> 58

ccacacacag cctactttcc aagcggagcc atgtctggta acggcaatgc ggctgcaacg 60

gcggaagaaa acagcccaaa gatgagagtg attcgcgtgg gta 103

<210> 59
<211> 2724
<212> DNA
<213> Homo sapiens

<400> 59

ggtgccatgg ctgagtcaca cctgctgcag tggctgctgc tgctgctgcc cacgctctgt 60
ggcccaggca ctgctgcctg gaccacctca tccttggcct gtgcccaggg ccctgagttc 120
tggtgccaaa gcctggagca agcattgcag tgcagagccc tagggcattg cctacaggaa 180
gtctggggac atgtgggagc cgatgaccta tgccaagagt gtgaggacat cgtccacatc 240
cttaacaaga tggccaagga ggccattttc caggacacga tgaggaagtt cctggagcag 300
gagtgcaacg tcctcccctt gaagctgctc atgccccagt gcaaccaagt gcttgacgac 360
tacttccccc tggtcatcga ctacttccag aaccagactg actcaaacgg catctgtatg 420
cacctgggcc tgtgcaaatc ccggcagcca gagccagagc aggagccagg gatgtcagac 480
cccctgccca aacctctgcg ggaccctctg ccagaccctc tgctggacaa gctcgtcctc 540
cctgtgctgc ccggggccct ccaggcgagg cctgggcctc acacacagga tctctccgag 600
cagcaattcc ccattcctct cccctattgc tggctctgca gggctctgat caagcggatc 660
caagccatga ttcccaaggg tgcgctagct gtggcagtgg cccaggtgtg ccgcgtggta 720
cctctggtgg cgggcggcat ctgccagtgc ctggctgagc gctactccgt catcctgctc 780
gacacgctgc tgggccgcat gctgccccag ctggtctgcc gcctcgtcct ccggtgctcc 840
atggatgaca gcgctggccc aaggtcgccg acaggagaat ggctgccgcg agactctgag 900
tgccacctct gcatgtccgt gaccacccag gccgggaaca gcagcgagca ggccatacca 960
caggcaatgc tccaggcctg tgttggctcc tggctggaca gggaaaagtg caagcaattt 1020
gtggagcagc acacgcccca gctgctgacc ctggtgccca ggggctggga tgcccacacc 1080
acctgccagg ccctcggggt gtgtgggacc atgtccagcc ctctccagtg tatccacagc 1140
cccgaccttt gatgagaact cagctgtcca gaaaaagaca ccgtccttta aagtgctgca 1200
gtatggccag acgtggtggc tcacacctgc aatcccagca ccttaggagg ccgaggcagg 1260
aggatccttg aggtcaggag ttcgagacca gcctcgccaa catggtgaaa ccccatttct 1320
actaaaaata caaaaaatta gccaagtgtg gtggcatatg cctgtaatcc caactactca 1380
gaaggccgag gcaggagaat tacttgaacg caggagaatc actgcagccc aggaggcaga 1440
ggttgcagtg agccgagatt gcaccactgc actccagcct gggtgacaga gcaagactcc 1500
atctcagtaa ataaataaat aaataaaaag cgctgcagta gctgtggcct caccctgaag 1560
tcagcgggcc caggcctacc tcactctctc ccttggcaga gaagcagacg tccatagctc 1620
ctctccctca caagcgctcc cagcctgccc tccagctgct gctctcccct cccagtctct 1680
actcactggg atgaggttag gtcatgagga caccaaaaac ctaaaaataa acaaaaagcc 1740
aaacaagcct tagcttttct taaagactga aatgcctgga agtgtccctt tatttataaa 1800
ataacttttg tcatatttct tatacatgtt tcttgtaaga aattcagaaa ctacagacaa 1860
agagagtgga aattacccac tgtcaggcct ctgagcccaa gctaagccat catatcccct 1920

gtgccctgca cgtatacacc cagatggcct gaagcaactg aagatccaca aaagaagtga 1980
aaatagccag ttcctgcctt aactgatgac attccaccat tgtgatttgt tcctgcccca 2040
ccctaactga tcaattgacc ttgtgacaat acaccttccc cacccttgag aaggtgcttt 2100
gtaatattct ccccacccac cccacgcccg cacccccgca cccttaagaa ggtattttgt 2160
aatattctct ccgccattga gaatgtgctt tgtaagatcc accccctgcc cacaaaaaat 2220
tgctcctaac tccaccgcct atcccaaacc tacaagaact aatgataatc ccaccaccct 2280
ttgctgactc tttttggact cagcccacct gcacccaggt gattaaaaag ctttattgtt 2340
cacacaaagc ctgtttggta gtctcttcac agggaagcat gtgacaccca caatcccacc 2400
tagcccagga gagagctacg gcagggtgtg tgttttgaca ctgagcttgg ggctttttcc 2460
atcttctccc cacagcctct ggctccacac ctccaccgtt caagcgccag aaagagctgt 2520
ctatgcagcc tgctcttggg cctggggatg agacacacaa ttcattggct cctggatttt 2580
aagtagacat ttgtaaatct atagctaact actgtcctta aagccattgt ttccattaca 2640
aaatccaact ctctgagaga aaagggtgtt ttaaatttaa aaaaataaaa acaaaaaagt 2700
ttgattgaga aaaaaaaaaa aaaa 2724

<210> 60
<211> 2352
<212> DNA
<213> Homo sapiens

<400> 60

```
gaaacttaaa ggtgtttacc ttgtcatcag catgtaagct aattatctcg ggcaagatgt     60
aggcttctat tgtcttgttg ctttagcgct tacgccccgc ctctggtggc tgcctaaaac    120
ctggcgccgg gctaaaacaa acgcgaggca gcccccgagc ctccactcaa gccaattaag    180
gaggactcgg tccactccgt tacgtgtaca tccaacaaga tcggcgttaa ggtaacacca    240
gaatatttgg caaagggaga aaaaaaaagc agcgaggctt cgccttcccc ctctcccttt    300
tttttcctcc tcttccttcc tcctccagcc gccgccgaat catgtcgatg agtccaaagc    360
acacgactcc gttctcagtg tctgacatct tgagtcccct ggaggaaagc tacaagaaag    420
tgggcatgga gggcggcggc ctcggggctc cgctggcggc gtacaggcag ggccaggcgg    480
caccgccaac agcggccatg cagcagcacg ccgtggggca ccacggcgcc gtcaccgccg    540
cctaccacat gacggcggcg ggggtgcccc agctctcgca ctccgccgtg gggggctact    600
gcaacggcaa cctgggcaac atgagcgagc tgccgccgta ccaggacacc atgaggaaca    660
gcgcctctgg ccccggatgg tacggcgcca acccagaccc gcgcttcccc gccatctccc    720
gcttcatggg cccggcgagc ggcatgaaca tgagcggcat gggcggcctg ggctcgctgg    780
gggacgtgag caagaacatg gccccgctgc caagcgcgcc gcgcaggaag cgccgggtgc    840
tcttctcgca ggcgcaggtg tacgagctgg agcgacgctt caagcaacag aagtacctgt    900
cggcgccgga gcgcgagcac ctggccagca tgatccacct gacgcccacg caggtcaaga    960
tctggttcca gaaccaccgc tacaaaatga agcgccaggc caaggacaag gcggcgcagc   1020
```

agcaactgca gcaggacagc ggcggcggcg ggggcggcgg gggcaccggg tgcccgcagc 1080
agcaacaggc tcagcagcag tcgccgcgac gcgtggcggt gccggtcctg gtgaaagacg 1140
gcaaaccgtg ccaggcgggt gcccccgcgc cgggcgccgc cagcctacaa ggccacgcgc 1200
agcagcaggc gcagcaccag gcgcaggccg cgcaggcggc ggcagcggcc atctccgtgg 1260
gcagcggtgg cgccggcctt ggcgcacacc cgggccacca gccaggcagc gcaggccagt 1320
ctccggacct ggcgcaccac gccgccagcc ccgcggcgct gcagggccag gtatccagcc 1380
tgtcccacct gaactcctcg ggctcggact acggcaccat gtcctgctcc accttgctat 1440
acggtcggac ctggtgagag gacgccgggc cggccctagc ccagcgctct gcctcaccgc 1500
ttccctcctg cccgccacac agaccaccat ccaccgctgc tccacgcgct tcgacttttc 1560
ttaacaacct ggccgcgttt agaccaagga acaaaaaaac cacaaaggcc aaactgctgg 1620
acgtctttct ttttttcccc ccctaaaatt tgtgggtttt ttttttaaa aaaagaaaat 1680
gaaaaacaac caagcgcatc caatctcaag gaatctttaa gcagagaagg gcataaaaca 1740
gctttggggt gtcttttttt ggtgattcaa atgggttttc cacgctaggg cggggcacag 1800
attggagagg gctctgtgct gacatggctc tggactctaa agaccaaact tcactctggg 1860
cacactctgc cagcaaagag gactcgcttg taaataccag gatttttttt ttttttttgaa 1920
gggaggacgg gagctgggga gaggaaagag tcttcaacat aacccacttg tcactgacac 1980
aaaggaagtg cccccctcccc ggcaccctct ggccgcctag gctcagcggc gaccgccctc 2040
cgcgaaaata gtttgtttaa tgtgaacttg tagctgtaaa acgctgtcaa aagttggact 2100
aaatgcctag tttttagtaa tctgtacatt ttgttgtaaa aagaaaaacc actcccagtc 2160
cccagccctt cacatttttt atgggcattg acaaatctgt gtatattatt tggcagtttg 2220
gtatttgcgg cgtcagtctt tttctgttgt aacttatgta gatatttggc ttaaatatag 2280
ttcctaagaa gcttctaata aattatacaa attaaaaaga ttcttttct gattaaaaaa 2340
aaaaaaaaaa aa 2352

<210> 61
<211> 3336
<212> DNA
<213> Homo sapiens

<400> 61

```
ttttcttaga cattaactgc agacggctgg caggatagaa gcagcggctc acttggactt    60
tttcaccagg gaaatcagag acaatgatgg ggctcttccc cagaactaca ggggctctgg   120
ccatcttcgt ggtggtcata ttggttcatg gagaattgcg aatagagact aaaggtcaat   180
atgatgaaga agagatgact atgcaacaag ctaaaagaag gcaaaaacgt gaatgggtga   240
aatttgccaa accctgcaga gaaggagaag ataactcaaa aagaaaccca attgccaaga   300
ttacttcaga ttaccaagca acccagaaaa tcacctaccg aatctctgga gtgggaatcg   360
atcagccgcc ttttggaatc tttgttgttg acaaaaacac tggagatatt aacataacag   420
ctatagtcga ccgggaggaa actccaagct tcctgatcac atgtcgggct ctaaatgccc   480
```

aaggactaga tgtagagaaa ccacttatac taacggttaa aattttggat attaatgata 540
atcctccagt attttcacaa caaattttca tgggtgaaat tgaagaaaat agtgcctcaa 600
actcactggt gatgatacta aatgccacag atgcagatga accaaaccac ttgaattcta 660
aaattgcctt caaaattgtc tctcaggaac cagcaggcac acccatgttc ctcctaagca 720
gaaacactgg ggaagtccgt actttgacca attctcttga ccgagagcaa gctagcagct 780
atcgtctggt tgtgagtggt gcagacaaag atggagaagg actatcaact caatgtgaat 840
gtaatattaa agtgaaagat gtcaacgata acttcccaat gtttagagac tctcagtatt 900
cagcacgtat tgaagaaaat attttaagtt ctgaattact tcgatttcaa gtaacagatt 960
tggatgaaga gtacacagat aattggcttg cagtatattt ctttacctct gggaatgaag 1020
gaaattggtt tgaaatacaa actgatccta gaactaatga aggcatcctg aaagtggtga 1080
aggctctaga ttatgaacaa ctacaaagcg tgaaacttag tattgctgtc aaaaacaaag 1140
ctgaatttca ccaatcagtt atctctcgat accgagttca gtcaacccca gtcacaattc 1200
aggtaataaa tgtaagagaa ggaattgcat tccgtcctgc ttccaagaca tttactgtgc 1260
aaaaaggcat aagtagcaaa aaattggtgg attatatcct gggaacatat caagccatcg 1320
atgaggacac taacaaagct gcctcaaatg tcaaatatgt catgggacgt aacgatggtg 1380
gatacctaat gattgattca aaaactgctg aaatcaaatt tgtcaaaaat atgaaccgag 1440
attctacttt catagttaac aaaacaatca cagctgaggt tctggccata gatgaataca 1500
cgggtaaaac ttctacaggc acggtatatg ttagagtacc cgatttcaat gacaattgtc 1560
caacagctgt cctcgaaaaa gatgcagttt gcagttcttc accttccgtg gttgtctccg 1620
ctagaacact gaataataga tacactggcc cctatacatt tgcactggaa gatcaacctg 1680
taaagttgcc tgccgtatgg agtatcacaa ccctcaatgc tacctcggcc ctcctcagag 1740
cccaggaaca gatacctcct ggagtatacc acatctccct ggtacttaca gacagtcaga 1800
acaatcggtg tgagatgcca cgcagcttga cactggaagt ctgtcagtgt gacaacaggg 1860
gcatctgtgg aacttcttac ccaaccacaa gccctgggac caggtatggc aggccgcact 1920
cagggaggct ggggcctgcc gccatcggcc tgctgctcct tggtctcctg ctgctgctgt 1980
tggccccct tctgctgttg acctgtgact gtggggcagg ttctactggg ggagtgacag 2040
gtggttttat cccagttcct gatggctcag aaggaacaat tcatcagtgg ggaattgaag 2100
gagcccatcc tgaagacaag gaaatcacaa atatttgtgt gcctcctgta acagccaatg 2160
gagccgattt catggaaagt tctgaagttt gtacaaatac gtatgccaga ggcacagcgg 2220
tggaaggcac ttcaggaatg gaaatgacca ctaagcttgg agcagccact gaatctggag 2280
gtgctgcagg ctttgcaaca gggacagtgt caggagctgc ttcaggattc ggagcagcca 2340
ctggagttgg catctgttcc tcagggcagt ctggaaccat gagaacaagg cattccactg 2400
gaggaaccaa taaggactac gctgatgggg cgataagcat gaattttctg gactcctact 2460
tttctcagaa agcatttgcc tgtgcggagg aagacgatgg ccaggaagca aatgactgct 2520

tgttgatcta tgataatgaa ggcgcagatg ccactggttc tcctgtgggc tccgtgggtt 2580
gttgcagttt tattgctgat gacctggatg acagcttctt ggactcactt ggacccaaat 2640
ttaaaaaact tgcagagata agccttggtg ttgatggtga aggcaaagaa gttcagccac 2700
cctctaaaga cagcggttat gggattgaat cctgtggcca tcccatagaa gtccagcaga 2760
caggatttgt taagtgccag actttgtcag gaagtcaagg agcttctgct ttgtccgcct 2820
ctgggtctgt ccagccagct gtttccatcc ctgaccctct gcagcatggt aactatttag 2880
taacggagac ttactcggct tctggttccc tcgtgcaacc ttccactgca ggctttgatc 2940
cacttctcac acaaaatgtg atagtgacag aaagggtgat ctgtcccatt tccagtgttc 3000
ctggcaacct agctggccca acgcagctac gagggtcaca tactatgctc tgtacagagg 3060
atccttgctc ccgtctaata tgaccagaat gagctggaat accacactga ccaaatctgg 3120
atctttggac taaagtattc aaaatagcat agcaaagctc actgtattgg gctaataatt 3180
tggcacttat tagcttctct cataaactga tcacgattat aaattaaatg tttgggttca 3240
taccccaaaa gcaatatgtt gtcactccta attctcaagt actattcaaa ttgtagtaaa 3300
tcttaaagtt tttcaaaacc ctaaaatcat attcgc 3336

<210> 62
<211> 3697
<212> DNA
<213> Homo sapiens

<400> 62

```
agggagtgtt cccgggggag atactccagt cgtagcaaga gtctcgacca ctgaatggaa    60
gaaaaggact tttaaccacc attttgtgac ttacagaaag gaatttgaat aaagaaaact   120
atgatacttc aggcccatct tcactccctg tgtcttctta tgctttattt ggcaactgga   180
tatggccaag aggggaagtt tagtggaccc ctgaaaccca tgacattttc tatttatgaa   240
ggccaagaac cgagtcaaat tatattccag tttaaggcca atcctcctgc tgtgactttt   300
gaactaactg gggagacaga caacatattt gtgatagaac gggagggact tctgtattac   360
aacagagcct tggacaggga aacaagatct actcacaatc tccaggttgc agccctggac   420
gctaatggaa ttatagtgga gggtccagtc cctatcacca tagaagtgaa ggacatcaac   480
gacaatcgac ccacgtttct ccagtcaaag tacgaaggct cagtaaggca gaactctcgc   540
ccaggaaagc ccttcttgta tgtcaatgcc acagacctgg atgatccggc cactcccaat   600
ggccagcttt attaccagat tgtcatccag cttcccatga tcaacaatgt catgtacttt   660
cagatcaaca acaaaacggg agccatctct cttacccgag agggatctca ggaattgaat   720
cctgctaaga atccttccta taatctggtg atctcagtga aggacatggg aggccagagt   780
gagaattcct tcagtgatac cacatctgtg gatatcatag tgacagagaa tatttggaaa   840
gcaccaaaac ctgtggagat ggtggaaaac tcaactgatc ctcaccccat caaaatcact   900
caggtgcggt ggaatgatcc cggtgcacaa tattccttag ttgacaaaga gaagctgcca   960
agattcccat tttcaattga ccaggaagga gatatttacg tgactcagcc cttggaccga  1020
```

gaagaaaagg atgcatatgt tttttatgca gttgcaaagg atgagtacgg aaaaccactt 1080
tcatatccgc tggaaattca tgtaaaagtt aaagatatta atgataatcc acctacatgt 1140
ccgtcaccag taaccgtatt tgaggtccag gagaatgaac gactgggtaa cagtatcggg 1200
acccttactg cacatgacag ggatgaagaa aatactgcca acagttttct aaactacagg 1260
attgtggagc aaactcccaa acttcccatg gatggactct tcctaatcca aacctatgct 1320
ggaatgttac agttagctaa acagtccttg aagaagcaag atactcctca gtacaactta 1380
acgatagagg tgtctgacaa agatttcaag acccttttgtt ttgtgcaaat caacgttatt 1440
gatatcaatg atcagatccc catctttgaa aaatcagatt atggaaacct gactcttgct 1500
gaagacacaa acattgggtc caccatctta accatccagg ccactgatgc tgatgagcca 1560
tttactggga gttctaaaat tctgtatcat atcataaagg gagacagtga gggacgcctg 1620
ggggttgaca cagatcccca taccaacacc ggatatgtca taattaaaaa gcctcttgat 1680
tttgaaacag cagctgtttc caacattgtg ttcaaagcag aaaatcctga gcctctagtg 1740
tttggtgtga agtacaatgc aagttctttt gccaagttca cgcttattgt gacagatgtg 1800
aatgaagcac ctcaattttc ccaacacgta ttccaagcga aagtcagtga ggatgtagct 1860
ataggcacta aagtgggcaa tgtgactgcc aaggatccag aaggtctgga cataagctat 1920
tcactgaggg gagacacaag aggttggctt aaaattgacc acgtgactgg tgagatcttt 1980
agtgtggctc cattggacag agaagccgga agtccatatc gggtacaagt ggtggccaca 2040
gaagtagggg ggtcttcctt gagctctgtg tcagagttcc acctgatcct tatggatgtg 2100
aatgacaacc ctcccaggct agccaaggac tacacgggct tgttcttctg ccatcccctc 2160
agtgcacctg gaagtctcat tttcgaggct actgatgatg atcagcactt atttcggggt 2220
ccccatttta cattttccct cggcagtgga agcttacaaa acgactggga agtttccaaa 2280
atcaatggta ctcatgcccg actgtctacc aggcacacag agtttgagga gagggagtat 2340
gtcgtcttga tccgcatcaa tgatgggggt cggccacccct tggaaggcat tgtttcttta 2400
ccagttacat tctgcagttg tgtggaagga agttgtttcc ggccagcagg tcaccagact 2460
gggataccca ctgtgggcat ggcagttggt atactgctga ccacccttct ggtgattggt 2520
ataattttag cagttgtgtt tatccgcata aagaaggata aaggcaaaga taatgttgaa 2580
agtgctcaag catctgaagt caaacctctg agaagctgaa tttgaaaagg aatgtttgaa 2640
tttatatagc aagtgctatt tcagcaacaa ccatctcatc ctattacttt tcatctaacg 2700
tgcattataa ttttttaaac agatattccc tcttgtcctt taatatttgc taaatatttc 2760
ttttttgagg tggagtcttg ctctgtcgcc caggctggag tacagtggtg tgatcccagc 2820
tcactgcaac ctccgcctcc tgggttcaca tgattctcct gcctcagctt cctaagtagc 2880
tgggtttaca ggcacccacc accatgccca gctaattttt gtatttttaa tagagacggg 2940
gtttcgccat ttggccaggc tggtcttgaa ctcctgacgt caagtgatct gcctgccttg 3000
gtctcccaat acaggcatga accactgcac ccacctactt agatatttca tgtgctatag 3060
acattagaga gatttttcat ttttccatga catttttcct ctctgcaaat ggcttagcta 3120

```
cttgtgtttt tccctttgg ggcaagacag actcattaaa tattctgtac attttttctt    3180
tatcaaggag atatatcagt gttgtctcat agaactgcct ggattccatt tatgtttttt   3240
ctgattccat cctgtgtccc cttcatcctt gactcctttg gtatttcact gaatttcaaa   3300
catttgtcag agaagaaaaa cgtgaggact caggaaaaat aaataaataa aagaacagcc   3360
ttttccctta gtattaacag aaatgtttct gtgtcattaa ccatctttaa tcaatgtgac   3420
atgttgctct ttggctgaaa ttcttcaact tggaaatgac acagacccac agaaggtgtt   3480
caaacacaac ctactctgca aaccttggta aaggaaccag tcagctggcc agatttcctc   3540
actacctgcc atgcatacat gctgcgcatg ttttcttcat tcgtatgtta gtaaagtttt   3600
ggttattata tatttaacat gtggaagaaa acaagacatg aaaagagtgg tgacaaatca   3660
agaataaaca ctggttgtag tcagttttgt ttgttaa                            3697
```

<210> 63
<211> 503
<212> DNA
<213> Homo sapiens

<400> 63

```
gacagcggct tccttgatcc ttgccacccg cgactgaaca ccgacagcag cagcctcacc     60
atgaagttgc tgatggtcct catgctggcg gccctctccc agcactgcta cgcaggctct    120
ggctgcccct tattggagaa tgtgatttcc aagacaatca atccacaagt gtctaagact    180
gaatacaaag aacttcttca agagttcata gacgacaatg ccactacaaa tgccatagat    240
gaattgaagg aatgttttct taaccaaacg gatgaaactc tgagcaatgt tgaggtgttt    300
atgcaattaa tatatgacag cagtctttgt gatttatttt aactttctgc aagacctttg    360
gctcacagaa ctgcagggta tggtgagaaa ccaactacgg attgctgcaa accacacctt    420
ctctttctta tgtcttttta ctacaaacta caagacaatt gttgaaacct gctatacatg    480
tttattttaa taaattgatg gca                                            503
```

<210> 64
<211> 1894
<212> DNA
<213> Homo sapiens

<400> 64

gtctgacttc ctcccagcac attcctgcac tctgccgtgt ccacactgcc ccacagaccc 60
agtcctccaa gcctgctgcc agctccctgc aagcccctca ggttgggcct tgccacggtg 120
ccagcaggca gccctgggct gggggtaggg gactccctac aggcacgcag ccctgagacc 180
tcagagggcc acccccttgag ggtggccagg cccccagtgg ccaacctgag tgctgcctct 240
gccaccagcc ctgctggccc ctggttccgc tggcccccca gatgcctggc tgagacacgc 300
cagtggcctc agctgcccac acctcttccc ggcccctgaa gttggcactg cagcagacag 360
ctccctgggc accaggcagc taacagacac agccgccagc ccaaacagca gcggcatggg 420
cagcgccagc ccgggtctga gcagcgtatc ccccagccac ctcctgctgc cccccgacac 480

ggtgtcgcgg acaggcttgg agaaggcggc agcgggggca gtgggtctcg agagacggga 540
ctggagtccc agtccacccg ccacgcccga gcagggcctg tccgccttct acctctccta 600
ctttgacatg ctgtaccctg aggacagcag ctgggcagcc aaggcccctg gggccagcag 660
tcgggaggag ccacctgagg agcctgagca gtgcccggtc attgacagcc aagccccagc 720
gggcagcctg gacttggtgc ccggcgggct gaccttggag gagcactcgc tggagcaggt 780
gcagtccatg gtggtgggcg aagtgctcaa ggacatcgag acggcctgca agctgctcaa 840
catcaccgca gatcccatgg actggagccc cagcaatgtg cagaagtggc tcctgtggac 900
agagcaccaa taccggctgc cccccatggg caaggccttc caggagctgg cgggcaagga 960
gctgtgcgcc atgtcggagg agcagttccg ccagcgctcg cccctgggtg gggatgtgct 1020
gcacgcccac ctggacatct ggaagtcagc ggcctggatg aaagagcgga cttcacctgg 1080
ggcgattcac tactgtgcct cgaccagtga ggagagctgg accgacagcg aggtggactc 1140
atcatgctcc gggcagccca tccacctgtg gcagttcctc aaggagttgc tactcaagcc 1200
ccacagctat ggccgcttca ttaggtggct caacaaggag aagggcatct tcaaaattga 1260
ggactcagcc caggtggccc ggctgtgggg catccgcaag aaccgtcccg ccatgaacta 1320
cgacaagctg agccgctcca tccgccagta ttacaagaag ggcatcatcc ggaagccaga 1380
catctcccag cgcctcgtct accagttcgt gcaccccatc tgagtgcctg gcccagggcc 1440
tgaaacccgc cctcaggggc ctctctcctg cctgccctgc ctcagccagg ccctgagatg 1500
ggggaaaacg ggcagtctgc tctgctgctc tgaccttcca gagcccaagg tcagggaggg 1560
gcaaccaact gccccagggg gatatgggtc ctctggggcc ttcgggacca tggggcaggg 1620
gtgcttcctc ctcaggccca gctgctcccc tggaggacag agggagacag ggctgctccc 1680
caacacctgc ctctgacccc agcatttcca gagcagagcc tacagaaggg cagtgactcg 1740
acaaaggcca caggcagtcc aggcctctct ctgctccatc cccctgcctc ccattctgca 1800
ccacacctgg catggtgcag ggagacatct gcacccctga gttgggcagc caggagtgcc 1860
cccgggaatg gataataaag atactagaga actg 1894

<210> 65
<211> 3029
<212> DNA

<213> Homo sapiens

<400> 65

```
ccaggcagct ggggtaagga gttcaaggca gcgcccacac ccgggggctc tccgcaaccc     60
gaccgcctgt ccgctccccc acttcccgcc ctccctccca cctactcatt cacccaccca    120
cccacccaga gccgggacgg cagcccaggc gcccgggccc cgccgtctcc tcgccgcgat    180
cctggacttc ctcttgctgc aggacccggc ttccacgtgt gtcccggagc cggcgtctca    240
gcacacgctc cgctccgggc ctgggtgcct acagcagcca gagcagcagg gagtccggga    300
cccgggcggc atctgggcca agttaggcgc cgccgaggcc agcgctgaac gtctccaggg    360
ccggaggagc cgcggggcgt ccgggtctga gccgcagcaa atgggctccg acgtgcggga    420
```

```
cctgaacgcg ctgctgcccg ccgtcccctc cctgggtggc ggcggcggct gtgccctgcc    480
tgtgagcggc gcggcgcagt gggcgccggt gctggacttt gcgcccccgg gcgcttcggc    540
ttacgggtcg ttgggcggcc ccgcgccgcc accggctccg ccgccacccc cgccgccgcc    600
gcctcactcc ttcatcaaac aggagccgag ctggggcggc gcggagccgc acgaggagca    660
gtgcctgagc gccttcactg tccacttttc cggccagttc actggcacag ccggagcctg    720
tcgctacggg cccttcggtc ctcctccgcc cagccaggcg tcatccggcc aggccaggat    780
gtttcctaac gcgccctacc tgcccagctg cctcgagagc cagcccgcta ttcgcaatca    840
gggttacagc acggtcacct tcgacgggac gcccagctac ggtcacacgc cctcgcacca    900
tgcggcgcag ttccccaacc actcattcaa gcatgaggat cccatgggcc agcagggctc    960
gctgggtgag cagcagtact cggtgccgcc cccggtctat ggctgccaca cccccaccga   1020
cagctgcacc ggcagccagg ctttgctgct gaggacgccc tacagcagtg acaatttata   1080
ccaaatgaca tcccagcttg aatgcatgac ctggaatcag atgaacttag gagccacctt   1140
aaagggagtt gctgctggga gctccagctc agtgaaatgg acagaagggc agagcaacca   1200
cagcacaggg tacgagagcg ataaccacac aacgcccatc ctctgcggag cccaatacag   1260
aatacacacg cacggtgtct tcagaggcat tcaggatgtg cgacgtgtgc ctggagtagc   1320
cccgactctt gtacggtcgg catctgagac cagtgagaaa cgccccttca tgtgtgctta   1380
cccaggctgc aataagagat attttaagct gtcccactta cagatgcaca gcaggaagca   1440
cactggtgag aaaccatacc agtgtgactt caaggactgt gaacgaaggt tttctcgttc   1500
agaccagctc aaaagacacc aaaggagaca tacaggtgtg aaaccattcc agtgtaaaac   1560
ttgtcagcga aagttctccc ggtccgacca cctgaagacc cacaccagga ctcatacagg   1620
taaaacaagt gaaaagccct tcagctgtcg gtggccaagt tgtcagaaaa agtttgcccg   1680
gtcagatgaa ttagtccgcc atcacaacat gcatcagaga aacatgacca aactccagct   1740
ggcgctttga ggggtctccc tcggggaccg ttcagtgtcc caggcagcac agtgtgtgaa   1800
ctgctttcaa gtctgactct ccactcctcc tcactaaaaa ggaaacttca gttgatcttc   1860
ttcatccaac ttccaagaca agataccggt gcttctggaa actaccaggt gtgcctggaa   1920
gagttggtct ctgccctgcc tacttttagt tgactcacag gccctggaga agcagctaac   1980
aatgtctggt tagttaaaag cccattgcca tttggtgtgg attttctact gtaagaagag   2040
ccatagctga tcatgtcccc ctgacccttc ccttcttttt ttatgctcgt tttcgctggg   2100
gatggaatta ttgtaccatt ttctatcatg gaatatttat aggccagggc atgtgtatgt   2160
gtctgctaat gtaaactttg tcatggtttc catttactaa cagcaacagc aagaaataaa   2220
tcagagagca aggcatcggg ggtgaatctt gtctaacatt cccgaggtca gccaggctgc   2280
taacctggaa agcaggatgt agttctgcca ggcaactttt aaagctcatg catttcaagc   2340
agctgaagaa aaaatcagaa ctaaccagta cctctgtata gaaatctaaa agaattttac   2400
cattcagtta attcaatgtg aacactggca cactgctctt aagaaactat gaagatctga   2460
gatttttttg tgtatgtttt tgactctttt gagtggtaat catatgtgtc tttatagatg   2520
```

tacatacctc cttgcacaaa tggaggggaa ttcattttca tcactgggag tgtccttagt 2580
gtataaaaac catgctggta tatggcttca agttgtaaaa atgaaagtga ctttaaaaga 2640
aaatagggga tggtccagga tctccactga taagactgtt tttaagtaac ttaaggacct 2700
ttgggtctac aagtatatgt gaaaaaaatg agacttactg ggtgaggaaa tccattgttt 2760
aaagatggtc gtgtgtgtgt gtgtgtgtgt gtgtgtgtgt gtgttgtgtt gtgttttgtt 2820
ttttaaggga gggaatttat tatttaccgt tgcttgaaat tactgtgtaa atatatgtct 2880
gataatgatt tgctctttga caactaaaat taggactgta taagtactag atgcatcact 2940
gggtgttgat cttacaagat attgatgata acacttaaaa ttgtaacctg catttttcac 3000
tttgctctca attaaagtct attcaaaag 3029

<210> 66
<211> 1064
<212> DNA
<213> Homo sapiens

<400> 66

tttgaggcca tataaagtca cctgaggccc tctccaccac agcccaccag tgaccatgaa 60
ggctgtgctg cttgccctgt tgatggcagg cttggccctg cagccaggca ctgccctgct 120
gtgctactcc tgcaaagccc aggtgagcaa cgaggactgc ctgcaggtgg agaactgcac 180
ccagctgggg gagcagtgct ggaccgcgcg catccgcgca gttggcctcc tgaccgtcat 240
cagcaaaggc tgcagcttga actgcgtgga tgactcacag gactactacg tgggcaagaa 300
gaacatcacg tgctgtgaca ccgacttgtg caacgccagc ggggcccatg ccctgcagcc 360
ggctgctgcc atccttgcgc tgctccctgc actcggcctg ctgctctggg gacccggcca 420
gctctaggct ctgggggggc ccgctgcagc ccacactggg tgtggtgccc caggcctctg 480
tgccactcct cacacacccg gcccagtggg agcctgtcct ggttcctgag gcacatccta 540
acgcaagtct gaccatgtat gtctgcgccc ctgtccccca ccctgacccct cccatggccc 600
tctccaggac tcccacccgg cagatcggct ctattgacac agatccgcct gcagatggcc 660
cctccaaccc tctctgctgc tgtttccatg gcccagcatt ctccaccctt aaccctgtgc 720
tcaggcacct cttcccccag gaagccttcc ctgcccaccc catctatgac ttgagccagg 780
tctggtccgt ggtgtccccc gcacccagca ggggacaggc actcaggagg gcccggtaaa 840
ggctgagatg aagtggactg agtagaactg gaggacagga gtcgacgtga gttcctggga 900
gtctccagag atggggcctg gaggcctgga ggaaggggcc aggcctcaca ttcgtggggc 960
tccctgaatg gcagcctcag cacagcgtag gcccttaata aacacctgtt ggataagcca 1020
aaaaaaaaaa aaaaaaaaaa aaaaaaaaaa aaaaaaaaaa aaaa 1064

<210> 67
<211> 6962
<212> DNA
<213> Homo sapiens

<400> 67

gcaagaactg caggggagga ggacgctgcc acccacagcc tctagagctc attgcagctg 60
ggacagcccg gagtgtggtt agcagctcgg caagcgctgc ccaggtcctg gggtggtggc 120
agccagcggg agcaggaaag gaagcatgtt cccaggctgc ccacgcctct gggtcctggt 180
ggtcttgggc accagctggg taggctgggg gagccaaggg acagaagcgg cacagctaag 240
gcagttctac gtggctgctc agggcatcag ttggagctac cgacctgagc ccacaaactc 300
aagtttgaat ctttctgtaa cttcctttaa gaaaattgtc tacagagagt atgaaccata 360
ttttaagaaa gaaaaaccac aatctaccat ttcaggactt cttgggccta ctttatatgc 420
tgaagtcgga gacatcataa aagttcactt taaaaataag gcagataagc ccttgagcat 480
ccatcctcaa ggaattaggt acagtaaatt atcagaaggt gcttcttacc ttgaccacac 540
attccctgcg gagaagatgg acgacgctgt ggctccaggc cgagaataca cctatgaatg 600
gagtatcagt gaggacagtg gacccaccca tgatgaccct ccatgcctca cacacatcta 660
ttactcccat gaaaatctga tcgaggattt caactcgggg ctgattgggc cctgcttat 720
ctgtaaaaaa gggaccctaa ctgagggtgg gacacagaag acgtttgaca agcaaatcgt 780
gctactattt gctgtgtttg atgaaagcaa gagctggagc cagtcatcat ccctaatgta 840
cacagtcaat ggatatgtga atgggacaat gccagatata acagtttgtg cccatgacca 900
catcagctgg catctgctgg gaatgagctc ggggccagaa ttattctcca ttcatttcaa 960
cggccaggtc ctggagcaga accatcataa ggtctcagcc atcacccttg tcagtgctac 1020
atccactacc gcaaatatga ctgtgggccc agagggaaag tggatcatat cttctctcac 1080
cccaaaacat ttgcaagctg ggatgcaggc ttacattgac attaaaaact gcccaaagaa 1140
aaccaggaat cttaagaaaa taactcgtga gcagaggcgg cacatgaaga ggtgggaata 1200
cttcattgct gcagaggaag tcatttggga ctatgcacct gtaataccag cgaatatgga 1260
caaaaaatac aggtctcagc atttggataa tttctcaaac caaattggaa aacattataa 1320
gaaagttatg tacacacagt acgaagatga gtccttcacc aaacatacag tgaatcccaa 1380
tatgaaagaa gatgggattt tgggtcctat tatcagagcc caggtcagag acacactcaa 1440
aatcgtgttc aaaaatatgg ccagccgccc ctatagcatt taccctcatg gagtgacctt 1500
ctcgccttat gaagatgaag tcaactcttc tttcacctca ggcaggaaca acaccatgat 1560
cagagcagtt caaccagggg aaacctatac ttataagtgg aacatcttag agtttgatga 1620
acccacagaa aatgatgccc agtgcttaac aagaccatac tacagtgacg tggacatcat 1680
gagagacatc gcctctgggc taataggact acttctaatc tgtaagagca gatccctgga 1740
caggcgagga atacagaggg cagcagacat cgaacagcag gctgtgtttg ctgtgtttga 1800
tgagaacaaa agctggtacc ttgaggacaa catcaacaag ttttgtgaaa atcctgatga 1860
ggtgaaacgt gatgacccca agttttatga atcaaacatc atgagcacta tcaatggcta 1920
tgtgcctgag agcataacta ctcttggatt ctgctttgat gacactgtcc agtggcactt 1980
ctgtagtgtg gggacccaga atgaaatttt gaccatccac ttcactgggc actcattcat 2040
ctatggaaag aggcatgagg acaccttgac cctcttcccc atgcgtggag aatctgtgac 2100

ggtcacaatg gataatgttg gaacttggat gttaacttcc atgaattcta gtccaagaag 2160
caaaaagctg aggctgaaat tcagggatgt taaatgtatc ccagatgatg atgaagactc 2220
atatgagatt tttgaacctc cagaatctac agtcatggct acacggaaaa tgcatgatcg 2280
tttagaacct gaagatgaag agagtgatgc tgactatgat taccagaaca gactggctgc 2340
agcattagga atcaggtcat tccgaaactc atcattgaat caggaagaag aagagttcaa 2400
tcttactgcc ctagctctgg agaatggcac tgaattcgtt tcttcaaaca cagatataat 2460
tgttggttca aattattctt ccccaagtaa tattagtaag ttcactgtca ataaccttgc 2520
agaacctcag aaagcccctt ctcaccaaca agccaccaca gctggttccc cactgagaca 2580
cctcattggc aagaactcag ttctcaattc ttccacagca gagcattcca gcccatattc 2640
tgaagaccct atagaggatc ctctacagcc agatgtcaca gggatacgtc tactttcact 2700
tggtgctgga gaattcaaaa gtcaagaaca tgctaagcat aagggaccca aggtagaaag 2760
agatcaagca gcaaagcaca ggttctcctg gatgaaatta ctagcacata aagttgggag 2820
acacctaagc caagacactg gttctccttc cggaatgagg ccctgggagg accttcctag 2880
ccaagacact ggttctcctt ccagaatgag gccctggaag gaccctccta gtgatctgtt 2940
actcttaaaa caaagtaact catctaagat tttggttggg agatggcatt tggcttctga 3000
gaaaggtagc tatgaaataa tccaagatac tgatgaagac acagctgtta acaattggct 3060
gatcagcccc cagaatgcct cacgtgcttg ggggagaaagc acccctcttg ccaacaagcc 3120
tggaaagcag agtggccacc caaagtttcc tagagttaga cataaatctc tacaagtaag 3180
acaggatgga ggaaagagta gactgaagaa aagccagttt ctcattaaga cacgaaaaaa 3240
gaaaaaagag aagcacacac accatgctcc tttatctccg aggacctttc accctctaag 3300
aagtgaagcc tacaacacat tttcagaaag aagacttaag cattcgttgg tgcttcataa 3360
atccaatgaa acatctcttc ccacagacct caatcagaca ttgccctcta tggattttgg 3420
ctggatagcc tcacttcctg accataatca gaattcctca aatgacactg gtcaggcaag 3480
ctgtcctcca ggtctttatc agacagtgcc cccagaggaa cactatcaaa cattccccat 3540
tcaagaccct gatcaaatgc actctacttc agaccccagt cacagatcct cttctccaga 3600
gctcagtgaa atgcttgagt atgaccgaag tcacaagtcc ttccccacag atataagtca 3660
aatgtcccct tcctcagaac atgaagtctg gcagacagtc atctctccag acctcagcca 3720
ggtgaccctc tctccagaac tcagccagac aaacctctct ccagacctca gccacacgac 3780
tctctctcca gaactcattc agagaaacct ttccccagcc ctcggtcaga tgcccatttc 3840
tccagacctc agccatacaa ccctttctcc agacctcagc catacaaccc tttctttaga 3900
cctcagccag acaaacctct ctccagaact cagtcagaca aacctttctc cagccctcgg 3960
tcagatgccc ctttctccag acctcagcca tacaaccctt tctctagact tcagccagac 4020
aaacctctct ccagaactca gccatatgac tctctctcca gaactcagtc agacaaacct 4080
ttccccagcc ctcggtcaga tgcccatttc tccagacctc agccatacaa ccctttctct 4140

agacttcagc cagacaaacc tctctccaga actcagtcaa acaaaccttt ccccagccct 4200
cggtcagatg cccctttctc cagaccccag ccatacaacc ctttctctag acctcagcca 4260
gacaaacctc tctccagaac tcagtcagac aaacctttcc ccagacctca gtgagatgcc 4320
cctctttgca gatctcagtc aaattcccct taccccagac ctcgaccaga tgacactttc 4380
tccagacctt ggtgagacag atctttcccc aaactttggt cagatgtccc tttccccaga 4440
cctcagccag gtgactctct ctccagacat cagtgacacc acccttctcc cggatctcag 4500
ccagatatca cctcctccag accttgatca gatattctac ccttctgaat ctagtcagtc 4560
attgcttctt caagaattta atgagtcttt tccttatcca gaccttggtc agatgccatc 4620
tccttcatct cctactctca atgatacttt tctatcaaag gaatttaatc cactggttat 4680
agtgggcctc agtaaagatg gtacagatta cattgagatc attccaaagg aagaggtcca 4740
gagcagtgaa gatgactatg ctgaaattga ttatgtgccc tatgatgacc cctacaaaac 4800
tgatgttagg acaaacatca actcctccag agatcctgac aacattgcag catggtacct 4860
ccgcagcaac aatggaaaca gaagaaatta ttacattgct gctgaagaaa tatcctggga 4920
ttattcagaa tttgtacaaa gggaaacaga tattgaagac tctgatgata ttccagaaga 4980
taccacatat aagaaagtag tttttcgaaa gtacctcgac agcactttta ccaaacgtga 5040
tcctcgaggg gagtatgaag agcatctcgg aattcttggt cctattatca gagctgaagt 5100
ggatgatgtt atccaagttc gttttaaaaa tttagcatcc agaccgtatt ctctacatgc 5160
ccatggactt tcctatgaaa aatcatcaga gggaaagact tatgaagatg actctcctga 5220
atggtttaag gaagataatg ctgttcagcc aaatagcagt tatacctacg tatggcatgc 5280
cactgagcga tcagggccag aaagtcctgg ctctgcctgt cgggcttggg cctactactc 5340
agctgtgaac ccagaaaaag atattcactc aggcttgata ggtcccctcc taatctgcca 5400
aaaaggaata ctacataagg acagcaacat gcctatggac atgagagaat ttgtcttact 5460
atttatgacc tttgatgaaa agaagagctg gtactatgaa aagaagtccc gaagttcttg 5520
gagactcaca tcctcagaaa tgaaaaaatc ccatgagttt cacgccatta atgggatgat 5580
ctacagcttg cctggcctga aaatgtatga gcaagagtgg gtgaggttac acctgctgaa 5640
cataggcggc tcccaagaca ttcacgtggt tcactttcac ggccagacct tgctggaaaa 5700
tggcaataaa cagcaccagt taggggtctg gccccttctg cctggttcat ttaaaactct 5760
tgaaatgaag gcatcaaaac ctggctggtg gctcctaaac acagaggttg gagaaaacca 5820
gagagcaggg atgcaaacgc catttcttat catggacaga gactgtagga tgccaatggg 5880
actaagcact ggtatcatat ctgattcaca gatcaaggct tcagagtttc tgggttactg 5940
ggagcccaga ttagcaagat taaacaatgg tggatcttat aatgcttgga gtgtagaaaa 6000
acttgcagca gaatttgcct ctaaaccttg gatccaggtg gacatgcaaa aggaagtcat 6060
aatcacaggg atccagaccc aaggtgccaa acactacctg aagtcctgct ataccacaga 6120
gttctatgta gcttacagtt ccaaccagat caactggcag atcttcaaag ggaacagcac 6180
aaggaatgtg atgtatttta atggcaattc agatgcctct acaataaaag agaatcagtt 6240

```
tgacccacct attgtggcta gatatattag gatctctcca actcgagcct ataacagacc   6300
taccttcga ttggaactgc aaggttgtga ggtaaatgga tgttccacac ccctgggtat    6360
ggaaaatgga aagatagaaa acaagcaaat cacagcttct tcgtttaaga aatcttggtg   6420
gggagattac tgggaaccct tccgtgcccg tctgaatgcc cagggacgtg tgaatgcctg   6480
gcaagccaag gcaaacaaca ataagcagtg gctagaaatt gatctactca agatcaagaa   6540
gataacggca attataacac agggctgcaa gtctctgtcc tctgaaatgt atgtaaagag   6600
ctataccatc cactacagtg agcagggagt ggaatggaaa ccatacaggc tgaaatcctc   6660
catggtggac aagatttttg aaggaaatac taataccaaa ggacatgtga agaacttttt   6720
caaccccca atcatttcca ggtttatccg tgtcattcct aaaacatgga atcaaagtat    6780
tgcacttcgc ctggaactct ttggctgtga tatttactag aattgaacat tcaaaaaccc   6840
ctggaagaga ctctttaaga cctcaaacca tttagaatgg gcaatgtatt ttacgctgtg   6900
ttaaatgtta acagttttcc actatttctc tttcttttct attagtgaat aaaattttat   6960
ac                                                                  6962
```

<210> 68
<211> 1464
<212> DNA
<213> Homo sapiens

<400> 68

```
agccccaagc ttaccacctg cacccggaga gctgtgtcac catgtgggtc ccggttgtct     60
tcctcaccct gtccgtgacg tggattggtg ctgcacccct catcctgtct cggattgtgg    120
gaggctggga gtgcgagaag cattcccaac cctggcaggt gcttgtggcc tctcgtggca    180
gggcagtctg cggcggtgtt ctggtgcacc cccagtgggt cctcacagct gcccactgca    240
tcaggaacaa aagcgtgatc ttgctgggtc ggcacagcct gtttcatcct gaagacacag    300
gccaggtatt tcaggtcagc cacagcttcc cacacccgct ctacgatatg agcctcctga    360
agaatcgatt cctcaggcca ggtgatgact ccagccacga cctcatgctg ctccgcctgt    420
cagagcctgc cgagctcacg gatgctgtga aggtcatgga cctgcccacc caggagccag    480
cactggggac cacctgctac gcctcaggct ggggcagcat tgaaccagag gagttcttga    540
ccccaaagaa acttcagtgt gtggacctcc atgttatttc caatgacgtg tgtgcgcaag    600
ttcaccctca gaaggtgacc aagttcatgc tgtgtgctgg acgctggaca gggggcaaaa    660
gcacctgctc gggtgattct gggggcccac ttgtctgtaa tggtgtgctt caaggtatca    720
cgtcatgggg cagtgaacca tgtgccctgc ccgaaaggcc ttccctgtac accaaggtgg    780
tgcattaccg gaagtggatc aaggacacca tcgtggccaa cccctgagca cccctatcaa    840
ccccctattg tagtaaactt ggaaccttgg aaatgaccag gccaagactc aagcctcccc    900
agttctactg acctttgtcc ttaggtgtga ggtccagggt tgctaggaaa agaaatcagc    960
agacacaggt gtagaccaga gtgtttctta aatggtgtaa ttttgtcctc tctgtgtcct   1020
ggggaatact ggccatgcct ggagacatat cactcaattt ctctgaggac acagatagga   1080

tggggtgtct gtgttatttg tggggtacag agatgaaaga ggggtgggat ccacactgag   1140
agagtggaga gtgacatgtg ctggacactg tccatgaagc actgagcaga agctggaggc   1200
acaacgcacc agacactcac agcaaggatg gagctgaaaa cataacccac tctgtcctgg   1260
aggcactggg aagcctagag aaggctgtga gccaaggagg gagggtcttc ctttggcatg   1320
ggatggggat gaagtaagga gagggactgg acccccctgga agctgattca ctatgggggg   1380
aggtgtattg aagtcctcca gacaaccctc agatttgatg atttcctagt agaactcaca   1440
gaaataaaga gctgttatac tgtg                                           1464
```

<210> 69
<211> 1793
<212> DNA
<213> Homo sapiens

<400> 69

```
cgcgtccgcc ccgcgagcac agagcctcgc ctttgccgat ccgccgcccg tccacacccg      60
ccgccagctc accatggatg atgatatcgc cgcgctcgtc gtcgacaacg gctccggcat     120
gtgcaaggcc ggcttcgcgg gcgacgatgc cccccgggcc gtcttcccct ccatcgtggg     180
gcgccccagg caccagggcg tgatggtggg catgggtcag aaggattcct atgtgggcga     240
cgaggcccag agcaagagag gcatcctcac cctgaagtac cccatcgagc acggcatcgt     300
caccaactgg gacgacatgg agaaaatctg gcaccacacc ttctacaatg agctgcgtgt     360
ggctcccgag gagcaccccg tgctgctgac cgaggccccc ctgaacccca aggccaaccg     420
cgagaagatg acccagatca tgtttgagac cttcaacacc ccagccatgt acgttgctat     480
ccaggctgtg ctatccctgt acgcctctgg ccgtaccact ggcatcgtga tggactccgg     540
tgacggggtc acccacactg tgcccatcta cgaggggtat gccctccccc atgccatcct     600
gcgtctggac ctggctggcc gggacctgac tgactacctc atgaagatcc tcaccgagcg     660
cggctacagc ttcaccacca cggccgagcg ggaaatcgtg cgtgacatta aggagaagct     720
gtgctacgtc gccctggact tcgagcaaga gatggccacg gctgcttcca gctcctccct     780
ggagaagagc tacgagctgc ctgacggcca ggtcatcacc attggcaatg agcggttccg     840
ctgccctgag gcactcttcc agccttcctt cctgggcatg gagtcctgtg gcatccacga     900
aactaccttc aactccatca tgaagtgtga cgtggacatc cgcaaagacc tgtacgccaa     960
cacagtgctg tctggcggca ccaccatgta ccctggcatt gccgacagga tgcagaagga    1020
gatcactgcc ctggcaccca gcacaatgaa gatcaagatc attgctcctc ctgagcgcaa    1080
gtactccgtg tggatcggcg gctccatcct ggcctcgctg tccaccttcc agcagatgtg    1140
gatcagcaag caggagtatg acgagtccgg cccctccatc gtccaccgca aatgcttcta    1200
ggcggactat gacttagttg cgttacaccc tttcttgaca aaacctaact tgcgcagaaa    1260
acaagatgag attggcatgg ctttatttgt ttttttgtt ttgttttggt ttttttttt    1320
tttttggctt gactcaggat ttaaaaactg gaacggtgaa ggtgacagca gtcggttgga    1380
gcgagcatcc cccaaagttc acaatgtggc cgaggacttt gattgcacat tgttgttttt    1440

ttaatagtca ttccaaatat gagatgcatt gttacaggaa gtcccttgcc atcctaaaag    1500
ccaccccact tctctctaag gagaatggcc cagtcctctc ccaagtccac acaggggagg    1560
tgatagcatt gctttcgtgt aaattatgta atgcaaaatt tttttaatct tcgccttaat    1620
actttttat tttgttttat tttgaatgat gagccttcgt gcccccctt cccccttttt    1680
gtcccccaac ttgagatgta tgaaggcttt tggtctccct gggagtgggt ggaggcagcc    1740
agggcttacc tgtacactga cttgagacca gttgaataaa agtgcacacc tta           1793
```

<210> 70
<211> 1526
<212> DNA
<213> Homo sapiens

<400> 70

ccggaagtga cgcgaggctc tgcggagacc aggagtcaga ctgtaggacg acctcgggtc 60
ccacgtgtcc ccggtactcg ccggccggag cccccggctt cccggggccg ggggacctta 120
gcggcaccca cacacagcct actttccaag cggagccatg tctggtaacg gcaatgcggc 180
tgcaacggcg gaagaaaaca gcccaaagat gagagtgatt cgcgtgggta cccgcaagag 240
ccagcttgct cgcatacaga cggacagtgt ggtggcaaca ttgaaagcct cgtaccctgg 300
cctgcagttt gaaatcattg ctatgtccac cacaggggac aagattcttg atactgcact 360
ctctaagatt ggagagaaaa gcctgtttac caaggagctt gaacatgccc tggagaagaa 420
tgaagtggac ctggttgttc actccttgaa ggacctgccc actgtgcttc ctcctggctt 480
caccatcgga gccatctgca agcgggaaaa ccctcatgat gctgttgtct ttcacccaaa 540
atttgttggg aagaccctag aaaccctgcc agagaagagt gtggtgggaa ccagctccct 600
gcgaagagca gcccagctgc agagaaagtt cccgcatctg gagttcagga gtattcgggg 660
aaacctcaac acccggcttc ggaagctgga cgagcagcag gagttcagtg ccatcatcct 720
ggcaacagct ggcctgcagc gcatgggctg gcacaaccgg gtggggcaga tcctgcaccc 780
tgaggaatgc atgtatgctg tgggccaggg ggccttgggc gtggaagtgc gagccaagga 840
ccaggacatc ttggatctgg tgggtgtgct gcacgatccc gagactctgc ttcgctgcat 900
cgctgaaagg gccttcctga ggcacctgga aggaggctgc agtgtgccag tagccgtgca 960
tacagctatg aaggatgggc aactgtacct gactggagga gtctggagtc tagacggctc 1020
agatagcata caagagacca tgcaggctac catccatgtc cctgcccagc atgaagatgg 1080
ccctgaggat gacccacagt tggtaggcat cactgctcgt aacattccac gagggcccca 1140
gttggctgcc cagaacttgg gcatcagcct ggccaacttg ttgctgagca aaggagccaa 1200
aaacatcctg gatgttgcac ggcagcttaa cgatgcccat taactggttt gtggggcaca 1260
gatgcctggg ttgctgctgt ccagtgccta catcccgggc ctcagtgccc cattctcact 1320
gctatctggg gagtgattac cccgggagac tgaactgcag ggttcaagcc ttccagggat 1380
ttgcctcacc ttggggcctt gatgactgcc ttgcctcctc agtatgtggg ggcttcatct 1440
ctttagagaa gtccaagcaa cagcctttga atgtaaccaa tcctactaat aaaccagttc 1500

tgaaggtgta aaaaaaaaaa aaaaaa 1526

<210> 71
<211> 2397
<212> DNA
<213> Homo sapiens

<400> 71

gcaagaactg aaacgaatgg ggattgaact gctttgcctg ttctttctat ttctaggaag 60
gaatgatcac gtacaaggtg gctgtgccct gggaggtgca gaaacctgtg aagactgcct 120
gcttattgga cctcagtgtg cctggtgtgc tcaggagaat tttactcatc catctggagt 180
tggcgaaagg tgtgataccc cagcaaacct tttagctaaa ggatgtcaat taaacttcat 240
cgaaaaccct gtctcccaag tagaaatact taaaaataag cctctcagtg taggcagaca 300
gaaaaatagt tctgacattg ttcagattgc gcctcaaagc ttgatcctta agttgagacc 360
aggtggtgcg cagactctgc aggtgcatgt ccgccagact gaggactacc cggtggattt 420
gtattacctc atggacctct ccgcctccat ggatgacgac ctcaacacaa taaaggagct 480
gggctcccgg ctttccaaag agatgtctaa attaaccagc aactttagac tgggcttcgg 540
atcttttgtg gaaaaacctg tatccccttt cgtgaaaaca acaccagaag aaattgccaa 600
cccttgcagt agtattccat acttctgttt acctacattt ggattcaagc acattttgcc 660
attgacaaat gatgctgaaa gattcaatga aattgtgaag aatcagaaaa tttctgctaa 720
tattgacaca cccgaaggtg gatttgatgc aattatgcaa gctgctgtgt gtaaggaaaa 780
aattggctgg cggaatgact ccctccacct cctggtcttt gtgagtgatg ctgattctca 840
ttttggaatg gacagcaaac tagcaggcat cgtcattcct aatgacgggc tctgtcactt 900
ggacagcaag aatgaatact ccatgtcaac tgtcttggaa tatccaacaa ttggacaact 960
cattgataaa ctggtacaaa acaacgtgtt attgatcttc gctgtaaccc aagaacaagt 1020
tcatttatat gagaattacg caaaacttat tcctggagct acagtaggtc tacttcagaa 1080
ggactccgga aacattctcc agctgatcat ctcagcttat gaagaactgc ggtctgaggt 1140
ggaactggaa gtattaggag acactgaagg actcaacttg tcatttacag ccatctgtaa 1200
caacggtacc ctcttccaac accaaaagaa atgctctcac atgaaagtgg gagacacagc 1260
ttccttcagc gtgactgtga atatcccaca ctgcgagaga agaagcaggc acattatcat 1320
aaagcctgtg gggctggggg atgccctgga attacttgtc agcccagaat gcaactgcga 1380
ctgtcagaaa gaagtggaag tgaacagctc caaatgtcac cacgggaacg gctctttcca 1440
gtgtggggtg tgtgcctgcc accctggcca catggggcct cgctgtgagt gtggcgagga 1500
catgctgagc acagattcct gcaaggaggc cccagatcat ccctcctgca gcggaagggg 1560
tgactgctac tgtgggcagt gtatctgcca cttgtctccc tatggaaaca tttatgggcc 1620
ttattgccag tgtgacaatt tctcctgcgt gagacacaaa gggctgctct gcggaggtaa 1680
cggcgactgt gactgtggtg aatgtgtgtg caggagcggc tggactggcg agtactgcaa 1740
ctgcaccacc agcacggact cctgcgtctc tgaagatgga gtgctctgca gcgggcgcgg 1800

ggactgtgtt tgtggcaagt gtgtttgcac aaaccctgga gcctcaggac caacctgtga 1860
acgatgtcct acctgtggtg acccctgtaa ctctaaacgg agctgcattg agtgccacct 1920
gtcagcagct ggccaagccc gagaagaatg tgtggacaag tgcaaactag ctggtgcgac 1980
catcagtgaa gaagaagatt tctcaaagga tggttctgtt tcctgctctc tgcaaggaga 2040
aaatgaatgt cttattacat tcctaataac tacagataat gaggggaaaa ccatcattca 2100
cagcatcaat gaaaaagatt gtccgaagcc tccaaacatt cccatgatca tgttaggggt 2160
ttccctggct attcttctca tcggggttgt cctactgtgc atctggaagc tactggtgtc 2220
atttcatgat cgtaaagaag ttgccaaatt tgaagcagaa cgatcaaaag ccaagtggca 2280
aacgggaacc aatccactct acagaggatc cacaagtact tttaaaaatg taacttataa 2340
acacagggaa aaacaaaagg tagacctttc cacagattgc tagaactact ttatgca 2397

<210> 72
<211> 2118
<212> DNA
<213> Homo sapiens

<400> 72

```
tggggagccc aagcagaaac gcaagctggt ggctgaggtg tccctgcaga acccgctccc      60
tgtggccctg gaaggctgca ccttcactgt ggagggggcc ggcctgactg aggagcagaa     120
gacggtggag atcccagacc ccgtggaggc aggggaggaa gttaaggtga gaatggacct     180
gctgccgctc cacatgggcc tccacaagct ggtggtgaac ttcgagagcg acaagctgaa     240
ggctgtgaag ggcttccgga atgtcatcat tggccccgcc taagggaccc ctgctcccag     300
cctgctgaga gcccccacct tgatcccaat ccttatccca agctagtgag caaaatatgc     360
cccttcttgg gccccagacc ccagggcagg gtgggcagcc tatgggggct ctcggaaatg     420
gaatgtgccc ctggcccatc tcagcctcct gagcctgtgg gtccccactc acccccttg     480
ctgtgaggaa tgctctgtgc cagaaacagt gggagccctg accttggctg actggggctg     540
gggtgagaga ggaaagacct acattccctc tcctgcccag atgccctttg gaaagccatt     600
gaccacccac catattgttt gatctacttc atagctcctt ggagcaggca aaaaagggac     660
agcatgcccc ttggctggat cagggaatcc agctccctag actgcatccc gtacctcttc     720
ccatgactgc acccagctcc aggggccctt gggacagcca gagctgggtg gggacagtga     780
taggcccaag gtcccctcca catcccagca gcccaagctt aatagccctc cccctcaacc     840
tcaccattgt gaagcaccta ctatgtgctg ggtgcctccc acacttgctg gggctcacgg     900
ggcctccaac ccatttaatc accatgggaa actgttgtgg gcgctgcttc caggataagg     960
agactgaggc ttagagagag gaggcagccc cctccacacc agtggcctcg tggttattag    1020
caaggctggg taatgtgaag gcccaagagc agagtctggg cctctgactc tgagtccact    1080
gctccattta taaccccagc ctgacctgag actgtcggag aggctgtctg ggcctttat    1140
caaaaaaaga ctcagccaag acaaggaggt agagagggga ctgggggact gggagtcaga    1200
gccctggctg ggttcaggtc ccacgtctgg ccaggcactg ccttctcctc tctgggcctt    1260
```

```
tgtttccttg ttggtcagag gagtgattga accagctcat ctccaaggat cctctccact   1320
ccatgtttgc aatgctttta tatggcccag ccttgtaaat aaccacaagg tccactccct   1380
gctccacgaa gccttaagcc ataggcccag gatatttctg agagtgaaac catgactgtg   1440
accaccttct gtccccagcc ctgtcctggt tccttcctat gcccaggtac cacccttcag   1500
accccagttc taggggagaa gagccctgga cacccctgct ctacccatga gcctgcccgc   1560
tgcaatgcct agacttccca acagccttag ctgccagtgc tggtcactaa ccaacaaggt   1620
tggcacccca gctacccctt ctttgcaggg ctaaggcccc caaacatagc ccctgccccg   1680
gaggaagctt ggggaaccca tgagttgtca gctttgactt tatctcctgc tctttctaca   1740
tgactgggcc tcccttgggc tggaagaatt ggggattctc tattggaggt gagatcacag   1800
cctccagggc cccccaaatc ccagggaagg acttggagag aatcatgctg ttgcatttag   1860
aactttctgc tttgcacagg aaagagtcac acaattaatc aacatgtata ttttctctat   1920
acatagagct ctatttctct acggttttat aaaagccttg ggttccaacc aggcagtaga   1980
tgtgcttctg aaccgcaagg agcaaacact gaaataaaat agtttatttt tcacactcaa   2040
aaaaaaaaaa aaaaaaaaaa aaaaaaaaaa aaaaaaaaaa aaaaaaaaaa aaaaaaaaaa   2100
aaaaaaaaaa aaaaaaaa                                                 2118
```

<210> 73
<211> 2832
<212> DNA
<213> Homo sapiens

<400> 73

```
aaagctcaaa ccgacaccct cacgcagatg atgacatcaa ctcttttttc ttccccaagt   60
gtacacaatg tgatggagac tgttacgcag gagacagctc ctccagatga aatgaccaca  120
tcatttccct ccagtgtcac caacacactc atgatgacat caaagactat aacaatgaca  180
acctccacag actccactct tggaaacaca gaagagacat caacagcagg aactgaaagt  240
tctaccccag tgacctcagc agtctcaata acagctggac aggaaggaca atcacgaaca  300
acttcctgga ggacctctat ccaagacaca tcagcttctt ctcagaacca ctggactcgg  360
agcacgcaga ccaccaggga atctcaaacc agcaccctaa cacacagaac cacttcaact  420
ccttctttct ctccaagtgt acacaatgtg acagggactg tttctcagaa gacatctcct  480
tcaggtgaaa cagctacctc atccctctgt agtgtcacaa acacatccat gatgacatca  540
gagaagataa cagtgacaac ctccacaggc tccactcttg gaaacccagg ggagacatca  600
tcagtacctg ttactggaag tcttatgcca gtcacctcag cagccttagt aacagttgat  660
ccagaaggac aatcaccagc aactttctca aggacttcta ctcaggacac aacagctttt  720
tctaagaacc accagactca gagcgtggag accaccagag tatctcaaat caacaccctc  780
aacaccctca caccggttac aacatcaact gttttatcct caccaagtgg attcaaccca  840
agtggaacag tttctcagga gacattccct tctggtgaaa caaccatctc atccccttcc  900
agtgtcagca atacattcct ggtaacatca aaggtgttca gaatgccaat ctccagagac  960
```

```
tctactcttg gaaacacaga ggagacatca ctatctgtaa gtggaaccat ttctgcaatc    1020
acttccaaag tttcaaccat atggtggtca gacactctgt caacagcact ctcccccagt    1080
tctctacctc caaaaatatc cacagctttc cacacccagc agagtgaagg tgcagagacc    1140
acaggacggc ctcatgagag gagctcattc tctccaggtg tgtctcaaga aatatttact    1200
ctacatgaaa caacaacatg gccttcctca ttctccagca aaggccacac aacttggtca    1260
caaacagaac tgccctcaac atcaacaggt gctgccacta ggcttgtcac aggaaatcca    1320
tctacaggga cagctggcac tattccaagg gtcccctcta aggtctcagc aataggggaa    1380
ccaggagagc ccaccacata ctcctcccac agcacaactc tcccaaaaac aacaggggca    1440
ggcgcccaga cacaatggac acaagaaacg gggaccactg gagaggctct tctcagcagc    1500
ccaagctaca gtgtgactca gatgataaaa acggccacat ccccatcttc ttcacctatg    1560
ctggatagac acacatcaca acaaattaca acggcaccat caacaaatca ttcaacaata    1620
cattccacaa gcacctctcc tcaggaatca ccagctgttt cccaaagggg tcacactcaa    1680
gccccgcaga ccacacaaga atcacaaacc acgaggtccg tctcccccat gactgacacc    1740
aagacagtca ccaccccagg ttcttccttc acagccagtg ggcactcgcc ctcagaaatt    1800
gttcctcagg acgcacccac cataagtgca gcaacaacct ttgccccagc tcccaccggg    1860
gatggtcaca caacccaggc cccgaccaca gcactgcagg cagcacccag cagccatgat    1920
gccaccctgg ggccctcagg aggcacgtca ctttccaaaa caggtgccct tactctggcc    1980
aactctgtag tgtcaacacc agggggccca gaaggacaat ggacatcagc ctctgccagc    2040
acctcacctg acacagcagc agccatgacc catacccacc aggctgagag cacagaggcc    2100
tctggacaaa cacagaccag cgaaccggcc tcctcagggt cacgaaccac ctcagcgggc    2160
acagctaccc cttcctcatc cggggcgagt ggcacaacac cttcaggaag cgaaggaata    2220
tccacctcag gagagacgac aaggttttca tcaaacccct ccagggacag tcacacaacc    2280
cagtcaacaa ccgaattgct gtccgcctca gccagtcatg gtgccatccc agtaagcaca    2340
ggaatggcgt cttcgatcgt ccccggcacc tttcatccca ccctctctga ggcctccact    2400
gcagggagac cgacaggaca gtcaagccca acttctccca gtgcctctcc tcaggagaca    2460
gccgccattt cccggatggc ccagactcag aggacaagaa ccagcagagg gtctgacact    2520
atcagcctgg cgtcccaggc aaccgacacc ttctcaacag tcccacccac acctccatcg    2580
atcacatcca ctgggcttac atctccacaa acccagaccc acactctgtc accttcaggg    2640
tctggtaaaa ccttcaccac ggccctcatc agcaacgcca cccctcttcc tgtcacctac    2700
gcttcctcgg catccacagg tcacaccacc cctcttcatg tcaccgatgc ttcctcagta    2760
tccacaggtc acgccacccc tcttcctgtc accagccctt cctcagtatc cacaggtcac    2820
accacccctc tt                                                        2832
```

<210> 74
<211> 1607
<212> DNA
<213> Homo sapiens

<400> 74

```
aatgactcct ttcggtaagt gcagtggaag ctgtacactg cccaggcaaa gcgtccgggc     60
agcgtaggcg ggcgactcag atcccagcca gtggacttag cccctgtttg ctcctccgat    120
aactggggtg accttggtta atattcacca gcagcctccc ccgttgcccc tctggatcca    180
ctgcttaaat acggacgagg acagggccct gtctcctcag cttcaggcac caccactgac    240
ctgggacagt gaatcgacaa tgccgtcttc tgtctcgtgg ggcatcctcc tgctggcagg    300
cctgtgctgc ctggtccctg tctccctggc tgaggatccc cagggagatg ctgcccagaa    360
gacagataca tcccaccatg atcaggatca cccaaccttc aacaagatca cccccaacct    420
ggctgagttc gccttcagcc tataccgcca gctggcacac cagtccaaca gcaccaatat    480
cttcttctcc ccagtgagca tcgctacagc ctttgcaatg ctctccctgg ggaccaaggc    540
tgacactcac gatgaaatcc tggagggcct gaatttcaac ctcacggaga ttccggaggc    600
tcagatccat gaaggcttcc aggaactcct ccgtaccctc aaccagccag acagccagct    660
ccagctgacc accggcaatg gcctgttcct cagcgagggc ctgaagctag tggataagtt    720
tttggaggat gttaaaaagt tgtaccactc agaagccttc actgtcaact tcggggacac    780
cgaagaggcc aagaaacaga tcaacgatta cgtggagaag ggtactcaag ggaaaattgt    840
ggatttggtc aaggagcttg acagagacac agtttttgct ctggtgaatt acatcttctt    900
taaaggcaaa tgggagagac cctttgaagt caaggacacc gaggaagagg acttccacgt    960
ggaccaggtg accaccgtga aggtgcctat gatgaagcgt ttaggcatgt ttaacatcca   1020
gcactgtaag aagctgtcca gctgggtgct gctgatgaaa tacctgggca atgccaccgc   1080
catcttcttc ctgcctgatg aggggaaact acagcacctg gaaaatgaac tcacccacga   1140
tatcatcacc aagttcctgg aaaatgaaga cagaaggtct gccagcttac atttacccaa   1200
actgtccatt actggaacct atgatctgaa gagcgtcctg ggtcaactgg gcatcactaa   1260
ggtcttcagc aatggggctg acctctccgg ggtcacagag gaggcacccc tgaagctctc   1320
caaggccgtg cataaggctg tgctgaccat cgacgagaaa gggactgaag ctgctggggc   1380
catgttttta gaggccatac ccatgtctat ccccccgag gtcaagttca acaaaccctt   1440
tgtcttctta atgattgaac aaaataccaa gtctcccctc ttcatgggaa aagtggtgaa   1500
tcccacccaa aaataactgc ctctcgctcc tcaacccctc ccctccatcc ctggccccct   1560
ccctggatga cattaaagaa gggttgagct ggtccctgcc tgcaaaa                 1607
```

<210> 75
<211> 1753
<212> DNA
<213> Homo sapiens

<400> 75

cagccccgcc cctacctgtg gaagcccagc cgcccgctcc cgcggataaa aggcgcggag 60
tgtccccgag gtcagcgagt gcgcgctcct cctcgcccgc cgctaggtcc atcccggccc 120
agccaccatg tccatccact tcagctcccc ggtattcacc tcgcgctcag ccgccttctc 180

gggccgcggc gcccaggtgc gcctgagctc cgctcgcccc ggcggccttg gcagcagcag 240
cctctacggc ctcggcgcct cacggccgcg cgtggccgtg cgctctgcct atgggggccc 300
ggtgggcgcc ggcatccgcg aggtcaccat taaccagagc ctgctggccc cgctgcggct 360
ggacgccgac ccctccctcc agcgggtgcg ccaggaggag agcgagcaga tcaagaccct 420
caacaacaag tttgcctcct tcatcgacaa ggtgcggttt ctggagcagc agaacaagct 480
gctggagacc aagtggacgc tgctgcagga gcagaagtcg gccaagagca gccgcctccc 540
agacatcttt gaggcccaga ttgctggcct tcggggtcag cttgaggcac tgcaggtgga 600
tgggggccgc ctggaggcgg agctgcggag catgcaggat gtggtggagg acttcaagaa 660
taagtacgaa gatgaaatta accaccgcac agctgctgag aatgagtttg tggtgctgaa 720
gaaggatgtg gatgctgcct acatgagcaa ggtggagctg gaggccaagg tggatgccct 780
gaatgatgag atcaacttcc tcaggaccct caatgagacg gagttgacag agctgcagtc 840
ccagatctcc gacacatctg tggtgctgtc catggacaac agtcgctccc tggacctgga 900
cggcatcatc gctgaggtca aggcgcagta tgaggagatg gccaaatgca gccgggctga 960
ggctgaagcc tggtaccaga ccaagtttga gaccctccag gcccaggctg ggaagcatgg 1020
ggacgacctc cggaataccc ggaatgagat ttcagagatg aaccgggcca tccagaggct 1080
gcaggctgag atcgacaaca tcaagaacca gcgtgccaag ttggaggccg ccattgccga 1140
ggctgaggag cgtggggagc tggcgctcaa ggatgctcgt gccaagcagg aggagctgga 1200
agccgccctg cagcggggca agcaggatat ggcacggcag ctgcgtgagt accaggaact 1260
catgagcgtg aagctggccc tggacatcga gatcgccacc taccgcaagc tgctggaggg 1320
cgaggagagc cggttggctg gagatggagt gggagccgtg aatatctctg tgatgaattc 1380
cactggtggc agtagcagtg gcggtggcat tgggctgacc ctcgggggaa ccatgggcag 1440
caatgccctg agcttctcca gcagtgcggg tcctgggctc ctgaaggctt attccatccg 1500
gaccgcatcc gccagtcgca ggagtgcccg cgactgagcc gcctcccacc actccactcc 1560
tccagccacc acccacaatc acaagaagat tcccacccct gcctcccatg cctggtccca 1620
agacagtgag acagtctgga aagtgatgtc agaatagctt ccaataaagc agcctcattc 1680
tgaggcctga gtgatccacg tgaaaaaaaa aaaaaaaaaa aaaaaaaaaa aaaaaaaaaa 1740
aaaaaaaaaa aaa 1753

<210> 76
<211> 2255
<212> DNA
<213> Homo sapiens

<400> 76

```
gatggctccg gccgcctggc tccgcagcgc ggccgcgcgc gccctcctgc ccccgatgct     60
gctgctgctg ctccagccgc cgccgctgct ggcccgggct ctgccgccgg acgcccacca    120
cctccatgcc gagaggaggg ggccacagcc ctggcatgca gccctgccca gtagcccggc    180
acctgcccct gccacgcagg aagccccccg gcctgccagc agcctcaggc ctccccgctg    240
```

```
tggcgtgccc gacccatctg atgggctgag tgcccgcaac cgacagaaga ggttcgtgct    300
ttctggcggg cgctgggaga agacggacct cacctacagg atccttcggt tcccatggca    360
gttggtgcag gagcaggtgc ggcagacgat ggcagaggcc ctaaaggtat ggagcgatgt    420
gacgccactc acctttactg aggtgcacga gggccgtgct gacatcatga tcgacttcgc    480
caggtactgg catggggacg acctgccgtt tgatgggcct gggggcatcc tggcccatgc    540
cttctccccc aagactcacc gagaaggga tgtccacttc gactatgatg agacctggac    600
tatcggggat gaccagggca cagacctgct gcaggtggca gcccatgaat ttggccacgt    660
gctggggctg cagcacacaa cagcagccaa ggccctgatg tccgccttct acacctttcg    720
ctacccactg agtctcagcc cagatgactg caggggcgtt caacacctat atggccagcc    780
ctggcccact gtcacctcca ggaccccagc cctgggcccc caggctggga tagacaccaa    840
tgagattgca ccgctggagc cagacgcccc gccagatgcc tgtgaggcct cctttgacgc    900
ggtctccacc atccgaggcg agctcttttt cttcaaagcg ggctttgtgt ggcgcctccg    960
tgggggccag ctgcagcccg gctacccagc attggcctct cgccactggc agggactgcc   1020
cagccctgtg gacgctgcct tcgaggatgc ccagggccac atttggttct tccaaggtgc   1080
tcagtactgg gtgtacgacg gtgaaaagcc agtcctgggc cccgcacccc tcaccgagct   1140
gggcctggtg aggttcccgg tccatgctgc cttggtctgg ggtcccgaga agaacaagat   1200
ctacttcttc cgaggcaggg actactggcg tttccacccc agcacccggc gtgtagacag   1260
tcccgtgccc cgcagggcca ctgactggag aggggtgccc tctgagatcg acgctgcctt   1320
ccaggatgct gatggctatg cctacttcct gcgcggccgc ctctactgga agtttgaccc   1380
tgtgaaggtg aaggctctgg aaggcttccc ccgtctcgtg ggtcctgact tctttggctg   1440
tgccgagcct gccaacactt tcctctgacc atggcttgga tgccctcagg ggtgctgacc   1500
cctgccaggc cacgaatatc aggctagaga cccatggcca tctttgtggc tgtgggcacc   1560
aggcatggga ctgagcccat gtctcctcag ggggatgggg tggggtacaa ccaccatgac   1620
aactgccggg agggccacgc aggtcgtggt cacctgccag cgactgtctc agactgggca   1680
gggaggcttt ggcatgactt aagaggaagg gcagtcttgg gcccgctatg caggtcctgg   1740
caaacctggc tgccctgtct ccatccctgt ccctcagggt agcaccatgg caggactggg   1800
ggaactggag tgtccttgct gtatccctgt tgtgaggttc cttccagggg ctggcactga   1860
agcaagggtg ctggggcccc atggccttca gccctggctg agcaactggg ctgtagggca   1920
gggccacttc ctgaggtcag gtcttggtag gtgcctgcat ctgtctgcct tctggctgac   1980
aatcctggaa atctgttctc cagaatccag gccaaaaagt tcacagtcaa atggggaggg   2040
gtattcttca tgcaggagac cccaggccct ggaggctgca acatacctca atcctgtccc   2100
aggccggatc ctcctgaagc ccttttcgca gcactgctat cctccaaagc cattgtaaat   2160
gtgtgtacag tgtgtataaa ccttcttctt cttttttttt ttttaaactg aggattgtca   2220
ttaaacacag ttgttttcta aaaaaaaaaa aaaaa                               2255
```

<210> 77
<211> 462
<212> DNA
<213> Homo sapiens

<400> 77

```
agctctattg ccaccatgag tttctccggc aagtaccaac tgcagagcca ggaaaacttt     60
gaagccttca tgaaggcaat cggtctgccg gaagagctca tccagaaggg gaaggatatc    120
aagggggtgt cggaaatcgt gcagaatggg aagcacttca agttcaccat caccgctggg    180
tccaaagtga tccaaaacga attcacggtg gggaggaat gtgagctgga gacaatgaca    240
ggggagaaag tcaagacagt ggttcagttg gaaggtgaca ataaactggt gacaactttc    300
aaaaacatca agtctgtgac cgaactcaac ggcgacataa tcaccaatac catgacattg    360
ggtgacattg tcttcaagag aatcagcaag agaatttaaa caagtctgca tttcatatta    420
ttttagtgtg taaaattaat gtaataaagt gaactttgtt tt                      462
```

<210> 78
<211> 2108
<212> DNA
<213> Homo sapiens

<400> 78

```
gggaccgcct cggaggcaga agagccgcga ggagccagcg gagcaccgcg ggctggggcg     60
cagccacccg ccgctcctcg agtcccctcg cccctttccc ttcgtgcccc ccggcagcct    120
ccagcgtcgg tccccaggca gcatggtgag gtctgctccc ggaccctcgc caccatgtac    180
gtgagctacc tcctggacaa ggacgtgagc atgtacccta gctccgtgcg ccactctggc    240
ggcctcaacc tggcgccgca gaacttcgtc agcccccgc agtacccgga ctacggcggt     300
taccacgtgg cggccgcagc tgcagcggca gcgaacttgg acagcgcgca gtccccgggg    360
ccatcctggc cggcagcgta tggcgcccca ctccgggagg actggaatgg ctacgcgccc    420
ggaggcgccg cggccgccgc caacgccgtg gctcacggcc tcaacggtgg ctccccggcc    480
gcagccatgg gctacagcag ccccgcagac taccatccgc accaccaccc gcatcaccac    540
ccgcaccacc cggccgccgc gccttcctgc gcttctgggc tgctgcaaac gctcaacccc    600
ggccctcctg ggcccgccgc caccgctgcc gccgagcagc tgtctcccgg cggccagcgg    660
cggaacctgt gcgagtggat gcggaagccg gcgcagcagt ccctcggcag ccaagtgaaa    720
accaggacga aagacaaata tcgagtggtg tacacggacc accagcggct ggagctggag    780
aaggagtttc actacagtcg ctacatcacc atccggagga aagccgagct agccgccacg    840
ctggggctct ctgagaggca ggttaaaatc tggtttcaga accgcagagc aaaggagagg    900
aaaatcaaca agaagaagtt gcagcagcaa cagcagcagc agccaccaca gccgcctccg    960
ccgccaccac agcctcccca gcctcagcca ggtcctctga gaagtgtccc agagcccttg   1020
agtccggtgt cttccctgca agcctcagtg tctggctctg tccctggggt tctggggcca   1080
actggggggg tgctaaaccc caccgtcacc cagtgaccca ccggggtctg cagcggcaga   1140
gcaattccag gctgagccat gaggagcgtg gactctgcta gactcctcag gagagacccc   1200
```

tcccctccca cccacagcca tagacctaca gacctggctc tcagaggaaa aatgggagcc 1260
aggagtaaga caagtgggat ttggggcctc aagaaatata ctctcccaga tttttacttt 1320
ttcccatctg gctttttctg ccactgagga gacagaaagc ctccgctggg cttcattccg 1380
gactggcaga agcattgcct ggactgacca caccaaccag gccttcatcc tcctccccag 1440
ctcttctctt cctagatctg caggctacac ctctggctag agccgagggg agagagggac 1500
tcaagggaaa ggcaagcttg aggccaagat ggctgctgcc tgctcatggc cctcggaggt 1560
ccagctgggc ctcctgcctc cgggcaggca aggtttacac tgcggaagcc aaaggcagct 1620
aagatagaaa gctggactga ccaaagactg cagaaccccc aggtggcctg cgtctttttt 1680
ctcttccctt cccagaccag gaaaggcttg gctggtgtat gcacagggtg tggtatgagg 1740
gggtggttat tggactccag gcctgaccag ggggcccgaa cagggacttg tttagagagc 1800
ctgtcaccag agcttctctg ggctgaatgt atgtcagtgc tataaatgcc agagccaacc 1860
tggacttcct gtcattttca caatcttggg gctgatgaag aagggggtgg ggggagtttg 1920
tgttgttgtt gctgctgttt gggttgttgg tctgtgtaac atccaagcca gagtttttaa 1980
agccttctgg atccatgggg ggagaagtga tatggtgaag ggaagtgggg agtatttgaa 2040
cacagttgaa ttttttctaa aaagaaaaag agataaatga gctttccaga aaaaaaaaaa 2100
aaaaaaaa 2108

<210> 79
<211> 3745
<212> DNA
<213> Homo sapiens

<400> 79

```
cgcaaagcaa gtgggcacaa ggagtatggt tctaacgtga ttggggtcat gaagacgttg      60
ctgttggact tggctttgtg gtcactgctc ttccagcccg ggtggctgtc ctttagttcc     120
caggtgagtc agaactgcca caatggcagc tatgaaatca gcgtcctgat gatgggcaac     180
tcagcctttg cagagcccct gaaaaacttg gaagatgcgg tgaatgaggg gctggaaata     240
gtgagaggac gtctgcaaaa tgctggccta aatgtgactg tgaacgctac tttcatgtat     300
tcggatggtc tgattcataa ctcaggcgac tgccggagta gcacctgtga aggcctcgac     360
ctactcagga aaatttcaaa tgcacaacgg atgggctgtg tcctcatagg gccctcatgt     420
acatactcca ccttccagat gtaccttgac acagaattga gctaccccat gatctcagct     480
ggaagttttg gattgtcatg tgactataaa gaaaccttaa ccaggctgat gtctccagct     540
agaaagttga tgtacttctt ggttaacttt tggaaaacca acgatctgcc cttcaaaact     600
tattcctgga gcacttcgta tgtttacaag aatggtacag aaactgagga ctgtttctgg     660
taccttaatg ctctggaggc tagcgtttcc tatttctccc acgaactcgg ctttaaggtg     720
gtgttaagac aagataagga gtttcaggat atcttaatgg accacaacag gaaaagcaat     780
gtgattatta tgtgtggtgg tccagagttc ctctacaagc tgaagggtga ccgagcagtg     840
gctgaagaca ttgtcattat tctagtggat cttttcaatg accagtactt ggaggacaat     900
```

gtcacagccc ctgactatat gaaaaatgtc cttgttctga cgctgtctcc tgggaattcc 960
cttctaaata gctctttctc caggaatcta tcaccaacaa aacgagactt tgctcttgcc 1020
tatttgaatg gaatcctgct ctttggacat atgctgaaga tatttcttga aaatggagaa 1080
aatattacca cccccaaatt tgctcatgct ttcaggaatc tcacttttga agggtatgac 1140
ggtccagtga ccttggatga ctggggggat gttgacagta ccatggtgct tctgtatacc 1200
tctgtggaca ccaagaaata caaggttctt ttgacctatg atacccacgt aaataagacc 1260
tatcctgtgg atatgagccc cacattcact tggaagaact ctaaacttcc taatgatatt 1320
acaggccggg gccctcagat cctgatgatt gcagtcttca ccctcactgg agctgtggtg 1380
ctgctcctgc tcgtcgctct cctgatgctc agaaaatata gaaaagatta tgaacttcgt 1440
cagaaaaaat ggtcccacat tcctcctgaa aatatctttc ctctggagac caatgagacc 1500
aatcatgtta gcctcaagat cgatgatgac aaaagacgag atacaatcca gagactacga 1560
cagtgcaaat acgacaaaaa gcgagtgatt ctcaaagatc tcaagcacaa tgatggtaat 1620
ttcactgaaa aacagaagat agaattgaac aagttgcttc agattgacta ttacaacctg 1680
accaagttct acggcacagt gaaacttgat accatgatct tcggggtgat agaatactgt 1740
gagagaggat ccctccggga agttttaaat gacacaattt cctaccctga tggcacattc 1800
atggattggg agtttaagat ctctgtcttg tatgacattg ctaagggaat gtcatatctg 1860
cactccagta agacagaagt ccatggtcgt ctgaaatcta ccaactgcgt agtggacagt 1920
agaatggtgg tgaagatcac tgattttggc tgcaattcca ttttacctcc aaaaaaggac 1980
ctgtggacag ctccagagca cctccgccaa gccaacatct ctcagaaagg agatgtgtac 2040
agctatggga tcatcgcaca ggagatcatt ctgcggaaag aaaccttcta cactttgagc 2100
tgtcgggacc ggaatgagaa gattttcaga gtggaaaatt ccaatggaat gaaacccttc 2160
cgcccagatt tattcttgga aacagcagag gaaaaagagc tagaagtgta cctacttgta 2220
aaaaactgtt gggaggaaga tccagaaaag agaccagatt tcaaaaaaat tgagactaca 2280
cttgccaaga tatttggact tttcatgac caaaaaaatg aaagctatat ggataccttg 2340
atccgacgtc tacagctata ttctcgaaac ctggaacatc tggtagagga aaggacacag 2400
ctgtacaagg cagagaggga cagggctgac agacttaact ttatgttgct tccaaggcta 2460
gtggtaaagt ctctgaagga gaaaggcttt gtggagccgg aactatatga ggaagttaca 2520
atctacttca gtgacattgt aggtttcact actatctgca aatacagcac cccccatggaa 2580
gtggtggaca tgcttaatga catctataag agttttgacc acattgttga tcatcatgat 2640
gtctacaagg tggaaaccat cggtgatgcg tacatggtgg ctagtggttt gcctaagaga 2700
aatggcaatc ggcatgcaat agacattgcc aagatggcct tggaaatcct cagcttcatg 2760
gggacctttg agctggagca tcttcctggc ctcccaatat ggattcgcat tggagttcac 2820
tctggtccct gtgctgctgg agttgtggga atcaagatgc ctcgttattg tctatttgga 2880
gatacggtca acacagcctc taggatggaa tccactggcc tccctttgag aattcacgtg 2940

agtggctcca ccatagccat cctgaagaga actgagtgcc agttccttta tgaagtgaga 3000
ggagaaacat acttaaaggg aagaggaaat gagactacct actggctgac tgggatgaag 3060
gaccagaaat tcaacctgcc aacccctcct actgtggaga atcaacagcg tttgcaagca 3120
gaattttcag acatgattgc caactcttta cagaaaagac aggcagcagg gataagaagc 3180
caaaaaccca gacgggtagc cagctataaa aaaggcactc tggaatactt gcagctgaat 3240
accacagaca aggagagcac ctatttttaa acctaaatga ggtataagga ctcacacaaa 3300
ttaaaataca gctgcactga ggcagcgacc tcaagtgtcc tgaaagctta cattttcctg 3360
agacctcaat gaagcagaaa tgtacttagg cttggctgcc ctgtctggaa catggacttt 3420
cttgcatgaa tcagatgtgt gttctcagtg aaataactac cttccactct ggaaccttat 3480
tccagcagtt gttccaggga gcttctacct ggaaaagaaa agaaatgaat agactatcta 3540
gaacttgaga agattttatt cttatttcat ttattttttg tttgtttatt tttatcgttt 3600
ttgtttactg gctttccttc tgtattcata agatttttta aattgtcata attatatttt 3660
aaatacccat cttcattaaa gtatatttaa ctcataattt ttgcagaaaa tatgctatat 3720
attaggcaag aataaaagct aaagg 3745

<210> 80
<211> 901
<212> DNA
<213> Homo sapiens

<400> 80

agccccaaac tcaccacctg gccgtggaca cctgtgtcag catgtgggac ctggttctct 60
ccatcgcctt gtctgtgggg tgcactggtg ccgtgcccct catccagtct cggattgtgg 120
gaggctggga gtgtgagaag cattcccaac cctggcaggt ggctgtgtac agtcatggat 180
gggcacactg tggggggtgtc ctggtgcacc cccagtgggt gctcacagct gcccattgcc 240
taaagaagaa tagccaggtc tggctgggtc ggcacaacct gtttgagcct gaagacacag 300
gccagagggt ccctgtcagc cacagcttcc cacacccgct ctacaatatg agccttctga 360
agcatcaaag ccttagacca gatgaagact ccagccatga cctcatgctg cttcgcctgt 420
cagagcctgc caagatcaca gatgttgtga aggtcctggg cctgcccacc caggagccag 480
cactggggac cacctgctac gcctcaggct ggggcagcat cgaaccagag gagttcttgc 540
gccccaggag tcttcagtgt gtgagcctcc atctcctgtc caatgacatg tgtgctagag 600
cttactctga gaaggtgaca gagttcatgt tgtgtgctgg gctctggaca ggtggtaaag 660
acacttgtgg gggtgattct gggggtccac ttgtctgtaa tggtgtgctt caaggtatca 720
catcatgggg ccctgagcca tgtgccctgc ctgaaaagcc tgctgtgtac accaaggtgg 780
tgcattaccg gaagtggatc aaggacacca tcgcagccaa cccctgagtg cccctgtccc 840
acccctacct ctagtaaatt taagtccacc tcaaaaaaaa aaaaaaaaaa aaaaaaaaaa 900
a 901

<210> 81
<211> 618
<212> DNA
<213> Homo sapiens

<400> 81

ggggaccact tctctgggac acattgcctt ctgttttctc cagcatgcgc ttgctccagc 60
tcctgttcag ggccagccct gccaccctgc tcctggttct ctgcctgcag ttgggggcca 120
acaaagctca ggacaacact cggaagatca taataaagaa ttttgacatt cccaagtcag 180
tacgtccaaa tgacgaagtc actgcagtgc ttgcagttca aacagaattg aaagaatgca 240
tggtggttaa aacttacctc attagcagca tccctctaca aggtgcattt aactataagt 300
atactgcctg cctatgtgac gacaatccaa aaaccttcta ctgggacttt tacaccaaca 360
gaactgtgca aattgcagcc gtcgttgatg ttattcggga attaggcatc tgccctgatg 420
atgctgctgt aatccccatc aaaaacaacc ggttttatac tattgaaatc ctaaaggtag 480
aataatggaa gccctgtctg tttgccacac ccaggtgatt tcctctaaag aaacttggct 540
ggaatttctg ctgtggtcta taaaataaac ttcttaacat gcttctacaa aaaaaaaaaa 600
aaaaaaaaaa aaaaaaaa 618

<210> 82
<211> 594
<212> DNA
<213> Homo sapiens

<400> 82

```
gtcggtttag gactttctgc ctccactatt gctatcggta ctggaatagc aggcatttca      60
acatctgtca cgaccttcca tagcctatat aatgacttat ctgctagcat cacagacata     120
tcacaaactt tatcagtcct ccaggcccaa gttgaatctt tagctgcagt tgtcctccaa     180
aaccgccgag gccttgactt acttactgct taaagaggag gactctgcat attcttaaat     240
gaggagtgtt gtttttacat aaatcaatct ggcctggtgt atgacaacat aaaaaaattc     300
aaggatagag cccaaaaact taccaaccaa gcaagtaatt tcactgaacc cccttgggca     360
ctccctaatt gggtgtcctg ggtcctccca attcttagtc ctttaatacc catttttctc     420
ctccttttat tcagaccttg tatcttctgt ttagcttctc aattcatcca aaaccatatc     480
caggccatca ccaatcattc tatacgacaa atgtttctta taacatcccc acaatatcac     540
cccttaccac aagacctccc ttcaacttaa tctctcccga tataggttcc caca           594
```

<210> 83
<211> 1372
<212> DNA
<213> Homo sapiens

<400> 83

```
gaattcggcg atgcctcaca actccatcag atctggccat ggagggctga accagctggg      60
aggggccttt gtgaatggca gacctctgcc ggaagtggtc cgccagcgca tcgtagacct     120
ggcccaccag ggtgtaaggc cctgcgacat ctctcgccag ctccgcgtca gccatggttg     180
cgtcagcaag atccttggca ggtactacga gactggcagc atccggcctg gagtgatagg     240
```

```
gggctccaag cccaaggtgg ccaccccca  ggtggtggag aagattgggg actacaaacg     300
ccagaaccct accatgtttg cctgggagat ccgagaccgg ctcctggctg agggcgtctg     360
tgacaatgac actgtgccca gtgtcagctc cattaataga atcatccgga ccaaagtgca     420
gcaaccattc aacctcccta tggacagctg cgtggccacc aagtccctga gtcccggaca     480
cacgctgatc cccagctcag ctgtaactcc cccggagtca ccccagtcgg attccctggg     540
ctccacctac tccatcaatg ggctcctggg catcgctcag cctggcagcg acaagaggaa     600
aatggatgac agtgatcagg atagctgccg actaagcatt gactcacaga gcagcagcag     660
cggaccccga aagcaccttc gcacggatgc cttcagccag caccacctcg agccgctcga     720
gtgcccattt gagcggcagc actacccaga ggcctatgcc tcccccagcc acaccaaagg     780
cgagcagggc ctctacccgc tgcccttgct caacagcacc ctggacgacg ggaaggccac     840
cctgacccct tccaacacgc cactggggcg caacctctcg actcaccaga cctaccccgt     900
ggtggcagat cctcactcac ccttcgccat aaagcaggaa acccccgagg tgtccagttc     960
tagctccacc ccttcctctt tatctagctc cgcctttttg gatctgcagc aagtcggctc    1020
cggggtcccg cccttcaatg cctttcccca tgctgcctcc gtgtacgggc agttcacggg    1080
ccaggccctc ctctcagggc gagagatggt ggggcccacg ctgcccggat acccacccca    1140
catccccacc agcggacagg gcagctatgc ctcctctgcc atcgcaggca tggtggcagg    1200
aagtgaatac tctggcaatg cctatggcca cacccccctac tcctcctaca gcgaggcctg    1260
gcgcttcccc aactccagct tgctgagttc ccccatattat tacagttcca catcaaggcc    1320
gagtgcaccg cccaccactg ccacggcctt tgaccatctg tagttgaagc tt            1372
```

<210> 84
<211> 2983
<212> DNA
<213> Homo sapiens

<400> 84

```
gcccagatag gggagcggag gtggcggcgg cggcggtagc ggtggccttg gttgtcttcc    60
agtctcctcg gctcgccctt tagccggcac cgctcccctt ccctccccct tcctctcttc   120
cttccttccc tccccttccc tttttccctt ccccgtcggt gagcggcggg ggtggctcca   180
gcaacggctg ggcccaagct gtgtagaggc cttaaccaac gataacggcg gcgacggcga   240
aacctcggag ctcgcagggc gggggcaagg cccgggcctt ggagatggag aattctcagt   300
tgtgtaagct gttcatcggc ggcctcaatg tgcagacgag tgagtcgggc ctgcgcggcc   360
actttgaggc ctttgggact ctgacggact gcgtggtggt ggtgaatccc cagaccaagc   420
gctcccgttg ctttggcttc gtgacctact ccaatgtgga ggaggcggac gccgccatgg   480
ccgcctcgcc ccatgccgtg gacggcaaca ctgtggagct gaagcgggcg gtgtcccggg   540
aggattcggc gcggcccggt gcccacgcca aggttaagaa gctctttgtc ggaggcctta   600
aaggagacgt ggctgagggc gacctgatcg agcacttctc gcagtttggc accgtggaaa   660
aggccgagat tattgccgac aagcagtccg gcaagaagcg tggattcggc ttcgtgtatt   720
```

tccagaatca cgacgcggca gacaaggccg cggtggtcaa gttccatccg attcagggcc 780
atcgcgtgga ggtgaagaaa gcagtcccca aggaggatat ctactccggt gggggtggag 840
gcggctcccg atcctcccgg ggcggccgag gcggccgggg gcgcggcggt ggtcgagacc 900
agaacggcct ttccaagggc ggcggcggcg gttacaacag ctacggtggt tacggcggcg 960
gcggaggcgg cggctacaat gcctacggag gcggcggcgg cggttcgtcc tacggtggga 1020
gcgactacgg taacggcttc ggcggcttcg gcagctacag ccagcatcag tcctcctatg 1080
ggcccatgaa gagcggcggc ggcggcggcg gtggaggcag tagctggggc ggtcgcagta 1140
atagtggacc ttacagaggc ggctatggcg gtgggggtgg ctatggaggc agctccttct 1200
aaaagaaaat ttaaaatgcc tgggagtggc tataggggta gctctttcca acagcccaag 1260
tggggtcaac tcctaagccc caccccctca cacacaccgc cttccctgtt ttgcccttgg 1320
gggagccact tctaaggctg cttacccttg gggtgttcc tctatttgcc tgccacctct 1380
cttgtctctc cctctgaaga tggactcggc cccacataca catttttgtg ttacagtcat 1440
tgatggactc tattttttta ttattacttg gaccttggtc gtttttatac tagcaaaatg 1500
tcttgtttta atttgtgttt tttgggggga gggagggagt gaacttgctg attctgtagc 1560
aaaacctggg tgggggttgg ggtgggggt agtttacttt gttgtaagga cttgataacc 1620
tggctacagc gtttctatg aaatctactt ggatcccatg cctgaaattt ggaagcatat 1680
gtacaaaaat catttttacg ttttattttt aataaatcat tgtgtttgac cgtacatgtc 1740
taacattttt tttctaggat ccattccgta ccgtttttta agggatattt gtttaagact 1800
ttacgtgtta attctttatt cttgatgtgt acttagagaa acttaagagg tcctgtggtt 1860
tttttcccct ctcctgttgc cctgctagtt gcgtgttgaa ttatatccct tacaggcaaa 1920
acttttgaag tggtggatgt ggctttttaa actcttaagt ttctgtgcat ccatctcttg 1980
tactaagcga attgtttatc atcttgacat ggttggtcat ttctatgaca atttacttca 2040
aactgtgtac tgtgtagttc tatatagttt gtgttaagca tgtcattcat ataaactgtt 2100
taaaattttt cagatggcct agtttcatcc ctcttactgg tttgtctgta atgaatggtt 2160
aaaaataagg gttatatttt accctcaaat gcgtttttgt actttcagag caggtttaaa 2220
cgtttttttt ttttttttcc tatatccgaa ctgttggcct catggaaatc cctttcccga 2280
tctttgtagc accatctact ggcagaatgg cagagtagct gcgaaacaat ttgtttaaaa 2340
acttgcttaa gacaattgca tcagatttgg aagttttgcc atcaaaattc tttgcagaat 2400
tggaagttaa cacatttgct tgtaactgag atgggcttca caggaatgta gttgccagtt 2460
catatcacaa tagccctttc tatatgaggt ttgaaaatgt aaactgctat gcatagcttg 2520
ggcaatagcc ctaaattgct atgacaacta atgaaccagc tacgtatact ggtattttag 2580
gtgcaagttg taaagcaaaa tatctgtgta ttctgcttgg ttaacaaatg tatatttgta 2640
gccctttcct gcaatagcat tcaagttgtt gtttataaga gaagaacaaa agtgataata 2700
ggtgaaaatt gcctttctgg atagaaatag agaatagcaa cgtttatgga tatcacaaat 2760
aaagaattca attctttaca tgattgagtg agagtatgta taacctggtg ggtgggttca 2820

```
gagtaccttt taatctagta tgcttaactt gatgttaata tttaacttaa atatttgact   2880
tacatgttga cgttgaaggc tcaaagctat actaagaagc tttctgaaag attgggcttt   2940
aaaataaaat aatattttaa tattgaaaaa aaaaaaaaaa aaa                     2983
```

<210> 85
<211> 3345
<212> DNA
<213> Homo sapiens

<400> 85

```
gaattccgtc tcgaccactg aatggaagaa aaggactttt aaccaccatt ttgtgactta     60
cagaaaggaa tttgaataaa gaaaactatg atacttcagg cccatcttca ctccctgtgt    120
cttcttatgc tttatttggc aactggatat ggccaagagg ggaagtttag tggacccctg    180
aaacccatga cattttctat ttatgaaggc caagaaccga gtcaaattat attccagttt    240
aaggccaatc ctcctgctgt gacttttgaa ctaactgggg agacagacaa catatttgtg    300
atagaacggg agggacttct gtattacaac agagccttgg acagggaaac aagatctact    360
cacaatctcc aggttgcagc cctggacgct aatggaatta tagtggaggg tccagtccct    420
atcaccatag aagtgaagga catcaacgac aatcgaccca cgtttctcca gtcaaagtac    480
gaaggctcag taaggcagaa ctctcgccca ggaaagccct tcttgtatgt caatgccaca    540
gacctggatg atccggccac tcccaatggc cagctttatt accagattgt catccagctt    600
cccatgatca acaatgtcat gtactttcag atcaacaaca aaacgggagc catctctctt    660
acccgagagg gatctcagga attgaatcct gctaagaatc cttcctataa tctggtgatc    720
tcagtgaagg acatgggagg ccagagtgag aattccttca gtgataccac atctgtggat    780
atcatagtga cagagaatat ttggaaagca ccaaaacctg tggagatggt ggaaaactca    840
actgatcctc accccatcaa aatcactcag gtgcggtgga atgatcccgg tgcacaatat    900
tccttagttg acaaagagaa gctgccaaga ttcccatttt caattgacca ggaaggagat    960
atttacgtga ctcagccctt ggaccgagaa gaaaaggatg catatgtttt ttatgcagtt   1020
gcaaaggatg agtacggaaa accactttca tatccgctgg aaattcatgt aaaagttaaa   1080
gatattaatg ataatccacc tacatgtccg tcaccagtaa ccgtatttga ggtccaggag   1140
aatgaacgac tgggtaacag tatcgggacc cttactgcac atgacaggga tgaagaaaat   1200
actgccaaca gttttctaaa ctacaggatt gtggagcaaa ctcccaaact tcccatggat   1260
ggactcttcc taatccaaac ctatgctgga atgttacagt tagctaaaca gtccttgaag   1320
aagcaagata ctcctcagta caacttaacg atagaggtgt ctgacaaaga tttcaagacc   1380
ctttgttttg tgcaaatcaa cgttattgat atcaatgatc agatccccat ctttgaaaaa   1440
tcagattatg gaaacctgac tcttgctgaa gacacaaaca ttgggtccac catcttaacc   1500
atccaggcca ctgatgctga tgagccattt actgggagtt ctaaaattct gtatcatatc   1560
ataaagggag acagtgaggg acgcctgggg gttgacacag atccccatac caacaccgga   1620
tatgtcataa ttaaaaagcc tcttgatttt gaaacagcag ctgtttccaa cattgtgttc   1680
```

aaagcagaaa atcctgagcc tctagtgttt ggtgtgaagt acaatgcaag ttcttttgcc 1740
aagttcacgc ttattgtgac agatgtgaat gaagcacctc aattttccca acacgtattc 1800
caagcgaaag tcagtgagga tgtagctata ggcactaaag tgggcaatgt gactgccaag 1860
gatccagaag gtctggacat aagctattca ctgaggggag acacaagagg ttggcttaaa 1920
attgaccacg tgactggtga gatctttagt gtggctccat tggacagaga agccggaagt 1980
ccatatcggg tacaagtggt ggccacagaa gtaggggggt cttccttaag ctctgtgtca 2040
gagttccacc tgatccttat ggatgtgaat gacaaccctc ccaggctagc caaggactac 2100
acgggcttgt tcttctgcca tcccctcagt gcacctggaa gtctcatttt cgaggctact 2160
gatgatgatc agcacttatt tcggggtccc cattttacat tttccctcgg cagtggaagc 2220
ttacaaaacg actgggaagt ttccaaaatc aatggtactc atgcccgact gtctaccagg 2280
cacacagact ttgaggagag ggcgtatgtc gtcttgatcc gcatcaatga tgggggtcgg 2340
ccacccttgg aaggcattgt ttctttacca gttacattct gcagttgtgt ggaaggaagt 2400
tgtttccggc cagcaggtca ccagactggg atacccactg tgggcatggc agttggtata 2460
ctgctgacca cccttctggt gattggtata attttagcag ttgtgtttat ccgcataaag 2520
aaggataaag gcaaagataa tgttgaaagt gctcaagcat ctgaagtcaa acctctgaga 2580
agctgaattt gaaaaggaat gtttgaattt atatagcaag tgctatttca gcaacaacca 2640
tctcatccta ttacttttca tctaacgtgc attataattt tttaaacaga tattccctct 2700
tgtcctttaa tatttgctaa atatttcttt tttgaggtgg agtcttgctc tgtcgcccag 2760
gctggagtac agtggtgtga tcccagctca ctgcaacctc cgcctcctgg gttcacatga 2820
ttctcctgcc tcagcttcct aagtagctgg gtttacaggc acccaccacc atgcccagct 2880
aatttttgta tttttaatag agacggggtt tcgccatttg gccaggctgg tcttgaactc 2940
ctgacgtcaa gtgatctgcc tgccttggtc tcccaataca ggcatgaacc actgcaccca 3000
cctacttaga tatttcatgt gctatagaca ttagagagat ttttcatttt tccatgacat 3060
ttttcctctc tgcaaatggc ttagctactt gtgtttttcc cttttggggc aagacagact 3120
cattaaatat tctgtacatt ttttctttat caaggagata tatcagtgtt gtctcataga 3180
actgcctgga ttccatttat gtttttctg attccatcct gtgtcccctt catccttgac 3240
tcctttggta tttcactgaa tttcaaacat ttgtcagaga agaaaaaagt gaggactcag 3300
gaaaaataaa taaataaaag aacagccttt tgcggccgcg aattc 3345

<210> 86
<211> 990
<212> DNA
<213> Homo sapiens

<400> 86

agccccaagc ttaccacctg cacccggaga gctgtgtcac catgtgggtc ccggttgtct 60
tcctcaccct gtccgtgacg tggattggtg ctgcacccct catcctgtct cggattgtgg 120
gaggctggga gtgcgagaag cattcccaac cctggcaggt gcttgtggcc tctcgtggca 180

gggcagtctg cggcggtgtt ctggtgcacc cccagtgggt cctcacagct gcccactgca 240
tcaggaacaa aagcgtgatc ttgctgggtc ggcacagcct gtttcatcct gaagacacag 300
gccaggtatt tcaggtcagc cacagcttcc cacacccgct ctacgatatg agcctcctga 360
agaatcgatt cctcaggcca ggtgatgact ccagccacga cctcatgctg ctccgcctgt 420
cagagcctgc cgagctcacg gatgctgtga aggtcatgga cctgcccacc caggagccag 480
cactggggac cacctgctac gcctcaggct ggggcagcat tgaaccagag gagttcttga 540
ccccaaagaa acttcagtgt gtggacctcc atgttatttc caatgacgtg tgtgcgcaag 600
ttcaccctca gaaggtgacc aagttcatgc tgtgtgctgg acgctggaca gggggcaaaa 660
gcacctgctc gggtgattct gggggcccac ttgtctgtaa tggtgtgctt caaggtatca 720
cgtcatgggg cagtgaacca tgtgccctgc ccgaaaggcc ttccctgtac accaaggtgg 780
tgcattaccg gaagtggatc aaggacacca tcgtggccaa cccctgagca cccctatcaa 840
ccccctattg tagtaaactt ggaaccttgg aaatgaccag gccaagactc aagcctcccc 900
agttctactg acctttgtcc ttaggtgtga ggtccagggt tgctaggaaa agaaatcagc 960
agacacaggt gtagaccaga gtgtttctta 990

<210> 87
<211> 1805
<212> DNA
<213> Homo sapiens

<400> 87

```
gcgcacactc tcctaagccc tctcatctcc tggaaccatg gccagcacat ccaccaccat      60
caggagccac agcagcagcc gccggggttt cagtgccaac tcagccaggc tccctggggt     120
cagccgctct ggcttcagca gcatctccgt gtcccgctcc aggggcagtg gtggcctggg     180
tggcgcatgt ggaggagctg gctttggcag ccgcagtctg tatggcctgg ggggctccaa     240
gaggatctcc attggagggg gcagctgtgc catcagtggc ggctatggca gcagagccgg     300
aggcagctat ggctttggtg gcgccgggag tggatttggt ttcggtggtg gagccggcat     360
tggctttggt ctgggtggtg gagccggcct tgctggtggc tttgggggcc ctggcttccc     420
tgtgtgcccc cctggaggca tccaagaggt cactgtcaac cagagtctcc tgactcccct     480
caacctgcaa attgaccccg ccatccagcg ggtgcgggcc gaggagcgtg agcagatcaa     540
gaccctcaac aacaagtttg cctccttcat cgacaaggtg cggttcctag agcagcagaa     600
caaggttctg gacaccaagt ggaccctgct gcaggagcag ggcaccaaga ctgtgaggca     660
gaacctggag ccgttgttcg agcagtacat caacaacctc aggaggcagc tggacaacat     720
cgtgggggaa cggggtcgtc tggactcgga gctgagaaac atgcaggacc tggtggagga     780
cctcaagaac aaatatgagg atgaaatcaa caagcgcaca gcagcagaga atgaatttgt     840
gactctgaag aaggatgtgg atgctgccta catgaacaag gttgaactgc aagccaaggc     900
agacactctt acagatgaga tcaacttcct gagagccttg tatgatgcag agctgtccca     960
gatgcagacc cacatctcag acacatccgt ggtgctatcc atggacaaca accgcaacct    1020

ggacctggac agcatcatcg ctgaggtcaa ggcccaatat gaggagattg ctcagaggag    1080
cagggctgag gctgagtcct ggtaccagac aaagtacgag gagctgcaga tcacagcagg    1140
cagacatggg gacgacctgc gcaacaccaa gcaggagatt gctgagatca accgcatgat    1200
ccagaggctg agatctgaga tcgaccacgt caagaagcag tgtgccaacc tacaggccgc    1260
cattgctgat gctgagcagc gtggggagat ggccctcaag gatgctaaga acaagctgga    1320
agggctggag gatgccctgc agaaggccaa gcaggacctg gcccggctgc tgaaggagta    1380
ccaggagctg atgaacgtca agctggccct ggacgtggag atcgccacct accgcaagct    1440
gctggagggc gaggagtgca ggctgaatgg cgaaggcgtt ggacaagtca acatctctgt    1500
agtgcagtcc accgtctcca gtggctatgg cggtgccagc ggtgtcggca gtggcttagg    1560
cctgggtgga ggaagcagct actcctatgg cagtggtctt ggcgttggag gcggctttag    1620
ttccagcagc ggcagagcca ctgggggtgg cctcagctct gttggaggcg gcagttccac    1680
catcaagtac accaccacct cctcctccag caggaagagc tacaagcact gaagtcgtgc    1740
cgccagctct cagtcccaca gctctcaggc ccctctctgg cagcagagcc ctctcctcag    1800
gttgc                                                                1805
```

<210> 88
<211> 2820
<212> DNA
<213> Homo sapiens

<400> 88

```
tggcaaaatc ctggagccag aagaaaggac agcagcattg atcaatctta cagctaacat     60
gttgtacctg gaaaacaatg cccagactca atttagtgag ccacagtaca cgaacctggg    120
gctcctgaac agcatggacc agcagattcg gaacggctcc tcgtccacca gtccctataa    180
cacagaccac gcgcagaaca gcgtcacggc gccctcgccc tacgcacagc ccagccccac    240
cttcgatgct ctctctccat cacccgccat cccctccaac accgactacc caggcccgca    300
cagttccgac gtgtccttcc agcagtcgag caccgccaag tcggccacct ggacgtattc    360
cactgaactg aagaaactct actgccaaat tgcaaagaca tgccccatcc agatcaaggt    420
gatgacccca cctcctcagg gagctgttat ccgcgccatg cctgtctaca aaaaagctga    480
gcacgtcacg gaggtggtga agcggtgccc caaccatgag ctgagccgtg agttcaacga    540
gggacagatt gcccctccta gtcatttgat tcgagtagag gggaacagcc atgcccagta    600
tgtagaagat cccatcacag gaagacagag tgtgctggta ccttatgagc caccccaggt    660
tggcactgaa ttcacgacag tcttgtacaa tttcatgtgt aacagcagtt gtgttggagg    720
gatgaaccgc cgtccaattt taatcattgt tactctggaa accagagatg ggcaagtcct    780
gggccgacgc tgctttgagg cccggatctg tgcttgccca ggaagagaca ggaaggcgga    840
tgaagatagc atcagaaagc agcaagtttc ggacagtaca aagaacggtg atggtacgaa    900
gcgcccgttt cgtcagaaca cacatggtat ccagatgaca tccatcaaga aacgaagatc    960
cccagatgat gaactgttat acttaccagt gaggggccgt gagacttatg aaatgctgtt   1020
```

gaagatcaaa gagtccctgg aactcatgca gtaccttcct cagcacacaa ttgaaacgta 1080
caggcaacag caacagcagc agcaccagca cttacttcag aaacagacct caatacagtc 1140
tccatcttca tatggtaaca gctccccacc tctgaacaaa atgaacagca tgaacaagct 1200
gccttctgtg agccagctta tcaaccctca gcagcgcaac gccctcactc ctacaaccat 1260
tcctgatggc atgggagcca acattcccat gatgggcacc cacatgccaa tggctggaga 1320
catgaatgga ctcagcccca cccaggcact ccctccccca ctctccatgc catccacctc 1380
ccactgcaca cccccacctc cgtatcccac agattgcagc attgtcagtt tcttagcgag 1440
gttgggctgt tcatcatgtc tggactattt cacgacccag ggggctgacca ccatctatca 1500
gattgagcat tactccatgg atgatctggc aagtctgaaa atccctgagc aatttcgaca 1560
tgcgatctgg aagggcatcc tggaccaccg gcagctccac gaattctcct cccccttctca 1620
tctcctgcgg accccaagca gtgcctctac agtcagtgtg ggctccagtg agacccgggg 1680
tgagcgtgtt attgatgctg tgcgattcac cctccgccag accatctctt tcccaccccg 1740
agatgagtgg aatgacttca actttgacat ggatgctcgc cgcaataagc aacagcgcat 1800
caaagaggag ggggagtgag cctcaccatg tgagctcttc ctatccctct cctaactgcc 1860
agccccctaa aagcactcct gcttaatctt caaagccttc tccctagctc ctccccttcc 1920
tcttgtctga tttcttaggg gaaggagaag taagaggcta cctcttacct aacatctgac 1980
ctggcatcta attctgattc tggctttaag ccttcaaaac tatagcttgc agaactgtag 2040
ctgccatggc taggtagaag tgagcaaaaa agagttgggt gtctccttaa gctgcagaga 2100
tttctcattg acttttataa agcatgttca cccttatagt ctaagactat atatataaat 2160
gtataaatat acagtataga tttttgggtg gggggcattg agtattgttt aaaatgtaat 2220
ttaaatgaaa gaaaattgag ttgcacttat tgaccatttt ttaatttact tgttttggat 2280
ggcttgtcta tactccttcc cttaaggggt atcatgtatg gtgataggta tctagagctt 2340
aatgctacat gtgagtgcga tgatgtacag attctttcag ttctttggat tctaaataca 2400
tgccacatca aacctttgag tagatccatt tccattgctt attatgtagg taagactgta 2460
gatatgtatt cttttctcag tgttggtata ttttatatta ctgacatttc ttctagtgat 2520
gatggttcac gttggggtga tttaatccag ttataagaag aagttcatgt ccaaacggtc 2580
ctctttagtt tttggttggg aatgaggaaa attcttaaaa ggcccatagc agccagttca 2640
aaaacacccg acgtcatgta tttgagcata tcagtaaccc ccttaaattt aatacccaga 2700
taccttatct tacaatgttg attgggaaaa catttgctgc ccattacaga ggtattaaaa 2760
ctaaatttca ctactagatt gactaactca aatacacatt tgctactgtt gtaagaattc 2820

<210> 89
<211> 991
<212> DNA
<213> Homo sapiens

<400> 89

cttatctcgg cttcgtttct ggagggccag gaacaaacag gcttcaaagc caagggcttg 60

gctggcacac agggggcttg gtccttcacc tctgtcccct ctccctacgg acacatataa 120
gaccctggtc acacctggga gaggaggaga ggagagcata gcacctgcag caagatggat 180
gtgggcagca aagaggtcct gatggagagc ccgccggact actccgcagc tccccggggc 240
cgatttggca ttccctgctg cccagtgcac ctgaaacgcc ttcttatcgt ggtggtggtg 300
gtggtcctca tcgtcgtggt gattgtggga gccctgctca tgggtctcca catgagccag 360
aaacacacgg agatggttct ggagatgagc attggggcgc cggaagccca gcaacgcctg 420
gccctgagtg agcacctggt taccactgcc accttctcca tcggctccac tggcctcgtg 480
gtgtatgact accagcagct gctgatcgcc tacaagccag cccctggcac ctgctgctac 540
atcatgaaga tagctccaga gagcatcccc agtcttgagg ctctcaatag aaaagtccac 600
aacttccaga tggaatgctc tctgcaggcc aagcccgcag tgcctacgtc taagctgggc 660
caggcagagg ggcgagatgc aggctcagca ccctccggag gggacccggc cttcctgggc 720
atggccgtga acaccctgtg tggcgaggtg ccgctctact acatctagga cgcctccggt 780
gagcagggtc agtggaagcc ccaacgggaa aggaaacgcc ccgggcaaag ggtcttttgc 840
agcttttgca gacgggcaag aagctgcttc tgcccacacc gcagggacaa accctggaga 900
aatgggagct tggggagagg atgggagtgg gcagaggtgg cacccagggg cccgggaact 960
cctgccacaa cagaataaag cagcctgatt g 991

<210> 90
<211> 1580
<212> DNA
<213> Homo sapiens

<400> 90

```
catcctgcca cccctagcct tgctggggac gtgaaccctc tccccgcgcc tgggaagcct     60
tcttggcacc gggacccgga gaatccccac ggaagccagt tccaaaaggg atgaaaaggg    120
ggcgtttcgg gcactgggag aagcctgtat tccagggccc ctcccagagc aggaatctgg    180
gacccaggag tgccagcctc acccacgcag atcctggcca tgagagctcc gcacctccac    240
ctctccgccg cctctggcgc ccgggctctg gcgaagctgc tgccgctgct gatggcgcaa    300
ctctgggccg cagaggcggc gctgctcccc caaaacgaca cgcgcttgga ccccgaagcc    360
tatggctccc cgtgcgcgcg cggctcgcag ccctggcagg tctcgctctt caacggcctc    420
tcgttccact gcgcgggtgt cctggtggac cagagttggg tgctgacggc cgcgcactgc    480
ggaaacaagc cactgtgggc tcgagtaggg gatgaccacc tgctgcttct tcagggagag    540
cagctccgcc ggaccactcg ctctgttgtc catcccaagt accaccaggg ctcaggcccc    600
atcctgccaa ggcgaacgga tgagcacgat ctcatgttgc tgaagctggc caggcccgta    660
gtgctggggc cccgcgtccg ggccctgcag cttccctacc gctgtgctca gcccggagac    720
cagtgccagg ttgctggctg gggcaccacg gccgcccgga gagtgaagta caacaagggc    780
ctgacctgct ccagcatcac tatcctgagc cctaaagagt gtgaggtctt ctaccctggc    840
gtggtcacca acaacatgat atgtgctgga ctggaccggg gccaggaccc ttgccagagt    900

gactctggag gcccccctggt ctgtgacgag accctccaag gcatcctctc gtggggtgtt   960
taccccctgtg gctctgccca gcatccagct gtctacaccc agatctgcaa atacatgtcc  1020
tggatcaata aagtcatacg ctccaactga tccagatgct acgctccagc tgatccagat   1080
gttatgctcc tgctgatcca gatgcccaga ggctccatcg tccatcctct tcctccccag   1140
tcggctgaac tctccccttg tctgcactgt tcaaacctct gccgccctcc acacctctaa   1200
acatctcccc tctcacctca ttcccccacc tatccccatt ctctgcctgt actgaagctg   1260
aaatgcagga agtggtggca aaggtttatt ccagagaagc caggaagccg gtcatcaccc   1320
agcctctgag agcagttact ggggtcaccc aacctgactt cctctgccac tccctgctgt   1380
gtgactttgg gcaagccaag tgccctctct gaacctcagt ttcctcatct gcaaaatggg   1440
aacaatgacg tgcctacctc ttagacatgt tgtgaggaga ctatgatata acatgtgtat   1500
gtaaatcttc atggtgattg tcatgtaagg cttaacacag tgggtggtga gttctgacta   1560
aaggttacct gttgtcgtga                                               1580
```

<210> 91
<211> 3359
<212> DNA
<213> Homo sapiens

<400> 91

```
cacaccttcg gcagcaggag ggcggcagct tctcgcaggc ggcagggcgg gcggccagga    60
tcatgtccac caccacatgc caagtggtgg cgttcctcct gtccatcctg gggctggccg   120
gctgcatcgc ggccaccggg atggacatgt ggagcaccca ggacctgtac gacaaccccg   180
tcacctccgt gttccagtac gaagggctct ggaggagctg cgtgaggcag agttcaggct   240
tcaccgaatg caggccctat ttcaccatcc tgggacttcc agccatgctg caggcagtgc   300
gagccctgat gatcgtaggc atcgtcctgg gtgccattgg cctcctggta tccatctttg   360
ccctgaaatg catccgcatt ggcagcatgg aggactctgc caaagccaac atgacactga   420
cctccgggat catgttcatt gtctcaggtc tttgtgcaat tgctggagtg tctgtgtttg   480
ccaacatgct ggtgactaac ttctggatgt ccacagctaa catgtacacc ggcatgggtg   540
ggatggtgca gactgttcag accaggtaca catttggtgc ggctctgttc gtgggctggg   600
tcgctggagg cctcacacta attgggggtg tgatgatgtg catcgcctgc cggggcctgg   660
caccagaaga aaccaactac aaagccgttt cttatcatgc ctcaggccac agtgttgcct   720
acaagcctgg aggcttcaag gccagcactg gctttgggtc caacaccaaa aacaagaaga   780
tatacgatgg aggtgcccgc acagaggacg aggtacaatc ttatccttcc aagcacgact   840
atgtgtaatg ctctaagacc tctcagcacg ggcggaagaa actcccggag agctcaccca   900
aaaaacaagg agatcccatc tagatttctt cttgcttttg actcacagct ggaagttaga   960
aaagcctcga tttcatcttt ggagaggcca aatggtctta gcctcagtct ctgtctctaa  1020
atattccacc ataaaacagc tgagttattt atgaattaga ggctatagct cacattttca  1080
atcctctatt tcttttttta aatataactt tctactctga tgagagaatg tggttttaat  1140
```

```
ctctctctca cattttgatg atttagacag actccccctc ttcctcctag tcaataaacc    1200
cattgatgat ctatttccca gcttatcccc aagaaaactt ttgaaaggaa agagtagacc    1260
caaagatgtt attttctgct gtttgaattt tgtctcccca cccccaactt ggctagtaat    1320
aaacacttac tgaagaagaa gcaataagag aaagatattt gtaatctctc cagcccatga    1380
tctcggtttt cttacactgt gatcttaaaa gttaccaaac caaagtcatt ttcagtttga    1440
ggcaaccaaa cctttctact gctgttgaca tcttcttatt acagcaacac cattctagga    1500
gtttcctgag ctctccactg gagtcctctt tctgtcgcgg gtcagaaatt gtccctagat    1560
gaatgagaaa attatttttt ttaatttaag tcctaaatat agttaaaata aataatgttt    1620
tagtaaaatg atacactatc tctgtgaaat agcctcaccc ctacatgtgg atagaaggaa    1680
atgaaaaaat aattgctttg acattgtcta tatggtactt tgtaaagtca tgcttaagta    1740
caaattccat gaaaagctca ctgatcctaa ttctttccct ttgaggtctc tatggctctg    1800
attgtacatg atagtaagtg taagccatgt aaaaagtaaa taatgtctgg gcacagtggc    1860
tcacgcctgt aatcctagca ctttgggagg ctgaggagga aggatcactt gagcccagaa    1920
gttcgagact agcctgggca acatggagaa gccctgtctc tacaaaatac agagagaaaa    1980
aatcagccag tcatggtggc ctacacctgt agtcccagca ttccgggagg ctgaggtggg    2040
aggatcactt gagcccaggg aggttggggc tgcagtgagc catgatcaca ccactgcact    2100
ccagccaggt gacatagcga gatcctgtct aaaaaaataa aaaataaata atggaacaca    2160
gcaagtccta ggaagtaggt taaaactaat tctttaaaaa aaaaaaaaag ttgagcctga    2220
attaaatgta atgtttccaa gtgacaggta tccacatttg catggttaca agccactgcc    2280
agttagcagt agcactttcc tggcactgtg gtcggttttg ttttgttttg ctttgtttag    2340
agacggggtc tcactttcca ggctggcctc aaactcctgc actcaagcaa ttcttctacc    2400
ctggcctccc aagtagctgg aattacaggt gtgcgccatc acaactagct ggtggtcagt    2460
tttgttactc tgagagctgt tcacttctct gaattcacct agagtggttg gaccatcaga    2520
tgtttgggca aaactgaaag ctctttgcaa ccacacacct tccctgagct tacatcactg    2580
cccttttgag cagaaagtct aaattccttc caagacagta gaattccatc ccagtaccaa    2640
agccagatag gccccctagg aaactgaggt aagagcagtc tctaaaaact acccacagca    2700
gcattggtgc aggggaactt ggccattagg ttattatttg agaggaaagt cctcacatca    2760
atagtacata tgaaagtgac ctccaagggg attggtgaat actcataagg atcttcaggc    2820
tgaacagact atgtctgggg aaagaacgga ttatgcccca ttaaataaca agttgtgttc    2880
aagagtcaga gcagtgagct cagaggccct tctcactgag acagcaacat ttaaaccaaa    2940
ccagaggaag tatttgtgga actcactgcc tcagtttggg taaaggatga gcagacaagt    3000
caactaaaga aaaaagaaaa gcaaggagga gggttgagca atctagagca tggagtttgt    3060
taagtgctct ctggatttga gttgaagagc atccatttga gttgaaggcc acagggcaca    3120
atgagctctc ccttctacca ccagaaagtc cctggtcagg tctcaggtag tgcggtgtgg    3180
```

ctcagctggg tttttaatta gcgcattctc tatccaacat ttaattgttt gaaagcctcc 3240

atatagttag attgtgcttt gtaattttgt tgttgttgct ctatcttatt gtatatgcat 3300

tgagtattaa cctgaatgtt ttgttactta aatattaaaa acactgttat cctacagtt 3359

<210> 92
<211> 733
<212> DNA
<213> Homo sapiens

<400> 92

gggatccgga gcccaaatct tctgacaaaa ctcacacatg cccaccgtgc ccagcacctg 60

aattcgaggg tgcaccgtca gtcttcctct tccccccaaa acccaaggac accctcatga 120

tctcccggac tcctgaggtc acatgcgtgg tggtggacgt aagccacgaa gaccctgagg 180

tcaagttcaa ctggtacgtg gacggcgtgg aggtgcataa tgccaagaca aagccgcggg 240

aggagcagta caacagcacg taccgtgtgg tcagcgtcct caccgtcctg caccaggact 300

ggctgaatgg caaggagtac aagtgcaagg tctccaacaa agccctccca acccccatcg 360

agaaaaccat ctccaaagcc aaagggcagc cccgagaacc acaggtgtac accctgcccc 420

catcccggga tgagctgacc aagaaccagg tcagcctgac ctgcctggtc aaaggcttct 480

atccaagcga catcgccgtg gagtgggaga gcaatgggca gccggagaac aactacaaga 540

ccacgcctcc cgtgctggac tccgacggct ccttcttcct ctacagcaag ctcaccgtgg 600

acaagagcag gtggcagcag gggaacgtct tctcatgctc cgtgatgcat gaggctctgc 660

acaaccacta cacgcagaag agcctctccc tgtctccggg taaatgagtg cgacggccgc 720

gactctagag gat 733

<210> 93
<211> 1076
<212> DNA
<213> Homo sapiens

<400> 93

atggtggttg aggttgattc catgccggct gcctcttctg tgaagaagcc atttggtctc 60
aggagcaaga tgggcaagtg gtgctgccgt tgcttcccct gctgcaggga gagcggcaag 120
agcaacgtgg gcacttctgg agaccacgac gactctgcta tgaagacact caggagcaag 180
atgggcaagt ggtgccgcca ctgcttcccc tgctgcaggg ggagtggcaa gagcaacgtg 240
ggcgcttctg gagaccacga cgactctgct atgaagacac tcaggaacaa gatgggcaag 300
tggtgctgcc actgcttccc ctgctgcagg gggagcggca agagcaaggt gggcgcttgg 360
ggagactacg atgacagtgc cttcatggag cccaggtacc acgtccgtgg agaagatctg 420
gacaagctcc acagagctgc ctggtggggt aaagtcccca gaaaggatct catcgtcatg 480
ctcagggaca ctgacgtgaa caagaaggac aagcaaaaga ggactgctct acatctggcc 540
tctgccaatg ggaattcaga agtagtaaaa ctcctgctgg acagacgatg tcaacttaat 600
gtccttgaca acaaaaagag gacagctctg ataaaggccg tacaatgcca ggaagatgaa 660
tgtgcgttaa tgttgctgga acatggcact gatccaaata ttccagatga gtatggaaat 720

accactctgc actacgctat ctataatgaa gataaattaa tggccaaagc actgctctta 780
tatggtgctg atatcgaatc aaaaaacaag catggcctca caccactgtt acttggtgta 840
catgagcaaa aacagcaagt cgtgaaattt ttaatcaaga aaaaagcgaa tttaaatgca 900
ctggatagat atggaaggac tgctctcata cttgctgtat gttgtggatc agcaagtata 960
gtcagccttc tacttgagca aaatattgat gtatcttctc aagatctatc tggacagacg 1020
gccagagagt atgctgtttc tagtcatcat catgtaattt gccagttact ttctga 1076

<210> 94
<211> 3675
<212> DNA
<213> Homo sapiens

<400> 94

```
tccgagctga ttacagacac caaggaagat gctgtaaaga gtcagcagcc acagccctgg     60
ctagctggcc ctgtgggcat ttattagtaa agttttaatg acaaaagctt tgagtcaaca    120
cacccgtggg taattaacct ggtcatcccc accctggaga gccatcctgc ccatgggtga    180
tcaaagaagg aacatctgca ggaacacctg atgaggctgc acccttggcg gaaagaacac    240
ctgacacagc tgaaagcttg gtggaaaaaa cacctgatga ggctgcaccc ttggtggaaa    300
gaacacctga cacggctgaa agcttggtgg aaaaaacacc tgatgaggct gcatccttgg    360
tggagggaac atctgacaaa attcaatgtt tggagaaagc gacatctgga aagttcgaac    420
agtcagcaga agaaacacct agggaaatta cgagtcctgc aaaagaaaca tctgagaaat    480
ttacgtggcc agcaaaagga agacctagga agatcgcatg ggagaaaaaa gaagacacac    540
ctagggaaat tatgagtccc gcaaaagaaa catctgagaa atttacgtgg gcagcaaaag    600
gaagacctag gaagatcgca tgggagaaaa aagaaacacc tgtaaagact ggatgcgtgg    660
caagagtaac atctaataaa actaaagttt tggaaaaagg aagatctaag atgattgcat    720
gtcctacaaa agaatcatct acaaaagcaa gtgccaatga tcagaggttc ccatcagaat    780
ccaaacaaga ggaagatgaa gaatattctt gtgattctcg gagtctcttt gagagttctg    840
caaagattca agtgtgtata cctgagtcta tatatcaaaa agtaatggag ataaatagag    900
aagtagaaga gcctcctaag aagccatctg ccttcaagcc tgccattgaa atgcaaaact    960
ctgttccaaa taaagccttt gaattgaaga atgaacaaac attgagagca gatccgatgt   1020
tcccaccaga atccaaacaa aaggactatg aagaaaattc ttgggattct gagagtctct   1080
gtgagactgt ttcacagaag gatgtgtgtt tacccaaggc tacacatcaa aaagaaatag   1140
ataaaataaa tggaaaatta gaagagtctc ctaataaaga tggtcttctg aaggctacct   1200
gcggaatgaa agtttctatt ccaactaaag ccttagaatt gaaggacatg caaactttca   1260
aagcagagcc tccggggaag ccatctgcct tcgagcctgc cactgaaatg caaaagtctg   1320
tcccaaataa agccttggaa ttgaaaaatg aacaaacatt gagagcagat gagatactcc   1380
catcagaatc caaacaaaag gactatgaag aaagttcttg ggattctgag agtctctgtg   1440
agactgtttc acagaaggat gtgtgtttac ccaaggctcc atcaaaaaga aatagataaa   1500
```

ataaatggaa aattagaagg gtctcctgtt aaagatggtc ttctgaaggc taactgcgga 1560
atgaaagttt ctattccaac taaagcctta gaattgatgg acatgcaaac tttcaaagca 1620
gagcctcccg agaagccatc tgccttcgag cctgccattg aaatgcaaaa gtctgttcca 1680
aataaagcct tggaattgaa gaatgaacaa acattgagag cagatgagat actcccatca 1740
gaatccaaac aaaaggacta tgaagaaagt tcttgggatt ctgagagtct ctgtgagact 1800
gtttcacaga aggatgtgtg tttacccaag gctccatcaa aaagaaatag ataaaataaa 1860
tggaaaatta gaagagtctc ctgataatga tggttttctg aaggctccct gcagaatgaa 1920
agtttctatt ccaactaaag ccttagaatt gatggacatg caaactttca aagcagagcc 1980
tcccgagaag ccatctgcct tcgagcctgc cattgaaatg caaaagtctg ttccaaataa 2040
agccttggaa ttgaagaatg aacaaacatt gagagcagat cagatgttcc cttcagaatc 2100
aaaacaaaag aagttgaaga aaattcttgg gattctgaga gtctccgtga gactgtttca 2160
cagaaggatg tgtgtgtacc caaggctaca catcaaaaag aaatggataa aataagtgga 2220
aaattagaag attcaactag cctatcaaaa atcttggata cagttcattc ttgtgaaaga 2280
gcaagggaac ttcaaaaaga tcactgtgaa caacgtacag gaaaaatgga acaaatgaaa 2340
aagaagtttt gtgtactgaa aaagaaactg tcagaagcaa aagaaataaa atcacagtta 2400
gagaaccaaa aagttaaatg ggaacaagag ctctgcagtg tgaggtttct cacactcatg 2460
aaaatgaaaa ttatctctta catgaaaatt gcatgttgaa aaaggaaatt gccatgctaa 2520
aactggaaat agccacactg aaacaccaat accaggaaaa ggaaaataaa tactttgagg 2580
acattaagat tttaaaagaa aagaatgctg aacttcagat gaccctaaaa ctgaaagagg 2640
aatcattaac taaaagggca tctcaatata gtgggcagct taaagttctg atagctgaga 2700
acacaatgct cacttctaaa ttgaaggaaa aacaagacaa agaaatacta gaggcagaaa 2760
ttgaatcaca ccatcctaga ctggcttctg ctgtacaaga ccatgatcaa attgtgacat 2820
caagaaaaag tcaagaacct gctttccaca ttgcaggaga tgcttgtttg caaagaaaaa 2880
tgaatgttga tgtgagtagt acgatatata acaatgaggt gctccatcaa ccactttctg 2940
aagctcaaag gaaatccaaa agcctaaaaa ttaatctcaa ttatgcggag atgctctaag 3000
agaaaataca ttggtttcag aacatgcaca aagagaccaa cgtgaaacac agtgtcaaat 3060
gaaggaagct gaacacatgt atcaaaacga acaagataat gtgaacaaac acactgaaca 3120
gcaggagtct ctagatcaga aattatttca actacaaagc aaaaatatgt ggcttcaaca 3180
gcaattagtt catgcacata agaaagctga caacaaaagc aagataacaa ttgatattca 3240
ttttcttgag aggaaaatgc aacatcatct cctaaaagag aaaaatgagg agatatttaa 3300
ttacaataac catttaaaaa accgtatata tcaatatgaa aaagagaaag cagaaacaga 3360
aaactcatga gagacaagca gtaagaaact tcttttggag aaacaacaga ccagatcttt 3420
actcacaact catgctagga ggccagtcct agcatcacct tatgttgaaa atcttaccaa 3480
tagtctgtgt caacagaata cttattttag aagaaaaatt catgatttct tcctgaagcc 3540

tacagacata aaataacagt gtgaagaatt acttgttcac gaattgcata aagctgcaca 3600

ggattcccat ctaccctgat gatgcagcag acatcattca atccaaccag aatctcgctc 3660

tgtcactcag gctgg 3675

<210> 95
<211> 2658
<212> DNA
<213> Homo sapiens

<400> 95

acccagaaga ccgtgccttg cctggaagtc ctgcctgtag gcctgaagga cttgccctaa 60
cagagcctca acaactacct ggtgattcct acttcagccc cttggtgtga gcagcttctc 120
aacatgaact acagcctcca cttggccttc gtgtgtctga gtctcttcac tgagaggatg 180
tgcatccagg ggagtcagtt caacgtcgag gtcggcagaa gtgacaagct ttccctgcct 240
ggctttgaga acctcacagc aggatataac aaatttctca ggcccaattt tggtggagaa 300
cccgtacaga tagcgctgac tctggacatt gcaagtatct ctagcatttc agagagtaac 360
atggactaca cagccaccat atacctccga cagcgctgga tggaccagcg gctggtgttt 420
gaaggcaaca agagcttcac tctggatgcc cgcctcgtgg agttcctctg ggtgccagat 480
acttacattg tggagtccaa gaagtccttc ctccatgaag tcactgtggg aaacaggctc 540
atccgcctct tctccaatgg cacggtcctg tatgccctca gaatcacgac aactgttgca 600
tgtaacatgg atctgtctaa ataccccatg gacacacaga catgcaagtt gcagctggaa 660
agctggggct atgatggaaa tgatgtggag ttcacctggc tgagagggaa cgactctgtg 720
cgtggactgg aacacctgcg gcttgctcag tacaccatag agcggtattt caccttagtc 780
accagatcgc agcaggagac aggaaattac actagattgg tcttacagtt tgagcttcgg 840
aggaatgttc tgtatttcat tttggaaacc tacgttcctt ccactttcct ggtggtgttg 900
tcctgggttt cattttggat ctctctcgat tcagtccctg caagaacctg cattggagtg 960
acgaccgtgt tatcaatgac cacactgatg atcgggtccc gcacttctct tcccaacacc 1020
aactgcttca tcaaggccat cgatgtgtac ctggggatct gctttagctt tgtgtttggg 1080
gccttgctag aatatgcagt tgctcactac agttccttac agcagatggc agccaaagat 1140
agggggacaa caaaggaagt agaagaagtc agtattacta atatcatcaa cagctccatc 1200
tccagcttta aacggaagat cagctttgcc agcattgaaa tttccagcga caacgttgac 1260
tacagtgact tgacaatgaa aaccagcgac aagttaaagt ttgtcttccg agaaaagatg 1320
ggcaggattg ttgattattt cacaattcaa aaccccagta atgttgatca ctattccaaa 1380
ctactgtttc ctttgatttt tatgctagcc aatgtatttt actgggcata ctacatgtat 1440
ttttgagtca atgttaaatt tcttgcatgc cataggtctt caacaggaca agataatgat 1500
gtaaatggta ttttaggcca agtgtgcacc cacatccaat ggtgctacaa gtgactgaaa 1560
taatatttga gtctttctgc tcaaagaatg aagctccaac cattgttcta agctgtgtag 1620
aagtcctagc attataggat cttgtaatag aaacatcagt ccattcctct ttcatcttaa 1680

```
tcaaggacat tcccatggag cccaagatta caaatgtact cagggctgtt tattcggtgg    1740
ctccctggtt tgcatttacc tcatataaag aatgggaagg agaccattgg gtaaccctca    1800
agtgtcagaa gttgtttcta aagtaactat acatgttttt tactaaatct ctgcagtgct    1860
tataaaatac attgttgcct atttagggag taacattttc tagtttttgt ttctggttaa    1920
aatgaaatat gggcttatgt caattcattg gaagtcaatg cactaactca ataccaagat    1980
gagtttttaa ataatgaata ttatttaata ccacaacaga attatcccca atttccaata    2040
agtcctatca ttgaaaattc aaatataagt gaagaaaaaa ttagtagatc aacaatctaa    2100
acaaatccct cggttctaag atacaatgga ttccccatac tggaaggact ctgaggcttt    2160
attcccccac tatgcatatc ttatcatttt attattatac acacatccat cctaaactat    2220
actaaagccc ttttcccatg catggatgga aatggaagat tttttttaa cttgttctag    2280
aagtcttaat atgggctgtt gccatgaagg cttgcagaat tgagtccatt ttctagctgc    2340
ctttattcac atagtgacgg ggtactaaaa gtactgggtt gactcagaga gtcgctgtca    2400
ttctgtcatt gctgctactc taacactgag caacactctc ccagtggcag atcccctgta    2460
tcattccaag aggagcattc atccctttgc tctaatgatc aggaatgatg cttattagaa    2520
aacaaactgc ttgacccagg aacaagtggc ttagcttaag taaacttggc tttgctcaga    2580
tccctgatcc ttccagctgg tctgctctga gtggcttatc ccgcatgagc aggagcgtgc    2640
tggccctgag tactgaac                                                  2658
```

<210> 96
<211> 2531
<212> DNA
<213> Homo sapiens

<400> 96

gcagtgtgag gcaatcgctc tatccttgac cccttccttt gcacagtgag tgatggcgtt 60
tttatctcct gatgatgatg cacagccttc agcgggggac atttaagacg cagaacacca 120
ggtccaggct gcagctgcgg gactcagagg cgaagcttga ggggctcagg aaggacgaag 180
aaccaccctt gagagaagag gcagcagcag cggcggcagc agcagcggca gcgaccccac 240
cactgccaca tttgccagga aacaatgctg ctagcgacat tcaagctgtg cgctgggagc 300
tcctacagac acatgcgcaa catgaagggg ctgaggcaac aggctgtgat ggccatcagc 360
caggagctga accggagggc cctgggggc cccacccta gcacgtggat taaccaggtt 420
cggcggcgga gctctctact cggttctcgg ctggaagaga ctctctacag tgaccaggag 480
ctggcctatc tccagcaggg ggaggaggcc atgcagaagg ccttgggcat ccttagcaac 540
caagagggct ggaagaagga gagtcagcag gacaatgggg acaaagtgat gagtaaagtg 600
gtcccagatg tgggcaaggt gttccggctg gaggtcgtgg tggaccagcc catggagagg 660
ctctatgaag agctcgtgga gcgcatggaa gcaatggggg agtggaaccc caatgtcaag 720
gagatcaagg tcctgcagaa gatcgggggg ccccgtgact ttgtgagcgt gcgctgtgcc 780
aagcgccgag gctccacctg tgtgctggct ggcatggcca cagacttcgg gaacatgcct 840

gagcagaagg gtgtcatcag ggcggagcac ggtcccactt gcatggtgct tcacccgttg 900
gctggaagtc cctctaagac caaacttacg tggctactca gcatcgacct caaggggtgg 960
ctgcccaaga gcatcatcaa ccaggtcctg tcccagaccc aggtggattt tgccaaccac 1020
ctgcgcaagc gcctggagtc ccaccctgcc tctgaagcca ggtgttgaag accagcctgc 1080
tgttcccaac tgtgcccagc tgcactggta cacacgctca tcaggagaat ccctactgga 1140
agcctgcaag tctaagatct ccatctggtg acagtgggat gggtggggtt cgtgtttaga 1200
gtatgacact aggattcaga ttggtgaaag tttttagtac caagaaaaca gggatgaggc 1260
tcttggatta aaaggtaact tcattcactg attagctatg acatgagggt tcaggcccct 1320
aaaataattg taaaactttt tttctgggcc cttatgtacc cacctaaaac catctttaaa 1380
atgctagtgg ctgatatggg tgtgggggat gctaaccaca gggcctgaga agtcttgctt 1440
tatgggctca agaatgccat gcgctggcag tacatgtgca caaagcagaa tctcagaggg 1500
tctcctgcag ccctctgctc ctcccggccg ctgcacagca acaccacaga acaagcagca 1560
ccccacagtg ggtgccttcc agaaatatag tccaagcttt ctctgtggaa aaagacaaaa 1620
ctcattagta gacatgtttc cctattgctt tcataggcac cagtcagaat aaagaatcat 1680
aattcacaca aacatcagtc tttgttttaa tattgtactg ttaaaaaaat ctatgcagct 1740
gggtgcagtg gctcacgcct gtaatcccag cattttggga ggctgaggta ggcggatcac 1800
aaggtcagga gatcgagacc atcctggcca acatggtgaa accccgtctc tactaaaata 1860
caaaaaatta gctgggtgtg gtggcgcaaa cctgtagtcg tagctacttg ggaggctgag 1920
gcaggggaat cacttgaacc ccggaggcgg aggttgtagt gggccgagat tgtgccactg 1980
cgctccagcg tgggcgacag agtgagactc catctcaaaa aaaaaaaaaa aaaaatctat 2040
gctagtagat tacaacttca cactagagga gttctggaca aagcttttaa ttagtcaaac 2100
taaattaagg ctcattaaaa ggaaaggaac tactgggaaa ttatgcaatt caataattta 2160
gactctgtta ccaggatctt tcataaaaat ttaatttcca taatcataac ctaaatgagt 2220
tcttaaagaa ttctataagc aatagctgat taatgggccc tggaagatga agattataac 2280
tgtttattta cctaattaaa aggaaaggca gtgccaaata tgagaggata aacaatatta 2340
gttaacattt ctgttattta tgatgccaat tagtagtaag ataattccac agctgtcaac 2400
tttgtttggg gctggcaact tctctgctta aacaggctaa aagtttagta ttctgggaga 2460
agtggctgga agaaggggta atatggtgaa agcaaattcc ctttcccagg agtcaagaga 2520
atttatgtga g 2531

<210> 97
<211> 6164
<212> DNA
<213> Homo sapiens

<220>
<221> misc_feature
<222> (2851)..(2851)

<223> n is a, c, g, or t

<220>
<221> misc_feature
<222> (2856)..(2856)
<223> n is a, c, g, or t

<400> 97

cgggcgccgc aggagcgagt gagctgggag cgaggggcga aggcgcggag aagcccggcc 60
gcccggtggg cggcagaagg ctcagccgag gcggcggcgc cgactccgtt ccactctcgg 120
cccggatcca ggcctccggg ttcccaggcg ctcacctccc tctgacgcac tttaaagagt 180
ctcccccctt ccacctcagg gcgagtaata gcgaccaatc atcaagccat ttaccaggct 240
tcggaggaag ctgtttatgt gatccccgca ctaattaggc tcatgaacta acaaatcgtt 300
tgcacaactt gtgaagaagc gaacacttcc atggattgtc cttggactta gggcgccctg 360
cccgcctttt gcagaggaga aaaaactttt tttttttttt gcctccccccg agaactttcc 420
ccccttctcc tccctgcctc taactccgat ccccccacgc catctcgcca aaaaaaaaaa 480
aaaaaaaaaa aaagaaaaaa aaagaaaaaa aaagaaaaaa aattacccca atccacgcct 540
gcaaattctt ctggaaggat tttccccccct ctcttcaggt tgggcgcgtt tggtgcaaga 600
ttctcgggat cctcggcttt gcctctccct ctccctcccc cctcctttcc tttttccttt 660
cctttccttt ctttcttcct ttccttcccc ccaccccac ccccacccca aacaaacgag 720
tccccaattc tcgtccgtcc tcgccgcggg cagcgggcgg cggaggcagc gtgcggcggt 780
cgccaggagc tgggagccca gggcgcccgc tcctcggcgc agcatgttcc agccggcgcc 840
caagcgctgc ttcaccatcg agtcgctggt ggccaaggac agtcccctgc ccgcctcgcg 900
ctccgaggac cccatccgtc ccgcggcact cagctacgct aactccagcc ccataaatcc 960
gttcctcaac ggcttccact cggccgccgc cgccgccgcc ggtaggggcg tctactccaa 1020
cccggacttg gtgttcgccg aggcggtctc gcacccgccc aaccccgccg tgccagtgca 1080
cccggtgccg ccgccgcacg ccctggccgc ccacccccta ccctcctcgc actcgccaca 1140
ccccctattc gcctcgcagc agcgggatcc gtccaccttc taccccctggc tcatccaccg 1200
ctaccgatat ctgggtcatc gcttccaagg gaacgacact agccccgaga gtttcctttt 1260
gcacaacgcg ctggcccgaa agcccaagcg gatccgaacc gccttctccc cgtcccagct 1320
tctaaggctg gaacacgcct ttgagaagaa tcactacgtg gtgggcgccg aaaggaagca 1380
gctggcacac agcctcagcc tcacggaaac tcaggtaaaa gtatggtttc agaaccgaag 1440
aacaaagttc aaaaggcaga agctggagga agaaggctca gattcgcaac aaaagaaaaa 1500
agggacgcac catattaacc ggtggagaat cgccaccaag caggcgagtc cggaggaaat 1560
agacgtgacc tcagatgatt aaaaacataa acctaacccc acagaaacgg acaacatgga 1620
gcaaaagaga cagggagagg tggagaagga aaaaacccta caaaacaaaa acaaaccgca 1680
tacacgttca ccgagaaagg gagagggaat cggagggagc agcggaatgc ggcgaagact 1740
ctggacagcg agggcacagg gtcccaaacc gaggccgcgc caagatggca gaggatggag 1800
gctccttcat caacaagcga ccctcgtcta aagaggcagc tgagtgagag acacagagag 1860

aaggagaaag agggagggag agagagaaag agagagaaag agagagagag agagagagag 1920

agaaagctga acgtgcactc tgacaagggg agctgtcaat caaacaccaa accggggaga 1980

caagatgatt ggcaggtatt ccgtttatca cagtccactt aaaaaatgat gatgatgata 2040

aaaaccacga cccaaccagg cacaggactt ttttgttttt tgcacttcgc tgtgtttccc 2100

ccccatcttt aaaaataatt agtaataaaa aacaaaaatt ccatatctag ccccatccca 2160

cacctgtttc aaatccttga aatgcatgta gcagttgttg ggcgaatggt gtttaaagac 2220

cgaaaatgaa ttgtaatttt cttttccttt taaagacagg ttctgtgtgc tttttatttt 2280

gatttttttt cccaagaaat gtgcagtctg taaacacttt ttgatacctt ctgatgtcaa 2340

agtgattgtg caagctaaat gaagtaggct cagcgatagt ggtcctctta cagagaaacg 2400

gggagcagga cgacgggggg gctgggggtg gcgggggagg gtgcccacaa aaagaatcag 2460

gacttgtact gggaaaaaaa cccctaaatt aattatattt cttggacatt ccctttccta 2520

acatcctgag gcttaaaacc ctgatgcaaa cttctccttt cagtggttgg agaaattggc 2580

cgagttcaac cattcactgc aatgcctatt ccaaacttta aatctatcta ttgcaaaacc 2640

tgaaggactg tagttagcgg ggatgatgtt aagtgtggcc aagcgcacgg cggcaagttt 2700

tcaagcactg agtttctatt ccaagatcat agacttacta aagagagtga caaatgcttc 2760

cttaatgtct tctataccag aatgtaaata tttttgtgtt ttgtgttaat ttgttagaat 2820

tctaacacac tatatacttc caagaagtad nahmanaaaa gatagatcct gctccaggag 2880

ccgggaagcc tcgccctggc cagctgtgct gggcacctcc cctgcctgct tcctggccca 2940

cttgcaggca aggtgagggc atgcgaatgg ctgccactgc ctgggcgggg ctccaagggc 3000

cacccctccc caccctctgt cccgcagtga ggacgggact ctactgccga gaccaggctc 3060

acgctgagag gtgggccatg acctccgaga cctcttccgg aagccactgt gccaggagca 3120

ggatgctgcg gcgacgggcc caggaagagg acagcaccgt cctgatcgat gtgagccccc 3180

ctgaggcaga gaagaggggc tcttacggga gcacagccca cgcctcggag ccaggtggac 3240

agcaagcggc cgcctgcaga gctgggagtc ctgccaagcc ccggatcgca gacttcgtcc 3300

tcgtttggga ggaggacctg aagctagaca ggcagcagga cagtgccgcc cgggacagaa 3360

cagacatgca caggacctgg cgggagactt ttctggataa tcttcgtgcg gctgggctgt 3420

gtgtagacca gcaggacgtc caggacggga acaccacagt gcactacgcc ctcctcagcg 3480

cctcctgggc tgtgctctgc tactacgccg aagacctgcg cctgaagctg cccttgcagg 3540

agttacccaa ccaggcctcc aactggtcgg ccggcctgct ggcatggctg ggcatcccca 3600

acgtcctgct ggaggttgtg ccagacgtac cccccgagta ctactcctgc cggttcagag 3660

tgaacaagct gccacgcttc ctcgggagtg acaaccagga caccttcttc acaagcacca 3720

agaggcacca aattctgttt gagatcctgg ccaagacccc gtatggccac gagaagaaaa 3780

acctgcttgg gatccaccag ctgctggcag agggtgtcct cagtgccgcc ttcccccctgc 3840

atgacggccc cttcaagacg cccccagagg gcccgcaggc tccacgcctc aaccagcgcc 3900

aagtcctttt ccagcactgg gcgcgctggg gcaagtggaa caagtaccag cccctggacc 3960

acgtgcgcag gtacttcggg gagaaggtgg ccctctactt cgcctggctc gggttttaca 4020
caggctggct cctgccagcg gcagtggtgg gcacactggt gttcctggtg ggctgcttcc 4080
tggtgttctc agacataccc acgcaggaac tgtgtggcag caaggacagc ttcgagatgt 4140
gcccactttg cctcgactgc cctttctggc tgctctccag cgcctgtgcc ctggcccagg 4200
ccggccggct gttcgaccac ggcggcaccg tgttcttcag cttgttcatg gcactgtggg 4260
ccgtgctgct gctggagtac tggaagcgga agagcgccac gctggcctac cgctgggact 4320
gctctgacta cgaggacact gaggagaggc ctcggcccca gtttgccgcc tcagccccca 4380
tgacagcccc gaaccccatc acgggtgagg acgagcccta cttccctgag aggagccgcg 4440
cgcgccgcat gctggccggc tctgtggtga tcgtggtgat ggtggccgtg gtggtcatgt 4500
gcctcgtgtc tatcatcctg taccgtgcca tcatggccat cgtggtgtcc aggtcgggca 4560
acacccttct cgcagcctgg gcctctcgca tcgccagcct cacggggtct gtagtgaacc 4620
tcgtcttcat cctcatcctc tccaagatct atgtatccct ggcccacgtc ctgacacgat 4680
gggaaatgca ccgcacccag accaagttcg aggacgcctt caccctcaag gtgttcatct 4740
tccagttcgt caacttctac tcctcacccg tctacattgc cttcttcaag ggcaggtttg 4800
tgggataccc aggcaactac cacaccttgt ttggagtccg caatgaggag tgcgcggctg 4860
gaggctgcct gatcgagctg gcacaggagc tcctggtcat catggtgggc aagcaggtca 4920
tcaacaacat gcaggaggtc ctcatcccga agctaaaggg ctggtggcag aagttccggc 4980
ttcgctccaa gaagaggaag gcgggagctt ctgcaggggc tagccagggg ccctgggagg 5040
acgactatga gcttgtgccc tgtgagggtc tgtttgacga gtacctggaa atggtgctgc 5100
agttcggctt cgtcaccatc ttcgtggccg cctgtccgct cgcgccgctc ttcgccctgc 5160
tcaacaactg ggtggagatc cgcttggacg cgcgcaagtt cgtctgcgag taccggcgcc 5220
ctgtggccga gcgcgcccag gacatcggca tctggttcca catcctggcg ggcctcacgc 5280
acctggcggt catcagcaac gccttcctcc tggccttctc gtccgacttc ctgccgcgcg 5340
cctactaccg gtggacccgc gcccacgacc tgcgcggctt cctcaacttc acgctggcgc 5400
gagccccgtc ctccttcgcc gccgcgcaca accgcacgtg caggtatcgg gctttccggg 5460
atgacgatgg acattattcc cagacctact ggaatcttct tgccatccgc ctggccttcg 5520
tcattgtgtt tgagcatgtg gttttctccg ttggccgcct cctggacctc ctggtgcctg 5580
acatcccaga gtctgtggag atcaaagtga agcgggagta ctacctggct aagcaggcac 5640
tggctgagaa tgaggttctt tttggaacga acggaacaaa ggatgagcag cccaagggct 5700
cagagctcag ctcccactgg acacccttca cggttcccaa ggccagccag ctgcagcagt 5760
gacgcctgga aggacatctg gtggtcctta ggggagtggc ccctcctgag ccctgcgagc 5820
agcgtccttt tcctcttccc tcaggcagcg gctgtgtgaa ccgctggctg ctgttgtgcc 5880
tcatctctgg gcacattgcc tgcttccccc cagcgccggc ttctctcctc agagcgcctg 5940
tcactccatc cccggcaggg agggaccgtc agctcacaag gccctctttg tttcctgctc 6000

ccagacataa gcccaagggg cccctgcacc caagggaccc tgtccctcgg tggcctcccc 6060

aggcccctgg acacgacagt tctcctcagg caggtgggct ttgtggtcct cgccgcccct 6120

ggccacatcg ccctctcctc ttacacctgg tgaccttcga atgt 6164

<210> 98
<211> 551
<212> DNA
<213> Homo sapiens

<400> 98

accccatccg ctggctctca cccctcggag acgctcgccc gacagcatag tacttgccgc 60

ccagccacgc ccgcgcgcca gccaccatgc taggtaacaa gcgactgggg ctgtccggac 120

tgaccctcgc cctgtccctg ctcgtgtgcc tgggtgcgct ggccgaggcg taccccctcca 180

agccggacaa cccgggcgag gacgcaccag cggaggacat ggccagatac tactcggcgc 240

tgcgacacta catcaacctc atcaccaggc agagatatgg aaaacgatcc agcccagaga 300

cactgatttc agacctcttg atgagagaaa gcacagaaaa tgttcccaga actcggcttg 360

aagaccctgc aatgtggtga tgggaaatga gacttgctct ctggcctttt cctattttca 420

gcccatattt catcgtgtaa aacgagaatc cacccatcct accaatgcat gcagccactg 480

tgctgaattc tgcaatgttt tcctttgtca tcattgtata tatgtgtgtt taaataaagt 540

atcatgcatt c 551

<210> 99
<211> 1607
<212> DNA
<213> Homo sapiens

<400> 99

aatgactcct ttcggtaagt gcagtggaag ctgtacactg cccaggcaaa gcgtccgggc 60
agcgtaggcg ggcgactcag atcccagcca gtggacttag cccctgtttg ctcctccgat 120
aactggggtg accttggtta atattcacca gcagcctccc ccgttgcccc tctggatcca 180
ctgcttaaat acggacgagg acagggccct gtctcctcag cttcaggcac caccactgac 240
ctgggacagt gaatcgacaa tgccgtcttc tgtctcgtgg ggcatcctcc tgctggcagg 300
cctgtgctgc ctggtccctg tctccctggc tgaggatccc cagggagatg ctgcccagaa 360
gacagataca tcccaccatg atcaggatca cccaaccttc aacaagatca cccccaacct 420
ggctgagttc gccttcagcc tataccgcca gctggcacac cagtccaaca gcaccaatat 480
cttcttctcc ccagtgagca tcgctacagc ctttgcaatg ctctccctgg ggaccaaggc 540
tgacactcac gatgaaatcc tggagggcct gaatttcaac ctcacggaga ttccggaggc 600
tcagatccat gaaggcttcc aggaactcct ccgtaccctc aaccagccag acagccagct 660
ccagctgacc accggcaatg gcctgttcct cagcgagggc ctgaagctag tggataagtt 720
tttggaggat gttaaaaagt tgtaccactc agaagccttc actgtcaact tcggggacac 780
cgaagaggcc aagaaacaga tcaacgatta cgtggagaag ggtactcaag ggaaaattgt 840
ggatttggtc aaggagcttg acagagacac agtttttgct ctggtgaatt acatcttctt 900

taaaggcaaa tgggagagac cctttgaagt caaggacacc gaggaagagg acttccacgt 960
ggaccaggtg accaccgtga aggtgcctat gatgaagcgt ttaggcatgt ttaacatcca 1020
gcactgtaag aagctgtcca gctgggtgct gctgatgaaa tacctgggca atgccaccgc 1080
catcttcttc ctgcctgatg aggggaaact acagcacctg gaaaatgaac tcacccacga 1140
tatcatcacc aagttcctgg aaaatgaaga cagaaggtct gccagcttac atttacccaa 1200
actgtccatt actggaacct atgatctgaa gagcgtcctg ggtcaactgg gcatcactaa 1260
ggtcttcagc aatggggctg acctctccgg ggtcacagag gaggcacccc tgaagctctc 1320
caaggccgtg cataaggctg tgctgaccat cgacgagaaa gggactgaag ctgctggggc 1380
catgttttta gaggccatac ccatgtctat ccccccccgag gtcaagttca acaaaccctt 1440
tgtcttctta atgattgaac aaaataccaa gtctcccctc ttcatgggaa aagtggtgaa 1500
tcccacccaa aaataactgc ctctcgctcc tcaacccctc ccctccatcc ctggccccct 1560
ccctggatga cattaaagaa gggttgagct ggtccctgcc tgcaaaa 1607

<210> 100
<211> 1753
<212> DNA
<213> Homo sapiens

<400> 100

cagccccgcc cctacctgtg gaagcccagc cgcccgctcc cgcggataaa aggcgcggag 60
tgtccccgag gtcagcgagt gcgcgctcct cctcgcccgc cgctaggtcc atcccggccc 120
agccaccatg tccatccact tcagctcccc ggtattcacc tcgcgctcag ccgccttctc 180
gggccgcggc gcccaggtgc gcctgagctc cgctcgcccc ggcggccttg gcagcagcag 240
cctctacggc ctcggcgcct cacggccgcg cgtggccgtg cgctctgcct atgggggccc 300
ggtgggcgcc ggcatccgcg aggtcaccat taaccagagc ctgctggccc cgctgcggct 360
ggacgccgac ccctccctcc agcgggtgcg ccaggaggag agcgagcaga tcaagaccct 420
caacaacaag tttgcctcct tcatcgacaa ggtgcggttt ctggagcagc agaacaagct 480
gctggagacc aagtggacgc tgctgcagga gcagaagtcg gccaagagca gccgcctccc 540
agacatcttt gaggcccaga ttgctggcct tcggggtcag cttgaggcac tgcaggtgga 600
tgggggccgc ctggaggcgg agctgcggag catgcaggat gtggtggagg acttcaagaa 660
taagtacgaa gatgaaatta accaccgcac agctgctgag aatgagtttg tggtgctgaa 720
gaaggatgtg gatgctgcct acatgagcaa ggtggagctg gaggccaagg tggatgccct 780
gaatgatgag atcaacttcc tcaggaccct caatgagacg gagttgacag agctgcagtc 840
ccagatctcc gacacatctg tggtgctgtc catggacaac agtcgctccc tggacctgga 900
cggcatcatc gctgaggtca aggcgcagta tgaggagatg gccaaatgca gccgggctga 960
ggctgaagcc tggtaccaga ccaagtttga gaccctccag gcccaggctg ggaagcatgg 1020
ggacgacctc cggaataccc ggaatgagat ttcagagatg aaccgggcca tccagaggct 1080
gcaggctgag atcgacaaca tcaagaacca gcgtgccaag ttggaggccg ccattgccga 1140

ggctgaggag cgtggggagc tggcgctcaa ggatgctcgt gccaagcagg aggagctgga 1200
agccgccctg cagcggggca agcaggatat ggcacggcag ctgcgtgagt accaggaact 1260
catgagcgtg aagctggccc tggacatcga gatcgccacc taccgcaagc tgctggaggg 1320
cgaggagagc cggttggctg gagatggagt gggagccgtg aatatctctg tgatgaattc 1380
cactggtggc agtagcagtg gcggtggcat tgggctgacc ctcgggggaa ccatgggcag 1440
caatgccctg agcttctcca gcagtgcggg tcctgggctc ctgaaggctt attccatccg 1500
gaccgcatcc gccagtcgca ggagtgcccg cgactgagcc gcctcccacc actccactcc 1560
tccagccacc acccacaatc acaagaagat tcccacccct gcctcccatg cctggtccca 1620
agacagtgag acagtctgga aagtgatgtc agaatagctt ccaataaagc agcctcattc 1680
tgaggcctga gtgatccacg tgaaaaaaaa aaaaaaaaaa aaaaaaaaaa aaaaaaaaaa 1740
aaaaaaaaaa aaa 1753

<210> 101
<211> 2276
<212> DNA
<213> Homo sapiens

<400> 101

aagcccagca gccccggggc ggatggctcc ggccgcctgg ctccgcagcg cggccgcgcg 60
cgccctcctg cccccgatgc tgctgctgct gctccagccg ccgccgctgc tggcccgggc 120
tctgccgccg gacgcccacc acctccatgc cgagaggagg gggccacagc cctggcatgc 180
agccctgccc agtagcccgg cacctgcccc tgccacgcag gaagcccccc ggcctgccag 240
cagcctcagg cctccccgct gtggcgtgcc cgacccatct gatgggctga gtgcccgcaa 300
ccgacagaag aggttcgtgc tttctggcgg gcgctgggag aagacggacc tcacctacag 360
gatccttcgg ttcccatggc agttggtgca ggagcaggtg cggcagacga tggcagaggc 420
cctaaaggta tggagcgatg tgacgccact cacctttact gaggtgcacg agggccgtgc 480
tgacatcatg atcgacttcg ccaggtactg gcatggggac gacctgccgt ttgatgggcc 540
tgggggcatc ctggcccatg ccttcttccc caagactcac cgagaagggg atgtccactt 600
cgactatgat gagacctgga ctatcgggga tgaccagggc acagacctgc tgcaggtggc 660
agcccatgaa tttggccacg tgctggggct gcagcacaca acagcagcca aggccctgat 720
gtccgccttc tacacctttc gctacccact gagtctcagc ccagatgact gcaggggcgt 780
tcaacaccta tatggccagc cctggcccac tgtcacctcc aggaccccag ccctgggccc 840
ccaggctggg atagacacca atgagattgc accgctggag ccagacgccc cgccagatgc 900
ctgtgaggcc tcctttgacg cggtctccac catccgaggc gagctctttt tcttcaaagc 960
gggctttgtg tggcgcctcc gtgggggcca gctgcagccc ggctacccag cattggcctc 1020
tcgccactgg cagggactgc ccagccctgt ggacgctgcc ttcgaggatg cccagggcca 1080
catttggttc ttccaaggtg ctcagtactg ggtgtacgac ggtgaaaagc cagtcctggg 1140
ccccgcaccc ctcaccgagc tgggcctggt gaggttcccg gtccatgctg ccttggtctg 1200

gggtcccgag aagaacaaga tctacttctt ccgaggcagg gactactggc gtttccaccc 1260
cagcacccgg cgtgtagaca gtcccgtgcc ccgcagggcc actgactgga gaggggtgcc 1320
ctctgagatc gacgctgcct tccaggatgc tgatggctat gcctacttcc tgcgcggccg 1380
cctctactgg aagtttgacc ctgtgaaggt gaaggctctg gaaggcttcc cccgtctcgt 1440
gggtcctgac ttctttggct gtgccgagcc tgccaacact ttcctctgac catggcttgg 1500
atgccctcag ggtgctgac ccctgccagg ccacgaatat caggctagag acccatggcc 1560
atctttgtgg ctgtgggcac caggcatggg actgagccca tgtctcctca gggggatggg 1620
gtggggtaca accaccatga caactgccgg gagggccacg caggtcgtgg tcacctgcca 1680
gcgactgtct cagactgggc agggaggctt tggcatgact taagaggaag ggcagtcttg 1740
ggcccgctat gcaggtcctg gcaaacctgg ctgccctgtc tccatccctg tccctcaggg 1800
tagcaccatg gcaggactgg gggaactgga gtgtccttgc tgtatccctg ttgtgaggtt 1860
ccttccaggg gctggcactg aagcaagggt gctggggccc catggccttc agccctggct 1920
gagcaactgg gctgtagggc agggccactt cctgaggtca ggtcttggta ggtgcctgca 1980
tctgtctgcc ttctggctga caatcctgga aatctgttct ccagaatcca ggccaaaaag 2040
ttcacagtca aatggggagg ggtattcttc atgcaggaga ccccaggccc tggaggctgc 2100
aacatacctc aatcctgtcc caggccggat cctcctgaag ccctttcgc agcactgcta 2160
tcctccaaag ccattgtaaa tgtgtgtaca gtgtgtataa accttcttct tcttttttt 2220
tttttaaact gaggattgtc attaaacaca gttgttttct aaaaaaaaaa aaaaaa 2276

<210> 102
<211> 7381
<212> DNA
<213> Homo sapiens

<400> 102

```
tacagcccca aggtcgctcc ctctggggcc ctttcttccc cattcttccc agcagcccaa     60
agctctggtg ggacaggggc agcccctggg gagggaggag aggacccagg aacccggcta    120
ggagggtggc ccacccattt ccagtgtgac ctgttcccat tcccccatgt ctcctcccat    180
ccctcccgcc actcagctca ggctgatgag aagcagagca acgggtgtat cggtgttttc    240
tttcctggtg gggtagtggg gtggggctga ggagagaaaa gggtgattag cgtggggccc    300
cgccctcttt tgtcctcttc ccaggttccc tggccccttc ggagaaacgc acttggttcg    360
ggccagccgc ctgaggggac gggctcacgt ctgctcctca cactgcagct gctgggccgt    420
ggagcttccc cagggagcca gggggacttt tgccgcagcc atgaaggggg cacgctggag    480
gagggtcccc tgggtgtccc tgagctgcct gtgtctctgc ctccttccgc atgtggtccc    540
aggaaccaca gaggacacat taataactgg aagtaaaact cctgccccag tcacctcaac    600
aggctcaaca acagcgacac tagagggaca atcaactgca gcttcttcaa ggacctctaa    660
tcaggacata tcagcttcat ctcagaacca ccagactaag agcacggaga ccaccagcaa    720
agctcaaacc gacaccctca cgcagatgat gacatcaact ctttttcttc ccccaagtgt    780
```

acacaatgtg atggagactg ttacgcagga gacagctcct ccagatgaaa tgaccacatc 840
atttccctcc agtgtcacca acacactcat gatgacatca aagactataa caatgacaac 900
ctccacagac tccactcttg gaaacacaga agagacatca acagcaggaa ctgaaagttc 960
taccccagtg acctcagcag tctcaataac agctggacag gaaggacaat cacgaacaac 1020
ttcctggagg acctctatcc aagacacatc agcttcttct cagaaccact ggactcggag 1080
cacgcagacc accagggaat ctcaaaccag caccctaaca cacagaacca cttcaactcc 1140
ttctttctct ccaagtgtac acaatgtgac agggactgtt tctcagaaga catctccttc 1200
aggtgaaaca gctacctcat ccctctgtag tgtcacaaac acatccatga tgacatcaga 1260
gaagataaca gtgacaacct ccacaggctc cactcttgga aacccagggg agacatcatc 1320
agtacctgtt actggaagtc ttatgccagt cacctcagca gccttagtaa cagttgatcc 1380
agaaggacaa tcaccagcaa ctttctcaag gacttctact caggacacaa cagctttttc 1440
taagaaccac cagactcaga gcgtggagac caccagagta tctcaaatca acaccctcaa 1500
caccctcaca ccggttacaa catcaactgt tttatcctca ccaagtggat tcaacccaag 1560
tggaacagtt tctcaggaga cattcccttc tggtgaaaca accatctcat ccccttccag 1620
tgtcagcaat acattcctgg taacatcaaa ggtgttcaga atgccaatct ccagagactc 1680
tactcttgga aacacagagg agacatcact atctgtaagt ggaaccattt ctgcaatcac 1740
ttccaaagtt tcaaccatat ggtggtcaga cactctgtca acagcactct cccccagttc 1800
tctacctcca aaaatatcca cagctttcca cacccagcag agtgaaggtg cagagaccac 1860
aggacggcct catgagagga gctcattctc tccaggtgtg tctcaagaaa tatttactct 1920
acatgaaaca acaacatggc cttcctcatt ctccagcaaa ggccacacaa cttggtcaca 1980
aacagaactg ccctcaacat caacaggtgc tgccactagg cttgtcacag gaaatccatc 2040
tacaagggca gctggcacta ttccaagggt cccctctaag gtctcagcaa taggggaacc 2100
aggagagccc accacatact cctcccacag cacaactctc ccaaaaacaa caggggcagg 2160
cgcccagaca caatggacac aagaaacggg gaccactgga gaggctcttc tcagcagccc 2220
aagctatagt gtgattcaga tgataaaaac ggccacatcc ccatcttctt cacctatgct 2280
ggatagacac acatcacaac aaattacaac ggcaccatca acaaatcatt caacaataca 2340
ttccacaagc acctctcctc aggaatcacc agctgtttcc caaaggggtc acactcgagc 2400
cccgcagacc acacaagaat cacaaaccac gaggtccgtc tcccccatga ctgacaccaa 2460
gacagtcacc accccaggtt cttccttcac agccagtggg cactcgccct cagaaattgt 2520
tcctcaggac gcacccacca taagtgcagc aacaaccttt gccccagctc ccaccgggaa 2580
tggtcacaca acccaggccc cgaccacagc actgcaggca gcacccagca gccatgatgc 2640
caccctgggg ccctcaggag gcacgtcact ttccaaaaca ggtgccctta ctctggccaa 2700
ctctgtagtg tcaacaccag ggggcccaga aggacaatgg acatcagcct ctgccagcac 2760
ctcacctgac acagcagcag ccatgaccca tacccaccag gctgagagca cagaggcctc 2820

tggacaaaca cagaccagcg aaccggcctc ctcagggtca cgaaccacct cagcgggcac 2880
agctacccct tcctcatccg gggcgagtgg cacaacacct tcaggaagcg aaggaatatc 2940
cacctcagga gagacgacaa ggttttcatc aaacccctcc agggacagtc acacaaccca 3000
gtcaacaacc gaattgctgt ccgcctcagc cagtcatggt gccatcccag taagcacagg 3060
aatggcgtct tcgatcgtcc ccggcacctt tcatcccacc ctctctgagg cctccactgc 3120
agggagaccg acaggacagt caagcccaac ttctcccagt gcctctcctc aggagacagc 3180
cgccatttcc cggatggccc agactcagag gacaggaacc agcagagggt ctgacactat 3240
cagcctggcg tcccaggcaa ccgacacctt ctcaacagtc ccacccacac ctccatcgat 3300
cacatccagt gggcttacat ctccacaaac ccagacccac actctgtcac cttcagggtc 3360
tggtaaaacc ttcaccacgg ccctcatcag caacgccacc cctcttcctg tcaccagcac 3420
ctcctcagcc tccacaggtc acgccacccc tcttgctgtc agcagtgcta cctcagcttc 3480
cacagtatcc tcggactccc ctctgaagat ggaaacatca ggaatgacaa caccgtcact 3540
gaagacagac ggtgggagac gcacagccac atcaccaccc cccacaacct cccagaccat 3600
catttccacc attcccagca ctgccatgca cacccgctcc acagctgccc ccatccccat 3660
cctgcctgag agaggagttt ccctcttccc ctatggggca ggcgccgggg acctggagtt 3720
cgtcaggagg accgtggact tcacctcccc actcttcaag ccggcgactg gcttccccct 3780
tggctcctct ctccgtgatt ccctctactt cacagacaat ggccagatca tcttcccaga 3840
gtcagactac cagattttct cctaccccaa cccactccca acaggcttca caggccggga 3900
ccctgtggcc ctggtggctc cgttctggga cgatgctgac ttctccactg gtcgggggac 3960
cacattttat caggaatacg agacgttcta tggtgaacac agcctgctag tccagcaggc 4020
cgagtcttgg attagaaaga tgacaaacaa cgggggctac aaggccaggt gggccctaaa 4080
ggtcacgtgg gtcaatgccc acgcctatcc tgcccagtgg accctcggga gcaacaccta 4140
ccaagccatc ctctccacgg acgggagcag gtcctatgcc ctgtttctct accagagcgg 4200
tgggatgcag tgggacgtgg cccagcgctc aggcaacccg gtgctcatgg gcttctctag 4260
tggagatggc tatttcgaaa acagcccact gatgtcccag ccagtgtggg agaggtatcg 4320
ccctgataga ttcctgaatt ccaactcagg cctccaaggg ctgcagttct acaggctaca 4380
ccgggaagaa aggcccaact accgtctcga gtgcctgcag tggctgaaga gccagcctcg 4440
gtggcccagc tggggctgga accaggtctc ctgcccttgt tcctggcagc agggacgacg 4500
ggacttacga ttccaacccg tcagcatagg tcgctggggc ctcggcagta ggcagctgtg 4560
cagcttcacc tcttggcgag gaggcgtgtg ctgcagctac gggccctggg gagagtttcg 4620
tgaaggctgg cacgtgcagc gtccttggca gttggcccag gaactggagc cacagagctg 4680
gtgctgccgc tggaatgaca agccctacct ctgtgccctg taccagcaga ggcggcccca 4740
cgtgggctgt gctacataca ggcccccaca gcccgcctgg atgttcgggg acccccacat 4800
caccaccttg gatggtgtca gttacacctt caatgggctg gggggacttcc tgctggtcgg 4860
ggcccaagac gggaactcct ccttcctgct tcagggccgc accgcccaga ctggctcagc 4920

ccaggccacc aacttcatcg cctttgcggc tcagtaccgc tccagcagcc tgggccccgt 4980
cacggtccaa tggctccttg agcctcacga cgcaatccgt gtcctgctgg ataaccagac 5040
tgtgacattt cagcctgacc atgaagacgg cggaggccag gagacgttca acgccaccgg 5100
agtcctcctg agccgcaacg gctctgaggt ctcggccagc ttcgacggct gggccaccgt 5160
ctcggtgatc gcgctctcca acatcctcca cgcctccgcc agcctcccgc ccgagtacca 5220
gaaccgcacg gaggggctcc tgggggtctg gaataacaat ccagaggacg acttcaggat 5280
gcccaatggc tccaccattc cccagggag ccctgaggag atgcttttcc actttggaat 5340
gacctggcag atcaacggga caggcctcct tggcaagagg aatgaccagc tgccttccaa 5400
cttcacccct gttttctact cacaactgca aaaaaacagc tcctgggctg aacatttgat 5460
ctccaactgt gacggagata gctcatgcat ctatgacacc ctggccctgc gcaacgcaag 5520
catcggactt cacacgaggg aagtcagtaa aaactacgag caggcgaacg ccaccctcaa 5580
tcagtacccg ccctccatca atggtggtcg tgtgattgaa gcctacaagg ggcagaccac 5640
gctgattcag tacaccagca atgctgagga tgccaacttc acgctcagag acagctgcac 5700
cgacttggag ctctttgaga atgggacgtt gctgtggaca cccaagtcgc tggagccatt 5760
cactctggag attctagcaa gaagtgccaa gattggcttg gcatctgcac tccagcccag 5820
gactgtggtc tgccattgca atgcagagag ccagtgtttg tacaatcaga ccagcagggt 5880
gggcaactcc tccctggagg tggctggctg caagtgtgac gggggcacct tcggccgcta 5940
ctgcgagggc tccgaggatg cctgtgagga gccgtgcttc ccgagtgtcc actgcgttcc 6000
tgggaagggc tgcgaggcct gccctccaaa cctgactggg gatgggcggc actgtgcggc 6060
tctggggagc tctttcctgt gtcagaacca gtcctgccct gtgaattact gctacaatca 6120
aggccactgc tacatctccc agactctggg ctgtcagccc atgtgcacct gcccccccagc 6180
cttcactgac agccgctgct tcctggctgg gaacaacttc agtccaactg tcaacctaga 6240
acttccctta agagtcatcc agctcttgct cagtgaagag gaaaatgcct ccatggcaga 6300
ggtcaacgcc tcggtggcat acagactggg gaccctggac atgcgggcct ttctccgcaa 6360
cagccaagtg gaacgaatcg attctgcagc accggcctcg ggaagcccca tccaacactg 6420
gatggtcatc tcggagttcc agtaccgccc tcggggcccg gtcattgact tcctgaacaa 6480
ccagctgctg gccgcggtgg tggaggcgtt cttataccac gttccacgga ggagtgagga 6540
gcccaggaac gacgtggtct tccagcccat ctccggggaa gacgtgcgcg atgtgacagc 6600
cctgaacgtg agcacgctga aggcttactt cagatgcgat ggctacaagg gctacgacct 6660
ggtctacagc cccccagagcg gcttcacctg cgtgtccccg tgcagtaggg gctactgtga 6720
ccatggaggc cagtgccagc acctgcccag tgggccccgc tgcagctgtg tgtccttctc 6780
catctacacg gcctggggcg agcactgtga gcacctgagc atgaaactcg acgcgttctt 6840
cggcatcttc tttggggccc tgggcggcct cttgctgctg ggggtcggga cgttcgtggt 6900
cctgcgcttc tggggttgct ccggggccag gttctcctat ttcctgaact cagctgaggc 6960

cttgccttga aggggcagct gtggcctagg ctacctcaag actcacctca tccttaccgc 7020
acatttaagg cgccattgct tttgggagac tggaaaaggg aaggtgactg aaggctgtca 7080
ggattcttca aggagaatga atactgggaa tcaagacaag actatacctt atccataggc 7140
gcaggtgcac agggggaggc cataaagatc aaacatgcat ggatgggtcc tcacgcagac 7200
acacccacag aaggacacta gcctgtgcac gcgcgcgtgc acacacacac acacacacac 7260
gagttcataa tgtggtgatg gccctaagtt aagcaaaatg cttctgcaca caaaactctc 7320
tggtttactt caaattaact ctatttaaat aaagtctctc tgactttttg tgtctccaaa 7380
a 7381

<210> 103
<211> 2323
<212> DNA
<213> Homo sapiens

<400> 103

```
agctatgatc gcaacacctt ggtggccatc gtggtgggtg tggggcgcct catcactggc    60
atggaccgag gcctcatggg catgtgtgtc aacgagcggc gacgcctcat tgtgcctccc   120
cacctgggct atgggagcat cggcctggcg gggctcattc caccggatgc caccctctac   180
ttcgatgtgg ttctgctgga tgtgtggaac aaggaagaca ccgtgcaggt gagcacattg   240
ctgcgcccgc cccactgccc ccgcatggtc caggacggcg actttgtccg ctaccactac   300
aatggcaccc tgctggacgg cacctccttc gacaccagct acagtaaggg cggcacttat   360
gacacctacg tcggctctgg ttggctgatc aagggcatgg accaggggct gctgggcatg   420
tgtcctggag agagaaggaa gattatcatc cctccattcc tggcctatgg cgagaaaggc   480
tatgggacag tgatcccccc acaggcctcg ctggtctttc acgtcctcct gattgacgtg   540
cacaacccga aggacgctgt ccagctagag acgctggagc tcccccccgg ctgtgtccgc   600
agagccgggg ccggggactt catgcgctac cactacaatg gctccttgat ggacggcacc   660
ctcttcgatt ccagctactc ccgcaaccac acctacaata cctatatcgg gcagggttac   720
atcatccccg ggatggacca ggggctgcag ggtgcctgca tgggggaacg ccggagaatt   780
accatccccc cgcacctcgc ctatggggag aatggaactg gagacaagat ccctggctct   840
gccgtgctaa tcttcaacgt ccatgtcatt gacttccaca accctgcgga tgtggtggaa   900
atcaggacac tgtcccggcc atccgagacc tgcaatgaga ccaccaagct tggggacttt   960
gttcgatacc attacaactg ttctttgctg gacggcaccc agctgttcac ctcgcatgac  1020
tacggggccc cccaggaggc gactctcggg gccaacaagg tgatcgaagg cctggacacg  1080
ggcctgcagg gcatgtgtgt gggagagagg cggcagctca tcgtgccccc gcacctggcc  1140
cacggggaga gtggagcccg gggagtccca ggcagtgctg tgctgctgtt tgaggtggag  1200
ctggtgtccc gggaggatgg gctgcccaca ggctacctgt ttgtgtggca caaggaccct  1260
cctgccaacc tgtttgaaga catggacctc aacaaggatg gcgaggtccc tccggaggag  1320
ttctccacct tcatcaaggc tcaagtgagt gagggcaaag gacgcctcat gcctgggcag  1380
```

gaccctgaga aaaccatagg agacatgttc cagaaccagg accgcaacca ggacggcaag 1440
atcacagtcg acgagctcaa gctgaagtca gatgaggacg aggagcgggt ccacgaggag 1500
ctctgagggg cagggagcct ggccaggcct gagacacaga ggcccactgc gagggggaca 1560
gtggcggtgg gactgacctg ctgacagtca ccctccctct gctgggatga ggtccaggag 1620
ccaactaaaa caatggcaga ggagacatct ctggtgttcc caccacccta gatgaaaatc 1680
cacagcacag acctctaccg tgtttctctt ccatccctaa accacttcct taaaatgttt 1740
ggatttgcaa agccaatttg gggcctgtgg agcctggggt tggatagggc catggctggt 1800
cccccaccat acctcccctc cacatcactg acacagctga gcttgttatc catctcccca 1860
aactttctct ttctttgtac ttcttgtcat ccccactccc agcccctatt cctctatgtg 1920
acagctggct aggacccctc tgccttcctt cccaatcctg actggctcct agggaagggg 1980
aaggctcctg gagggcagcc ctacctctcc catgcccttt gccctcctcc ctcgcctcca 2040
gtggaggctg agctgaccct gggctgctgg aggccagact gggctgtagt tagcttttca 2100
tccctaaaga aggctttccc taaggaacca tagaagagag gaagaaaaca aagggcatgt 2160
gtgagggaag ctgcttgggt gggtgttagg gctatgaaat cttggatttg gggctgaggg 2220
gtgggaggga gggcagagct ctgcacactc aaaggctaaa ctggtgtcag tcctttttc 2280
ctttgttcca aataaaagat taaaccaaaa aaaaaaaaaa aaa 2323

<210> 104
<211> 741
<212> DNA
<213> Homo sapiens

<400> 104

tcacgtgacc cgggcgcgct gcggccgccc gcgcggaccc ggcgagaggc ggcggcggga 60
gcggcggtga tggacgggtc cggggagcag cccagaggcg gggggcccac cagctctgag 120
cagatcatga agacaggggc ccttttgctt caggggatga ttgccgccgt ggacacagac 180
tccccccgag aggtcttttt ccgagtggca gctgacatgt tttctgacgg caacttcaac 240
tggggccggg ttgtcgccct tttctacttt gccagcaaac tggtgctcaa ggccctgtgc 300
accaaggtgc cggaactgat cagaaccatc atgggctgga cattggactt cctccgggag 360
cggctgttgg gctggatcca agaccagggt ggttgggacg gcctcctctc ctactttggg 420
acgcccacgt ggcagaccgt gaccatcttt gtggcgggag tgctcaccgc ctcgctcacc 480
atctggaaga agatgggctg aggcccccag ctgccttgga ctgtgttttt cctccataaa 540
ttatggcatt tttctgggag gggtggggat tgggggacat gggcattttt cttacttttg 600
taattattgg ggggtgtggg gaagagtggt cttgaggggg taataaacct ccttcgggac 660
acaaaaaaaa aaaaaaaaaa aaaaaaaaaa aaaaaaaaaa aaaaaaaaaa aaaaaaaaaa 720
aaaaaaaaaa aaaaaaaaaa a 741

<210> 105
<211> 2373
<212> DNA
<213> Homo sapiens

<400> 105

cccaggccca ccccacccag cacccctggc gcagggactg ctggaacctg gctgtgcgcg 60
ctgtcgcttt aagacagact ctgccggcgc cgtccggagc cttagaaacc ggccccggat 120
cgcgagccgg agccggagcc ggagccgggg ccggccgggc tgctgaggcc cgagcggcag 180
gagcgcagcg cggagcgctg agccaggcgc ccagtcgcga gaagctgccg ccgcctctgc 240
ccgcccggcg ccgcagcccc gggcggtcca tggggcgggc acggcgtcgc tgcaggcgcc 300
ggcagccctg gagggcagcc gcttaggcgc tgcgctcttg tccccgcagg tcgcagccag 360
ggcggcgggg cgcgcccagc cccggcccct ggagcgcccg ccgcggtccc cacctccatg 420
gacgccttca aggggggcat gagcctggag cggctgccgg aggggctccg gccgccgccg 480
ccgccacccc atgacatggg gcccgccttc cacctggccc ggcccgccga cccccgcgag 540
ccgctcgaga actccgccag cgagtcgtct gacacggagc tgccagagaa ggagcgcggc 600
ggggaaccca aggggcccga ggacagtggt gcgggaggca cgggctgcgg cggcgcagac 660
gacccagcca agaagaagaa gcagcggcgg caacgtacgc acttcacaag ccagcagttg 720
caagagctag aggccacgtt ccagaggaac cgctaccccg acatgagcat gagggaggag 780
atcgccgtgt ggaccaacct caccgagccg cgcgtgcggg tctggttcaa gaaccggcga 840
gccaagtggc gtaagcgcga gcgtaaccag cagctggacc tgtgcaaggg tggctacgtg 900
ccgcagttca gcggcctagt gcagccctac gaggacgtgt acgccgccgg ctactcctac 960
aacaactggg ccgccaagag cctggcgcca gcgccgctct ccaccaagag cttcaccttc 1020
ttcaactcca tgagcccgct gtcgtcgcag tccatgttct cagcacccag ctccatctcc 1080
tccatgacca tgccgtccag catgggccca ggcgccgtgc ctggcatgcc caactcgggc 1140
ctcaacaaca tcaacaacct caccggctcc tcgctcaact cggccatgtc gccgggcgct 1200
tgcccgtacg gcactcccgc ctcgccctac agcgtctacc gggacacgtg caactcgagc 1260
ctagccagcc tgcggctcaa gtccaaacag cactcgtcgt ttggctacgg cgccctgcag 1320
ggcccggcct cgggcctcaa cgcgtgccag tacaacagct gaccgccccg ccgcaccacg 1380
cgggccggcg gccggagcgg ggaagggcgc gggcgcggag gacgcacgcg gggccccggc 1440
tcgcaagccc cagctcaccg cgccgcggac ctcacacctg cgcagccccc tcctcccact 1500
tcccactccg ggttggtttt gtgtttgctt ttccggaccc cactctgccc tccaaaaaga 1560
caaaaaaaaa aaaaaaaaaa aaagcaaaaa gacgtcggag aaaagtgccg cgaaaaaatg 1620
gatgagttgc aatttctctc gggatggcgc gggtggtgtg tgtgtgttcc cacgggcccc 1680
ggaggcccac tccgcggagg gcacgcggcg cggtaggcga gcgccgaggc ccagcggccg 1740
ggggaggacg acctcgtatc ccgcgtcccc gccgcgctgg atccggactg agtggccggg 1800
cctgcggact ggatgtgcgg ggcctggact tgcctaggat ttcccgaccc cgtacaaacc 1860
aagttgccct ctccgagcta ggcccggccg agagcgcctt agctcgagtc ggatccgtgt 1920
tggggcgggc gttgggtttg gggggacggt gccccccagcc caggatcggg cactcagtgg 1980

agccgcacac ggccccggcg cgcctggtag agcctcgctg gccccgcgcc ccggagccct 2040
atattaaggc cacggagcga cagcgggcag tgcgggcctg gcgggaggtg ggggaggtcc 2100
atctcagaac accccagcct tgagcttagc tgcaggccca ggccctctgc tctgctcccg 2160
ggctaggagg tggccctctg tctgggcgaa cagccccctc ctcaccgccc gccgtgcaag 2220
agtcgagccg gcagagcaag gggcgcggcc ccagggccct gcgcccactt tgcacacccg 2280
ctctccggcc cgcgcccctg tttacagcgt ccctgtgtat gttggactga ctgtaataaa 2340
tctgtctata tcgactaaaa aaaaaaaaaa aaa 2373

<210> 106
<211> 1314
<212> DNA
<213> Homo sapiens

<400> 106

aattcccggc tcggggacct ccacgcaccg cggctagcgc cgacaaccag ctagcgtgca 60
aggcgccgcg gctcagcgcg taccggcggg cttcgaaacc gcagtcctcc ggcgaccccg 120
aactccgctc cggagcctca gccccctgga aagtgatccc ggcatccgag agccaagatg 180
ccggcccact tgctgcagga cgatatctct agctcctata ccaccaccac caccattaca 240
gcgcctccct ccagggtcct gcagaatgga ggagataagt tggagacgat gcccctctac 300
ttggaagacg acattcgccc tgatataaaa gatgatatat atgaccccac ctacaaggat 360
aaggaaggcc caagccccaa ggttgaatat gtctggagaa acatcatcct tatgtctctg 420
ctacacttgg gagccctgta tgggatcact ttgattccta cctgcaagtt ctacacctgg 480
ctttgggggg tattctacta ttttgtcagt gccctgggca taacagcagg agctcatcgt 540
ctgtggagcc accgctctta caaagctcgg ctgcccctac ggctctttct gatcattgcc 600
aacacaatgg cattccagaa tgatgtctat gaatgggctc gtgaccaccg tgcccaccac 660
aagttttcag aaacacatgc tgatcctcat aattcccgac gtggcttttt cttctctcac 720
gtgggttggc tgcttgtgcg caaacaccca gctgtcaaag agaaggggag tacgctagac 780
ttgtctgacc tagaagctga gaaactggtg atgttccaga ggaggtacta caaacctggc 840
ttgctgatga tgtgcttcat cctgcccacg cttgtgccct ggtatttctg ggtgaaact 900
tttcaaaaca gtgtgttcgt tgccactttc ttgcgatatg ctgtggtgct taatgccacc 960
tggctggtga acagtgctgc ccacctcttc ggatatcgtc cttatgacaa gaacattagc 1020
ccccgggaga atatcctggt ttcacttgga gctgtgggtg agggcttcca caactaccac 1080
cactcctttc cctatgacta ctctgccagt gagtaccgct ggcacatcaa cttcaccaca 1140
ttcttcattg attgcatggc cgccctcggt ctggcctatg accggaagaa agtctccaag 1200
gccgccatct tggccaggat taaaagaacc ggagatggaa actaaaaaaa aaaaaaaaaa 1260
aaaaaaaaaa aaaaaaaaaa aaaaaaaaaa aaaaataaaa aaaaaaaaaa aaaa 1314

<210> 107
<211> 476
<212> DNA
<213> Homo sapiens

<400> 107

```
gaaaaaccag ccactgcttt acaggacagg gggttgaagc tgagccccgc ctcacaccca    60
cccccatgca ctcaaagatt ggattttaca gctacttgca attcaaaatt cagaagaata   120
aaaaatggga acatacagaa ctctaaaaga tagacatcag aaattgttaa gttaagcttt   180
ttcaaaaaat cagcaattcc ccagcgtagt caagggtgga cactgcacgc tctggcatga   240
tgggatggcg accgggcaag ctttcttcct cgagatgctc tgctgcttga gagctattgc   300
tttgttaaga tataaaaagg ggtttctttt tgtctttctg taaggtggac ttccagattt   360
tgattgaaag tcctagggtg attctatttc tgctgtgatt tatctgctga aagctcagct   420
ggggttgtgc aagctaggga cccattcctg tgtaatacaa tgtctgcacc aatgct        476
```

**Description**

FIELD OF THE INVENTION

This invention provides methods for identifying the origin of a carcinoma of unknown primary origin.

BACKGROUND OF THE INVENTION

Carcinoma of unknown primary (CUP) is a set of heterogeneous, biopsy-confirmed malignancies wherein metastatic disease presents without an identifiable primary tumor site or tissue of origin (ToO). This problem represents approximately 3-5% of all cancers, making it the seventh most common malignancy. Ghosh et al. (2005); and Mintzer et al. (2004). The prognosis and therapeutic regimen of patients are dependent on the origin of the primary tumor, underscoring the need to identify the site of the primary tumor. Greco et al. (2004); Lembersky et al. (1996); and Schlag et al. (1994).

A variety of methods are currently used to resolve this problem. Several methods followed are diagrammed in Figures 1-2. Serum tumor Markers can be used for differential diagnosis. Although they lack adequate specificity, they can be used in combination with pathologic and clinical information. Ghosh ct al. (2005). Immunohistochemical (IHC) methods can be used to identify tumor lineage but very few IHC Markers are 100% specific. Therefore, pathologists often use a panel of IHC Markers. Several studies have demonstrated accuracies of 66-88% using four to 14 IHC Markers. Brown et al. (1997); DeYoung et al. (2000); and Dennis et al. (2005a). More expensive diagnostic workups include imaging methods such as chest x-ray, computed tomographic (CT) scans, and positron emission tomographic (PET) scans. Each of these methods can identify the primary in 30 to 50% of cases. Ghosh et al. (2005); and Pavlidis et al. (2003). Despite these sophisticated technologies, the ability to resolve CUP cases is only 20-30% ante mortem. Pavlidis et al. (2003); and Varadhachary ct al. (2004).

A promising new approach lies in the ability of genome-wide gene expression profiling to identify the origin of tumors. Ma et al. (2006); Dennis et al. (2005b); Su et al. (2001); Ramaswamy et al. (2001); Bloom et al. (2004); Giordano et al. (2001); and 20060094035. These studies demonstrated the feasibility of tissue of origin identification based on the gene expression profile. In order for these expression profiling technologies to be useful in the clinical setting, two major obstacles must be overcome. First, since gene expression profiling was conducted entirely on primary tissues, gene marker candidates must be validated on metastatic tissues to confirm that their tissue specific expression is preserved in metastasis. Second, the gene expression profiling technology must be able to utilize formalin-fixed, paraffin-embedded (FFPE) tissue, since fixed tissue samples are the standard material in current practice. Formalin fixation results in degradation of the RNA (Lewis et al. (2001); and Masuda et al. (1999)) so existing microarray protocols will not perform as reliably. Bibikova et al. (2004). Additionally, the profiling technology must be robust, reproducible, and easily accessible.

Quantitative RTPCR (qRTPCR) has been shown to generate reliable results from FFPE tissue. Abrahamsen et al. (2003); Specht et al. (2001); Godfrey et al. (2000); and Cronin et al. (2004). Therefore, a more practical approach would be to use a genome-wide method as a discovery tool and dcvelop a diagnostic assay based on a more robust technology. Ramaswamy (2004). This paradigm, however, requires a smaller gene set to be developed. Oien and colleagues used serial analysis of gene expression (SAGE) to identify 61 tumor Markers from which they developed a RTPCR method based on eleven genes for five tumor types. Dennis et al. (2002). Another study which coupled SAGE and qRTPCR developed a panel of five genes for four tumor types and achieved an accuracy of 81 %. Buckhaults et al. (2003). A more recent study coupled microarray profiling with qRTPCR, but used 79 Markers. Tothill et al. (2005).

SUMMARY OF THE INVENTION

The present invention provides a method of identifying origin of a metastasis of unknown origin in a sample containing metastatic cells comprising the steps of a) measuring Biomarkers associated with at least six different carcinomas wherein the Biomarkers are levels of expression of Marker genes wherein the Marker genes are SP-B, TTFI, DSG3, PSCA, F5, HPT1, PSA, MGB/MB, WT1 and PDEF, b) combining the data from the Biomarkers into an algorithm where the algorithm: normalizes the Biomarkers against a reference; and imposes a cut-off which optimizes sensitivity and specificity of each Biomarker, weights the prevalence of the carcinomas and selects a tissue of origin; c) determining origin based on highest probability determined by the algorithm or determining that the carcinoma is not derived from a particular set of carcinomas; and d) of optionally measuring Biomarkers specific for one or more additional different carcinoma, and repeating steps c) and d) as necessary for additional Biomarkers.

BRIEF DESCRIPTION OF THE DRAWINGS

Figures 1-2 depict prior art methods of identifying origin of a metastasis of unknown origin.

Figure 3 depicts the present CUP diagnostic algorithm.

Figure 4 depicts microarray data showing intensities of two genes in a panel of tissues. (A) Prostate stem cell antigen (PSCA). (B) Coagulation factor V (F5). The bar graphs show the intensity on the y-axis and the tissue on the x-axis. Panc Ca, pancreatic cancer; Panc N, normal pancreas.

Figure 5 depicts electropherograms obtained from an Agilent Bioanalyzer.

RNA was isolated from FFPE tissue using a three hour (A) or sixteen hour (B) proteinase K digestion. Sample C22 (red) was a one-year old block while sample C23 (blue) was a five-year old block. A size ladder is shown in green.

Figure 6 depicts a comparison of Ct values obtained from three different qRTPCR methods: random hexamer priming in the reverse transcription followed by qPCR with the resulting cDNA (RH 2 step), gene-specific (reverse primer) priming in the reverse transcription followed by qPCR with the resulting cDNA (GSP 2 step), or gene-specific priming and qRTPCR in a one-step reaction (GSP 1 step). RNA from eleven samples was divided into the three methods and RNA levels for three genes were measured: β-actin (A), HUMSPB (B), and TTF (C). The median Ct value obtained with each method is indicated by the solid line.

Figure 7 depicts CUP assay plate diagrams.

Figure 8 is a series of graphs depicting the assay performance over a range of RNA concentrations.

Figure 9 is an experimental workflow diagram: Marker candidate nomination and validation (9A); and assay optimization and prediction algorithm building and testing (9B).

Figure 10 depicts expression of 10 selected tissue specific gene Marker candidates in FFPE metastatic carcinomas and prostate primary adenocarcinoma. For each plot the X axis represents the normalized Marker expression value.

Figure 11 depicts assay optimization. (A and B) Electropherograms obtained from an Agilent Bioanalyzer. RNA was isolated from FFPE tissue using a three hour (A) or sixteen hour (B) proteinase K digestion. Sample C22 (red) was a one-year old block while sample C23 (blue) was a five-year old block. A size ladder is shown in green. (C and D) Comparison of Ct values obtained from three different qRTPCR methods: random hexamer priming in the reverse transcription followed by qPCR with the resulting cDNA (RH 2 step), gene-specific (reverse primer) priming in the reverse transcription followed by qPCR with the resulting cDNA (GSP 2 step), or gene-specific priming and qRTPCR in a one-step reaction (GSP 1 step). RNA from eleven samples was divided into the three methods and RNA levels for two genes were measured: β-actin (C), HUMSPB (D). The median Ct value obtained with each method is indicated by the solid line.

Figure 12 is a heatmap showing the relative expression levels of the 10 Marker panel across 239 samples. Red indicates higher expression.

DETAILED DESCRIPTION

Identifying the primary site in patients with metastatic carcinoma of unknown primary (CUP) origin can enable the application of specific therapeutic regimens and may prolong survival. Marker candidates were then validated by reverse transcriptase polymerase chain reaction (RT-PCR) on 205 FFPE metastatic carcinomas originating from these six tissues as well as metastases originating from other cancer types to determine specificity. A ten-gene signature was selected that predicted the tissue of origin of metastatic carcinomas for these six cancer types. Next, the RNA isolation and qRTPCR methods were optimized for these ten Markers, and applied the qRTPCR assay to a set of 260 metastatic tumors, generating an overall accuracy of 78%. Lastly, an independent set of 48 metastatic samples were tested. Importantly, thirty-seven samples in this set had either a known primary or initially presented as CUP but were subsequently resolved, and the assay demonstrated an accuracy of 78%.

A Biomarker is any indicia of the level of expression of an indicated Marker gene. The indicia can be direct or indirect and measure over- or under-expression of the gene given the physiologic parameters and in comparison to an internal control, normal tissue or another carcinoma. Biomarkers include, without limitation, nucleic acids (both over and under-expression and direct and indirect). Using nucleic acids as Biomarkers can include any method known in the art including, without limitation, measuring DNA amplification, RNA, micro RNA, loss of heterozygosity (LOH), single nucleotide polymorphisms (SNPs, Brookes (1999)), microsatellite DNA, DNA hypo- or hyper-methylation. Using proteins as Biomarkers includes any method known in the art including, without limitation, measuring amount, activity, modifications such as glycosylation, phosphorylation, ADP-ribosylation, ubiquitination, etc., or imunohistochemistry (IHC). Other Biomarkers include imaging, cell count and apoptosis Markers.

The indicated genes provided herein are those associated with a particular tumor or tissue type. A Marker gene may be associated with numerous cancer types but provided that the expression of the gene is sufficiently associated with one tumor or tissue type to be identified using the algorithm described herein to be specific for a particular origin, the gene can be used in the claimed invention to determine tissue of origin for a carcinoma of unknown primary origin (CUP). Numerous genes associated with one or more cancers are known in the art. The present invention provides preferred Marker genes and even more preferred Marker gene combinations. These are described herein in detail. "Origin" as referred to in 'tissue of origin' means either the tissue type (lung, colon, etc.) or the histological type (adenocarcinoma, squamous cell carcinoma, etc.) depending on the particular medical circumstances and will be understood by anyone of skill in the art.

A Marker gene corresponds to the sequence designated by a SEQ ID NO when it contains that sequence. A gene segment or fragment corresponds to the sequence of such gene when it contains a portion of the referenced sequence or its complement sufficient to distinguish it as being the sequence of the gene. A gene expression product corresponds to such sequence when its RNA, mRNA, or cDNA hybridizes to the composition having such sequence (e.g. a probe) or, in the case of a peptide or protein, it is encoded by such mRNA. A segment or fragment of a gene expression product corresponds to the sequence of such gene or gene expression product when it contains a portion of the referenced gene expression product or its complement sufficient to distinguish it as being the sequence of the gene or gene expression product.

The inventive methods described and claimed in this specification include Marker genes, as defined in the claims. "Marker" or "Marker gene" is used throughout this specification to refer to genes and gene expression products that correspond with any gene the over- or under-expression of which is associated with a tumor or tissue type. The Marker genes are described in more detail in Table 1.

Table 1

| CUP panel | | | |
|---|---|---|---|
| SEQ ID NO: | Name | Chip designation | sequence |
| 1 | SP-B | 209810_at | gaaaaaccagccactgctttacaggacaggggggttgaagctgagccccgc ctcacacccacccccatgcactcaaagattggattttacagctacttgcaatt caaaattcagaagaataaaaaatgggaacatacagaactctaaaagataga catcagaaattgttaagttaagctttttcaaaaaatcagcaattccccagcgta gtcaagggtggacactgcacgctctggcatgatgggatggcgaccgggc aagctttcttcctcgagatgctctgctgcttgagagctattgctttgttaagatat aaaaaggggtttctttttgtctttctgtaaggtggacttccagattttgattgaaa gtcctagggtgattctatttctgctgtgatttatctgctgaaagctcagctggg gttgtgcaagctagggacccattcctgtgtaatacaatgtctgcaccaatgct |
| 2 | TTF1 | 211024_s_at | gtgattcaaatgggttttccacgctagggcggggcacagattggagagggc tctgtgctgacatggctctggactctaaagaccaaacttcactctgggcaca ctctgccagcaaagaggactcgcttgtaaataccaggattttttttttttttgaa gggaggacgggagctggggagaggaaagagtcttcaacataacccactt gtcactgacacaaaggaagtgccccctccccggcaccctctggccgccta ggctcagcggcgaccgccctccgcgaaaatagtttgtttaatgtgaacttgt agctgtaaaacgctgtcaaaagttggactaaatgcctagtttttagtaatctgt acattttgttgtaaaaagaaaaaccactcccagtccccagcccttcacattttt atgggcattgacaaatctgtgtatattatttggcagtttggtatttgcggcgtca gtctttttctgttgtaact |

(continued)

| CUP panel | | | |
|---|---|---|---|
| SEQ ID NO: | Name | Chip designation | sequence |
| 3 | DSG3 | 205595_at | ccatcccatagaagtccagcagacaggatttgttaagtgccagactttgtca<br>ggaagtcaaggagcttctgctttgtccgcctctgggtctgtccagccagctgt<br>ttccatccctgaccctctgcagcatggtaactatttagtaacggagacttactc<br>ggcttctggttccctcgtgcaaccttccactgcaggctttgatccacttctcac<br>acaaaatgtgatagtgacagaaagggtgatctgtcccatttccagtgttcctg<br>gcaacctagctggcccaacgcagctacgagggtcacatactatgctctgta<br>cagaggatccttgctcccgtctaatatgaccagaatgagctggaataccaca<br>ctgaccaaatctggatctttggactaaagtattcaaaatagcatagcaaagct<br>cactgtattgggctaataatttggcacttattagcttctctcataaactgatcac<br>gattataaattaaatgtttgggttcataccccaaaagcaatatgttgtcactcct<br>aattctcaagtac |
| 4 | HPT1 | 209847_at | ctgcacccacctacttagatatttcatgtgctatagacattagagagatttttca<br>tttttccatgacatttttcctctctgcaaatggcttagctacttgtgtttttcccttttt<br>ggggcaagacagactcattaaatattctgtacatttttttctttatcaaggagata<br>tatcagtgttgtctcatagaactgcctggattccatttatgtttttttctgattccatc<br>ctgtgtccccttcatccttgactcctttggtatttcactgaatttcaaacatttgtc |
| 5 | PSCA | 205319_at | ttcctgaggcacatcctaacgcaagtttgaccatgtatgtttgcaccccttttcc<br>ccnaaccctgaccttcccatgggccttttccaggattccnaccnggcagatc<br>agttttagtganacanatccgcntgcagatggcccctccaaccntttntgttg<br>ntgtttccatggcccagcattttccacccttaaccctgtgttcaggcacttnttc<br>ccccaggaagccttccctgcccaccccatttatgaattgagccaggtttggt<br>ccgtggtgtcccccgcacccagcaggggacaggcaatcaggagggccc<br>agtaaaggctgagatgaagtggactgagtagaactggaggacaagagttg<br>acgtgagttcctgggagtttccagagatg |
| 6 | F5 | 204713_s_at | atcctctacagccagatgtcacagggatacgtctactttcacttggtgctgga<br>gaattcanaagtcaagaacatgctaagcntaagggacccaaggtagaaag<br>agatcaagcagcaaagcacaggttctcctggatgaaattactagcacataa<br>agttgggagacacctaagccaagacactggttctccttccggaatgaggcc<br>ctgggaggaccttcctagccaagacactggttctccttccagaatgaggcc<br>ctggaaggaccctcctagtgatctgttactcttaaaacaaagtaactcatctaa<br>gattttggttgggagatggcatttggcttctgagaaaggtagctatgaaataat<br>ccaagatactgatgaagacacagctgttaacaattggctgatcagcccccca<br>gaatgcctcacgtgcttggggagaaagcacccctcttgccaacaagcctgg<br>aaag |
| 7 | MGB1 | 206378_at | gcagcagcctcaccatgaagttgctgatggtcctcatgctggcggccctctc<br>ccagcactgctacgcaggctctggctgccccttattggagaatgtgatttcca<br>agacaatcaatccacaagtgtctaagactgaatacaaagaacttcttcaaga<br>gttcatagacgacaatgccactacaaatgccatagatgaattgaaggaatgt<br>tttcttaaccaaacggatgaaactctgagcaatgttgaggtgtttatgcaatta<br>atatatgacagcagtctttgtgatttattttaactttctgcaagacctttggctca<br>cagaactgcagggtatggtgagaaaccaactacggattgctgcaaaccac<br>accttctctttcttatgtctttttact |

(continued)

| CUP panel | | | |
|---|---|---|---|
| SEQ ID NO: | Name | Chip designation | sequence |
| 8 | PDEF | 220192_x_at | gagtggggcccttaaactggattcaaaaaatgctctaaacataggaatggtt gaagaggtcttgcagtcttcagatgaaactaaatctctagaagaggcacaa gaatggctaaagcaattcatccaagggccaccggaagtaattagagctttg aaaaaatctgtttgttcaggcagagagctatatttggaggaagcattacagaa cgaaagagatcttttaggaacagtttggggtgggcctgcaaatttagaggct attgctaagaaaggaaaatttaataaataattggtttttcgtgtggatgtactcc aagtaaagctccagtgactaatatgtataaatgttaaatgatattaaatatgaa catcagttaaaaaaaaaattctttaaggctactattaatatgcagacttactttta atcatttgaaatctgaactcatttacctcatttcttgccaattactcccttgggtat ttactgcgta |
| 9 | PSA | 204582_s_at | tggtgtaattttgtcctctctgtgtcctggggaatactggccatgcctggagac atatcactcaatttctctgaggacacagataggatggggtgtctgtgttatttgt ggggtacagagatgaaagaggggtgggatccacactgagagagtggag agtgacatgtgctggacactgtccatgaagcactgagcagaagctggagg cacaacgcaccagacactcacagcaaggatggagctgaaaacataaccc actctgtcc |
| 10 | WT1 | 206067_s_at | atagatgtacatacctccttgcacaaatggaggggaattcattttcatcactgg gagtgtccttagtgtataaaaaccatgctggtatatggcttcaagttgtaaaaa tgaaagtgactttaaaagaaaataggggatggtccaggatctccactgataa gactgtttttaagtaacttaaggacctttgggtctacaagtatatgtgaaaaaa atgagacttactgggtgaggaaatccattgtttaaagatggtcgtgtgtgtgt gtgtgtgtgtgtgtgttgtgttgtgttttgtttttttaagggagggaatttattatt taccgttgcttgaaattactgtgtaaatatatgtctgataatgatttgctctttgac aactaaaattaggactgtataagtactagatgcatcactgggtgttgatcttac aagat |

The present invention provides a method of identifying origin of a metastasis of unknown origin by measuring Biomarkers associated with at least six different carcinomas in a sample containing metastatic cells wherein the Biomarkers are levels of expression of genes and wherein the Marker genes are SP-8, JTFI, DSG3, PSCA, F5, HPT1, PSA, MGB/MG. WT1 and PDEF; combining the data from the Biomarkers into an algorithm where the algorithm: normalizes the Biomarkers against a reference; and imposes a cut-off which optimizes sensitivity and specificity of each Biomarker, weights the prevalence of the carcinomas and selects a tissue of origin; determining origin based on highest probability determined by the algorithm or determining that the carcinoma is not derived from a particular set of carcinomas; and optionally measuring Biomarkers specific for one or more additional different carcinoma, and repeating steps as necessary for additional Biomarkers.

The present invention provides a method of identifying origin of a metastasis of unknown origin in a sample containing metastatic cells as defined in the claims, comprising; measuring Biomarkers associated with at least six different carcinomas; combining the data from the Biomarkers into an algorithm where the algorithm i) normalizes the Biomarkers against a reference; and ii) imposes a cut-off which optimizes sensitivity and specificity of each Biomarker, weights the prevalence of the carcinomas and selects a tissue of origin; determining origin based on highest probability determined by the algorithm or determining that the carcinoma is not derived from a particular set of carcinomas; and optionally measuring Biomarkers specific for one or more additional different carcinoma, and repeating steps c) and d) for the additional Biomarkers.

The Marker genes are i) SP-B, TTF1, DSG3, PSCA, F5, HPT1, PSA, MCB/MC, WT1 and PDEF The Marker genes may further include KRT6F, p73H, and/or SFTPC.

Preferably, the Marker genes can further include ITGB6, KLK10, CLDN18, TR10 and/or FKB 10.

In another embodiment, the Marker genes can further include CDX1 and/or FABP1.

In one embodiment, gene expression is measured using at least one of SEQ ID NOs: 11-58.

The present disclosure also provides methods that measure gene expression by obtaining and measuring the formation of at least one of the amplicons SEQ ID NOs: 14, 18, 22, 26, 30, 34, 38, 42, 46, 50, 54 and/or 58.

In one embodiment, the Marker genes can further comprise a gender specific Marker selected from at least one of: i) in the case of a male patient KLK2, NGEP or NPY; or ii) in the case of a female patient PIP, B305D, B726 or GABA-Pi; and/or PAX8, STAR or EMX2. Preferably the Marker genes further comprise KLK2. In this embodiment, the Marker genes can further comprise NGEP and/or NPY. In one embodiment, in the case of a female patient, the Marker genes further comprise PIP, B305D, B726 or GABA-Pi. In one embodiment , in the case of a female patient, the Marker genes further comprise, PAX8, STAR or EMX2.

The present disclosure provides methods of obtaining additional clinical information including the site of metastasis to determine the origin of the carcinoma; obtaining optimal biomarker sets for carcinomas comprising the steps of using metastases of know origin, determining Biomarkers therefor and comparing the Biomarkers to Biomarkers of metastases of unknown origin; providing direction of therapy by determining the origin of a metastasis of unknown origin and identifying the appropriate treatment therefor; and providing a prognosis by determining the origin of a metastasis of unknown origin and identifying the corresponding prognosis therefor.

The present disclosure further provides methods of finding Biomarkers by determining the expression level of a Marker gene in a particular metastasis, measuring a Biomarker for the Marker gene to determine expression thereof, analyzing the expression of the Marker gene according to any of the methods provided herein or known in the art and determining if the Marker gene is effectively specific for the tumor of origin.

The present disclosure further provides composition containing at least one isolated sequence selected from SEQ ID NOs: 11-58. The present invention further provides the use of kits for conducting an assay according to the methods provided herein, wherein the kit comprises RNA or cDNA which hybridises to the marker genes of the invention, a microarray or a gene chip.

The present invention further provides the use of microarrays or gene chips for performing the methods described herein.

The present disclosure further provides diagnostic/prognostic portfolios containing isolated nucleic acid sequences, their complements, or portions thereof of a combination of genes as described herein where the combination is sufficient to measure or characterize gene expression in a biological sample having metastatic cells relative to cells from different carcinomas or normal tissue.

Any method described in the present invention can further include measuring expression of at least one gene constitutively expressed in the sample.

The Markers for pancreatic cancer are coagulation factor V (F5), prostate stem cell antigen (PSCA), integrin, β6 (ITGB6), kallikrein 10 (KLK10), claudin 18 (CLDN18), trio isoform (TR10), and hypothetical protein FLJ22041 similar to FK506 binding proteins (FKBP10). Biomarkers for F5 and PSCA are measured together. Biomarkers forITGB6, KLK10, CLDN18, TR10, and FKBP10 can be measured in addition to F5 and PSCA. F5 is described for instance by 20040076955; 20040005563; and WO2004031412. PSCA is described for instance by WO1998040403; 20030232350; and WO2004063355. ITGB6 is described for instance by WO2004018999; and 6339148. KLK10 is described for instance by WO20040770 and 20030235820. CLDN18 is described for instance by WO2004063355; and WO2005005601. TR10 is described for instance by 20020055627. FKBP10 is described for instance by WO2000055320.

The Marker genes for colon cancer are intestinal peptide-associated transporter HPT-1 (CDH17), caudal type homeo box transcription factor 1 (CDX1) and fatty acid binding protein 1 (FABP1). Biomarkers for CDX1 and FABP1 can be measured in addition to a Biomarker for CDH 17. CDH17 is described for instance by Takamura et al. (2004); and WO2004063355. CDX1 is described for instance by Pilozzi et al. (2004); 20050059008; and 20010029020. FABP11 is described for instance by Borchers et al. (1997); Chan et al. (1985); Chen et al. (1986); and Lowe et al. (1985).

The Marker genes for lung cancer are surfactant protein-B (SP-B), thyroid transcription factor (TTF), desmoglein 3 (DSG3), Keratin 6 isoform 6F (KRT6F), p53-related gene (p73H), and surfactant protein C (SFTPC). Biomarkers for SP-B, TTF and DSG3 are measured together. Biomarkers for KRT6F, p73H and SFTPC can be measured in addition to any of the Biomarkers for SP-B, TTF and/or DSG3. SP-B is described for instance by Pilot-Mathias ct al. (1989); 20030219760; and 20030232350. TTF is described for instance by Jones et al. (2005); US20040219575; WO 1998056953; WO2002073204; 20030138793; and WO2004063355 DSG3 is described for instance by Wan et al. (2003); 20030232350; aWO2004030615; and WO2002101357. KRT6F is described for instance by Takahashi et al. (1995); 20040146862; and 20040219572. p73H is described for instance by Senoo et al. (1998); and 20030138793. SFTPC is described for instance by Glasser et al. (1988).

The Marker genes can be further selected from a gender specific Marker such as, in the case of a male patient KLK2, NGEP or NPY; or in the case of a female patient PIP, B305D, B726 or GABA-Pi; and/or PAX8, STAR or EMX2.

The Marker genes for breast cancer are prostate derived epithelial factor (PDEF), mammaglobin (MG), prolactin-inducible protein (PIP), B305D, B726, and GABA-π. Biomarkers for PDEF and MG are measured together. Biomarkers for PIP, B305D, B726 and GABA-Pi can be measured in addition to Biomarkers for PDEF and MG. PDEF is described for instance by WO2004030615 WO2000006589; WO2001073032; Wallace et al. (2005); Feldman et al. (2003); and Oettgen et al. (2000). MG is described for instance by WO2004030615; 20030124128; Fleming et al (2000); Watson et al. (1996 and 1998); and 5668267. PIP is described for instance by Autiero et al. (2002); Clark et al. (1999); Myal ct al. (1991) and Murphy et al. (1987). B305D, B726 and GABA-Pi are described by Reinholz et al. (2005). NGEP is described for instance by Bera et al. (2004).

The Markers for ovarian cancer are Wilm's tumor I (WT1), PAX8, steroidogenic acute regulatory protein (STAR) and EMX2. Preferably, Biomarkers for WT1 are measured. Biomarkers for STAR and EMX2 can be measured in addition to Biomarkers for WT1. WT1 is described for instance by 5350840; 6232073; 6225051; 20040005563; and Bentov et al. (2003). PAX8 is described for instance by 20050037010; Poleev et al. (1992); Di Palma et al. (2003); Marques et al. (2002); Cheung et al. (2003); Goldstein et al. (2002); Oji et al. (2003); Rauscher et al. (1993); Zapata-Benavides et al. (2002); and Dwight et al. (2003). STAR is described for instance by Gradi et al. (1995); and Kim et al. (2003). EMX2 is described for instance by Noonan et al. (2001).

The Markers for prostate cancer arc KLK3, KLK22, NGEP and NPY. Biomarkers for KLK3 are measured. Biomarkers for KLK2, NGEP and NPY can be measured in addition to KLK3, KLK2 and KLK3 are described for instance by Magklara et al. (2002). KLK2 is described for instance by US 20030215835; and US 5786148, KLK3 is described for instance by US 6261766.

The method can also include obtaining additional clinical information including the site of metastasis to determine the origin of the carcinoma. A flow diagram is provided in Figure 3.

The disclosure further provides a method for obtaining optimal Biomarker sets for carcinomas by using metastases of know origin, determining Biomarkers therefor and comparing the Biomarkers to Biomarkers of metastases of unknown origin.

The disclosure further provides a method for providing direction of therapy by determining the origin of a metastasis of unknown origin according to the methods described herein and identifying the appropriate treatment therefor.

The disclosure further provides a method for providing a prognosis by determining the origin of a metastasis of unknown origin according to the methods described herein and identifying the corresponding prognosis therefor.

The disclosure further provides a method for finding Biomarkers comprising determining the expression level of a Marker gene in a particular metastasis, measuring a Biomarker for the Marker gene to determine expression thereof, analyzing the expression of the Marker gene according to the methods described herein and determining if the Marker gene is effectively specific for the tumor of origin.

The disclosure further provides compositions comprising at least one isolated sequence selected from SEQ ID NOs: 11-58.

The invention further provides the use of kits, microarrays or gene chips for conducting the assays described herein, as defined in the claims.

The mere presence or absence of particular nucleic acid sequences in a tissue sample has only rarely been found to have diagnostic or prognostic value. Information about the expression of various proteins, peptides or mRNA, on the other hand, is increasingly viewed as important. The mere presence of nucleic acid sequences having the potential to express proteins, peptides, or mRNA (such sequences referred to as "genes") within the genome by itself is not determinative of whether a protein, peptide, or mRNA is expressed in a given cell. Whether or not a given gene capable of expressing proteins, peptides, or mRNA does so and to what extent such expression occurs, if at all, is determined by a variety of complex factors. Irrespective of difficulties in understanding and assessing these factors, assaying gene expression can provide useful information about the occurrence of important events such as tumorogenesis, metastasis, apoptosis, and other clinically relevant phenomena. Relative indications of the degree to which genes are active or inactive can be found in gene expression profiles. The gene expression profiles obtained from the methods of this invention are used to provide a diagnosis and treat patients for CUP.

Sample preparation requires the collection of patient samples. Patient samples used in the inventive method are those that are suspected of containing diseased cells such as cells taken from a nodule in a fine needle aspirate (FNA) of tissue. Bulk tissue preparation obtained from a biopsy or a surgical specimen and laser capture microdissection are also suitable for use. Laser Capture Microdissection (LCM) technology is one way to select the cells to be studied, minimizing variability caused by cell type heterogeneity. Consequently, moderate or small changes in Marker gene expression between normal or benign and cancerous cells can be readily detected. Samples can also comprise circulating epithelial cells extracted from peripheral blood. These can be obtained according to a number of methods but the most preferred method is the magnetic separation technique described in 6136182. Once the sample containing the cells of interest has been obtained, a gene expression profile is obtained using a Biomarker, for genes in the appropriate portfolios.

Preferred methods for establishing gene expression profiles include determining the amount of RNA that is produced by a gene that can code for a protein or peptide. This is accomplished by reverse transcriptase PCR (RT-PCR), competitive RT-PCR, real time RT-PCR, differential display RT-PCR, Northern Blot analysis and other related tests. While it is possible to conduct these techniques using individual PCR reactions, it is best to amplify complementary DNA (cDNA) or complementary RNA (cRNA) produced from mRNA and analyze it via microarray. A number of different array configurations and methods for their production are known to those of skill in the art and are described in for instance, 5445934; 5532128; 5556752; 5242974; 5384261; 5405783; 5412087; 5424186; 5429807; 5436327; 5472672; 5527681; 5529756; 5545531; 5554501; 5561071; 5571639; 5593839; 5599695; 5624711; 5658734; and 5700637.

Microarray technology allows for measuring the steady-state mRNA level of thousands of genes simultaneously providing a powerful tool for identifying effects such as the onset, arrest, or modulation of uncontrolled cell proliferation. Two microarray technologies are currently in wide use, cDNA and oligonucleotide arrays. Although differences exist in the construction of these chips, essentially all downstream data analysis and output are the same. The product of these analyses are typically measurements of the intensity of the signal received from a labeled probe used to detect a cDNA sequence from the sample that hybridizes to a nucleic acid sequence at a known location on the microarray. Typically, the intensity of the signal is proportional to the quantity of cDNA, and thus mRNA, expressed in the sample cells. A large number of such techniques are available and useful. Preferred methods for determining gene expression can be found in 6271002; 6218122; 6218114; and 6004755.

Analysis of the expression levels is conducted by comparing such signal intensities. This is best done by generating a ratio matrix of the expression intensities of genes in a test sample versus those in a control sample. For instance, the gene expression intensities from a diseased tissue can be compared with the expression intensities generated from benign or normal tissue of the same type. A ratio of these expression intensities indicates the fold-change in gene expression between the test and control samples.

The selection can be based on statistical tests that produce ranked lists related to the evidence of significance for each gene's differential expression between factors related to the tumor's original site of origin. Examples of such tests include ANOVA and Kruskal-Wallis. The rankings can be used as weightings in a model designed to interpret the summation of such weights, up to a cutoff, as the preponderance of evidence in favor of one class over another. Previous evidence as described in the literature may also be used to adjust the weightings.

In the present invention, 10 markers were chosen that showed significant evidence of differential expression amongst 6 tumor types. The selection process included an ad-hoc collection of statistical tests, mean-variance optimization, and expert knowledge. In an alternative embodiment the feature extraction methods could be automated to select and test markers through supervised learning approaches. As the database grows, the selection of markers can be repeated in order to produce the highest diagnostic accuracy possible at any given state of the database.

A preferred embodiment is to normalize each measurement by identifying a stable control set and scaling this set to zero variance across all samples. This control set is defined as any single endogenous transcript or set of endogenous transcripts affected by systematic error in the assay, and not known to change independently of this error. All markers are adjusted by the sample specific factor that generates zero variance for any descriptive statistic of the control set, such as mean or median, or for a direct measurement. Alternatively, if the premise of variation of controls related only to systematic error is not true, yet the resulting classification error is less when normalization is performed, the control set will still be used as stated. Non-endogenous spike controls could also be helpful, but are not preferred.

Following marker selection, these selected variables are used in a classifier designed to produce as high a classification accuracy as possible. A supervised learning algorithm designed to relate a set of input measurements to an output set of predictors in order to build a model from the 10 inputs to predict the tissue of origin can be used. The problem can be stated as: given training data $\{(\mathbf{x_1},y), ..., (\mathbf{x_n}, y)\}$ produce a classifier $h : \chi \rightarrow \gamma$ which maps a sample x $\in \chi$ to its tissue of orign label $y \in \gamma$. The predictions are based on the previously resolved cases that are contained in the database and thus compose the training set.

The supervised learning algorithm should find parameters based on the relationships of the input variables to the known outputs that will minimize the expected classification error. These parameters can then be used to predict the tissue of origin from a new sample's input. Examples of these algorithms include linear classification models, quadratic classifiers, tree-based methods, neural networks, and prototype methods such as a k-nearest neighbor classifier or learning vector quantization algorithms.

One specific embodiment to model the 10 normalized markers is the LDA method, using default parameters, as described in Venables and Ripley (2002). This method is based on Fisher's linear discriminant analysis, where given means $\mu_y$=0, $\mu_y$=1 and covariances $\Sigma_y$=0, $\Sigma_y$=1 for y class labels 0 and 1, we seek a linear combination of $\overline{w.x}$ which will have means $\overline{w}.\mu_{y=i}$ and variances $\overline{w}^T\Sigma_{y=i}\overline{w}$ that will maximize the ratio of the variance between the classes to the variance within the classes:

$$S = \frac{\sigma^2_{between}}{\sigma^2_{within}} = \frac{(\vec{w}.\vec{\mu}_{y=1} - \vec{w}.\vec{\mu}_{y=0})^2}{\vec{w}^T\Sigma_{y=1}\vec{w} + \vec{w}^T\Sigma_{y=0}\vec{w}} = \frac{(\vec{w}.(\vec{\mu}_{y=1} - \vec{\mu}_{y=0}))^2}{\vec{w}^T(\Sigma_{y=0} + \Sigma_{y=1})\vec{w}}$$

LDA can be generalized to a multiple class discriminant analysis, where *y* has *N* possible states, instead of only two. The class means and variances are estimated from the values contained in the database for the choosen markers. In a preferred embodiment, the covariance matrix is weighted by equal prior probabilities of each tumor type subject to the following. Male patients are predicted by a model where the priors are zero for each female reproductive organ tumor group. Likewise, female patients are predicted by a model where the prior is zero for male reproductive organs. In the present invention, the priors are zero for tests on females for prostate and zero for tested males for breast and ovary. Furthermore, samples with a background identical to a class label are tested by a model where the prior probability is zero for that particular class label.

The problem above can be viewed as a maximization of the Rayleigh quotient handled as a generalized eigenvalue problem. The reduced subspace are used in classification by calculating each sample's distance to the centroid in the chosen subspace. The model can be fitted by maximum likelihood, and the posterior probabilities are calculated using Bayes' theorem.

An alternative method may include finding a map of a the n-dimensional feature space, where n is the number of variables used, to a set of classification labels will involve partitioning the feature space into regions, then assigning a classification to each region. The scores of these nearest neighbor type algorithms are related to the distance between decision boundaries and are not necessarily translated into class probabilities.

If there are too many variables to select from, and many of them are random noise, then the variable selection and model risk over-fitting the problem. Therefore, ranked list at various cut-offs are often used as inputs in order to limit the number of variables. Search algorithms such as a genetic algorithm can also be used to select for a sub-set of variables as they test a cost function. Simulated annealing can be attempted to limit the risk of catching the cost function in a local minimum. Nevertheless, these procedures must be validated with samples independent to the selection and modeling process.

Latent variable approaches may also be used. Any unsupervised learning algorithm to estimate low dimensional manifolds from high dimensional space can be used to discover associations between the input variables and how well they can fit a smaller set of latent variables. Although estimations of the effectiveness of the reductions are subjective, a supervised algorithm can be applied on the reduced variable set in order to estimate classification accuracy. Thus a classifier, which can be constructed from the latent variables, can also be built from a set of variables significantly correlated with the latent variables. An example of this would include using variables correlated to the principle components, from a principle component analysis, as inputs to any supervised classification model.

These algorithms can be implemented in any software code that has methods for inputting the variables, training the samples with a function, testing a sample based on the model, and outputting the results to a console. R, Octave, C, C++, Fortran, Java, Perl, and Python all have libraries available under an open source license to perform many of the functions listed above. Commercial packages such as S+ and Matlab are also packaged with many of these methods.

The code performs the following steps in the following order using R version 2.2.1 (http://www.r-project.org) with the MASS (Venables et al. (2002)) library installed. The term LDA refers to the lda function in the MASS namespace.

1) CT values for 10 marker genes and 2 controls are stored on a hard drive for all available training set samples.

2) For each sample, subtracting the sample specific average of the controls from each marker normalizes the 10 marker gene values.

3) The training data set is composed of metastasis with known sites of origin where each sample has at least one of its target markers specific for the labeled tissue of origin with a normalized CT value less than 5.

4) LDA constructs 4 sets of 2 LDA models from the training data in (3). In each set, one model is specific for males, and has prior odds for breast and ovary set to zero as well as the prior odds for prostate set to the equivalent priors of the other class labels. The other model in each pair is specific for females with the prior odds of prostate set to zero, and with the priors for breast and ovary set to the equivalent priors found in the other class labels.

   a. The first set is used to test CUP samples found in the colon, the prior odds for colon are set to zero and all other non-reproductive class labels are set to equivalent priors.

   b. A second model set is specific for a CUP found in the ovary, with prior odds for ovary set to zero and all other non-reproductive class labels set to equivalent priors.

   c. A third set is for a CUP found in the lung, with prior odds for lung set to zero. All other non-reproductive class labels have equivalent priors.

   d. The general model used for all other background tissues. All priors are set equivalently with the exception of the reproductive specific class labels that are set as defined in 4.

In order to test a sample, we run an R program that performs the following.

1) Reads in a test data set.

2) Generates a sample specific average of both controls.

3) For each sample, uses the sample specific average to subtract from each marker.
4) Replaces any normalized CT generated from a raw CT of 40 with 12.
5) For each sample in the test set the following are tested.

a. If the average of both controls are greater than 34 than the sample is labeled as 'CTR_FAILURE' with zeros for posterior probabilities.
b. The backgrounds are checked for colon, ovary, or lung. If a match is found than the gender is checked as well. The background and gender specific model is then used to evaluate the sample.
c. If breast, pancreas, lungSCC, or prostate is found as the background label, then a label of 'FAILURE_ineligible_sample' is given to the sample, and the posterior probabilities are all set to zero.
d. The general model for either male or female is used for all other samples.

The results are formatted and written to a file.

The present disclosure includes gene expression portfolios obtained by this process.

Gene expression profiles can be displayed in a number of ways. The most common is to arrange raw fluorescence intensities or ratio matrix into a graphical dendogram where columns indicate test samples and rows indicate genes. The data are arranged so genes that have similar expression profiles are proximal to each other. The expression ratio for each gene is visualized as a color. For example, a ratio less than one (down-regulation) appears in the blue portion of the spectrum while a ratio greater than one (up-regulation) appears in the red portion of the spectrum. Commercially available computer software programs are available to display such data including "GeneSpring" (Silicon Genetics, Inc.) and "Discovery" and "Infer" (Partek, Inc.)

Measurements of the abundance of unique RNA species are collected from primary tumors or metastatic tumors from primaries of known origin. These readings along with clinical records including, but not limited to, a patient's age, gender, site of origin of primary tumor, and site of metastasis (if applicable) are used to generate a relational database. The database is used to select RNA transcripts and clinical factors that can be used as marker variables to predict the primary origin of a metastatic tumor.

In the case of measuring protein levels to determine gene expression, any method known in the art is suitable provided it results in adequate specificity and sensitivity. For example, protein levels can be measured by binding to an antibody or antibody fragment specific for the protein and measuring the amount of antibody-bound protein. Antibodies can be labeled by radioactive, fluorescent or other detectable reagents to facilitate detection. Methods of detection include, without limitation, enzyme-linked immunosorbent assay (ELISA) and immunoblot techniques.

Modulated genes used in the methods of the invention are described in the Examples. The genes that are differentially expressed are either up regulated or down regulated in patients with carcinoma of a particular origin relative to those with carcinomas from different origins. Up regulation and down regulation are relative terms meaning that a detectable difference (beyond the contribution of noise in the system used to measure it) is found in the amount of expression of the genes relative to some baseline. In this case, the baseline is determined based on the algorithm. The genes of interest in the diseased cells are then either up regulated or down regulated relative to the baseline level using the same measurement method. Diseased, in this context, refers to an alteration of the state of a body that interrupts or disturbs, or has the potential to disturb, proper performance of bodily functions as occurs with the uncontrolled proliferation of cells. Someone is diagnosed with a disease when some aspect of that person's genotype or phenotype is consistent with the presence of the disease. However, the act of conducting a diagnosis or prognosis may include the determination of disease/status issues such as determining the likelihood of relapse, type of therapy and therapy monitoring. In therapy monitoring, clinical judgments are made regarding the effect of a given course of therapy by comparing the expression of genes over time to determine whether the gene expression profiles have changed or are changing to patterns more consistent with normal tissue.

Genes can be grouped so that information obtained about the set of genes in the group provides a sound basis for making a clinically relevant judgment such as a diagnosis, prognosis, or treatment choice. These sets of genes make up the portfolios of the disclosure. As with most diagnostic Markers, it is often desirable to use the fewest number of Markers sufficient to make a correct medical judgment. This prevents a delay in treatment pending further analysis as well unproductive use of time and resources.

One method of establishing gene expression portfolios is through the use of optimization algorithms such as the mean variance algorithm widely used in establishing stock portfolios. This method is described in detail in 20030194734. Essentially, the method calls for the establishment of a set of inputs (stocks in financial applications, expression as measured by intensity here) that will optimize the return (e.g., signal that is generated) one receives for using it while minimizing the variability of the return. Many commercial software programs are available to conduct such operations. "Wagner Associates Mean-Variance Optimization Application," referred to as "Wagner Software" throughout this specification, is preferred. This software uses functions from the "Wagner Associates Mean-Variance Optimization Library" to determine an efficient frontier and optimal portfolios in the Markowitz sense is preferred. Markowitz (1952).

Use of this type of software requires that microarray data be transformed so that it can be treated as an input in the way stock return and risk measurements are used when the software is used for its intended financial analysis purposes.

The process of selecting a portfolio can also include the application of heuristic rules. Preferably, such rules are formulated based on biology and an understanding of the technology used to produce clinical results. More preferably, they are applied to output from the optimization method. For example, the mean variance method of portfolio selection can be applied to microarray data for a number of genes differentially expressed in subjects with cancer. Output from the method would be an optimized set of genes that could include some genes that are expressed in peripheral blood as well as in diseased tissue. If samples used in the testing method are obtained from peripheral blood and certain genes differentially expressed in instances of cancer could also be differentially expressed in peripheral blood, then a heuristic rule can be applied in which a portfolio is selected from the efficient frontier excluding those that are differentially expressed in peripheral blood. Of course, the rule can be applied prior to the formation of the efficient frontier by, for example, applying the rule during data pre-selection.

Other heuristic rules can be applied that are not necessarily related to the biology in question. For example, one can apply a rule that only a prescribed percentage of the portfolio can be represented by a particular gene or group of genes. Commercially available software such as the Wagner Software readily accommodates these types of heuristics. This can be useful, for example, when factors other than accuracy and precision (e.g., anticipated licensing fees) have an impact on the desirability of including one or more genes.

The gene expression profiles of this disclosure can also be used in conjunction with other non-genetic diagnostic methods useful in cancer diagnosis, prognosis, or treatment monitoring. For example, in some circumstances it is beneficial to combine the diagnostic power of the gene expression based methods described above with data from conventional Markers such as serum protein Markers (e.g., Cancer Antigen 27.29 ("CA 27.29")). A range of such Markers exists including such analytes as CA 27.29. In one such method, blood is periodically taken from a treated patient and then subjected to an enzyme immunoassay for one of the serum Markers described above. When the concentration of the Marker suggests the return of tumors or failure of therapy, a sample source amenable to gene expression analysis is taken. Where a suspicious mass exists, a fine needle aspirate (FNA) is taken and gene expression profiles of cells taken from the mass are then analyzed as described above. Alternatively, tissue samples may be taken from areas adjacent to the tissue from which a tumor was previously removed. This approach can be particularly useful when other testing produces ambiguous results.

Kits made according to the disclosure include formatted assays for determining the gene expression profiles. These can include all or some of the materials needed to conduct the assays such as reagents and instructions and a medium through which Biomarkers are assayed.

Articles of this disclosure include representations of the gene expression profiles useful for treating, diagnosing, prognosticating, and otherwise assessing diseases. These profile representations are reduced to a medium that can be automatically read by a machine such as computer readable media (magnetic, optical, and the like). The articles can also include instructions for assessing the gene expression profiles in such media. For example, the articles may comprise a CD ROM having computer instructions for comparing gene expression profiles of the portfolios of genes described above. The articles may also have gene expression profiles digitally recorded therein so that they may be compared with gene expression data from patient samples. Alternatively, the profiles can be recorded in different representational format. A graphical recordation is one such format. Clustering algorithms such as those incorporated in "DISCOVERY" and "INFER" software from Partek, Inc. mentioned above can best assist in the visualization of such data.

Different types of articles of manufacture according to the disclosure are media or formatted assays used to reveal gene expression profiles. These can comprise, for example, microarrays in which sequence complements or probes are affixed to a matrix to which the sequences indicative of the genes of interest combine creating a readable determinant of their presence. Alternatively, articles according to the disclosure can be fashioned into reagent kits for conducting hybridization, amplification, and signal generation indicative of the level of expression of the genes of interest for detecting cancer.

The following examples are provided to illustrate but not limit the claimed invention.

### Example 1

Materials and Methods

*Pancreatic cancer Markers gene discovery.*

RNA was isolated from pancreatic tumor, normal pancreatic, lung, colon, breast and ovarian tissues using Trizol. The RNA was then used to generate amplified, labeled RNA (Lipshutz et al. (1999)) which was then hybridized onto Affymetrix U133A arrays. The data were then analyzed in two ways.

In the first method, this dataset was filtered to retain only those genes with at least two present calls across the entire dataset. This filtering left 14,547 genes. 2,736 genes were determined to be overexpressed in pancreatic cancer versus normal pancreas with a p value of less than 0.05. Forty five genes of the 2,736 were also overexpressed by at least two-fold compared to the maximum intensity found from lung and colon tissues. Finally, six probe sets were found which were overexpressed by at least two-fold compared to the maximum intensity found from lung, colon, breast, and ovarian tissues.

In the second method, this dataset was filtered to retain only those genes with no more than two present calls in breast, colon, lung, and ovarian tissues. This filtering left 4,654 genes. 160 genes of the 4,654 genes were found to have at least two present calls in the pancreatic tissues (normal and cancer). Finally, eight probe sets were selected which showed the greatest differential expression between pancreatic cancer and normal tissues.

*Tissue samples.*

A total of 260 FFPE metastasis and primary tissues were acquired from a variety of commercial vendors. The samples tested included: 30 breast metastasis, 30 colorectal metastasis, 56 lung metastasis, 49 ovarian metastasis 43 pancreas metastasis, 18 prostate primary and 2 prostate metastases and 32 other origins (6 stomach, 6 kidney, 3 larynx, 2 liver, 1 esophagus, 1 pharynx, 1 bile duct, 1 pleura, 3 bladder, 5 melanoma, 3 lymphoma).

*RNA Extraction.*

RNA isolation from paraffin tissue sections was based on the methods and reagents described in the High Pure RNA Paraffin Kit manual (Roche) with the following modifications. Paraffin embedded tissue samples were sectioned according to size of the embedded metastasis (2-5mm = 9 X 10μm, 6-8mm = 6 X 10μm, 8 -≥ 10mm = 3 X 10 μm), and placed in RNase/DNase 1.5ml Eppendorf tubes. Sections were deparaffinized by incubation in 1ml of xylene for 2-5min at room temperature following a 10-20 second vortex. Tubes were then centrifuged and supernatant was removed and the deparaffmization step was repeated. After supernatant was removed, 1ml of ethanol was added and sample was vortexed for 1 minute, centrifuged and supernatant removed. This process was repeated one additional time. Residual ethanol was removed and the pellet was dried in a 55°C oven for 5-10 minutes and resuspended in 100μl of tissue lysis buffer, 16μl 10% SDS and 80μl Proteinase K. Samples were vortexed and incubated in a thermomixer set at 400 rpm for 2 hours at 55°C. 325μl binding buffer and 325 μl ethanol was added to each sample that was then mixed, centrifuged and the supernatant was added onto the filter column. Filter column along with collection tube were centrifuged for 1 minute at 8000rpm and flow through was discarded. A series of sequential washes were performed (500μl Wash Buffer I → 500μl Wash Buffer II → 300μl Wash Buffer II) in which each solution was added to the column, centrifuged and flow through discarded. Column was then centrifuged at maximum speed for 2 minutes, placed in a fresh 1.5ml tube and 90μl of elution buffer was added. RNA was obtained after a 1 minute incubation at room temperature followed by a 1 minute centrifugation at 8000 rpm. Sample was DNase treated with the addition of 10μl DNase incubation buffer, 2μl of DNase I and incubated for 30 minutes at 37°C. DNase was inactivated following the addition of 20μl of tissue lysis buffer, 18μl 10% SDS and 40μl Proteinase K. Again, 325μl binding buffer and 325μl ethanol was added to each sample that was then mixed, centrifuged and supernatant was added onto the filter column. Sequential washes and elution of RNA proceeded as stated above with the exception of 50μl of elution buffer being used to elute the RNA. To eliminate glass fiber contamination carried over from the column RNA was centrifuged for 2 minutes at full speed and supernatant was removed into a fresh 1.5ml Eppendorf tube. Samples were quantified by OD 260/280 readings obtained by a spectrophotometer and samples were diluted to 50ng/μl. The isolated RNA was stored in Rnase-free water at -80°C until use.

*TaqMan Primer and Probe Design.*

Appropriate mRNA reference sequence accession numbers in conjunction with Oligo 6.0 were used to develop TaqMan® CUP assays (lung Markers: human surfactant, pulmonary-associated protein B (HUMPSPBA), thyroid transcription factor 1 (TTF1), desmoglein 3 (DSG3), colorectal Marker: cadherin 17 (CDH17), breast Markers: mammaglobin (MG), prostate-derived ets transcription factor (PDEF), ovarian Marker: wilms tumor 1 (WT1), pancreas Markers: prostate stem cell antigen (PSCA), coagulation factor V (F5), prostate Marker kallikrein 3 (KLK3)) and housekeeping assays β actin, hydroxymethylbilane synthase (PBGD). Primers and hydrolysis probes for each assay are listed in Table 2. Genomic DNA amplification was excluded by designing assays around exon-intron splicing sites. Hydrolysis probes were labeled at the 5' nucleotide with FAM as the reporter dye and at 3' nucleotide with BHQ1-TT as the internal quenching dye.

*Quantitative Real-Time Polymerase Chain Reaction.*

Quantitation of gene-specific RNA was carried out in a 384 well plate on the ABI Prism 7900HT sequence detection system (Applied Biosystems). For each thermo-cycler run calibrators and standard curves were amplified. Calibrators for each Marker consisted of target gene in vitro transcripts that were diluted in carrier RNA from rat kidney at $1X10^5$ copies. Standard curves for housekeeping Markers consisted of target gene in vitro transcripts that were serially diluted in carrier RNA from rat kidney at $1X10^7$, $1X10^5$ and $1X10^3$ copies. No target controls were also included in each assay run to ensure a lack of environmental contamination. All samples and controls were run in duplicate. qRTPCR was performed with general laboratory use reagents in a 10μl reaction containing: RT-PCR Buffer (50nM Bicine/KOH pH 8.2, 115nM KAc, 8% glycerol, 2.5mM $MgCl_2$, 3.5mM $MnSO_4$, 0.5mM each of dCTP, dATP, dGTP and dTTP), Additives (2mM Tris-Cl pH 8, 0.2mM Albumin Bovine, 150mM Trehalose, 0.002% Tween 20), Enzyme Mix (2U Tth (Roche), 0.4mg/μl Ab TP6-25), Primer and Probe Mix (0.2μM Probe, 0.5μM Primers). The following cycling parameters were followed: 1 cycle at 95°C for 1 minute; 1 cycle at 55°C for 2 minutes; Ramp 5%; 1 cycle at 70°C for 2 minutes; and 40 cycles of 95°C for 15 seconds, 58°C for 30 seconds. After the PCR reaction was completed, baseline and threshold values were set in the ABI 7900HT Prism software and calculated Ct values were exported to Microsoft Excel.

*One-Step vs. Two-Step Reaction.*

First strand synthesis was carried out using either 100ng of random hexamers or gene specific primers per reaction. In the first step, 11.5μl of Mix-1 (primers and lug of total RNA) was heated to 65°C for 5 minutes and then chilled on ice. 8.5μl of Mix-2 (1x Buffer, 0.01mM DTT, 0.5mM each dNTP's, 0.25U/μl RNasin®, 10U/μl Superscript III) was added to Mix-1 and incubated at 50°C for 60 minutes followed by 95°C for 5 minutes. The cDNA was stored at -20°C until ready for use. qRTPCR for the second step of the 2-step reaction was performed as stated above with the cycling parameters: 1 cycle at 95°C for 1 minute; 40 cycles of 95°C for 15 seconds, 58°C for 30 seconds. qRTPCR for the one-step reaction was performed exactly as stated in the preceding paragraph. Both the one-step and two-step reactions were performed on 100ng of template (RNA/cDNA). After the PCR reaction was completed, baseline and threshold values were set in the ABI 7900HT Prism software and calculated Ct values were exported to Microsoft Excel.

*Generation of a heatmap.*

For each sample, a ΔCt was calculated by taking the mean Ct of each CUP Marker and subtracting the mean Ct of an average of the housekeeping Markers (ΔCt = Ct(CUP Marker) - Ct(Ave. HK Marker)). The minimal ΔCt for each tissue of origin Marker set (lung, breast, prostate, colon, ovarian and pancreas) was determined for each sample. The tissue of origin with the overall minimal ΔCt was scored one and all other tissue of origins scored zero. Data were sorted according to pathological diagnosis. Partek Pro was populated with the modified feasibility data and an intensity plot was generated.

**Results.**

*Discovery of novel pancreatic tumor of origin and cancer status* Markers.

First, five pancreas Marker candidates were analyzed: prostate stem cell antigen (PSCA), serine proteinase inhibitor, clade A member 1 (SERPINA1), cytokeratin 7 (KRT7), matrix metalloprotease 11 (MMP11), and mucin4 (MUC4) (Varadhachary et al (2004); Fukushima et al. (2004); Argani et al. (2001); Jones et al. (2004); Prasad et al. (2005); and Moniaux et al. (2004)) using DNA microarrays and a panel of 13 pancreatic ductal adenocarcinomas, five normal pancreas tissues, and 98 samples from breast, colorectal, lung, and ovarian tumors. Only PSCA demonstrated moderate sensitivity (six out of thirteen or 46% of pancreatic tumors were detected) at a high specificity (91 out of 98 or 93% were correctly identified as not being of pancreatic origin) (Figure 4A). In contrast, KRT7, SERPINA1, MMP11, and MUC4 demonstrated sensitivities of 38%, 31%, 85%, and 31%, respectively, at specificities of 66%, 91%, 82%, and 81 %, respectively. These data were in good agreement with qRTPCR performed on 27 metastases of pancreatic origin and 39 metastases of non-pancreatic origin for all Markers except for MMP11 which showed poorer sensitivity and specificity with qRTPCR and the metastases. In conclusion, the microarray data on snap frozen, primary tissue serves as a good indicator of the ability of the Marker to identify a FFPE metastasis as being pancreatic in origin using qRTPCR but that additional Markers may be useful for optimal performance.

Because pancreatic ductal adenocarcinoma develops from ductal epithelial cells that comprise only a small percentage of all pancreatic cells (with acinar cells and islet cells comprising the majority) and because pancreatic adenocarcinoma tissues contain a significant amount of adjacent normal tissue (Prasad et al. (2005); and Ishikawa et al. (2005)), it has been difficult to identify pancreatic cancer Markers (i.e., upregulated in cancer) which would also differentiate this organ from the organs. For use in a CUP panel such differentiation is necessary. The first query method (see Materials and Methods) returned six probe sets: coagulation factor V (F5), a hypothetical protein FLJ22041 similar to FK506 binding proteins (FKBP10), β 6 integrin (ITGB6), transglutaminase 2 (TGM2), heterogeneous nuclear ribonucleoprotein A0 (HNRP0), and BAX delta (BAX). The second query method (see Materials and Methods) returned eight probe sets: F5, TGM2, paired-like homeodomain transcription factor 1 (PITX1), trio isoform mRNA (TRIO), mRNA for p73H (p73), an unknown protein for MGC:10264 (SCD), and two probe sets for claudin18. F5 and TGM2 were present in both query results and, of the two, F5 looked the most promising (Figure 4B).

*Optimization of sample prep and qR TPCR using* FFPE *tissues.*

Next the RNA isolation and qRTPCR methods were optimized using fixed tissues before examining Marker panel performance. First the effect of reducing the proteinase K incubation time from sixteen hours to 3 hours was analyzed. There was no effect on yield. However, some samples showed longer fragments of RNA when the shorter proteinase K step was used (Figure 5). For example, when RNA was isolated from a one year old block (C22), there was no observed difference in the electropherograms. However, when RNA was isolated from a five year old block (C23), a larger fraction of higher molecular weight RNAs was observed, as assessed by the hump in the shoulder, when the shorter proteinase K digest was used. This trend generally held when other samples were processed, regardless of the organ of origin for the FFPE metastasis. In conclusion, shortening the proteinase K digestion time does not sacrifice RNA yields and may aid in isolating longer, less degraded RNA.

Next, three different methods of reverse transcription were compared: reverse transcription with random hexamers followed by qPCR (two step), reverse transcription with a gene-specific primer followed by qPCR (two step), and a one-step qRTPCR using gene-specific primers. RNA was isolated from eleven metastases and compared Ct values across the three methods for β-actin, human surfactant protein B (HUMSPB), and thyroid transcription factor (TTF) (Figure 6). There were statistically significant differences ($p < 0.001$) for all comparisons. For all three genes, the reverse transcription with random hexamers followed by qPCR (two step reaction) gave the highest Ct values while the reverse transcription with a gene-specific primer followed by qPCR (two step reaction) gave slightly (but statistically significant) lower Ct values than the corresponding 1 step reaction. However, the 2 step RTPCR with gene-specific primers had a longer reverse transcription step. When HUMSPB and TTF Ct values were normalized to the corresponding β-actin value for each sample, there were no differences in the normalized Ct values across the three methods. In conclusion, optimization of the RTPCR reaction conditions can generate lower Ct values, which may help in analyzing older paraffin blocks (Cronin et al (2004)), and a one step RTPCR reaction with gene-specific primers can generate Ct values comparable to those generated in the corresponding two step reaction.

*Diagnostic performance of a CUP qRTPCR assay.*

Next 12 qRTPCR reactions (10 Markers and two housekeeping genes) were performed on 239 FFPE metastases. The Markers used for the assay are shown in Table 2. The lung Markers were human surfactant pulmonary-associated protein B (HUMPSPB), thyroid transcription factor 1 (TTF1), and desmoglein 3 (DSG3). The colorectal Marker was cadherin 17 (CDH 17). The breast Markers were mammaglobin (MG) and prostate-derived Ets transcription factor (PDEF). The ovarian Marker was Wilms tumor 1 (WT1). The pancreas Markers were prostate stem cell antigen (PSCA) and coagulation factor V (F5), and the prostate Marker was kallikrein 3 (KLK3). For gene descriptions, see Table 31.

Table 2. Primer and probe sequences, accession numbers, and amplicon lengths.

| Target | SEQ ID NO | Sequence (5'-3') | Description | SEQ ID NO |
|---|---|---|---|---|
| SP-B | 59 | cacagccccgacctttgatga | Forward primer | 11 |
| | | ggtcccagagcccgtctca | Reverse primer | 12 |
| | | agctgtccagctgcaaaggaaaagcc | Probe* | 13 |
| | | cacagccccgacctttgatgagaactcagctgtccagctgcaaaggaaaagc caagtgagacgggctctgggacc | Amplicon | 14 |
| TTF1 | 60 | ccaacccagacccgcgc | Forward primer | 15 |
| | | cgcccatgccgctcatgttca | Reverse primer | 16 |
| | | cccgccatctcccgcttcatg | Probe* | 17 |
| | | ccaacccagacccgcgcttccccgccatctcccgcttcatgggcccggcgagc ggcatgaacatgagcggcatgggcg | Amplicon | 18 |
| DSG3 | 61 | gcagagaaggagaagataactcaa | Forward primer | 19 |
| | | actccagagattcggtaggtga | Reverse primer | 20 |
| | | attgccaagattacttcagattacca | Probe* | 21 |
| | | gcagagaaggagaagataactcaaaaagaaacccaattgccaagattacttc agattaccaagcaacccagaaaatcacctaccgaatctctggagt | Amplicon | 22 |
| CDH17 | 62 | tccctcggcagtggaagctta | Forward primer | 23 |
| | | tcctcaaactctgtgtgcctggta | Reverse primer | 24 |
| | | ccaaaatcaatggtactcatgcccgactg | Probe* | 25 |
| | | tccctcggcagtggaagcttacaaaacgactgggaagtttccaaaatcaatggt actcatgcccgactgtctaccaggcacacagagtttgagga | Amplicon | 26 |
| MG | 63 | agttgctgatggtcctcatgc | Forward primer | 27 |
| | | cacttgtggattgattgtcttgga | Reverse primer | 28 |
| | | ccctctcccagcactgctacgca | Probe* | 28 |
| | | agttgctgatggtcctcatgctggcggccctctcccagcactgctacgcaggctct ggctgccccttattggagaatgtgatttccaagacaatcaatccacaagtg | Amplicon | 30 |
| PDEF | 64 | cgcccacctggacatctgga | Forward primer | 31 |

(continued)

| Target | SEQ ID NO | Sequence (5'-3') | Description | SEQ ID NO |
|---|---|---|---|---|
| | | cactggtcgaggcacagtagtga | Reverse primer | 32 |
| | | gtcagcggcctggatgaaagagcgg | Probe* | 33 |
| | | cgcccacctggacatctggaagtcagcggcctggatgaaagagcggacttca cctggggcgattcactactgtgcctcgaccagtg | Amplicon | 34 |
| WT1 | 65 | gcggagcccaatacagaatacac | Forward primer | 35 |
| | | cggggctactccaggcaca | Reverse primer | 36 |
| | | tcagaggcattcaggatgtgcgacg | Probe* | 37 |
| | | gcggagcccaatacagaatacacacgcacggtgtcttcagaggcattcagga tgtgcgacgtgtgcctggagtagccccg | Amplicon | 38 |
| PSCA | 66 | ctgttgatggcaggcttggc | Forward primer | 39 |
| | | ttgctcacctgggctttgca | Reverse primer | 40 |
| | | gcagccaggcactgccctgct | Probe* | 41 |
| | | ctgttgatggcaggcttggccctgcagccaggcactgccctgctgtgctactcct gcaaagcccaggtgagcaa | Amplicon | 42 |
| F5 | 67 | tgaagaaatatcctgggattattca | Forward primer | 43 |
| | | tatgtggtatcttctggaatatcatca | Reverse primer | 44 |
| | | acaaagggaaacagatattgaagactc | Probe* | 45 |
| | | tgaagaaatatcctgggattattcagaatttgtacaaagggaaacagatattgaa gactctgatgatattccagaagataccacata | Amplicon | 46 |
| KLK3 | 68 | cccccagtgggtcctcaca | Forward primer | 47 |
| | | aggatgaaacaagctgtgccga | Reverse primer | 48 |
| | | caggaacaaaagcgtgatcttgctgg | Probe* | 49 |
| | | cccccagtgggtcctcacagctgcccactgcatcaggaacaaaagcgtgatct tgctgggtcggcacagcttgtttcatcct | Amplicon | 50 |
| β actin | 69 | gccctgaggcactcttcca | Forward primer | 51 |
| | | cggatgtccacgtcacacttca | Reverse primer | 52 |

(continued)

| Target | SEQ ID NO | Sequence (5'-3') | Description | SEQ ID NO |
|---|---|---|---|---|
| | | cttccttcctgggcatggagtcctg | Probe* | 53 |
| | | gccctgaggcactcttccagccttccttcctgggcatggagtcctgtggcatccac gaaactaccttcaactccatcatgaagtgtgacgtggacatccg | Amplicon | 54 |
| PBGD | 70 | ccacacacagcctactttccaa | Forward primer | 55 |
| | | tacccacgcgaatcactctca | Reverse primer | 56 |
| | | aacggcaatgcggctgcaacggcggaa | Probe* | 57 |
| | | ccacacacagcctactttccaagcggagccatgtctggtaacggcaatgcggc tgcaacggcggaagaaaacagcccaaagatgagagtgattcgcgtgggta | Amplicon | 58 |
| *Probes are 5'FAM-3'BHQ1-TT | | | | |

Analysis of the normalized Ct values in a heat map revealed the high specificity of the breast and prostate Markers, moderate specificity of the colon, lung, and ovarian, and somewhat lower specificity of the pancreas Markers. Combining the normalized qRTPCR data with computational refinement improves the performance of the Marker panel. Results were obtained from the combined normalized qRTPCR data with the algorithm and the accuracy of the qRTPCR assay was determined.

**Discussion.**

In this example, microarray-based expression profiling was used on primary tumors to identify candidate Markers for use with metastases. The fact that primary tumors can be used to discover tumor of origin Markers for metastases is consistent with several recent findings. For example, Weigelt and colleagues have shown that gene expression profiles of primary breast tumors are maintained in distant metastases. Weigelt et al. (2003). Italiano and coworkers found that EGFR status, as assessed by IHC, was similar in 80 primary colorectal tumors and the 80 related metastases. Italiano et al. (2005). Only five of the 80 showed discordance in EGFR status. Italiano et al. (2005). Backus and colleagues identified putative Markers for detecting breast cancer metastasis using a genome-wide gene expression analysis of breast and other tissues and demonstrated that mammaglobin and CK19 detected clinically actionable metastasis in breast sentinel lymph nodes with 90% sensitivity and 94% specificity. Backus et al. (2005).

The microarray-based studies with primary tissue confirmed the specificity and sensitivity of known Markers. As a result, with the exception of F5, all of the Markers used have high specificity for the tissues studied here. Argani et al (2001; Backus et al. (2005); Cunha et al. (2005); Borgono et al. (2004); McCarthy et al. (2003); Hwang et al. (2004); Fleming et al. (2000); Nakamura et al. (2002); and Khoor et al. (1997). A recent study determined that, using IHC, PSCA is overexpressed in prostate cancer metastases. Lam et al. (2005). Dennis et al. (2002) also demonstrated that PSCA could be used as a tumor of origin Marker for pancreas and prostate. As shown herein, strong expression of PSCA is found in some prostate tissues at the RNA level but, because by including PSA in the assay, one can now segregate prostate and pancreatic cancers. A novel finding of this study was the use of F5 as a complementary (to PSCA) Marker for pancreatic tissue of origin. In both the microarray data set with primary tissue and the qRTPCR data set with FFPE metastases, F5 complemented PSCA (Figure 4 and Table 3).

Table 3 feasibility data

| | Breast | Colon | Lung | Other | Ovary | Pancreas | Prostate | Total |
|---|---|---|---|---|---|---|---|---|
| Total tested | 30 | 30 | 56 | 32 | 49 | 43 | 20 | 260 |
| #Correct | 22 | 27 | 45 | 16 | 43 | 31 | 20 | 204 |
| #Other / No test | 1 | 1 | 3 | n/a | 1 | 4 | 0 | 10 |
| #Incorrect | 7 | 2 | 8 | 16 | 5 | 8 | 0 | 46 |
| % Tested | 96.67 | 96.67 | 94.64 | 100 | 97.96 | 90.70 | 100 | 96.15 |
| % Correct of tested | 75.86 | 93.10 | 84.91 | 0 | 89.58 | 79.49 | 100 | 81.60 |
| Correct of total (%) | 73.33 | 90.00 | 80.36 | 50.00 | 87.76 | 72.09 | 100 | 78.46 |

Previous investigators have generated CUP assays using IHC or microarrays. Su et al. (2001); Ramaswamy et al. (2001); and Bloom et al. (2004). More recently, SAGE has been coupled to a small qRTPCR Marker panel. Dennis (2002); and Buckhaults et al. (2003). This study is the first to combine microarray-based expression profiling with a small panel of qRTPCR assays. Microarray studies with primary tissue identified some, but not all, of the same tissue of origin Markers as those identified previously by SAGE studies. Some studies have demonstrated that a modest agreement between SAGE- and DNA microarray-based profiling data exists and that the correlation improves for genes with higher expression levels. van Ruissen et al. (2005); and Kim (2003). For example, Dennis and colleagues identified PSA, MG, PSCA, and HUMSPB while Buckhaults and coworkers (Dennis et al. (2002)) identified PDEF. Executing the CUP assay using qRTPCR is preferred because it is a robust technology and may have performance advantages over IHC. Al-Mulla et al. (2005); and Haas et al. (2005). As shown herein, the qRTPCR protocol was improved through the use of gene-specific primers in a one-step reaction. This is the first demonstration of the use of gene-specific primers in a one-step qRTPCR reaction with FFPE tissue. Other investigators have either done a two step qRTPCR (cDNA synthesis in one reaction followed by qPCR) or have used random hexamers or truncated gene-specific primers. Abrahamsen et al. (2003); Specht et al. (2001); Godfrey et al. (2000); Cronin et al. (2004); and Mikhitarian et al. (2004).

**Example 2**

CUP FFPE Total RNA Isolation Protocol
(Highpure kit Cat#3270289)

**Purpose:**

Isolation of total RNA from FFPE tissue

**Procedure:**

**Preparation of working solutions**

1. Proteinase K (PK) in kit

Dissolve lyophilizate in 4.5 ml Elution Buffer. Aliquot and store at -20°C, stable for 12 months.
PK-4x250mg (cat #3115852)
Dissolve lyophilizate in 12.5 ml of Elution Buffer (1x TE Buffer (pH 7.4-7). Aliquot and store at -20°C.

2. Wash Buffer I

Add 60 ml absolute ethanol to Wash Buffer I, store at RT.

3. Wash Buffer II

Add 200 ml absolute ethanol to Wash Buffer II, store at RT.

4. DNase I

Dissolve lyophilizate in 400 μl Elution Buffer. Aliquot and store at -20°C, stable for 12 months.

**Sectioning Paraffin Blocks -30-45 minutes for 12 blocks (12 blocks x 2 tubes = 24 tubes)**

Sections cut from the block should be processed immediately for RNA extraction
1. Use a clean sharp razor blade on Microtome to cut 6 X 10 micron thick sections from trimmed tissue blocks (size 3-4 x 5-10mm).
Note: New block-Discard wax sections until obtained tissue section. Used block-Discard first 3 tissue sections
2. Immediately place cut tissue in 1.5 mL microfuge tubes and tightly cap to minimize moisture.
3. It is recommended to take the number of sections based on size of tumor shown in Table 4.

Table 4

| Size of MET | Sections/Tube |
|---|---|
| 8-10 mm | 6 |
| 6-8 mm | 12 |
| 2-4 mm | 18 |

**Deparaffinization ~30-45 minutes**

1. Add 1.0 ml xylene to each sample and vortex vigorously for 10-20 sec and incubate RT 2-5min. Centrifuge at full speed 2min. Remove the supernatant carefully.
*Note: if the tissue appears to be floating, centrifuge for an additional 2min.*
2. Repeat step 1.
3. Centrifuge at full speed 2min. Remove the supernatant.

4. Add 1 ml ethanol abs. and vortex vigorously 1min. Centrifuge at full speed 2min. Remove the supernatant.
5. Repeat step 4.
6. Blot the tube briefly onto a paper towel to get rid of ethanol residues.
7. Dry the tissue pellet for 5-10 min at 55°C in oven.
Note: it is critical that the ethanol is completely removed and the pellets are thoroughly dry, residual ethanol can inhibit PK digestion.
***Note: if PK is in -20C, warm in RT 20-30min.***

RNA Extraction ~2.5-3 hours

1. Add 100 μl Tissue Lysis Buffer, 16 μl 10% SDS and **80** μl Proteinase K working solution to one tissue pellet, vortex briefly in several intervals and incubate **2hrs** at 55°C shaking 400rpm,
2. Add 325μl Binding Buffer and 325μl ethanol abs. Mix gently by pipetting up and down.
3. Centrifuge the lysate at full speed for 2min.
4. Combine the filter tube and the collection tube (12 tubes), and pipet the lysate supernatant into the filter.
5. Centrifuge for 30 sec at 8000 rpm and discard the flowthrough.
*Note: Step 4-5 can be repeated, if RNA needs to be pooled with 2 more tissue pellet preparations.*
6. Repeat the centrifugation at 8000 rpm for 30 sec to dry the filter.
7. Add 500 μl Wash Buffer I working solution to the column and centrifuge for 15-30 sec at 8000 rpm, discard the flowthrough.
8. Add 500 μl Wash Buffer II working solution. Centrifuge for 15-30 sec at 8000 rpm, discard the flowthrough.
9. Add 300 μl Wash Buffer II working solution, centrifuge for 15-30 sec at 8000 rpm, discard the flowthrough.
10. Centrifuge the High Pure filter for 2 min at maximum speed.
11. Place the High Pure filter tube into a fresh 1.5 ml tube and add 90 μl Elution Buffer. Incubate for 1-2 min at room temperature. Centrifuge 1 min at 8000 rpm. **DNase I Treatment ~1.5 hours**
12. Add 10 μl of 10x DNase Incubation Buffer and 1.0 μl DNase I working solution to the eluate and mix. Incubate for 45 min at 37°C **(or 2.0 μl DNase I for 30min).**
13. Add 20 μl Tissue Lysis Buffer, 18 μl 10% SDS and 40μl Proteinase K working solution. Vortex briefly. Incubate for **30 min** (30-60 min.) at 55°C.
14. Add 325 μl Binding Buffer and 325 μl ethanol abs. Mix and pipet into a fresh High Pure filter tube with collection tube (12 tubes).
15. Centrifuge for 30 sec at 8000 rpm and discard the flowthrough.
16. Repeat the centrifugation at 8000 rpm for 30 sec to dry the filter.
17. Add 500 μl Wash Buffer I working solution to the column. Centrifuge for 15 sec at 8000 rpm, discard the flowthrough.
18. Add 500 μl Wash Buffer II working solution. Centrifuge for 15 sec at 8000 rpm, discard the flowthrough.
19. Add 300 μl Wash Buffer II working solution. Centrifuge for 15 sec at 8000 rpm, discard the flowthrough.
20. Centrifuge the High Pure filter for 2 min at maximum speed.
21. Place the High Pure filter tube into a fresh 1.5 ml tube. Add 50 μl Elution Buffer; incubate for 1-2 min at room temperature. Centrifuge for 1 min at 8000 rpm to collect the eluated RNA.
22. Centrifuge the eluate for 2 min at full speed and transfer supernatant to a new tube without disturbing glass fibers at the bottom.
23. Take 260/280 OD reading and dilute to 50ng/μl. Store at -80°C.

**CUP ASR Assay Protocol** (ABI 7900)

**Purpose:** Use qRTPCR to determine tissue of origin of a CUP sample

**Control Setup:**

***1. Positive Controls (Refer to Table 5 and Plate C in Plate Setup, Figure 7)***

Table 5 Serial dilutions of IVT - 5μl 1X10$^8$ into 470μl $H_2O$ + 25μl of 10000 rRNA

| IVT Control | CE/μl | Sample | Water | Bkgd rRNA |
|---|---|---|---|---|
| BACTIN | 100E+05 | 50 | 425 | 25 |
| CDH 17 | 100E+05 | 50 | 425 | 25 |
| DSG3 | 100E+05 | 50 | 425 | 25 |
| F5 | 100E+05 | 50 | 425 | 25 |
| Hump | 100E+05 | 50 | 425 | 25 |
| MG | 100E+05 | 50 | 425 | 25 |
| PBGD | 100E+05 | 50 | 425 | 25 |
| PDEF | 100E+05 | 50 | 425 | 25 |
| PSCA | 100E+05 | 50 | 425 | 25 |
| TTF1 | 100E+05 | 50 | 425 | 25 |
| WT1 | 100E+05 | 50 | 425 | 25 |
| 1E6. Table 5. Dilute 50,000 CE/μl rRNA to 500 CE/μl - 5μl 50,000 CE/μl + 495 μl $H_2O$ | | | | |

*Aliqouts 10μl per strip tube (2 plates); Place Mix at -80°C until ready for use.*

***2. Standard Curves (Refer to Table 6 and Plate C in Plate Setup, Figure 7)***

Step1: Standard curve was setup exactly as shown in Table 6.

Table 7. Stock Solution - 1X10$^8$ IVT. Dilute 50,000 CE/μl rRNA to 500 CE/μl - 5μl 50,000 CE/μl + 495 μl $H_2O$

| IVT Control | CE/μl | Sample | Water | Bkgd rRNA |
|---|---|---|---|---|
| BACTIN-1 | 100E+07 | 50 | 425 | 25 |
| BACTIN-2 | 100E+06 | 50 | 425 | 25 |
| BACTIN-3 | 100E+05 | 50 | 425 | 25 |
| BACTIN-4 | 100E+04 | 50 | 425 | 25 |
| BACTIN-5 | 100E+03 | 50 | 425 | 25 |
| PBGD-1 | 100E+07 | 50 | 425 | 25 |
| PBGD-2 | 100E+06 | 50 | 425 | 25 |
| PBGD-3 | 100E+05 | 50 | 425 | 25 |
| PBGD-4 | 100E+04 | 50 | 425 | 25 |
| PBGD-5 | 100E+03 | 50 | 425 | 25 |

*Aliqouts 10μl per strip tube (2 plates); Place Mix at -80°C until ready for use.*

**Enzyme Mix:**

*1. **Master Mix:*** Enzyme (Tth) / Antibody (TP6-25), see Table 7.

Table 7

| Reagent | 2x |
|---|---|
| Enzyme Tth (5U/μl) | 600.00 |
| Antibody: TP6-25 (1mg/ml) | 600.00 |
| Water | 300.00 |
| Total | 1500.00 |

Aliquot 500μl */tube and freeze at -20°C.*

**CUP Master Mix:**

***1. 2.5X CUP Master Mix (Tables 8-11):***

Table 8

| ml | 5x Additives | 2.5x Conc. |
|---|---|---|
| 0.50 | 1M Tris-Cl pH 8 | 5mM |
| 1.25 | 40mg/ml Albumin, bovine | 500μg/ml |
| 37.50 | 1M stock Trehalose | 375mM |
| 2.5 | 20%v Tween 20 | 0.50% |
| 7.00 | $ddH_2O$ | |
| 48.75 | | |
| *Allow reagent to fully mix > 15 minutes* | | |

Table 9

| ml | 5x Additives | 2.5 x Conc |
|---|---|---|
| 12.50 | 1M Bicine/Potassium Hydroxide pH 8.2 | 125mM |
| 5.75 | 5M Potassium Acetate | 287.5mM |
| 20.00 | Glycerol (V x D = M -> 19.6 x 1.26 = 24.6 g) | 20% |
| 1.25 | 500mM Magnesium Chloride | 6.25mM |
| 1.75 | 500mM Manganese Chloride | 8.75mM |
| 5.00 | $ddH_2O$ | |
| 46.25 | | |
| *Allow reagent to fully mix > 15 minutes; Combine above mixes into sterile container - add the following* | | |

Table 10

| ml | 5x Additives | 2.5x Conc. |
|---|---|---|
| 1.25 | 100mM dATP | 1.25mM |
| 1.25 | 100mM dCTP | 1.25mM |
| 1.25 | 100mM dTTP | 1.25mM |

(continued)

| ml | 5x Additives | 2.5x Conc. |
|---|---|---|
| 1.25 | 100mM dGTP | 1.25mM |
| 100.00 | | |
| *Allow reagent to fully mix > 15 minutes; A liquot 1.8ml / tube and freeze at -20°C* | | |

Table 11

| Primer/Probe | Stock (μM) | FC (μM) | μl |
|---|---|---|---|
| Forward Primer | 100 | 10 | 100.0 |
| Reverse Primer | 100 | 10 | 100.0 |
| Probe (5'FAM/3'BHQ1-TT) | 100 | 4 | 40.0 |
| DI Water | | | 760.0 |
| Total | | | 1000.0 |

**Primer and Probe Mix:**

*Aliquot 250μl / tube and freeze at -20°C*

**Reaction Mix:**

*1. CUP Master Mix (CMM): (Refer to Tables 12-14 and Plate A in Plate Setup, Figure 7)*

Table 12

| Reagent | FC | X1 (10μl) | 450 |
|---|---|---|---|
| 2.5 x CUP Master Mix | 1X | 4.00 | 1800 |
| ROX | 1x | 0.20 | 90 |
| 2x TthAb Mix | 2U | 1.00 | 450 |
| Water | | 2.3 | 1035 |
| Total | | 7.50 | 3375 |

Preferably, each run / plate will have no more than 356 reactions: 12 samples with 12 Markers (288 reactions with 2 replicates for each) + 10 std curve controls in duplicate (20) + 2 positive and 2 negative controls for each Marker. (4x12=48)

*Adjust water for sample volume - 4.3μl Sample MAX; Mix Well*

Table 13

| Reagent | FC | X1 (10μl) | 34 |
|---|---|---|---|
| Primers 10μM/Probe 4μM | 0.5 μM/0.2 μM | 0.50 | 17 |
| CMM | 1x | 7.50 | 255 |
| Total | | 8.00 | 272 |

2. ***ToO Markers:*** *Mix Well*

Table 14

| Reagent | FC | X1 (10μl) | 44 |
|---|---|---|---|
| Primers 10μM/Probe 4μM | 0.5μM/0.2μM | 0.50 | 22 |
| CMM | 1x | 7.50 | 330 |
| Total | | 8.00 | 352 |

3. β***-Actin and PBGD Markers:*** *Mix Well*

**Sample Setup:**

Table 15

| Sample | Sample ID | Conc | Water Added = 50ng/μl |
|---|---|---|---|
| A1 | | | |
| A2 | | | |
| A3 | | | |
| A4 | | | |
| A5 | | | |
| A6 | | | |
| A7 | | | |
| A8 | | | |
| A9 | | | |
| A10 | | | |
| A11 | | | |
| A12 | | | |

***1. CUP Samples: 12 samples in 96 well plate: A1-A12 (Refer to Table 16 and Plate B in Plate Setup, Figure 7); Aliquot 50μl of 50ngl/μl (2μl/rxn)***

**Load Plate:**

***1. 384 Well Plate Setup: (Refer to Plate D in Plate Setup, Figure 7)***

2μl of sample and 8μl of CMM are loaded onto the plate. (sample=50ng/μl)

4μl of sample and 6μl of CMM are loaded on to the plate (sample=25ng/μl)

The plate is sealed and labeled. Centrifuge at 2000rpm for 1 min. ***ABI* 7900HT Setup:** Place in the ABI 7900. Select the program "CUP 384" and hit start.

Table 16

| Thermocycling conditions |
|---|
| 95C x 60s |
| 55C x 2m |

(continued)

| Thermocycling conditions |
|---|
| RAMP 5% |
| 70C x 2m |
| 40 cycles of |
| 95C x 15s |
| 58C x 30s |
| ROX Turned On |

*Data are analyzed, Ct's extracted and inserted in Algorithm*

**Example 3**

CUP Algorithm

The actin normalized ΔCt values for HPT, MGB, PDEF, PSA, SP-B, TFF, DSG, WT1, PSCA, and F5 are placed into 6 sets based on the tissue of origin from which originally selected. The constants 9.00, 11.00, 7.50, 5.00, 10.00, 9.50, 6.50, 8.00, 9.00, and 8.00 are subtracted from each ΔCt respectively. Then, for each sample the minimum CT value from each of the 6 sets (HPT, min (MGB, or PDEF), PSA, min (SP-B, TFF, or DSG), WT1, and min (PSCA, or F5)) is selected as the representative variable for the group.

These variables, and the metastatic site are used to classify the sample using linear discriminants. Two different models, one for males and one for females, should be constructed from the training data using the MASS library function 'lda' (Venables et al. (2002) in R (version 2.0.1). A posterior probability for each ToO is then calculated using the 'predict' function for either the male or female model.

The variables used in the male models are HPT, PSA, the minimum of ('SP-B', 'TFF', 'DSG3'), the minimum of ('PSCA', 'F5'), and the metastatic site. The metastatic site category has 4 levels corresponding to colon, lung, ovary, and all other tissues. For the female models, the variables are HPT, the minimum of ('MGB', 'PDEF'), the minimum of ('SP-B', 'TFF', 'DSG3'), WT1, the minimum of ('PSCA', 'F5'), and the metastatic site.

Example R Code:

```
#Training the male model
dat.m<-CUP2.MIN.NORM[,c
('HPT','PSA','SP.B.TTF.DSG3','PSCA.F5','Class','background')]
CUP.lda.m<-lda(Class~.,dat.m,prior=c(0,0.09,0.23,0.43,0,0.16,0.02)/sum(c
(0,0.09,0.23,0.43,0,0.16,0.02)))
#Training the female model
dat.f<-CUP2.MIN.NORM[,c
('HPT','MFB.PDEF','SP.B.TTF.DSG3','WT1','PSCA.F5','Class','background')]
CUP.lda.f<-lda(Class~.,dat.f,prior=c(0.03,0.09,0.23,0.43,0.04,0.16,0)/sum(c
(0.03,0.09,0.23,0.43.0.04,0.16,0)))
#if unknown sample (i) is male
predict(CUP.lda.m, CUP2.MIN.NORM.TEST[i,])
#if unknown sample (i) is female
predict(CUP.lda.f, CUP2.MIN.NORM.TEST[i,])
```

To run this code, a data frame called CUP2.MIN.NORM needs to contain the training data with the minimum value calculated for each tissue of origin set as described above.

Class corresponds to the tissue of origin, and background corresponds to the metastatic sites described above.

The test data can be contained in CUP2.MIN.NORM.TEST, and a specific sample at row i can be tested using the predict function. Again, the test data must be in the same format as the training set and have the minimum value adjustments applied to it as well.

**Example 4**

CUP resolved samples

48 CUP resolved and unresolved samples were compared to determine the correlation to true CUP samples. The methods used were those described in Examples 1-3. The results obtained are presented in Table 17. 11 samples were tested of unresolved CUP, diagnosis was made on 8 samples, 3 were of other category.

Table 17

| Sample category | Sample # | Correct | Incorrect | No test | Accuracy % |
|---|---|---|---|---|---|
| Known ToO | 15 | 11 | 3 | 1 | 79 |
| Resolved CUP | 22 | 17 | 4 | 1 | 81 |
| Unresolved CUP | 11 | 8 | N/a | 3 | 73 |

**Example 5**

CUP Assay Limits

Figure 8 depicts the results obtained, using the methods described in Examples 1-3, to determine the limits of the CUP assays. Assay performance was tested over a range of RNA concentrations and it was found that CUP assays are efficient in the range of from 100 - 12.5 ng RNA.

**Example 6**

qRTPCR assay

**Materials and Methods.** *Frozen Tissue Samples for Microarray Analysis.* A total of 700 frozen primary human tissues were used for gene expression microarray profiling. Samples were obtained from variety of academic institutions, including Washington University (St. Louis, MO), Erasmus Medical Center (Rotterdam, Netherlands), and commercial tissue bank companies, including Genomics Collaborative, Inc (Cambridge, MA), Asterand (Detroit, MI), Oncomatrix (La Jolla, CA) and Clinomics Biosciences (Pittsfield, MA). For each specimen, patient demographic, clinical and pathology information was collected as well. The histopathological features of each sample were reviewed to confirm diagnosis, and to estimate sample preservation and tumor content.

*RNA extraction and Affymetrix GeneChip Hybridization.* Frozen cancer samples with greater than 70% tumor cells, benign and normal samples were dissected and homogenized with mechanical homogenizer (UltraTurrex T8, Germany) in Trizol reagent (Invitrogen, Carlsbad, CA). Tissue was homogenized in Trizol reagent by following the standard Trizol protocol for RNA isolation from frozen tissues (Invitrogen, Carlsbad, CA). After centrifugation the top liquid phase was collected and total RNA was precipitated with isopropyl alcohol at -20°C. RNA pellets were washed with 75% ethanol, resolved in water and stored at -80°C until use.

RNA quality was examined with an Agilent 2100 Bioanalyzer RNA 6000 Nano Assay (Agilent Technologies, Palo Alto, CA). Labeled cRNA was prepared and hybridized with the high-density oligonucleotide array Hu133A Gene Chip (Affymetrix, Santa Clara, CA) containing a total of 22,000 probe sets according to standard manufacturer protocol. Arrays were scanned using Affymetrix protocols and scanners. For subsequent analysis, each probe set was considered a separate gene. Expression values for each gene were calculated using Affymetrix Gene Chip analysis software MAS 5.0. All chips met three quality control standards: the percent "present" call for the array was greater than 35%, the scale factor was less than 12 when scaled to a global target intensity of 600, and the average background level was less than 150.

*Marker Candidate Selection.* For selection of tissue of origin (ToO) Marker candidates for lung, colon, breast, ovarian, and prostate tissues, expression levels of the probe sets were measured in the RNA samples covering a total of 682 normal, benign, and cancerous tissues from breast, colon, lung, ovarian, prostate. Tissue specific Marker candidates were selected based on number of statistical queries.

In order to generate pancreatic candidates, gene expression profiles of 13 primary pancreas ductal adenocarcinoma, 5 pancreas normal and 98 lung, colon, breast and ovarian cancer specimens was used to select pancreas adenocarcinoma Markers. Two queries were performed. In the first query, data set containing 14547 genes with at least 2 "present" calls in pancreas samples was created. A total of 2736 genes that overexpressed in pancreas cancer compare to normal was identified by T-test ($p<0.05$) were identified. Genes which minimal expression at 11th percentile of pancreas cancer was at least 2 fold higher that the maximum in colon and lung cancer was selected, making 45 probe sets. As a final step, 6 genes with maximum expression at least 2 fold higher than maximum expression in colon, lung, breast, and ovarian cancers were selected. In a second query, data set of 4654 probe sets with at most 2 "present" calls in all breast, colon, lung and ovarian specimens was created. A total of 160 genes that have at least 2 "present" calls in pancreas normal and cancer samples were selected. Out of 160 genes, 10 genes were selected after comparing their expression level between pancreas and normal tissues. Results of both pancreas queries were combined.

In addition to gene expression profiles analysis, a few Markers were selected from literature. Results of all queries were combined to make a short list of ToO Marker candidates for each tissue type. Sensitivity and specificity of each Marker were estimated. Markers that demonstrated the best ability to differentiate tissues by their origin were nominated for RT-PCR testing based on Markers redundancy and complementarity.

*FFPE metastatic carcinoma of known origin and CUP tissues.* A total of 386 FFPE metastatic carcinomas (Stage III-IV) of known origin and 24 FFPE prostate primary adenocarcinomas were acquired from a variety of commercial vendors, including Proteogenex (Los Angeles, CA), Genomics Collaborative, Inc. (Cambridge, MA), Asterand (Detroit, MI), Ardais (Lexington, MA) and Oncomatrix (La Jolla, CA). An independent set of 48 metastatic carcinoma of known primary and CUP tissues was obtained from Albany Medical College (Albany, NY). For each specimen, patient demographic, clinical and pathology information was collected. The histopathologic features of each sample were reviewed to confirm diagnosis, and to estimate sample preservation and tumor content. For metastatic samples, diagnoses of metastatic carcinoma and ToO were unequivocally established based on patient's clinical history and histological evaluation of metastatic carcinoma in comparison to corresponding primaries.

*DNA Isolation from FFPE samples.* RNA isolation from paraffin tissue sections was as described in the High Pure RNA Paraffin Kit manual (Roche) with the following modifications. Paraffin embedded tissue samples were sectioned according to size of the embedded metastasis (2-5mm = 9 X 10μm, 6-8mm = 6 X 10μm, 8-≥10mm = 3 X 10μm). Sections were deparaffmized as described by Kit manual, the tissue pellet was dried in a 55°C oven for 5-10 minutes and resuspended in 100μl of tissue lysis buffer, 16μl 10% SDS and 80μl Proteinase K. Samples were vortexed and incubated in a thermomixer set at 400 rpm for 2 hours at 55°C. Subsequent sample processing was performed according High Pure RNA Paraffin Kit manual. Samples were quantified by OD 260/280 readings obtained by a spectrophotometer and samples were diluted to 50ng/μl. The isolated RNA was stored in RNase-free water at -80°C until use.

*qRTPCR for Marker candidates pre-screening.* One μg total RNA from each sample was reverse-transcribed with random hexamers using Superscript II reverse transcriptase according to the manufacturer's instructions (Invitrogen, Carlsbad, CA). Primers and MGB-probes for the tested gene Marker candidates and the control gene ACTB were designed using Primer Express software (Applied Biosystems, Foster City, CA) either ABI Assay-on-Demand (Applied Biosystems, Foster City, CA) were used. All in-house designed primers and probes were tested for optimal amplification efficiency above 90%. RT-PCR amplification was carried out in a 20 ml reaction mix containing 200 ng template cDNA, 2 x TaqMan® universal PCR master mix (10 ml) (Applied Biosystems, Foster City, CA), 500nM forward and reverse primers, and 250nM probe. Reactions were run on an ABI PRISM 7900HT Sequence Detection System (Applied Biosystems, Foster City, CA). The cycling conditions were: 2 min of AmpErase UNG activation at 50°C, 10 min of polymerase activation at 95°C and 50 cycles at 95°C for 15 sec and annealing temperature (60°C) for 60 sec. In each assay, "no-template" control along with template cDNA was included in duplicate for both the gene of interest and the control gene. The relative expression of each target gene was represented as ΔCt, which is equal to Ct of the target gene subtracted by Ct of the control gene (ACTB).

*Optimized One-step* qRTPCR. Appropriate mRNA reference sequence accession numbers in conjunction with Oligo 6.0 were used to develop TaqMan® CUP assays (lung Markers: human surfactant, pulmonary-associated protein B (HUMPSPBA), thyroid transcription factor 1 (TTF1), desmoglein 3 (DSG3), colorectal Marker: cadherin 17 (CDH17), breast Markers: mammaglobin (MG), prostate-derived ets transcription factor (PDEF), ovarian Marker: wilms tumor 1 (WT1), pancreas Markers: prostate stem cell antigen (PSCA), coagulation factor V (F5), prostate Marker kallikrein 3 (KLK3)) and housekeeping assays beta actin (β-Actin), hydroxymethylbilane synthase (PBGD). Gene specific primers and hydrolysis probes for the optimized one-step qRT-PCR assay are listed in Table 2 (SEQ ID NOs: 11-58). Genomic DNA amplification was excluded by designing the assays around exon-intron splicing sites. Hydrolysis probes were labeled at the 5' nucleotide with FAM as the reporter dye and at 3' nucleotide with BHQ1-TT as the internal quenching dye.

Quantitation of gene-specific RNA was carried out in a 384 well plate on the ABI Prism 7900HT sequence detection system (Applied Biosystems). For each thermo-cycler run calibrators and standard curves were amplified. Calibrators for each Marker consisted of target gene in vitro transcripts that were diluted in carrier RNA from rat kidney at $1X10^5$ copies. Standard curves for housekeeping Markers consisted of target gene in vitro transcripts that were serially diluted in carrier RNA from rat kidney at $1X10^7$, $1X10^5$ and $1X10^3$ copies. No target controls were also included in each assay run to ensure a lack of environmental contamination. All samples and controls were run in duplicate. qRTPCR was performed with general laboratory use reagents in a 10μl reaction containing: RT-PCR Buffer (50nM Bicine/KOH pH 8.2, 115nM KAc, 8% glycerol, 2.5mM $MgCl_2$, 3.5mM $MnSO_4$, 0.5mM each of dCTP, dATP, dGTP and dTTP), Additives (2mM Tris-Cl pH 8, 0.2mM Albumin Bovine, 150mM Trehalose, 0.002% Tween 20), Enzyme Mix (2U Tth (Roche),

0.4mg/μl Ab TP6-25), Primer and Probe Mix (0.2μM Probe, 0.5μM Primers). The following cycling parameters were followed: 1 cycle at 95°C for 1 minute; 1 cycle at 55°C for 2 minutes; Ramp 5%; 1 cycle at 70°C for 2 minutes; and 40 cycles of 95°C for 15 seconds, 58°C for 30 seconds. After the PCR reaction was completed, baseline and threshold values were set in the ABI 7900HT Prism software and calculated Ct values were exported to Microsoft Excel.

*One-Step vs. Two-Step Reaction.* For comparison of two-step with one-step RT-PCR reactions, first strand synthesis of two-step reaction was carried out using either 100ng of random hexamers or gene specific primers per reaction. In the first step, 11.5μl of Mix-1 (primers and 1μg of total RNA) was heated to 65°C for 5 minutes and then chilled on ice. 8.5μl of Mix-2 (1x Buffer, 0.01mM DTT, 0.5mM each dNTP's, 0.25U/μl RNasin®, 10U/μl Superscript III) was added to Mix-1 and incubated at 50°C for 60 minutes followed by 95°C for 5 minutes. The cDNA was stored at -20°C until ready for use. qRTPCR for the second step of the two-step reaction was performed as stated above with the following cycling parameters: 1 cycle at 95°C for 1 minute; 40 cycles of 95°C for 15 seconds, 58°C for 30 seconds. qRTPCR for the one-step reaction was performed exactly as stated in the preceding paragraph. Both the one-step and two-step reactions were performed on 100ng of template (RNA/cDNA). After the PCR reaction was completed baseline and threshold values were set in the ABI 7900HT Prism software and calculated Ct values were exported to Microsoft Excel.

*Algorithm development.* Linear discriminators were constructed using the MASS (Venables and Ripley) library function 'idea' in the R language (version 2.1.1). The model used is dependent on the tissue from which the metastasis was extracted from, as well as the gender of the patient. When a lung, colon, or ovarian site of metastasis is encountered, the class prior is set to zero for the class that is equivalent to the site of metastasis. Furthermore, the prior odds are set to zero for the breast and ovary class in male patients, whilst in female patients, the prostate class' prior is set to zero. All other prior odds used in the models are equivalent. Furthermore classification for each sample is based on the highest posterior probability determined by the model for each class. To estimate the models performance, leave-one-out cross-validation was performed. In addition to this, the data sets were randomly split in halves, while preserving the proportional relationship between each class, into training and testing sets. This random splitting was repeated three times.

**Results**. The goal of this study was to develop a qRTPCR assay to predict metastatic carcinoma tissue of origin. The experimental work consisted of two major parts. The first part included tissue-specific Marker candidates nomination, their validation on FFPE metastatic carcinoma tissues, and selection of ten Markers for the assay (Figure 9A.). The second part included qRTPCR assay optimization followed by assay implementation on another set of FFPE metastatic carcinomas, building of a prediction algorithm, its cross-validation and validation on an independent sample set. (Figure 9B).

*Sample characteristics.* RNA from a total of 700 frozen primary tissue samples was used for the gene expression profiling and tissue type specific gene identification. Samples included 545 primary carcinomas (29 lung, 13 pancreas, 315 breast, 128 colorectal, 38 prostate, 22 ovarian), 37 benign lesions (1 lung, 4 colorectal, 6 breast, 26 prostate) and 118 (36 lung, 5 pancreas, 36 colorectal, 14 breast, 3 prostate, 24 ovarian) normal tissues.

A total of 375 metastatic carcinomas of known origin (Stage III-IV) and 26 prostate primary adenocarcinoma samples were used in the study. The metastatic carcinomas originated from lung, pancreas, colorectal, ovarian, prostate as well as other cancers. The "other" sample category consisted of metastasis derived from tissues other than lung, pancreas, colon, breast, ovary and prostate. Patients' characteristics are summarized in Table 18.

Table 18

| | | Metastatic CUP | Sample Set |
|---|---|---|---|
| Total Number | | 401 | 48 |
| Average Age | | 57.8 ± 11* | 62.13 ± 11.7 |
| Gender | Female | 241 | 20 |
| | Male | 160 | 28 |
| Tissue of Origin | | | |
| Lung | | 65 | 9 |
| Pancreas | | 63 | 2 |
| Colorectal | | 61 | 4 |
| Breast | | 63 | 5 |
| Ovarian | | 82 | 2 |
| Prostate | | 27 | 2 |
| Kidney | | 8 | 8 |
| Stomach | | 7 | 0 |
| Other** | | 25 | 5 |
| Carcinoma of Unknown Primary | | | 11 |

(continued)

| | Metastatic CUP | Sample Set |
|---|---|---|
| Histopathological Diagnosis | | |
| Adenocarcinoma, moderately/well differentiated | 306 | 27 |
| Adenocarcinoma, poorly differentiated | 49 | 4 |
| Squamous cell carcinoma | 16 | 5 |
| Poorly differentiated carcinoma | 16 | 10 |
| Small cell carcinoma | 3 | |
| Melanoma | 5 | |
| Lymphoma | 3 | |
| Hepatocellular carcinoma | 2 | |
| Mesothelioma | 1 | |
| Other*** | 14 | 2 |
| Metastatic Site | | |
| Lymph Nodes | 73 | 1 |
| Brain | 17 | 14 |
| Lung | 20 | 7 |
| Liver | 75 | 11 |
| Pelvic region (ovary, bladder, fallopian tubes) | 53 | 2 |
| Abdomen (Omentum (omentum, mesentery, colon, peritoneum) | 91 | 5 |
| Other (skin, thyroid, chest wall, umbilicus) | 44 | 8 |
| Unknown | 2 | |
| Primary (prostate) | 26 | |

* Age is unknown for 26 patients
**esophagus, bladder, pleura, liver gallbladder, bile ducts, larynx, pharynx, Non-Hodgkin lymphoma
***small cell, mesothelioma, hepatocellular, melanoma, lymphoma

Samples were separated into two sets: the validation set (205 specimens) that was used to validate Marker candidates' tissue-specific differential expression and the training set (260 specimens) that was used for testing of the optimized one-step qRTPCR procedure and training of a prediction algorithm. The first set of 205 samples included 25 lung, 41 pancreas, 31 colorectal, 33 breast, 33 ovarian, 1 prostate, 23 other cancer metastasis and 18 prostate primary cancers. The second set consisted of 260 samples included 56 lung, 43 pancreas, 30 colorectal, 30 breast, 49 ovarian, 32 other cancer metastasis and 20 primary prostate cancers. Sixty-four specimens, including 16 lung, 21 pancreas, 15 other metastatic, and 12 prostate primary carcinomas were from the same patient in both sets.

The independent sample set obtained from Albany Medical College was comprised of 33 CUP specimens with a primary suggested for 22 of them, and 15 metastatic carcinomas of known origin. For CUPs having a suggested primary, a diagnosis was rendered based on morphological features, and/or results of testing with a panel of IHC Markers. Patient demographic, clinical and pathology characteristics are presented in Table 18.

*Marker candidate selection.* Analysis of gene expression profiles of 5 primary tissues types (lung, colon, breast, ovary, prostate) resulted in nomination of 13 tissue specific Marker candidates for qRTPCR testing. Top candidates have been identified in previous studies of cancers in situ. Argani et al. (2001); Backus et al. (2005); Cunha et al. (2005); Borgono et al. (2004); McCarthy et al. (2003); Hwang et al. (2004); Fleming et al. (2000); Nakamura et al. (2002); and Khoor et al. (1997). In addition to the analysis of the microarray data, two Markers were selected from the literature, including a complementary lung squamous cell carcinoma Marker DSG3 and the breast Marker PDEF. Backus et al. (2005). The microarray data confirmed the high sensitivity and specificity of these Markers.

A special approach was used to identify pancreas specific Markers. First, five pancreas Marker candidates were analyzed: prostate stem cell antigen (PSCA), serine proteinase inhibitor, clade A member 1 (SERPINA1), cytokeratin 7 (KRT7), matrix metalloprotease 11 (MMP11), and mucin 4 (MUC4) (Varadhachary et al. (2004); Argani et al. (2001); Jones et al. (2004); Prasad et al. (2005); and Moniaux et al. (2004)) using DNA microarrays and a panel of 13 pancreatic ductal adenocarcinomas, five normal pancreas tissues, and 98 samples from breast, colorectal, lung, and ovarian tumors. Only PSCA demonstrated moderate sensitivity (six out of thirteen or 46% of pancreatic tumors were detected) at a high specificity (91 out of 98 or 93% were correctly identified as not being of pancreatic origin). In contrast, KRT7, SERPINA1, MMP11, and MUC4 demonstrated sensitivities of 38%, 31%, 85%, and 31%, respectively, at specificities of 66%, 91%, 82%, and 81%, respectively. These data were in good agreement with qRTPCR performed on 27 metastases of pancreatic origin and 39 metastases of non-pancreatic origin for all Markers except for MMP11 which showed poorer sensitivity and specificity with qRTPCR and the metastases. In conclusion, the microarray data on snap frozen, primary tissue serves as a good indicator of the ability of the Marker to identify a FFPE metastasis as being pancreatic in origin using qRTPCR but that additional Markers may be useful for optimal performance.

Pancreatic ductal adenocarcinoma develops from ductal epithelial cells that comprise only a small percentage of all pancreatic cells (with acinar and islet cells comprising the majority) in the normal pancreas. Furthermore, pancreatic adenocarcinoma tissues contain a significant amount of adjacent normal tissue. Prasad et al. (2005); and Ishikawa et al. (2005). Because of this the candidate pancreas Markers were enriched for genes elevated in pancreas adenocarcinoma relative to normal pancreas cells. The first query method returned six probe sets: coagulation factor V (F5), a hypothetical protein FLJ22041 similar to FK506 binding proteins (FKBP10), beta 6 integrin (ITGB6), transglutaminase 2 (TGM2), heterogeneous nuclear ribonucleoprotein A0 (HNRP0), and BAX delta (BAX). The second query method (see Materials and Methods section for details) returned eight probe sets: F5, TGM2, paired-like homeodomain transcription factor 1 (PITX1), trio isoform mRNA (TRIO), mRNA for p73H (p73), an unknown protein for MGC:10264 (SCD), and two probe sets for claudin18.

A total of 23 tissue specific Marker candidates were selected for further RT-PCR validation on metastatic carcinoma FFPE tissues by qRT-PCR. Marker candidates were tested on 205 FFPE metastatic carcinomas, from lung, pancreas, colon, breast, ovary, prostate and prostate primary carcinomas. Table 19 provides the gene symbols of the tissue specific Markers selected for RT-PCR validation and also summarizes the results of testing performed with these Markers.

Table 19

| Tissue type | SEQ ID NOs | ID method | | Marker selection filters | | | Marker adequate? |
|---|---|---|---|---|---|---|---|
| | | Micro array | Lit | Low exp corres met tissue | Marker redundancy | Tissue cross reactivity | |
| Lung | 1/59 | X | X | | | | X |
| | 60 | X | X | | | | X |
| | 61 | | X | | X | | X |
| Pancreas | 66 | | X | | | | X |
| | 67 | X | | | | | X |
| | 71 | X | | | X | | |
| | 72 | X | | X | | | |
| | 73 | | X | | | | |
| | 74 | | X | | | | |
| | 75 | | X | | | | |
| | 76 | | X | | | | |
| Colon | 4/85 | X | X | | | | X |
| | 77 | X | X | | | | |
| | 78 | X | X | | X | | |
| | 79 | X | X | | X | | |
| Prostate | 9/86 | X | X | | | | X |
| | 80 | X | X | | X | | |
| Breast | 63 | X | X | | | | X |
| | 81 | X | X | | | X | |
| | 64 | | X | | | | X |
| Ovarian | 82 | X | X | | | X | |
| | 83 | X | X | | | X | |

(continued)

| Tissue type | SEQ ID NOs | ID method | | Marker selection filters | | | Marker adequate? |
|---|---|---|---|---|---|---|---|
| | | Micro array | Lit | Low exp corres met tissue | Marker redundancy | Tissue cross reactivity | |
| | 65 | X | X | | | | X |

Out of 23 tested Markers, thirteen were rejected based on their cross reactivity, low expression level in the corresponding metastatic tissues, or redundancy. Ten Markers were selected for the final version of assay. The lung Markers were human surfactant pulmonary-associated protein B (HUMPSPB), thyroid transcription factor 1 (TTF1), and desmoglein 3 (DSG3). The pancreas Markers were prostate stem cell antigen (PSCA) and coagulation factor V (F5), and the prostate Marker was kallikrein 3 (KLK3). The colorectal Marker was cadherin 17 (CDH17). Breast Markers were mammaglobin (MG) and prostate-derived Ets transcription factor (PDEF). The ovarian Marker was Wilms tumor 1 (WT1). Mean normalized relative expression values of selected Markers in different metastatic tissues are presented on Figure 10.

*Optimization of sample preparation and qRT-PCR using FFPE tissues.* Next the RNA isolation and qRTPCR methods were optimized using fixed tissues before examining the performance of the Marker panel. First the effect of reducing the proteinase K incubation time from sixteen hours to 3 hours was analyzed. There was no effect on yield. However, some samples showed longer fragments of RNA when the shorter proteinase K step was used (Figure 11A, B). For example, when RNA was isolated from a one-year-old block (C22), no difference was observed in the electropherograms. However, when RNA was isolated from a five-year-old block (C23), a larger fraction of higher molecular weight RNAs were observed, as assessed by the hump in the shoulder, when the shorter proteinase K digest was used. This trend generally held when other samples were processed, regardless of the organ of origin for the FFPE metastasis. In conclusion, shortening the proteinase K digestion time does not sacrifice RNA yields and may aid in isolating longer, less degraded RNA.

Next three different methods of reverse transcription were compared: reverse transcription with random hexamers followed by qPCR (two step), reverse transcription with a gene-specific primer followed by qPCR (two step), and a one-step qRTPCR using gene-specific primers. RNA was isolated from eleven metastases and compared Ct values across the three methods for β-actin, HUMSPB (Figure 11C, D) and TTF. The results showed statistically significant differences ($p < 0.001$) for all comparisons. For both genes, the reverse transcription with random hexamers followed by qPCR (two step reaction) gave the highest Ct values while the reverse transcription with a gene-specific primer followed by qPCR (two-step reaction) gave slightly (but statistically significant) lower Ct values than the corresponding 1 step reaction. However, the two-step RTPCR with gene-specific primers had a longer reverse transcription step. When HUMSPB Ct values were normalized to the corresponding β-action value for each sample, there were no differences in the normalized Ct values across the three methods. In conclusion, optimization of the RTPCR reaction conditions can generate lower Ct values, which aids in analyzing older paraffin blocks (Cronin et al. (2004)), and a one step RTPCR reaction with gene-specific primers can generate Ct values comparable to those generated in the corresponding two step reaction.

*Diagnostic performance of optimized qRTPCR assay.* 12 qRTPCR reactions (10 Markers and 2 housekeeping genes) were performed on new set of 260 FFPE metastases. Twenty-one samples gave high Ct values for the housekeeping genes so only 239 were used in a heat map analysis. Analysis of the normalized Ct values in a heat map revealed the high specificity of the breast and prostate Markers, moderate specificity of the colon, lung, and ovarian, and somewhat lower specificity of the pancreas Markers (Figure 12). Combining the normalized qRTPCR data with computational refinement improves performance of the Marker panel.

Using expression values, normalized to average of expression of two housekeeping genes, an algorithm to predict metastasis tissue of origin was developed by combining the normalized qRTPCR data with the algorithm and determined the accuracy of the qRTPCR assay by performing a leave-one-out-cross-validation test (LOOCV). For the six tissue types included in the assay, it was separately estimated that both the number of false-positive calls, when a sample was wrongly predicted as another tumor type included in the assay (pancreas as colon, for example), and the number of times a sample was not predicted as those included in the assay tissue types (other). Results of the LOOCV are presented on Table 20.

Table 20

| | Tissue of Origin | | | | | | | |
|---|---|---|---|---|---|---|---|---|
| Prediction | Breast | Colon | Lung | Ovary | Pancreas | Prostate | Other | Total |
| Breast | 22 | 0 | 2 | 1 | 1 | 0 | 0 | |

(continued)

| Tissue of Origin | | | | | | | | |
|---|---|---|---|---|---|---|---|---|
| Prediction | Breast | Colon | Lung | Ovary | Pancreas | Prostate | Other | Total |
| Colon | 1 | 27 | 3 | 2 | 4 | 0 | 4 | |
| Lung | 1 | 2 | 45 | 2 | 3 | 0 | 5 | |
| Other | 1 | 1 | 3 | 1 | 4 | 0 | 16 | |
| Ovary | 5 | 0 | 0 | 43 | 0 | 0 | 1 | |
| Pancreas | 0 | 0 | 3 | 0 | 31 | 0 | 6 | |
| Prostate | 0 | 0 | 0 | 0 | 0 | 20 | 0 | |
| | | | | | | | | |
| Total | 30 | 30 | 56 | 49 | 43 | 20 | 32 | 260 |
| # Correct | 22 | 27 | 45 | 43 | 31 | 20 | 16 | 204 |
| Accuracy | 72.3 | 90.0 | 87.8 | 87.8 | 72.1 | 100.0 | 50.0 | 78.5 |

The tissue of origin was predicted correctly for 204 out of 260 tested samples with an overall accuracy of 78%. A significant proportion of the false positive calls were due to the Markers' cross-reactivity in histologically similar tissues. For example, three squamous cell metastatic carcinomas originated from pharynx, larynx and esophagus were wrongly predicted as lung due to DSG3 expression in these tissues. Positive expression of CDH17 in other than colon GI carcinomas, including stomach and pancreas, caused false classification of 4 out of 6 tested stomach and 3 out of 43 tested pancreatic cancer metastasis as colon.

In addition to a LOOCV test, the data was randomly split into 3 separate pairs of training and test sets. Each split contained approximately 50% of the samples from each class. At 50/50 splits in three separate pairs of training and test sets, assay overall classification accuracies were 77%, 71% and 75%, confirming assay performance stability.

Last, another independent set of 48 FFPE metastatic carcinomas that included metastatic carcinoma of known primary, CUP specimens with a tissue of origin diagnosis rendered by pathological evaluation including IHC, and CUP specimens that remained CUP after IHC testing were tested. The tissue of origin prediction accuracy was estimated separately for each category of samples. Table 21 summarizes the assay results.

Table 21

| | Tested | correct | Accuracy |
|---|---|---|---|
| Known mets | 15 | 11 | 73.3 |
| Resolved CUP | 22 | 17 | 77.3 |
| Unresolved CUP | 11 | | |

The tissue of origin prediction was, with only a few exceptions, consistent with the known primary or tissue of origin diagnosis assessed by clinical/pathological evaluation including IHC. Similar to the training set, the assay was not able to differentiate squamous cell carcinomas originating from different sources and falsely predicted them as lung.

The assay also made putative tissue of origin diagnoses for eight out of eleven samples which remained CUP after standard diagnostic tests. One of the CUP cases was especially interesting. A male patient with a history of prostate cancer was diagnosed with metastatic carcinoma in lung and pleura. Serum PSA tests and IHC with PSA antibodies on metastatic tissue were negative, so the pathologist's diagnosis was CUP with an inclination toward gastrointestinal tumors. The assay strongly (posterior probability 0.99) predicted the tissue of origin as colon. **Discussion.** In this study, microarray-based expression profiling on primary tumors was used to identify candidate Markers for use with metastases. The fact that primary tumors can be used to discover tumor of origin Markers for metastases is consistent with several recent findings. For example, Weigelt and colleagues have shown that gene expression profiles of primary breast tumors are maintained in distant metastases. Weigelt et al. (2003). Backus and colleagues identified putative Markers for detecting breast cancer metastasis using a genome-wide gene expression analysis of breast and other tissues and demonstrated that mammaglobin and CK19 detected clinically actionable metastasis in breast sentinel lymph nodes with 90% sensitivity and 94% specificity. Backus et al. (2005).

During the development of the assay, selection was focused on six cancer types, including lung, pancreas and colon which are among the most prevalent in CUP (Ghosh et al. (2005); and Pavlidis et al. (2005)) and breast, ovarian and prostate for which treatment could be potentially most beneficial for patients. Ghosh et al. (2005). However, additional tissue types and Markers can be added to the panel as long as the overall accuracy of the assay is not compromised and, if applicable, the logistics of the RTPCR reactions are not encumbered.

The microarray-based studies with primary tissue confirmed the specificity and sensitivity of known Markers. As a result, the majority of tissue specific Markers have high specificity for the tissues studied here. A recent study found that, using IHC, PSCA is overexpressed in prostate cancer metastases. Lam et al. (2005). Dennis et al. (2002) also demonstrated that PSCA could be used as a tumor of origin Marker for pancreas and prostate. Strong expression of PSCA in some prostate tissues at the RNA level was present but, because due to inclusion of PSA in the assay, prostate and pancreatic cancers can now be segregated. A novel finding of this study was the use of F5 as a complementary (to PSCA) Marker for pancreatic tissue of origin. In both the microarray data set with primary tissue and the qRTPCR data set with FFPE metastases, F5 was found to complement PSCA.

Previous investigators have generated CUP assays using IHC (Brown et al. (1997); DeYoung et al. (2000); and Dennis et al. (2005a)) or microarrays. Su et al. (2001); Ramaswamy et al. (2001); and Bloom et al. (2004). More recently, SAGE has been coupled to a small qRTPCR Marker panel. Dennis et al. (2002); and Buckhaults et al. (2003). This study is the first to combine microarray-based expression profiling with a small panel of qRTPCR assays. The microarray studies with primary tissue identified some, but not all, of the same tissue of origin Markers as those identified previously by SAGE studies. This finding is not surprising given studies that have demonstrated that a modest agreement between SAGE- and DNA microarray-based profiling data exists and that the correlation improves for genes with higher expression levels. van Ruissen et al. (2005); and Kim et al. (2003). For example, Dennis and colleagues identified PSA, MG, PSCA, and HUMSPB while Buckhaults and coworkers (Buckhaults et al. (2003)) identified PDEF.

Execution of the CUP assay is preferably by qRTPCR because it is a robust technology and may have performance advantages over IHC. Al-Mulla et al. (2005); and Haas et al. (2005). Further, as shown herein, the qRTPCR protocol has been improved through the use of gene-specific primers in a one-step reaction. This is the first demonstration of the use of gene-specific primers in a one-step qRTPCR reaction with FFPE tissue. Other investigators have either done a two-step qRTPCR (cDNA synthesis in one reaction followed by qPCR) or have used random hexamers or truncated gene-specific primers. Abrahamsen et al. (2003); Specht et al. (2001); Godfrey et al. (2000); Cronin et al. (2004); and Mikhitarian et al. (2004).

In summary, the 78% overall accuracy of the assay for six tissue types compares favorably to other studies. Brown et al. (1997); DeYoung et al. (2000); Dennis et al. (2005a); Su et al. (2001); Ramaswamy et al. (2001); and Bloom et al. (2004).

**Example 7**

In this study classifier using gene marker portfolios were built by choosing from MVO and using this classifier to predict tissue origin and cancer status for five major cancer types including breast, colon, lung, ovarian and prostate. Three hundred and seventy eight primary cancer, 23 benign proliferative epithelial lesions and 103 normal snap-frozen human tissue specimens were analyzed by using Affymetrix human U133A GeneChip. Leukocyte samples were also analyzed in order to subtract gene expression potentially masked by co-expression in leukocyte background cells. A novel MVO-based bioinformatics method was developed to select gene marker portfolios for tissue of origin and cancer status. The data demonstrated that a panel of 26 genes could be used as a classifier to accurately predict the tissue of origin and cancer status among the 5 cancer types. Thus a multi-cancer classification method is obtainable by determining gene expression profiles of a reasonably small number of gene markers. Table 22 shows the Markers identified for the tissue origins indicated. For gene descriptions see Table 31.

Table 22

| Tissue | SEQ ID NO: | Name |
|---|---|---|
| Lung | 59 | SP-B |
| | 60 | TTF1 |
| | 61 | DSG3 |
| Pancreas | 66 | PSCA |
| | 67 | F5 |
| | 71 | ITGB6 |

(continued)

| Tissue | SEQ ID NO: | Name |
|---|---|---|
| | 72 | TGM2 |
| | 84 | HNRPA0 |
| Colon | 85 | HPT1 |
| | 77 | FABP1 |
| | 78 | CDX1 |
| | 79 | GUCY2C |
| Prostate | 86 | PSA |
| | 80 | hKLK2 |
| Breast | 63 | MGB1 |
| | 81 | PIP |
| | 64 | PDEF |
| Ovarian | 82 | HE4 |
| | 83 | PAX8 |
| | 65 | WT1 |

The sample set included a total of 299 metastatic colon, breast, pancreas, ovary, prostate, lung and other carcinomas and primary prostate cancer samples. QC based on histological evaluation, RNA yield and expression of control gene beta-actin was implemented. Other samples category included metastasis originated from stomach (5), kidney (6), cholangio/gallbladder (4), liver (2), head and neck (4), ileum (1) carcinomas and one mesothelioma. Table 23 summarizes the results.

Table 23

| Tissue type | Collected | Histology QC | RNA isolation QC | ACTB Cut-off QC |
|---|---|---|---|---|
| Lung | 41 | 37 | 36 | 25 |
| Pancreas | 63 | 57 | 49 | 41 |
| Colon | 45 | 42 | 42 | 31 |
| Breast | 40 | 35 | 35 | 34 |
| Ovarian | 37 | 36 | 35 | 33 |
| Prostate | 27 | 27 | 25 | 19 |
| Other | 46 | 34 | 29 | 23 |
| Total | 299 | 268 | 251 | 205 |

Testing the above samples resulted in the narrowing of the Marker set to those in Table 24 with the results seen in Table 25.

Table 24

| Final Marker Table | | |
|---|---|---|
| Lung | surfactant-associated protein | SP-B |
| | thyroid transcription factor 1 | TTF1 |
| | desmoglein 3 | DSG3 |
| Pancreas | prostate stem cell antigen | PSCA |

(continued)

| Final Marker Table | | |
|---|---|---|
| | coagulation factor 5 | F5 |
| Colon | intestinal peptide-associated transporter | HPT1 |
| Prostate | prostate-specific antigen | PSA |
| Breast | Mammaglobin | MGB |
| | Ets transcription factor | PDEF |
| Ovary | Wilms tumor 1 | WT1 |

Table 25

| Cancer | Samples # | Marker | Correct | Sens % | Wrong | Spec % |
|---|---|---|---|---|---|---|
| Lung | 25/180 | SP-B | 13/25 | 52 | 0/180 | 100 |
| | | TTF | 12/25 | 48 | 1/180 | 99 |
| | | DSG3 | 5/25 | 20 | 0/180 | 100 |
| Pancreas | 41/164 | PSCA | 24/41 | 59 | 6/164 | 96 |
| | | F5 | 6/41 | 15 | 4/164 | 98 |
| Colon | 31/174 | HPT1 | 22/31 | 71 | 2/174 | 99 |
| Breast | 33/172 | MGB | 23/33 | 70 | 3/172 | 98 |
| | | PDEF | 16/3 | 48 | 1/172 | 99 |
| Prostate | 19/186 | PSA | 19/19 | 100 | 0/186 | 100 |
| | | PDEF | 19/19 | 100 | 2/186 | 99 |
| Ovarian | 33/172 | WT1 | 24/33 | 71 | 1/172 | 99 |
| Total | 205 | | | | | |

The results showed that out of 205 paraffin embedded metastatic tumors; 166 samples (81%) had conclusive assay results, Table 26.

Table 26

| | Candidate | Correct | Incorrect | No | Accuracy (%) |
|---|---|---|---|---|---|
| Lung | SP-B + TFF+DSG3 | 19 | 0 | 6 | 76 |
| Pancreas | PSCA+F5 | 27 | 1 | 13 | 66 |
| Colon | HPT1 | 24 | 2 | 5 | 78 |
| Prostate | PSA | 19 | 0 | 0 | 100 |
| Breast | MGB + PDEF | 23 | 3 | 7 | 70 |
| Ovarian | WT1 | 23 | 2 | 8 | 70 |
| Other | | 20 | 3 | | 87 |
| Overall | | 155 | 11 | 39 | 76 |

Of the false positive results, many false derived from histologically and embryologically similar tissues, Table 27.

Table 27

| Sample ID | Diagnosis | Predicted |
|---|---|---|
| OV_26 | Ovarian | Breast |
| Br_24 | Breast | Colon |
| Br_37 | Breast | Colon |
| CRC_25 | Colon | Ovarian |
| Pn_59 | Pancreas | Colon |
| Cont_27 | Stomach | pancreas |
| Cont_34 | Stomach | Colon |
| Cont_35 | Stomach | Colon |
| Cont_43 | Bile duct | Pancreas |
| Cont_44 | Bile duct | Pancreas |
| Cong_25 | Liver | pancreas |

The following parameters were considered for the model development:

Separate markers on female and male sets and calculate CUP probability separately for male and female patients. The male set included: SP_B, TTF1, DSG3, PSCA, F5, PSA, HPT1; the female set included: SP_B, TTF1, DSG3, PSCA, F5, HPT1, MGB, PDEF, WT1. Background expression was excluded from the assay results: Lung: SP_B, TTF1, DSG3; Ovary: WT1; and Colon: HPT1.

The CUP model was adjusted to the CUP prevalence (%): lung 23, pancreas 16, colorectal 9, breast 3, ovarian 4, prostate 2, other 43. The prevalence for breast and ovarian adjusted to 0% for male patients, and prostate adjusted to 0% for female patients.

The following steps were taken: place markers on similar scale; reduce number of variables from 12 to 8 by selecting minimum value from each tissue specific set; leave out 1 sample; build model from remaining samples; test left out sample; repeat until 100% of samples are tested randomly leave out ~50% of samples (~50% per tissue); build model from remaining samples; test ~50% of samples; and rRepeat for 3 different random splits.

Classification accuracy was adjusted to cancer types prevalence to produce the results summarized in Table 28 with the raw data shown in Table 29.

Table 28

| | Breast | Colon | Lung | Other | Ovary | Panc | prostate | Over all | Ad justed |
|---|---|---|---|---|---|---|---|---|---|
| Correct | 23 | 29 | 22 | 19 | 24 | 35 | 19 | 171 | |
| NoTest | 3 | 2 | 2 | | 2 | 3 | 0 | 12 | |
| Incorrect | 7 | 0 | 1 | 4 | 7 | 3 | 0 | 22 | |
| Prevalence | 0.03 | 0.09 | 0.23 | 0.43 | 0.04 | 0.16 | 0.02 | | |
| | | | | | | | | | |
| Tested/total% | 91 | 94 | 92 | 100 | 94 | 93 | 100 | 94 | 95 |
| Correct/total% | 70 | 94 | 88 | 83 | 73 | 85 | 100 | 89 | 89 |
| NoTest% | 9 | 6 | 8 | n/a | 6 | 7 | 0 | 6 | 5 |
| | | | | | | | | | |
| Correct | 23 | 25 | 19 | 20 | 20 | 24 | 19 | 150 | |
| NoTest% | 7 | 6 | 5 | | 10 | 15 | 0 | 43 | |
| Incorrect | 3 | 0 | 1 | 3 | 3 | 2 | 0 | 12 | |
| Prevalence | 0.03 | 0/09 | 0.23 | 0.43 | 0.04 | 0.16 | 0.02 | | |
| | | | | | | | | | |

(continued)

| | Breast | Colon | Lung | Other | Ovary | Panc | prostate | Over all | Ad justed |
|---|---|---|---|---|---|---|---|---|---|
| Tested/total% | 79 | 81 | 80 | 100 | 70 | 63 | 100 | 79 | 83 |
| Correct/total% | 70 | 81 | 76 | 87 | 61 | 59 | 100 | 73 | 76 |
| Correct/tested% | 88 | 100 | 95 | 87 | 87 | 92 | 100 | 93 | 91 |
| NoTest% | 21 | 19 | 20 | n/a | 30 | 37 | 0 | 21 | 17 |

Table 29

| Sample ID | Gender | Origin | BK | Prediction | BACTIN | PBGD | Ave | CDH17 | DSG3 | F5 | HUMP | KLK3 | MG | PDEF | PSCA | TTF1 | WT1 |
|---|---|---|---|---|---|---|---|---|---|---|---|---|---|---|---|---|---|
| 128 | f | breast | lung | other | 23.37 | 30.04 | 26.71 | 40.00 | 37.78 | 35.74 | 22.19 | 40.00 | 40.00 | 30.36 | 29.96 | 29.39 | 34.85 |
| 134 | f | breast | uk | breast | 19.60 | 27.00 | 23.30 | 40.00 | 31.27 | 30.83 | 40.00 | 40.00 | 29.51 | 25.07 | 24.67 | 40.00 | 34.13 |
| 166 | f | breast | uk | breast | 23.47 | 27.95 | 25.71 | 40.00 | 40.00 | 26.66 | 40.00 | 28.20 | 24.78 | 25.19 | 30.69 | 40.00 | 35.32 |
| 331 | f | breast | ovary | breast | 25.12 | 31.40 | 28.26 | 40.00 | 40.00 | 40.00 | 40.00 | 40.00 | 22.26 | 26.01 | 40.00 | 40.00 | 40.00 |
| 356 | f | breast | uk | breast | 28.59 | 33.89 | 31.24 | 40.00 | 34.01 | 40.00 | 40.00 | 40.00 | 35.73 | 33.19 | 30.72 | 40.00 | 40.00 |
| 163 | f | colon | uk | colon | 24.69 | 30.34 | 27.52 | 29.39 | 40.00 | 26.52 | 40.00 | 40.00 | 40.00 | 37.72 | 40.00 | 40.00 | 36.17 |
| 184 | m | colon | uk | colon | 22.47 | 28.63 | 25.55 | 26.22 | 33.26 | 28.76 | 40.00 | 40.00 | 40.00 | 34.07 | 33.44 | 40.00 | 31.64 |
| 339 | f | colon | uk | colon | 28.35 | 34.29 | 31.32 | 33.76 | 40.00 | 40.00 | 40.00 | 40.00 | 40.00 | 35.99 | 40.00 | 40.00 | 40.00 |
| 346 | m | colon | lung | colon | 23.15 | 28.77 | 25.96 | 26.36 | 40.00 | 32.64 | 20.89 | 40.00 | 40.00 | 32.47 | 40.00 | 26.75 | 30.58 |
| 363 | m | colon | uk | colon | 24.46 | 30.62 | 27.54 | 26.20 | 31.84 | 29.98 | 34.44 | 40.00 | 40.00 | 30.45 | 35.00 | 40.00 | 30.35 |
| 101 | m | lung | uk | lung | 24.68 | 28.79 | 26.74 | 40.00 | 40.00 | 39.34 | 21.57 | 40.00 | 40.00 | 28.21 | 27.47 | 40.00 | 35.76 |
| 106 | m | lung | uk | lung | 22.05 | 27.50 | 24.78 | 40.00 | 40.00 | 32.24 | 23.68 | 40.00 | 40.00 | 25.79 | 25.02 | 26.42 | 37.27 |
| 110 | m | lung | uk | lung | 29.19 | 32.32 | 30.76 | 40.00 | 40.00 | 40.00 | 21.21 | 40.00 | 40.00 | 32.77 | 32.43 | 30.70 | 36.13 |
| 112 | m | lung | uk | other | 22.48 | 27.79 | 25.14 | 40.00 | 37.05 | 37.38 | 36.08 | 40.00 | 40.00 | 37.12 | 36.04 | 40.00 | 37.45 |
| 199 | f | lung | uk | lung | 21.21 | 27.07 | 24.14 | 35.65 | 25.56 | 31.23 | 40.00 | 40.00 | 28.94 | 32.19 | 27.95 | 32.14 | 31.60 |
| 200 | m | lung | uk | lung | 22.16 | 26.94 | 24.55 | 40.00 | 24.53 | 33.69 | 40.00 | 40.00 | 40.00 | 36.67 | 38.34 | 38.61 | 33.55 |
| 313 | m | lung | uk | other | 24.76 | 30.05 | 27.41 | 38.40 | 40.00 | 40.00 | 40.00 | 40.00 | 40.00 | 40.00 | 40.00 | 40.00 | 35.11 |
| 323 | m | lung | uk | pancreas | 23.82 | 30.24 | 27.03 | 32.43 | 31.82 | 33.81 | 40.00 | 40.00 | 40.00 | 33.60 | 28.12 | 40.00 | 31.87 |
| 325 | m | lung | uk | lung | 22.09 | 27.97 | 25.03 | 40.00 | 26.84 | 34.88 | 38.61 | 40.00 | 38.04 | 34.29 | 27.31 | 39.21 | 31.23 |
| 335 | m | lung | uk | other | 24.89 | 29.73 | 27.31 | 40.00 | 29.62 | 38.00 | 40.00 | 40.00 | 40.00 | 39.23 | 40.00 | 31.12 | 32.12 |
| 347 | m | lung | uk | lung | 23.40 | 29.08 | 26.24 | 40.00 | 26.72 | 37.21 | 40.00 | 40.00 | 40.00 | 36.10 | 30.76 | 40.00 | 39.44 |
| 374 | m | lung | uk | lung | 22.50 | 28.23 | 25.37 | 40.00 | 40.00 | 38.76 | 21.38 | 40.00 | 37.26 | 26.56 | 38.26 | 24.86 | 36.60 |
| 385 | f | lung | uk | lung | 21.65 | 26.44 | 24.05 | 37.05 | 40.00 | 34.51 | 19.89 | 40.00 | 40.00 | 27.36 | 40.00 | 23.72 | 37.09 |
| 114 | f | other | lung | other | 24.80 | 30.56 | 27.68 | 40.00 | 40.00 | 28.16 | 21.51 | 40.00 | 40.00 | 35.76 | 37.85 | 28.19 | 37.21 |
| 129 | m | other | lung | other | 21.49 | 28.25 | 24.87 | 39.47 | 40.00 | 28.86 | 20.65 | 40.00 | 40.00 | 32.98 | 40.00 | 28.14 | 31.11 |
| 179 | f | other | uk | other | 23.97 | 30.45 | 27.21 | 40.00 | 40.00 | 29.79 | 40.00 | 40.00 | 40.00 | 40.00 | 40.00 | 40.00 | 32.64 |
| 194 | m | other | uk | other | 25.28 | 32.47 | 28.88 | 40.00 | 40.00 | 28.90 | 40.00 | 40.00 | 40.00 | 40.00 | 40.00 | 34.75 | 35.41 |

| | | | | | | | | | | | | | | | | | |
|---|---|---|---|---|---|---|---|---|---|---|---|---|---|---|---|---|---|
| 302 | f | other | colon | breast | 25.67 | 31.47 | 28.57 | 34.17 | 40.00 | 40.00 | 40.00 | 40.00 | 40.00 | 30.55 | 32.47 | 40.00 | 38.20 |
| 305 | m | other | uk | other | 23.80 | 29.74 | 26.77 | 29.64 | 40.00 | 34.06 | 40.00 | 40.00 | 40.00 | 31.82 | 40.00 | 40.00 | 40.00 |
| 317 | m | other | uk | pancreas | 25.90 | 30.62 | 28.26 | 40.00 | 40.00 | 27.75 | 40.00 | 40.00 | 40.00 | 31.89 | 33.06 | 40.00 | 35.12 |
| 333 | f | other | uk | other | 22.45 | 28.82 | 25.64 | 30.54 | 40.00 | 37.01 | 40.00 | 40.00 | 40.00 | 37.85 | 40.00 | 40.00 | 40.00 |
| 334 | m | other | uk | other | 22.14 | 29.20 | 25.67 | 31.79 | 40.00 | 36.27 | 40.00 | 40.00 | 40.00 | 34.69 | 40.00 | 40.00 | 40.00 |
| 342 | f | other | uk | pancreas | 27.32 | 31.37 | 29.35 | 32.36 | 40.00 | 29.24 | 40.00 | 40.00 | 40.00 | 32.89 | 40.00 | 40.00 | 38.18 |
| 382 | m | other | uk | other | 25.04 | 30.22 | 27.63 | 40.00 | 40.00 | 36.13 | 40.00 | 40.00 | 40.00 | 38.30 | 40.00 | 40.00 | 34.91 |
| 404 | m | other | uk | other | 23.27 | 30.16 | 26.72 | 40.00 | 39.36 | 34.75 | 40.00 | 40.00 | 40.00 | 39.02 | 40.00 | 40.00 | 34.24 |
| 354 | f | ovary | uk | ovary | 24.62 | 31.54 | 28.08 | 40.00 | 40.00 | 34.90 | 40.00 | 40.00 | 40.00 | 36.62 | 40.00 | 40.00 | 29.71 |
| 148 | f | ovary | uk | pancreas | 23.55 | 29.88 | 26.72 | 40.00 | 40.00 | 30.60 | 38.84 | 40.00 | 40.00 | 32.12 | 31.76 | 40.00 | 38.59 |
| 417 | f | pancreas | uk | pancreas | 23.42 | 29.46 | 26.44 | 28.28 | 38.96 | 29.05 | 37.01 | 40.00 | 40.00 | 30.15 | 30.23 | 40.00 | 30.69 |
| 136 | m | prostate | lung | prostate | 22.37 | 26.95 | 24.66 | 40.00 | 40.00 | 29.47 | 23.69 | 21.38 | 40.00 | 24.70 | 24.28 | 30.89 | 31.16 |
| 407 | m | prostate | lung | prostate | 28.20 | 31.87 | 30.04 | 40.00 | 40.00 | 40.00 | 27.70 | 25.98 | 40.00 | 27.65 | 40.00 | 39.13 | 38.76 |
| 116 | f | CUP | uk | lungSCC | 21.66 | 27.31 | 24.49 | 28.95 | 27.86 | 31.06 | 40.00 | 40.00 | 30.28 | 33.49 | 29.31 | 40.00 | 38.11 |
| 123 | m | CUP | lung | colon | 27.09 | 30.59 | 28.84 | 27.92 | 36.01 | 40.00 | 40.00 | 40.00 | 40.00 | 40.00 | 40.00 | 40.00 | 36.65 |
| 157 | m | CUP | uk | pancreas | 26.81 | 31.94 | 29.38 | 40.00 | 40.00 | 26.82 | 40.00 | 40.00 | 40.00 | 36.68 | 40.00 | 40.00 | 40.00 |
| 177 | m | CUP | uk | pancreas | 25.44 | 31.52 | 28.48 | 40.00 | 40.00 | 27.15 | 40.00 | 40.00 | 40.00 | 39.67 | 40.00 | 40.00 | 34.71 |
| 306 | m | CUP | uk | lung | 23.15 | 28.38 | 25.77 | 37.30 | 40.00 | 34.94 | 19.71 | 40.00 | 40.00 | 30.81 | 40.00 | 25.45 | 39.28 |
| 360 | m | CUP | uk | other | 21.14 | 27.43 | 24.29 | 33.97 | 36.98 | 32.72 | 40.00 | 40.00 | 40.00 | 27.75 | 40.00 | 40.00 | 40.00 |
| 372 | f | CUP | uk | ovary | 23.16 | 29.12 | 26.14 | 40.00 | 40.00 | 34.07 | 40.00 | 40.00 | 40.00 | 32.93 | 40.00 | 40.00 | 25.28 |
| 187 | f | CUP | uk | colon | 24.44 | 29.80 | 27.12 | 26.83 | 35.91 | 26.32 | 30.55 | 40.00 | 40.00 | 40.00 | 40.00 | 29.75 | 40.00 |

**Example 8**

Prospective gene signature study of metastatic cancer of unknown primary site CUP to predict the tissue of origin

The specific aim of this study was to determine the ability of the 10-gene signature to predict tissue of origin of metastatic carcinoma in patients with carcinoma of unknown primary (CUP).

Primary objective: Confirm the feasibility of conducting gene analysis from core biopsy samples in consecutive patients with CUP.

Secondary objective: Correlate the results of the 10-gene signature RT-PCR assay with diagnostic work-up done at M.D. Anderson Cancer Center (MDACC). Third objective: Correlate prevalence of 6 cancer types predicted by assay with the prevalence derived from the literature and MDACC experience.

The method described herein was used to perform a microarray gene expression analysis of 700 frozen primary carcinoma, and benign and normal specimens and identified gene marker candidates, specific for lung, pancreas, colon, breast, prostate and ovarian carcinomas. Gene marker candidates were tested by RT-PCR on 205 formalin-fixed, paraffin-embedded (FFPE) specimens of metastatic carcinoma (Stage III-IV) originated from lung, pancreas, colon, breast, ovary and prostate as well as metastasis originated from other cancer types for specificity control. Other metastatic cancer types included gastric, renal cell, hepatocellular, cholangio/gallbladder and head and neck carcinomas. Results allowed selecting of 10-gene signature that predicted tissue of origin of metastatic carcinoma and gave an overall accuracy of 76%. The average CV for repeated measurements in RT-PCR experiments is 1.5%, calculated based on 4 replicate date points. Beta-actin (ACTB) was used as housekeeping gene and its median expression was the similar in metastatic samples of different origin (CV=5.6%).

Specific aim for this study was to validate the ability of 10-gene signature to predict metastatic carcinoma tissue of origin in the CUP patients compared to comprehensive diagnostic workup.

Patient Eligibility

Patient must be at least 18 years old with a ECOG performance status of 0-2. Patients with diagnosis adenocarcinoma or poorly differentiated carcinoma diagnosis were accepted. Adenocarcinoma patient's group include well, moderate and poor differentiated tumors.

Patients have fulfilled the criteria for CUP: no primary detected after a complete evaluation which is defined as complete history and physical examination, detailed laboratory examination, imaging studies and symptom or sign directed invasive studies. Only untreated patients were allowed on the study.

If a patient has been treated with chemotherapy or radiation, participation in the study is allowed if prior (to treatment) tissue is available as archived blocks within 10 years time period

Patients provided written consent/authorization to participate in this study.

Study Design

Patients with diagnosis of CUP who have undergone a core needle or excision biopsy of the most accessible metastatic lesion were allowed on the study. Patients with FNA biopsy only were not eligible. The first 60 consecutive presenting patients who met the inclusion criteria and consent to the study were enrolled. If repeated biopsy is required at MDACC for diagnostic purposes for their treatment, additional tissue was obtained for the study if patient consented. All participants were registered on the protocol in the institutional Protocol Data Management System (PDMS).

Complete diagnostic work-up, including clinical and pathological assessments, was performed on all enrolled patients according MDACC standards. Pathology part of diagnostic work-up may have included immunohistochemistry (IHC) assays with markers including CK-7, CK-20, TTF-1 and other as deemed indicated by the pathologist. This is part of routine work up of all patients who present with CUP.

Tissue sample collection

Study included formalin-fixed paraffin embedded metastatic carcinoma specimens collected from CUP patients.

Six 10 $\mu$m sections were used for RNA isolation, smaller tissue specimens will require nine 10 $\mu$m sections. Histopathology diagnosis and tumor content were confirmed for each sample used for RNA isolation on an additional section stained with hematoxylin and eosin (HE). The tumor sample should have had a greater than 30% of tumor content in the HE section.

Clinical data were anonymously supplied to Veridex and include patient age, gender, tumor histology by light microscopy, tumor grade (differentiation), site of metastasis, date of specimen collection, description of the diagnostic workup performed for individual patient.

Tissue processing and RT-PCR experiments

Total RNA was extracted from each tissue sample using the protocol described above. Only samples that yielded more than 1 μm of total RNA out of standard amount of tissue were used for subsequent RT-PCR testing. Samples with less RNA yield were considered degraded and excluded from subsequent experiments. RNA integrity control based on housekeeping expression were implemented in order to exclude samples with degraded RNA, according the standard Veridex procedure.

RT-PCR assay that includes panel of 10 genes and 1-2 control genes was used for the analysis of the RNA samples. The reverse transcription and the PCR assay are completed using the protocols described above.

Relative expression value for each tested gene presented as ΔCt, which is equal to Ct of the target gene subtracted by Ct of the control genes, was calculated and used for the tissue of origin prediction.

Sample size and Data interpretation

A limited sample size of 60 patients were studied due to the exploratory nature of the pilot study. Up to the date, 22 patients have been tested. One patient samples failed to yield enough RNA for RT-PCR test and 3 failed to pass QC control assessed by RT-PCR with control genes. A total of 18 patients were used for determine probability of patient's metastatic lesion.

The statistical model was used to determine probability of metastatic carcinoma tissue of origin of following seven categories: lung, pancreas, colon, breast, prostate, ovarian and no test (other). For each sample, the probability for each category are calculated from a linear classification model. Assay results are summarized in Table 30.

The probability of a patient's metastatic lesion (with known primaries) coming from one of these 6 sites (colon, pancreas, lung, prostate, ovary, breast) is about 76%. This number is derived from literature given the incidence of various cancers and potential for spread and unpublished data generated at M.D. Anderson from tumor registry. For the tested samples, prevalence of 6 sites was 67% (12 out 18 tested samples), which very close consistent with previous observations.

Table 30

| Patiient datta | | | ToO posterior probability (%) | | | | | | | |
|---|---|---|---|---|---|---|---|---|---|---|
| ID | M/F | prediction | Breast | Colon | Lung | LungSCC | Other | Ovary | Pancreas | prostate |
| 1 | M | Other | 0.00 | 0.00 | 0.81 | 0.00 | 98.68 | 0.00 | 0.51 | 0.00 |
| 4 | F | Colon | 0.00 | 99.70 | 0.00 | 0.00 | 0.09 | 0.20 | 0.01 | 0.00 |
| 5 | M | Lung | 0.00 | 33.29 | 52.27 | 0.01 | 13.30 | 0.00 | 1.13 | 0.00 |
| 6 | F | Colon | 0.00 | 99.91 | 0.00 | 0.00 | 0.09 | 0.00 | 0.00 | 0.00 |
| 2 | M | Colon | 0.00 | 93.19 | 0.01 | 0.00 | 2.90 | 0.00 | 3.90 | 0.00 |
| 10 | F | Other | 0.02 | 2.04 | 0.03 | 0.03 | 61.43 | 1.12 | 35.34 | 0.00 |
| 16 | F | Colon | 0.00 | 48.59 | 0.01 | 1.57 | 47.62 | 0.17 | 2.05 | 0.00 |
| 22 | M | LungSCC | 0.00 | 8.85 | 0.01 | 71.69 | 11.84 | 0.00 | 7.62 | 0.00 |
| 23 | M | Colon | 0.00 | 99.27 | 0.01 | 0.00 | 0.72 | 0.00 | 0.00 | 0.00 |
| 24 | F | Colon | 0.00 | 90.59 | 0.00 | 0.00 | 2.36 | 0.00 | 7.04 | 0.00 |
| 26 | F | Lung | 0.00 | 0.00 | 99.93 | 0.00 | 0.06 | 0.00 | 0.01 | 0.00 |
| 17 | M | Other | 0.00 | 0.07 | 0.02 | 0.09 | 94.06 | 0.00 | 5.77 | 0.00 |
| 19 | F | Other | 0.02 | 0.11 | 0.04 | 0.22 | 76.36 | 23.24 | 0.01 | 0.00 |
| 21 | F | Pancreas | 0.00 | 6.97 | 0.00 | 0.00 | 2.37 | 8.43 | 82.23 | 0.00 |
| 27 | F | Other | 0.00 | 0.04 | 0.04 | 0.59 | 99.06 | 0.14 | 0.13 | 0.00 |
| 11 | M | Other | 0.00 | 0.23 | 0.07 | 0.09 | 99.52 | 0.00 | 0.09 | 0.00 |
| 32 | F | Ovary | 0.00 | 0.01 | 0.00 | 0.00 | 7.23 | 92.63 | 0.13 | 0.00 |
| 34 | M | LungSCC | 0.00 | 0.03 | 0.00 | 65.64 | 7.96 | 0.00 | 26.38 | 0.00 |

(continued)

| Patiient datta | | | ToO posterior probability (%) | | | | | | | |
|---|---|---|---|---|---|---|---|---|---|---|
| ID | M/F | prediction | Breast | Colon | Lung | LungSCC | Other | Ovary | Pancreas | prostate |
| 3 | F | ctr failure | 0.00 | 0.00 | 0.00 | 0.00 | 0.00 | 0.00 | 0.00 | 0.00 |
| 8 | M | ctr failure | 0.00 | 0.00 | 0.00 | 0.00 | 0.00 | 0.00 | 0.00 | 0.00 |
| 20 | F | ctr failure | 0.00 | 0.00 | 0.00 | 0.00 | 0.00 | 0.00 | 0.00 | 0.00 |

Although the foregoing invention has been described in some detail by way of illustration and example for purposes of clarity of understanding, the descriptions and examples should not be construed as limiting the scope of the invention.

Table 31

| Name | SEQ ID NOs | Accession | Description |
|---|---|---|---|
| CDH17 | 62 | NM_004063 | Cadherin 17 |
| CDX1 | 78 | NM_001804 | Homeo box transcription factor 1 |
| DSG3 | 61/3 | NM_001944 | Desmoglein 3 |
| F5 | 67/6 | NM_000130 | Coagulation factor V |
| FABP1 | 71 | NM_001443 | Fatty acid binding protein 1, liver |
| GUCY2C | 79 | NM_004963 | Guanylate cyclase 2C |
| HE4 | 82 | NM_006103 | Putative ovarian carcinoma marker |
| KLK2 | 80 | BC005196 | Kallikrein 2, prostatic |
| HNRPA0 | 84 | NM_006805 | Heterogeneous nuclear ribonucleoprotein A0 |
| HPT1 | 85/4 | U07969 | Intestinal peptide-associated transporter |
| ITGB6 | 71 | NM_000888 | Integrin, beta 6 |
| KLK3 | 68 | NM_001648 | Kallikrein 3 |
| MGB1 | 63/7 | NM_002411 | Mammaglobin 1 |
| PAX8 | 83 | BC001060 | Paired box gene 8 |
| PBGD | 70 | NM_000190 | Hydroxymethylbilane synthase |
| PDEF | 64/8 | NM_012391 | Domain containing Ets transcription factor |
| PIP | 81 | NM_002652 | Prolactin-induced protein |
| PSA | 86/9 | U17040 | Prostate specific antigen precursor |
| PSCA | 66/5 | NM_005672 | Prostate stem cell antigen |
| SP-B | 59/1 | NM_198843 | Pulmonary surfactant-associated protein B |
| TGM2 | 72 | NM_004613 | Transglutaminase 2 |
| TTF1 | 60/2 | NM_003317 | Similar to thyroid transcription factor 1 |
| WT1 | 65/10 | NM_024426 | Wilms tumor 1 |
| β-actin | 69 | NM_001101 | β-actin |
| KRT6F | 87 | L42612 | keratin 6 isoform K6f |
| p73H | 88 | AB010153 | p53-related protein |
| SFTPC | 89 | NM_003018 | surfactant, pulmonary-associated protein C |
| KLK10 | 90 | NM_002776 | Kallikrein 10 |

(continued)

| Name | SEQ ID NOs | Accession | Description |
|---|---|---|---|
| CLDN18 | 91 | NM_016369 | Claudin 18 |
| TR10 | 92 | BD280579 | Tumor necrosis factor receptor |
| B305D | 93 | | |
| B726 | 94 | | |
| GABA-pi | 95 | BC109105 | gamma-aminobutyric acid A receptor, pi |
| StAR | 96 | NM_01007243 | steroidogenic acute regulator |
| EMX2 | 97 | NM_004098 | empty spiracles homolog 2 (Drosophila) |
| NGEP | 98 | AY617079 | NGEP long variant |
| NPY | 99 | NM_000905 | Neuropeptide Y |
| SERPINA1 | 100 | NM_000295 | serpin peptidase inhibitor, clade A member 1 |
| KRT7 | 101 | NM_005556 | Keratin 7 |
| MMP11 | 102 | NM_005940 | matrix metallopeptidase 11 (stromelysin 3) |
| MUC4 | 103 | NM_018406 | Mucin 4 cell-surface associated |
| FLJ22041 | 104 | AK025694 | |
| BAX | 105 | NM_138763 | BCL2-assoc X protein transcript variant Δ |
| PITX1 | 106 | NM_002653 | paired-like homeodomain trans factor 1 |
| MGC:10264 | 107 | BC005807 | stearoyl-CoA desaturase (Δ-9-desaturase) |

REFERENCES

US patent application publications and patents


| | | |
|---|---|---|
| 5242974 | 5700637 | 20030194733 |
| 5350840 | 5786148 | 20030198970 |
| 5384261 | 6004755 | 20030215803 |
| 5405783 | 6136182 | 20030215835 |
| 5412087 | 6218114 | 20030219760 |
| 5424186 | 6218122 | 20030219767 |
| 5429807 | 6225051 | 20030232350 |
| 5436327 | 6232073 | 20030235820 |
| 5445934 | 6261766 | 20040005563 |
| 5472672 | 6271002 | 20040009154 |
| 5527681 | 6339148 | 20040009489 |
| 5529756 | 20010029020 | 20040018969 |
| 5532128 | 20020055627 | 20040029114 |
| 5545531 | 20020068288 | 20040076955 |
| 5554501 | 20020168647 | 20040126808 |
| 5556752 | 20030044859 | 20040146862 |
| 5561071 | 20030087818 | 20040219572 |
| 5571639 | 20030104448 | 20040219575 |
| 5593839 | 20030124128 | 20050037010 |
| 5599695 | 20030124579 | 20050059008 |
| 5624711 | 20030138793 | 20060094035 |
| 5658734 | 20030190656 | |

Foreign patent publications and patents

| | | |
|---|---|---|
| WO1998040403 | WO2001073032 | WO2004030615 |
| WO1998056953 | WO2002046467 | WO2004031412 |
| WO2000006589 | WO2002073204 | WO2004063355 |
| WO2000055320 | WO2002101357 | WO2004077060 |
| WO2001031342 | WO2004018999 | WO2005005601 |

Journal articles

Abrahamsen et al. (2003) Towards quantitative mRNA analysis in paraffin-embedded tissues using real-time reverse transcriptase-polymerase chain reaction J Mol Diag 5:34-41

Al-Mulla et al. (2005) BRCA1 gene expression in breast cancer: a correlative study between real-time RT-PCR and immunohistochemistry J Histochem Cytochem 53:621-629

Argani et al. (2001) Discovery of new Markers of cancer through serial analysis of gene expression: prostate stem cell antigen is overexpressed in pancreatic adenocarcinoma Cancer Res 61:4320-4324

Autiero et al. (2002) Intragenic amplification and formation of extrachromosomal small circular DNA molecules from the PIP gene on chromosome 7 in primary breast carcinomas Int J Cancer 99:370-377

Backus et al. (2005) Identification and characterization of optimal gene expression Markers for detection of breast cancer metastasis J Mol Diagn 7:327-336

Bentov et. al. (2003) The WT1 Wilms' tumor suppressor gene: a novel target for insulin-like growth factor-I action Endocrinol 144:4276-4279

Bera et al. (2004) NGEP, a gene encoding a membrane protein detected only in prostate cancer and normal prostate Proc Natl Acad Sci USA 101:3059-3064 Bibikova et al (2004) Quantitative gene expression profiling in formalin-fixed, paraffin-embedded tissues using universal bead arrays Am j Pathol 165:1799-1807

Bloom et al. (2004) Multi-platform, multi-site, microarray-based human tumor classification Am J Pathol 164:9-16

Borchers et al. (1997) Heart-type fatty acid binding protein - involvement in growth inhibition and differentiation Prostaglandins Leukot Essent Fatty Acids 57:77-84 Borgono et al. (2004) Human tissue kallikreins: physiologic roles and applications in cancer Mol Cancer Res 2:257-280

Brookes (1999) The essence of SNPs Gene 23:177-186

Brown et al. (1997) Immunohistochemical identification of tumor Markers in metastatic adenocarcinoma. A diagnostic adjunct in the determination of primary site Am J Clin Pathol 107:12-19

Buckhaults et al. (2003) Identifying tumor origin using a gene expression-based classification map Cancer Res 63:4144-4149

Chan et al. (1985) Human liver fatty acid binding protein cDNA and amino acid sequence. Functional and evolutionary implications J Biol Chem 260:2629-2632 Chen et al. (1986) Human liver fatty acid binding protein gene is located on chromosome 2 Somat Cell Mol Genet 12:303-306 Cheung et al. (2003) Detection of the PAX8-PPAR gamma fusion oncogene in both follicular thyroid carcinomas and adenomas J Clin Endocrinol Metab 88:354-357 Clark et al. (1999) The potential role for prolactin-inducible protein (PIP) as a Marker of human breast cancer micrometastasis Br J Cancer 81:1002-1008

Cronin et al. (2004) Measurement of gene expression in archival paraffin-embedded tissue Am J Pathol 164:35-42

Cunha et al. (2006) Tissue-specificity of prostate specific antigens: Comparative analysis of transcript levels in prostate and non-prostatic tissues Cancer Lett 236:229-238

Dennis et al. (2002) Identification from public data of molecular Markers of adenocarcinoma characteristic of the site of origin Can Res 62:5999-6005

Dennis et al. (2005a) Hunting the primary: novel strategies for defining the origin of tumors J Pathol 205:236-247

Dennis et al. (2005b) Markers of adenocarcinoma characteristic of the site of origin: development of a diagnostic algorithm Clin Can Res 11:3766-3772

DeYoung et al. (2000) Immunohistologic evaluation of metastatic carcinomas of unknown origin: an algorithmic approach Semin Diagn Pathol 17:184-193

Di Palma et al. (2003) The paired domain-containing factor Pax8 and the homeodomain-containing factor TTF-1 directly interact and synergistically activate transcription Biol Chem 278:3395-3402

Dwight et al. (2003) Involvement of the PAX8 peroxisome proliferator-activated receptor gamma rearrangement in follicular thyroid tumors J Clin Endocrinol Metab 88:4440-4445

Feldman et al. (2003) PDEF expression in human breast cancer is correlated with invasive potential and altered gene expression Cancer Res 63:4626-4631 Fleming et al. (2000) Mammaglobin, a breast-specific gene, and its utility as a Marker for breast cancer Ann N Y Acad Sci 923:78-89

Fukushima et al. (2004) Characterization of gene expression in mucinous cystic neoplasms of the pancreas using oligonucleotide microarrays Oncogene 23:9042-9051

Ghosh et al (2005) Management of patients with metastatic cancer of unknown primary Curr Probl Surg 42:12-66

Giordano et al. (2001) Organ-specific molecular classification of primary lung, colon, and ovarian adenocarcinomas using gene expression profiles Am J Pathol.159:1231-1238

Glasser et al (1988) cDNA, deduced polypeptide structure and chromosomal assignment of human pulmonary surfactant proteolipid, SPL(pVal) J Biol Chem 263:9-12

Godfrey et al. (2000) Quantitative mRNA expression analysis from formalin-fixed, paraffin-embedded tissues using 5' nuclease quantitative reverse transcription-polymerase chain reaction J Mol Diag 2:84-91

Goldstein et al. (2002) WT1 immunoreactivity in uterine papillary serous carcinomas is different from ovarian serous carcinomas Am J Clin Pathol 117:541-545

Gradi et al. (1995) The human steroidogenic acute regulatory (StAR) gene is expressed in the urogenital system and encodes a mitochondrial polypeptide Biochim Biophys Acta 1258:228-233

Greco et al. (2004) Carcinoma of unknown primary site: sequential treatment with paclitaxel/carboplatin/etoposide and gemcitabine/irinotecan: A Minnie Pearl cancer research network phase II trial The Oncologist 9:644-652

Haas et al. (2005) Combined application of RT-PCR and immunohistochemistry on paraffin embedded sentinel lymph nodes of prostate cancer patients Pathol Res Pract 200:763-770

Hwang et al. (2004) Wilms tumor gene product: sensitive and contextually specific Marker of serous carcinomas of ovarian surface epithelial origin Appl Immunohistochem Mol Morphol 12:122-126

Ishikawa et al. (2005) Experimental trial for diagnosis of pancreatic ductal carcinoma based on gene expression profiles of pancreatic ductal cells Cancer Sci 96:387-393

Italiano et al. (2005) Epidermal growth factor receptor (EGFR) status in primary colorectal tumors correlates with EGFR expression in related metastatic sites: biological and clinical implications Ann Oncol 16:1503-1507

Jones et al. (2004) Comprehensive analysis of matrix metalloproteinase and tissue inhibitor expression in pancreatic cancer: increased expression of matrix metalloproteinase-7 predicts poor survival Clin Cancer Res 10:2832-2845

Jones et al. (2005) Thyroid transcription factor 1 expression in small cell carcinoma of the urinary bladder: an immunohistochemical profile of 44 cases Hum Pathol 36:718-723

Khoor et al. (1997) Expression of surfactant protein B precursor and surfactant protein B mRNA in adenocarcinoma of the lung Mod Pathol 10:62-67

Kim (2003) Comparison of oligonucleotide-microarray and serial analysis of gene expression (SAGE) in transcript profiling analysis of megakaryocytes derived from CD34+ cells Exp Mol Med 35:460-466

Kim et al. (2003) Steroidogenic acute regulatory protein expression in the normal human brain and intracranial tumors Brain Res 978:245-249

Lam et al. (2005) Prostate stem cell antigen is overexpressed in prostate cancer metastases Clin Can Res 11:2591-2596

Lembersky et al. (1996) Metastases of unknown primary site Med Clin North Am. 80:153-171

Lewis et al. (2001) Unlocking the archive-gene expression in paraffin-embedded tissue J Pathol 195:66-71

Lipshutz et al. (1999) High density synthetic oligonucleotide arrays Nature Genetics 21:S20-24

Lowe et al. (1985) Human liver fatty acid binding protein. Isolation of a full length cDNA and comparative sequence analyses of orthologous and paralogous proteins J Biol Chem 260:3413-3417

Ma et al. (2006) Molecular classification of human cancers using a 92-gene real-time quantitative polymerase chain reaction assay Arch Pathol Lab med 130:465-473 Magklara et al. (2002) Characterization of androgen receptor and nuclear receptor co-regulator expression in human breast cancer cell lines exhibiting differential regulation of kallikreins 2 and 3 Int J Cancer 100:507-514

Markowitz (1952) Portfolio Selection J Finance 7:77-91

Marques et al. (2002) Expression of PAX8-PPAR gamma 1 rearrangements in both follicular thyroid carcinomas and adenomas J Clin Endocrinol Metab 87:3947-3952

Masuda et al. (1999) Analysis of chemical modification of RNA from formalin-fixed samples and optimization of molecular biology applications for such samples Nucl Acids Res 27:4436-4443

McCarthy et al. (2003) Novel Markers of pancreatic adenocarcinoma in fine-needle aspiration: mesothelin and prostate stem cell antigen labeling increases accuracy in cytologically borderline cases Appl Immunohistochem Mol Morphol 11:238-243 Mikhitarian et al. (2004) Enhanced detection of RNA from paraffin-embedded tissue using a panel of truncated gene-specific primers for reverse transcription

BioTechniques 36:1-4 Mintzer et al. (2004) Cancer of unknown primary: changing approaches, a multidisciplinary case presentation from the Joan Karnell Cancer Center of

Pennsylvania Hospital The Oncologist 9:330-338 Moniaux et al. (2004) Multiple roles of mucins in pancreatic cancer, a lethal and challenging malignancy Br J Cancer 91:1633-1638
Murphy et al. (1987) Isolation and sequencing of a cDNA clone for a prolactin-inducible protein (PIP). Regulation of PIP gene expression in the human breast cancer cell line, T-47D J Biol Chem 262:15236-15241
Myal et al. (1991) The prolactin-inducible protein (PIPGCDFP-15) gene: cloning, structure and regulation J Mol Cell Endocrinol 80:165-175
Nakamura et al. (2002) Expression of thyroid transcription factor-1 in normal and neoplastic lung tissues Mod Pathol 15:1058-1067
Noonan et al. (2001) Characterization of the homeodomain gene EMX2: sequence conservation, expression analysis, and a search for mutations in endometrial cancers Genomics 76:37-44
Oettgen et al. (2000) PDEF, a novel prostate epithelium-specific Ets transcription factor, interacts with the androgen receptor and activates prostate-specific antigen gene expression J Biol Chem 275:1216-1225
Oji et al. (2003) Overexpression of the Wilms' tumor gene WT1 in head and neck squamous cell carcinoma Cancer Sci 94:523-529
Pavlidis et al. (2003) Diagnostic and therapeutic management of cancer of an unknown primary Eur J Can 39: 990-2005
Pilot-Mathias et al. (1989) Structure and organization of the gene encoding human pulmonary surfactant proteolipid SP-B DNA 8:75-86
Pilozzi et al. (2004) CDX1 expression is reduced in colorectal carcinoma and is associated with promoter hypermethylation J Pathol 204:289-295
Poleev et al. (1992) PAX8, a human paired box gene: isolation and expression in developing thyroid, kidney and Wilms' tumors Development 116:611-623
Prasad et al. (2005) Gene expression profiles in pancreatic intraepithelial neoplasia reflect the effects of Hedgehog signaling on pancreatic ductal epithelial cells Cancer Res 65:1619-1626
Ramaswamy (2004) Translating cancer genomics into clinical oncology N Engl J Med 350:1814-1816
Ramaswamy et al. (2001) Multiclass cancer diagnosis using tumor gene expression signatures Proc Natl Acad Sci USA 98:15149-15154
Rauscher (1993) The WT1 Wilms tumor gene product: a developmentally regulated transcription factor in the kidney that functions as a tumor suppressor FASEB J 7:896-903
Reinholz et al. (2005) Evaluation of a panel of tumor Markers for molecular detection of circulating cancer cells in women with suspected breast cancer Clin Cancer Res 11:3722
Schlag et al. (1994) Cancer of unknown primary site Ann Chir Gynaecol 83:8-12
Senoo et al. (1998) A second p53-related protein, p73L, with high homology to p73 Biochem Biophys Res Comm 248:603-607
Specht et al. (2001) Quantitative gene expression analysis in microdissected archival formalin-fixed and paraffin-embedded tumor tissue Amer J Pathol 158:419-429
Su et al. (2001) Molecular classification of human carcinomas by use of gene expression signatures Cancer Res 61:7388-7393
Takahashi et al. (1995) Cloning and characterization of multiple human genes and cDNAs encoding highly related type II keratin 6 isoforms J Biol Chem 270:18581-18592
Takamura et al. (2004) Reduced expression of liver-intestine cadherin is associated with progression and lymph node metastasis of human colorectal carcinoma Cancer Lett 212:253-259
Tothill et al. (2005) An expression-based site of origin diagnostic method designed for clinical application to cancer of unknown origin Can Res 65:4031-4040 van Ruissen et al. (2005) Evaluation of the similarity of gene expression data estimated with SAGE and Affymetrix GeneChips BMC Genomics 6:91
Varadhachary et al. (2004) Diagnostic strategies for unknown primary cancer Cancer 100:1776-1785
Venables et al. (2002) Modern Applied Statistics with S. Fourth edition. Springer
Wallace et al. (2005) Accurate Molecular detection of non-small cell lung cancer metastases in mediastinal lymph nodes sampled by endoscopic ultrasound-guided needle aspiration Cest 127:430-437
Wan et al. (2003) Desmosomal proteins, including desmoglein 3, serve as novel negative Markers for epidermal stem cell-containing population of keratinocytes J Cell Sci 116:4239-4248
Watson et al. (1996) Mammaglobin, a mammary-specific member of the uteroglobin gene family, is overexpressed in human breast cancer Cancer Res 56:860-865
Watson et al. (1998) Structure and transcriptional regulation of the human mammaglobin gene, a breast cancer associated member of the uteroglobin gene family localized to chromosome 11q13 Oncogene 16:817-824
Weigelt et al. (2003) Gene expression profiles of primary breast tumors maintained in distant metastases Proc Natl Acad Sci USA 100:15901-15905
Zapata-Benavides et al. (2002) Downregulation of Wilms' tumor 1 protein inhibits breast cancer proliferation Biochem Biophys Res Commun 295:784-790

**Claims**

1. A method of identifying origin of a metastasis of unknown origin comprising the steps of

   a. measuring Biomarkers associated with at least six different carcinomas in a sample containing metastatic cells wherein the Biomarkers are levels of expression of at least the following Marker genes: SP-B, TTF1, DSG3, PSCA, F5, HPT1, PSA, MGB/MG, WT1 and PDEF;
   b. combining the data from the Biomarkers into an algorithm where the algorithm

      i. normalizes the Biomarkers against a reference; and
      ii. imposes a cut-off which optimizes sensitivity and specificity of each Biomarker, weights the prevalence of the carcinomas and selects a tissue of origin;

   c. determining origin based on highest probability determined by the algorithm or determining that the carcinoma is not derived from a particular set of carcinomas; and
   d. optionally measuring Biomarkers specific for one or more additional different carcinoma, and repeating steps c) and d) for the additional Biomarkers.

2. The method according to claim 1 wherein the Marker genes further comprise

   (a) KRT6F, p73H, and/or SFTPC; and/or
   (b) ITGB6, KLK10, CLDN18, TR10 and/or FKBP10; and/or
   (c) CDX1 and/or FABP1

3. The method of one of claims 1 or 2 wherein gene expression is measured using at least one of SEQ ID NOs: 11-58.

4. The method of claim 3 wherein the Marker genes further comprise gender specific Marker genes selected from at least one of

   i. in the case of a male patient KLK2, NGEP or NPY; or
   ii. in the case of a female patient PIP, B305D, B726 or GABA-Pi; and/or PAX8, STAR or EMX2.

5. The method of claim 4 wherein, in the case of a male patient, the Marker genes further comprise KLK2.

6. The method of claim 5 wherein, in the case of a male patient, the Marker genes further comprise NGEP and/or NPY.

**7.** The method of claim 4 wherein, in the case of a female patient, the Marker genes further comprise, PIP, B305D, B726 or GABA-Pi.

**8.** The method of claim 4 wherein, in the case of a female patient, the Marker genes further comprise PAX8, STAR or EMX2.

**9.** The method of claim 8 wherein, in the case of a female patient, the Marker genes further comprise PIP, B305D, B726 or GABA-Pi.

**10.** The method according to one of claims 1-2, or 4-9 further comprising measuring expression of at least one gene constitutively expressed in the sample.

**11.** The method of claim 1 comprising further obtaining additional clinical information including the site of metastasis to determine the origin of the carcinoma.

**12.** A method of providing a prognosis or direction of therapy by determining the origin of a metastasis of unknown origin according to claim 1 and identifying the corresponding prognosis therefore or appropriate treatment therefor.

**13.** The use of a kit for performing the method of claim 1, wherein the kit comprises: RNA or cDNA which hybridizes to the marker genes of claim 1, a microarray or a gene chip.

**Patentansprüche**

**1.** Verfahren zur Identifizierung des Ursprungs einer Metastase unbekannten Ursprungs, umfassend die Schritte

a. Messen mit wenigstens sechs unterschiedlichen Karzinomen assoziierter Biomarker in einer Metastasenzellen enthaltenden Probe, wobei die Biomarker Expressionsniveaus wenigstens der folgenden Markergene SP-B, TTF1, DSG3, PSCA, F5, HPT1, PSA, MGB/MG, WT1 und PDEF sind;
b. Kombinieren der Daten von den Biomarkern zu einem Algorithmus, wobei der Algorithmus

i. die Biomarker auf eine Referenz normiert; und
ii. eine Ausschlussgrenze aufstellt, womit die Empfindlichkeit und Spezifität eines jeden Biomarkers optimiert werden, die Prävalenz der Karzinome gewichtet und ein Ursprungsgewebe ausgewählt wird;

c. Bestimmen des Ursprungs anhand der mit dem Algorithmus bestimmten höchsten Wahrscheinlichkeit oder Bestimmen, dass das Karzinom nicht von einem bestimmten Satz von Karzinomen stammt; und
d. gegebenenfalls Messen für ein oder mehrere weitere unterschiedliche Karzinome spezifischer Biomarker, wobei für die zusätzlichen Biomarker die Schritte c) und d) wiederholt werden.

**2.** Verfahren gemäß Anspruch 1, wobei die Markergene ferner umfassen:

(a) KRT6F, p73H, und/oder SFTPC; und/oder
(b) ITGB6, KLK10, CLDN18, TR10 und/oder FKBP10; und/oder
(c) CDX1 und/oder FABP1.

**3.** Verfahren nach einem der Ansprüche 1 oder 2, wobei die Genexpression unter Verwendung wenigstens einer der SEQ ID NO: 11-58 gemessen wird.

**4.** Verfahren nach Anspruch 3, wobei die Markergene ferner geschlechtsspezifische Markergene umfassen, die aus wenigstens einem der Folgenden ausgewählt sind:

i. im Falle eines männlichen Patienten KLK2, NGEP oder NPY; oder
ii. im Falle eines weiblichen Patienten PIP, B305D, B726 oder GABA-Pi; und/oder PAX8, STAR oder EMX2.

**5.** Verfahren nach Anspruch 4, wobei im Falle eines männlichen Patienten die Markergene ferner KLK2 umfassen.

**6.** Verfahren nach Anspruch 5, wobei im Falle eines männlichen Patienten die Markergene ferner NGEP und/oder NPY umfassen.

**7.** Verfahren nach Anspruch 4, wobei im Falle eines weiblichen Patienten die Markergene ferner PIP, B305D, B726 oder GABA-Pi umfassen.

**8.** Verfahren nach Anspruch 4, wobei im Falle eines weiblichen Patienten die Markergene ferner PAX8, STAR oder EMX2 umfassen.

**9.** Verfahren nach Anspruch 8, wobei im Falle eines weiblichen Patienten die Markergene ferner PIP, B305D, B726 oder GABA-Pi umfassen.

**10.** Verfahren gemäß einem der Ansprüche 1-2 oder 4-9, ferner umfassend Messen der Expression wenigstens eines in der Probe konstitutiv exprimierten Gens.

**11.** Verfahren nach Anspruch 1, umfassend weiteres Gewinnen zusätzlicher klinischer Informationen, einschließlich des Metastasenortes, zur Bestimmung des Ursprungs des Karzinoms.

**12.** Verfahren zur Bereitstellung einer Prognose oder Therapierichtung durch Bestimmen des Ursprungs einer Metastase unbekannten Ursprungs gemäß Anspruch 1 und Identifizieren der entsprechenden Prognose dafür oder geeigneten Behandlung dafür.

**13.** Verwendung eines Kits zur Durchführung des Verfahrens nach Anspruch 1, wobei das Kit umfasst: RNA oder cDNA, die an die Markergene nach Anspruch 1 hybridisiert, einen Mikroarray oder einen Gen-Chip.

**Revendications**

**1.** Procédé pour identifier l'origine d'une métastase d'origine inconnue, comprenant les étapes suivantes:

a. mesurer des marqueurs biologiques associés à au moins six carcinomes différents dans un échantillon contenant des cellules métastatiques, dans lequel les marqueurs biologiques sont des niveaux d'expression d'au moins les gènes marqueurs suivants: SP-B, TTF1, DSG3, PSCA, F5, HPT1, PSA, MGB/MG, WT1 et PDEF;
b. combiner les données en provenance des marqueurs biologiques en un algorithme, dans lequel l'algorithme:

i. normalise les marqueurs biologiques par rapport à une référence; et
ii. impose une coupure qui optimise la sensibilité et la spécificité de chaque marqueur biologique, pèse la prévalence des carcinomes et sélectionne un tissu d'origine;

c. déterminer l'origine sur la base de la probabilité la plus élevée déterminée par l'algorithme ou déterminer que le carcinome n'est pas dérivé d'un ensemble particulier de carcinomes; et
d. optionnellement, mesurer des marqueurs biologiques spécifiques pour un ou plusieurs carcinome(s) différent(s) supplémentaire(s), et répéter les étapes c) et d) pour les marqueurs biologiques supplémentaires.

**2.** Procédé selon la revendication 1, dans lequel les gènes marqueurs comprennent en outre:

a) KRT6F, p73H et/ou SFTPC; et/ou
b) ITGB6, KLK10, CLDN18, TR10 et/ou FKBP10; et/ou
c) CDX1 et/ou FABP1.

**3.** Procédé selon la revendication 1 ou 2, dans lequel l'expression génétique est mesurée en utilisant au moins une des SEQUENCES D'IDENTIFICATION N°. 11-58.

**4.** Procédé selon la revendication 3, dans lequel les gènes marqueurs comprennent en outre des gènes marqueurs spécifiques au genre sélectionnés parmi au moins un de:

i. dans le cas d'un patient mâle: KLK2, NGEP ou NPY; ou
ii. dans le cas d'un patient femelle: PIP, B305D, B726 ou GABA-Pi; et/ou PAX8, STAR ou EMX2.

**5.** Procédé selon la revendication 4, dans lequel, dans le cas d'un patient mâle, les gènes marqueurs comprennent en outre: KLK2.

**6.** Procédé selon la revendication 5, dans lequel, dans le cas d'un patient mâle, les gènes marqueurs comprennent en outre: NGEP et/ou NPY.

**7.** Procédé selon la revendication 4, dans lequel, dans le cas d'un patient femelle, les gènes marqueurs comprennent en outre: PIP, B305D, B726 ou GABA-Pi.

**8.** Procédé selon la revendication 4, dans lequel, dans le cas d'un patient femelle, les gènes marqueurs comprennent en outre: PAX8, STAR ou EMX2.

**9.** Procédé selon la revendication 8, dans lequel, dans le cas d'un patient femelle, les gènes marqueurs comprennent en outre: PIP, B305D, B726 ou GABA-Pi.

**10.** Procédé selon l'une des revendications 1 à 2 ou 4 à 9, comprenant en outre la mesure de l'expression d'au moins un gène exprimé de façon constitutive dans l'échantillon.

**11.** Procédé selon la revendication 1, comprenant en outre l'obtention d'informations cliniques supplémentaires comprenant le site de la métastase afin de déterminer l'origine du carcinome.

**12.** Procédé de fourniture d'un pronostic ou d'une orientation thérapeutique en déterminant l'origine d'une métastase d'origine inconnue selon la revendication 1 et d'identification du pronostic correspondant pour celle-ci ou du traitement approprié pour celle-ci.

**13.** Utilisation d'un kit pour exécuter le procédé selon la revendication 1, dans lequel le kit comprend: de l'ARN ou de l'ADN complémentaire qui s'hybride sur les gènes marqueurs selon la revendication 1, une micro-matrice d'ADN ou une bio-puce à ADN.

Figure 1

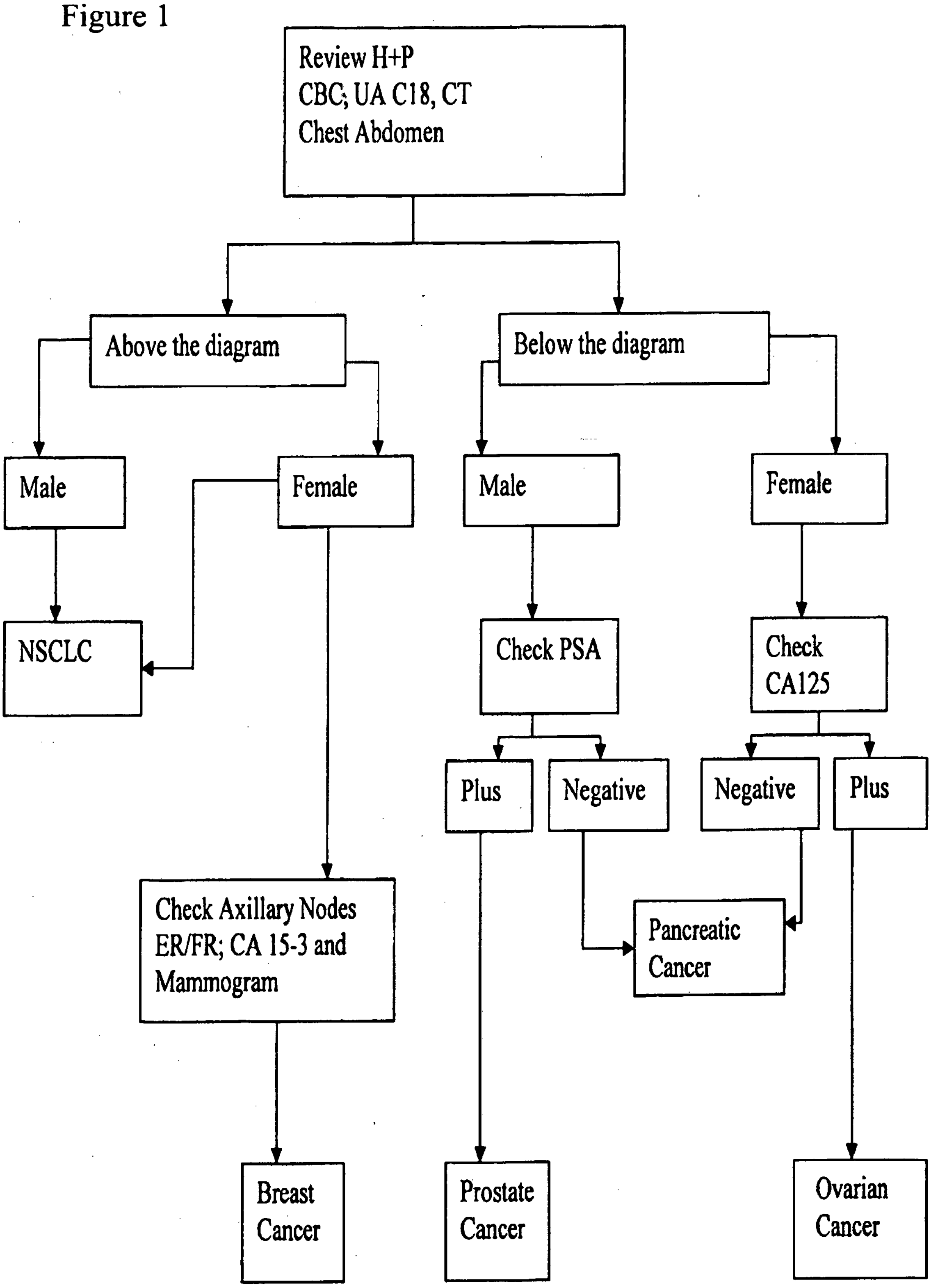
Review H+P
CBC; UA C18, CT
Chest Abdomen
Above the diagram
Below the diagram
Male
Female
Male
Female
NSCLC
Check PSA
Check
CA125
Plus
Negative
Negative
Plus
Check Axillary Nodes
ER/FR; CA 15-3 and
Mammogram
Pancreatic
Cancer
Breast
Cancer
Prostate
Cancer
Ovarian
Cancer

Figure 2

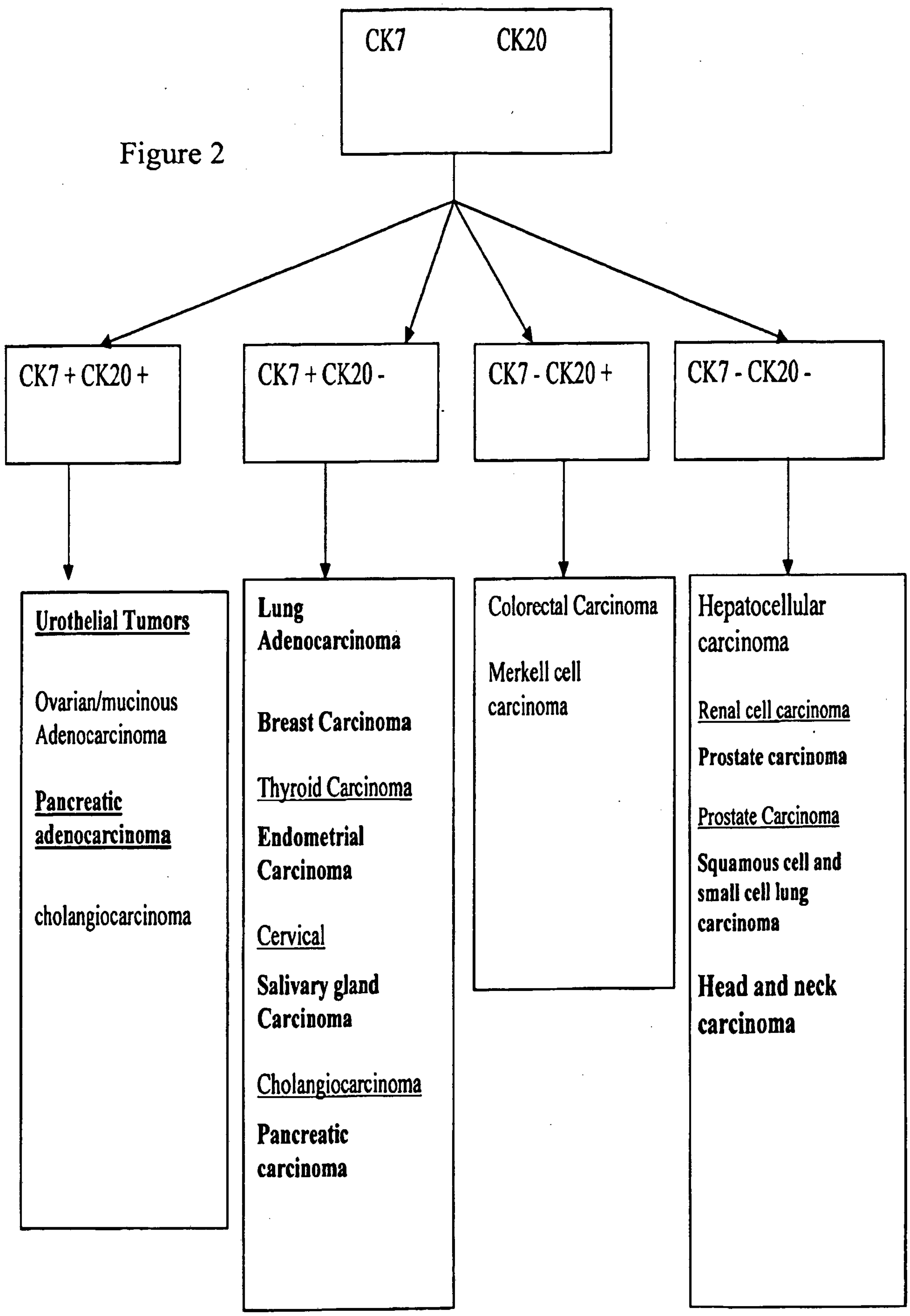
CK7 CK20
CK7 + CK20 +
CK7 + CK20 -
CK7 - CK20 +
CK7 - CK20 -
Urothelial Tumors
Ovarian/mucinous Adenocarcinoma
Pancreatic adenocarcinoma
cholangiocarcinoma
Lung Adenocarcinoma
Breast Carcinoma
Thyroid Carcinoma
Endometrial Carcinoma
Cervical
Salivary gland Carcinoma
Cholangiocarcinoma
Pancreatic carcinoma
Colorectal Carcinoma
Merkell cell carcinoma
Hepatocellular carcinoma
Renal cell carcinoma
Prostate carcinoma
Prostate Carcinoma
Squamous cell and small cell lung carcinoma
Head and neck carcinoma

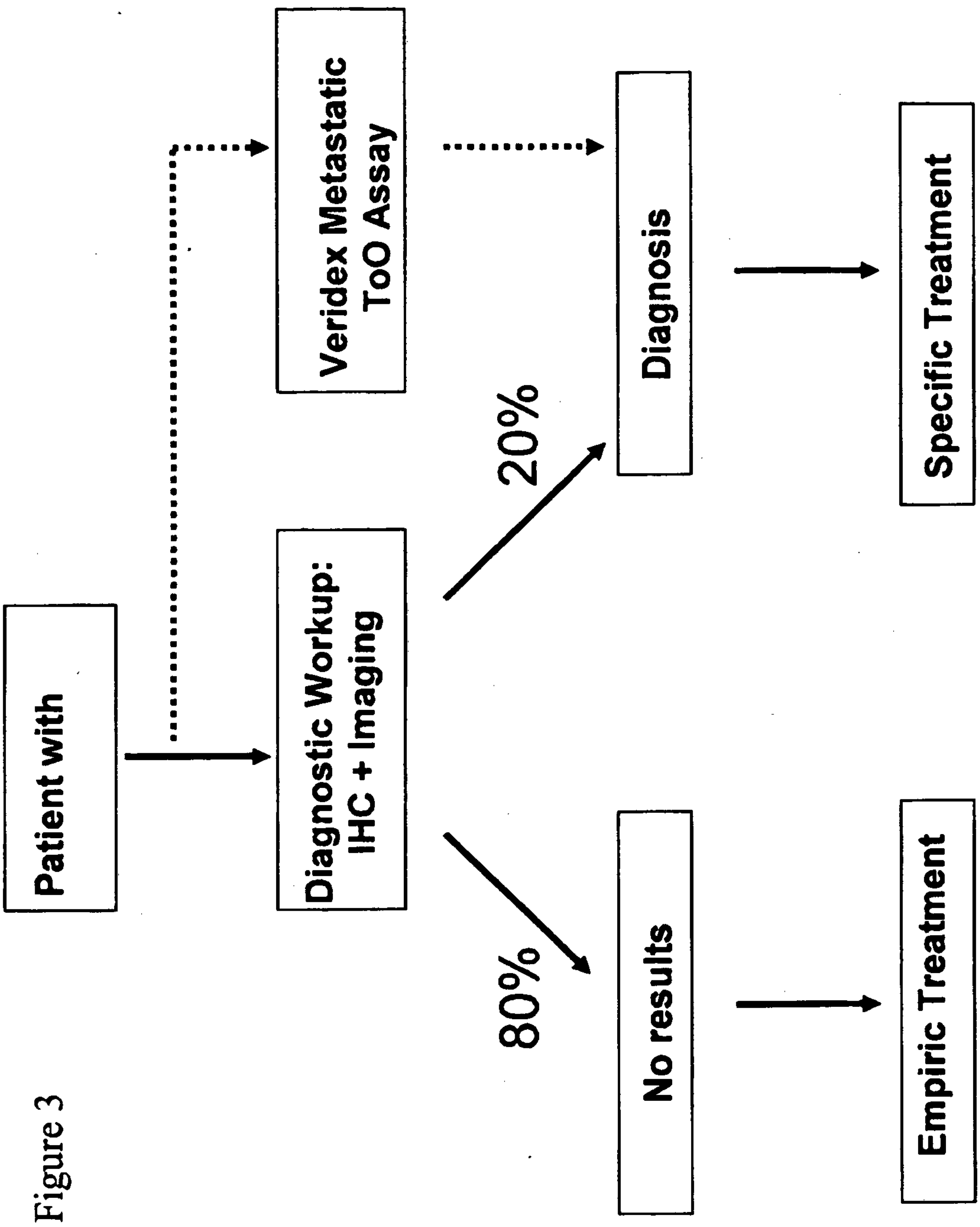
Patient with
Diagnostic Workup: IHC + Imaging
Veridex Metastatic ToO Assay
20%
80%
Diagnosis
No results
Specific Treatment
Empiric Treatment

Figure 3

Figure 4(A)

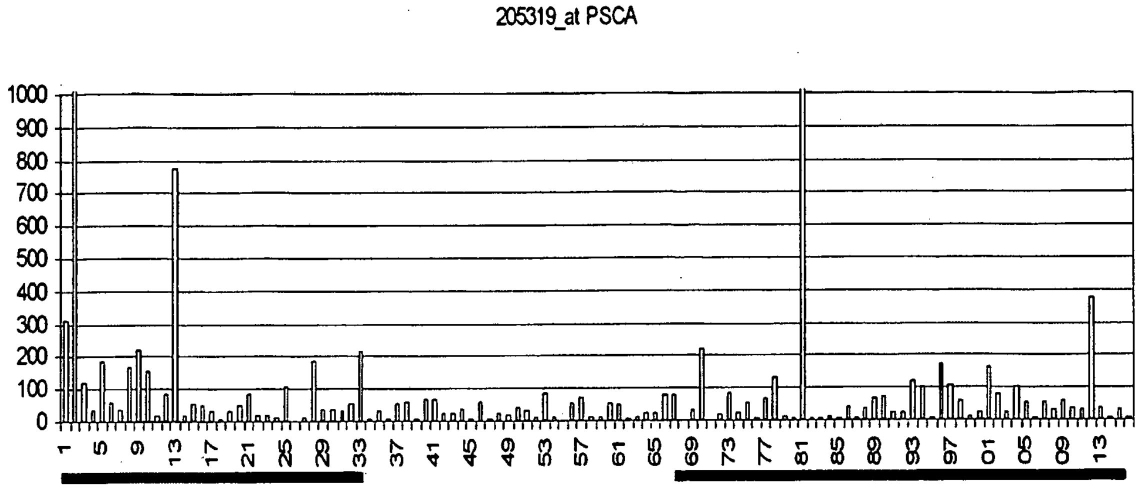


Figure 4(B)

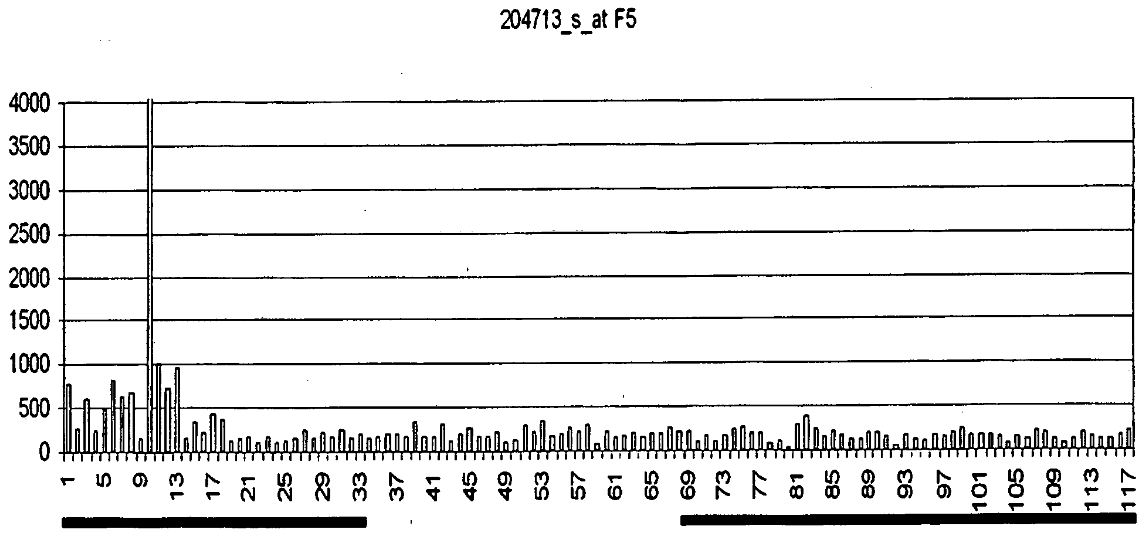

Figure 5(A)

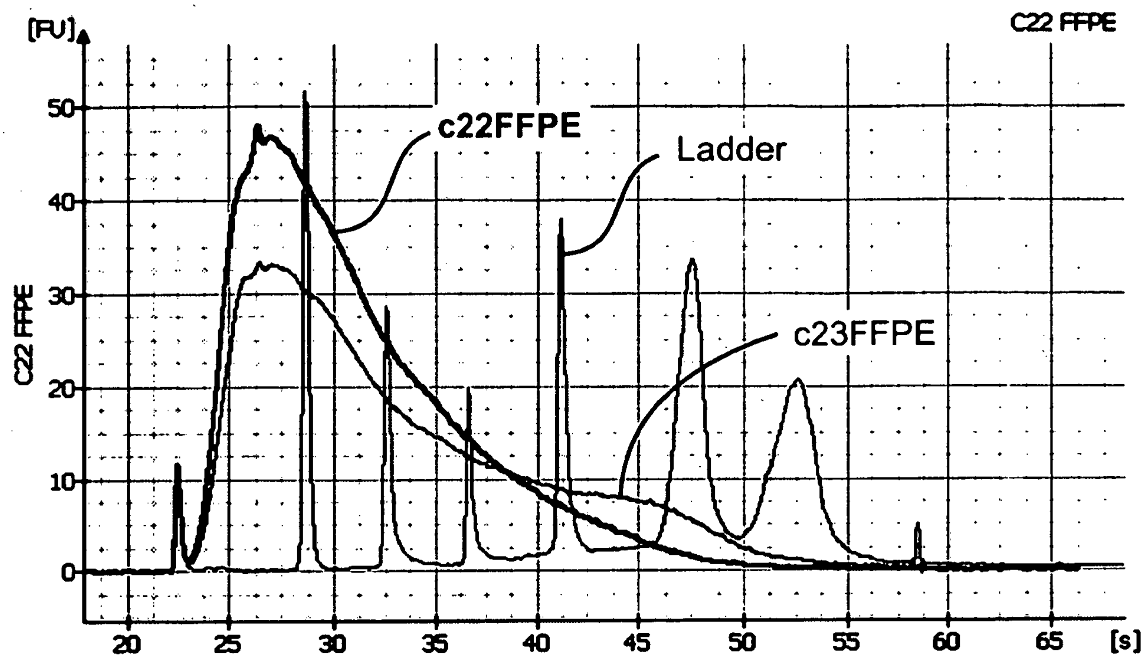


Figure 5(B)

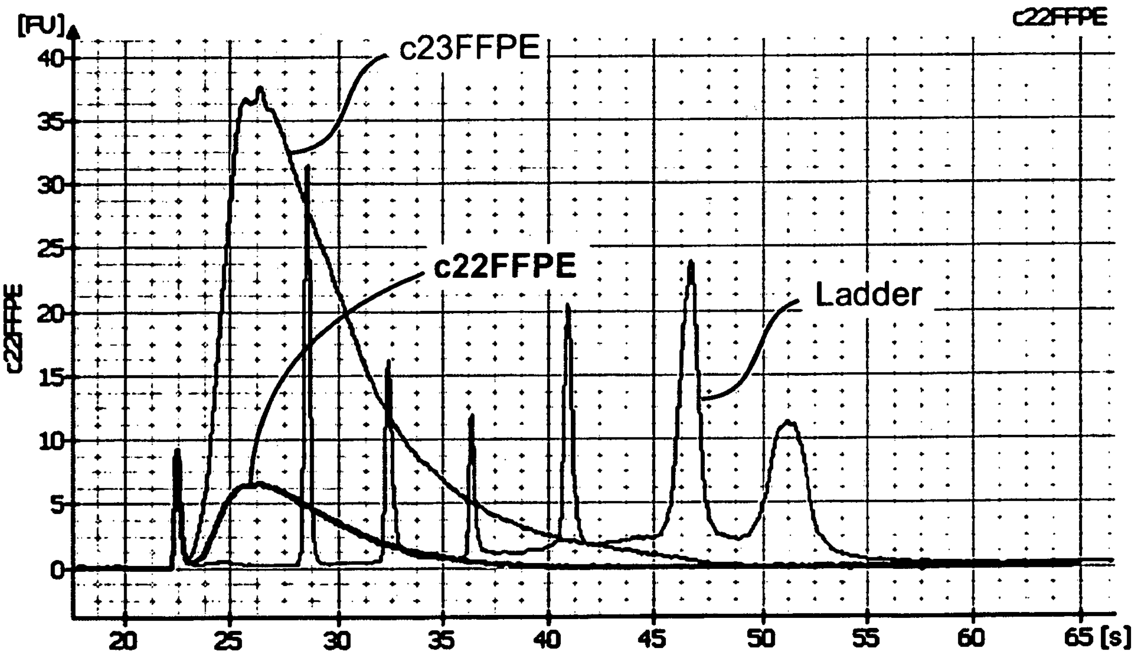

Figure 6(A)

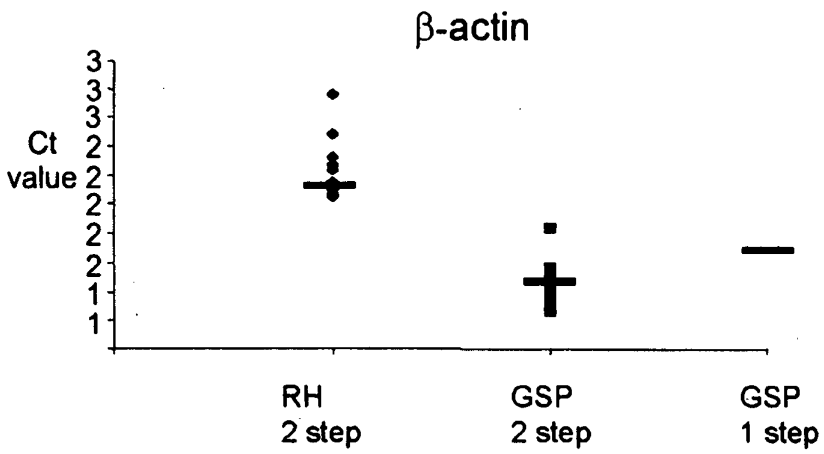


Figure 6(B)

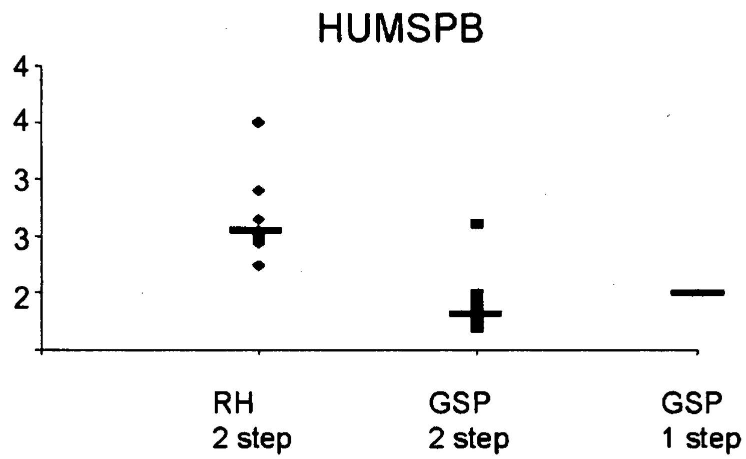


Figure 6(C)

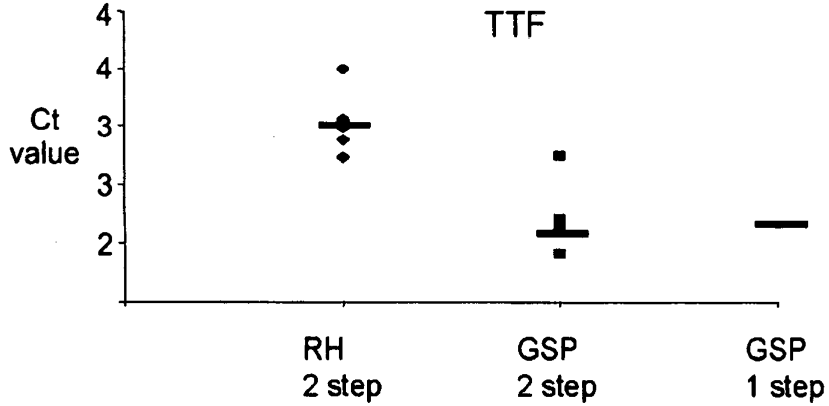

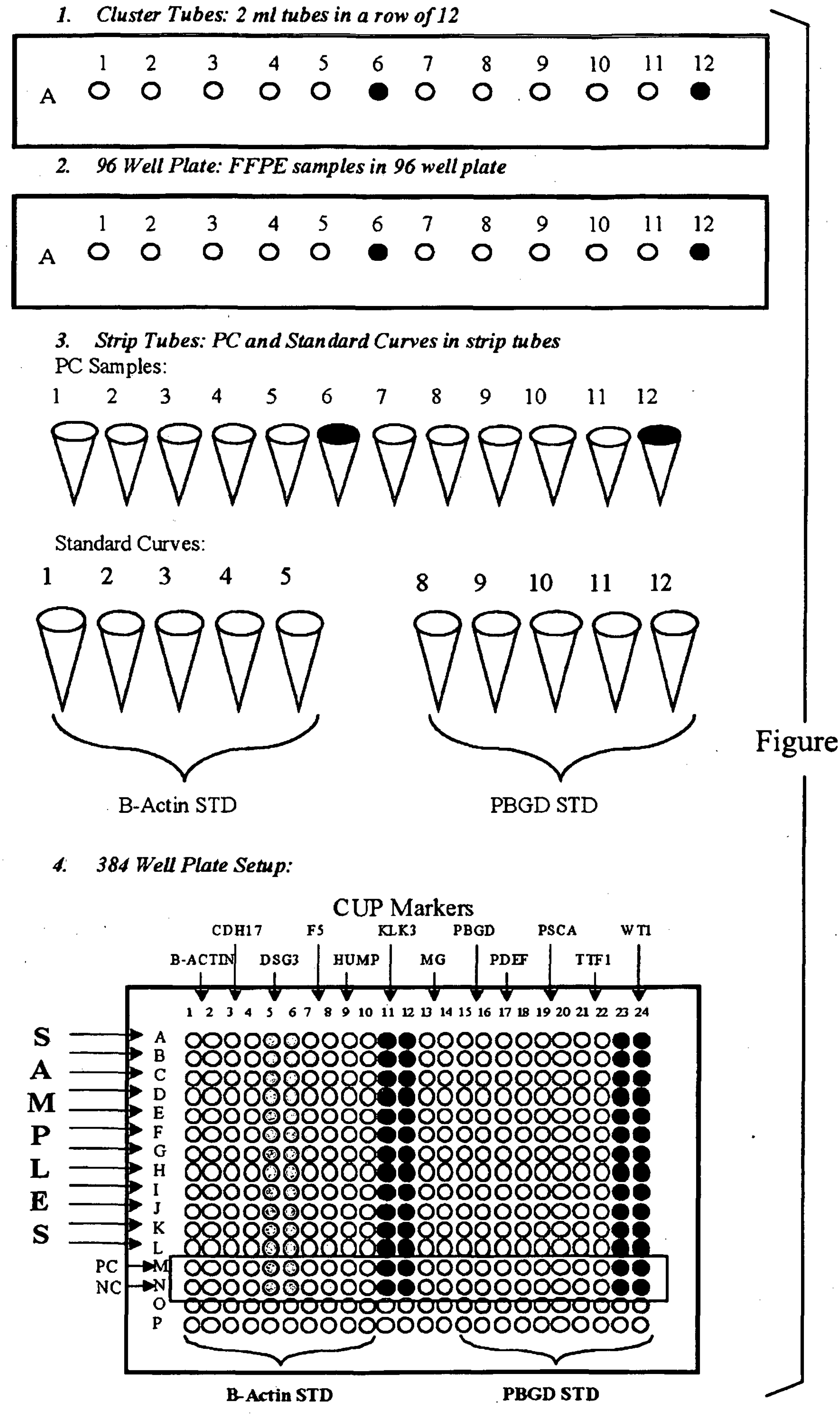
1. Cluster Tubes: 2 ml tubes in a row of 12
2. 96 Well Plate: FFPE samples in 96 well plate
3. Strip Tubes: PC and Standard Curves in strip tubes
PC Samples:
Standard Curves:
B-Actin STD
PBGD STD
4. 384 Well Plate Setup:
CUP Markers
CDH17
F5
KLK3
PBGD
PSCA
WT1
B-ACTIN
DSG3
HUMP
MG
PDEF
TTF1
SAMPLES
PC
NC
B-Actin STD
PBGD STD

Figure 7

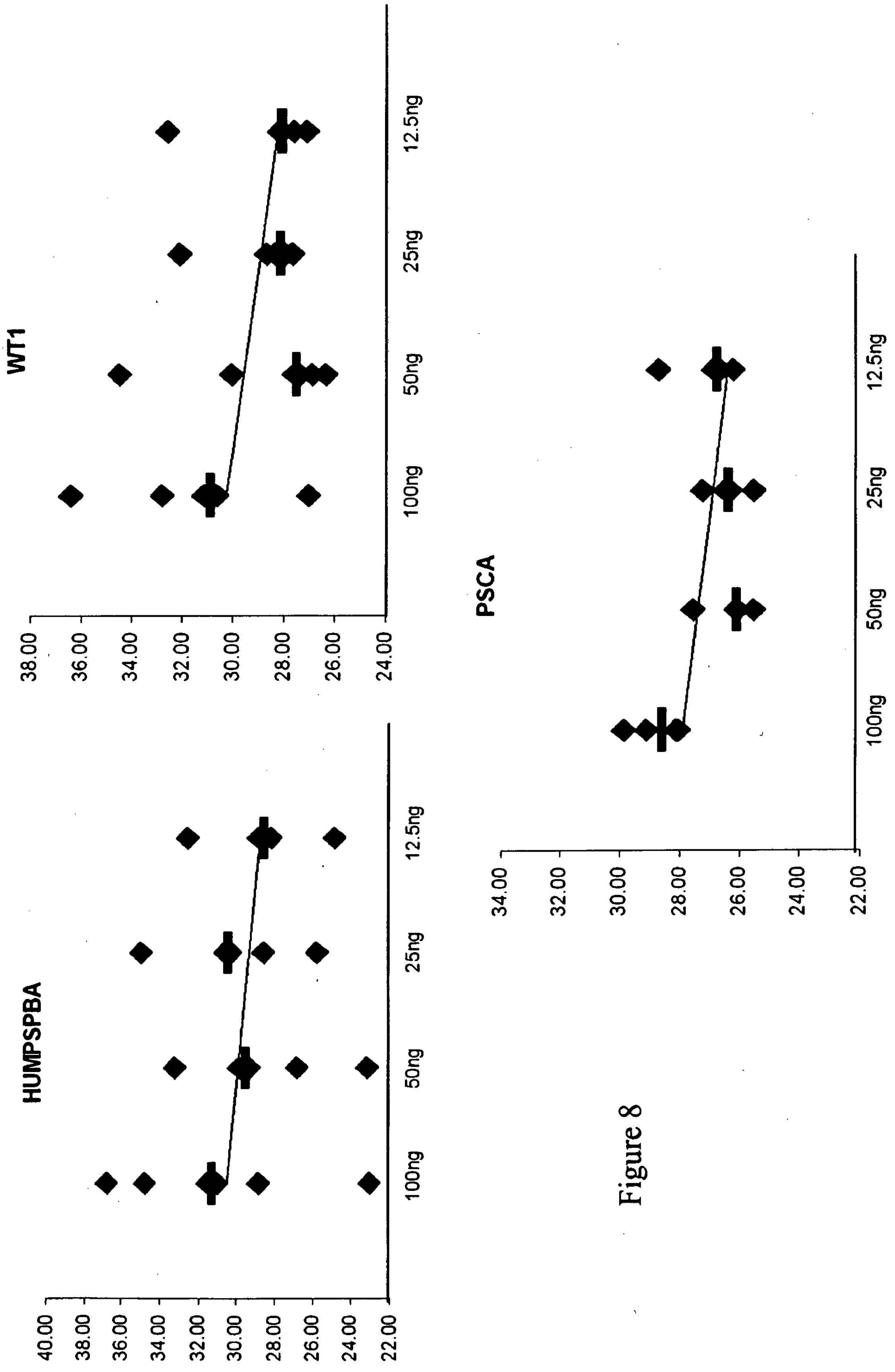

HUMPSPBA
40.00
38.00
36.00
34.00
32.00
30.00
28.00
26.00
24.00
22.00
100ng
50ng
25ng
12.5ng
WT1
38.00
36.00
34.00
32.00
30.00
28.00
26.00
24.00
100ng
50ng
25ng
12.5ng
PSCA
34.00
32.00
30.00
28.00
26.00
24.00
22.00
100ng
50ng
25ng
12.5ng


Figure 8

Figure 9A

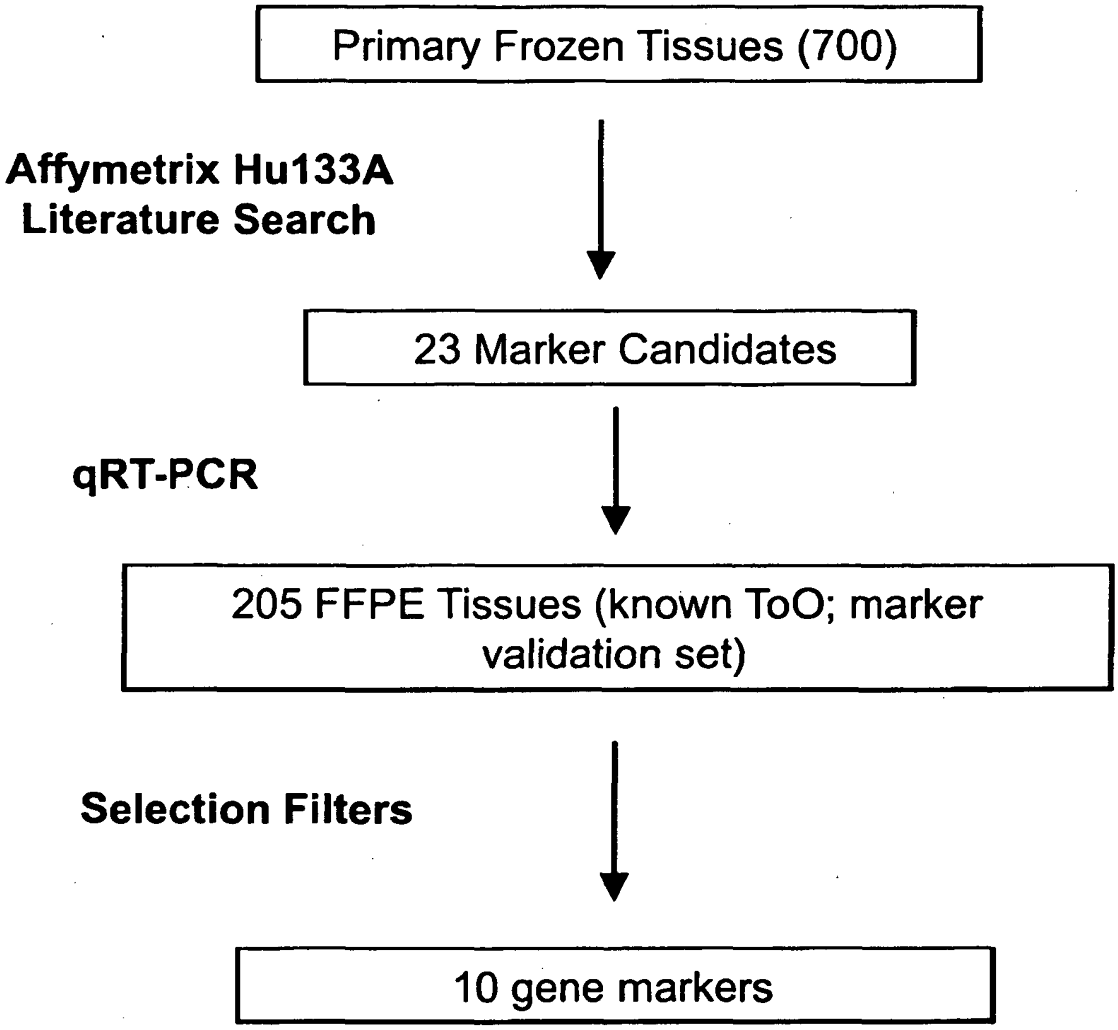

Primary Frozen Tissues (700)
Affymetrix Hu133A
Literature Search
23 Marker Candidates
qRT-PCR
205 FFPE Tissues (known ToO; marker validation set)
Selection Filters
10 gene markers

Figure 9B

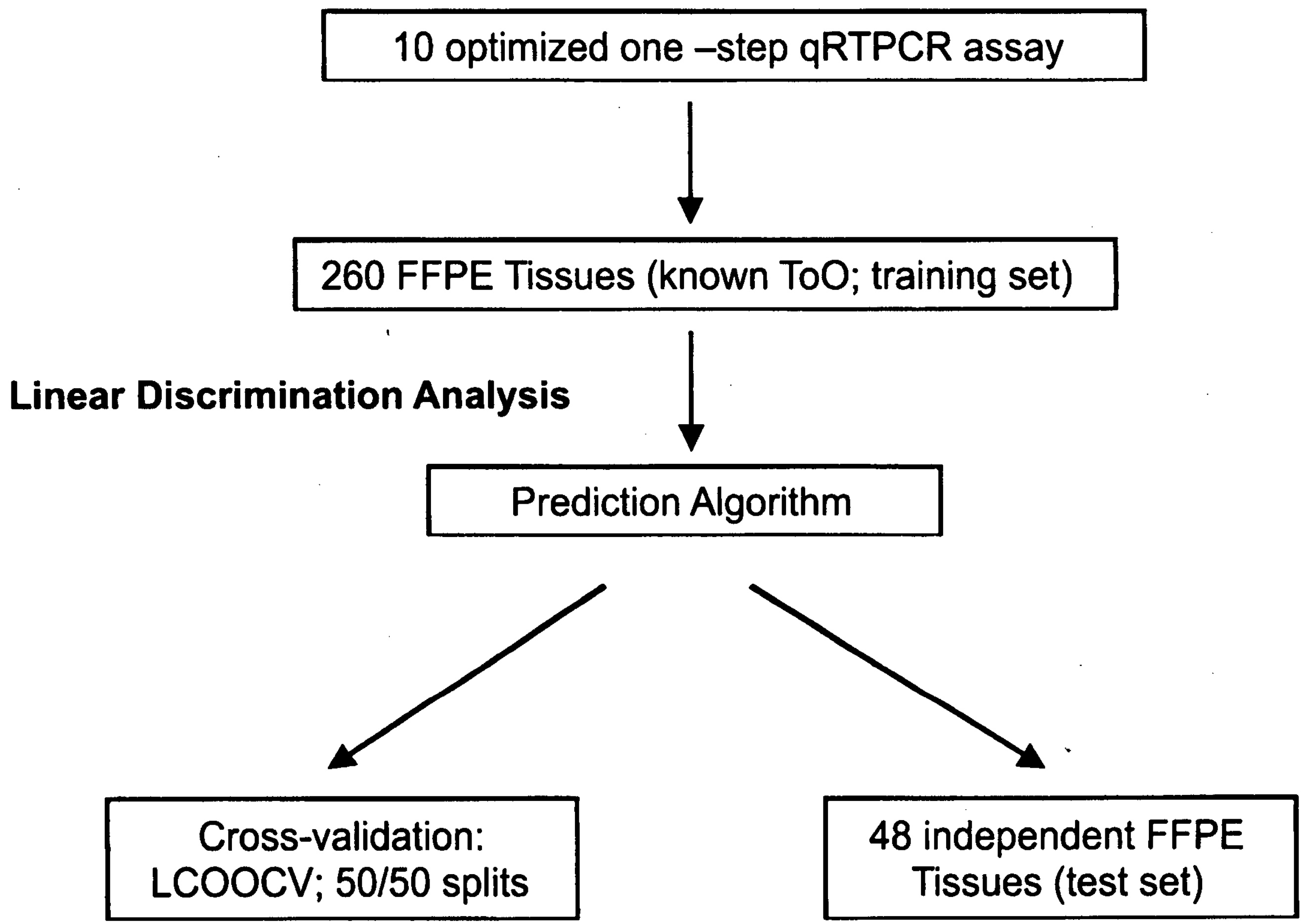
10 optimized one –step qRTPCR assay
260 FFPE Tissues (known ToO; training set)
Linear Discrimination Analysis
Prediction Algorithm
Cross-validation:
LCOOCV; 50/50 splits
48 independent FFPE
Tissues (test set)

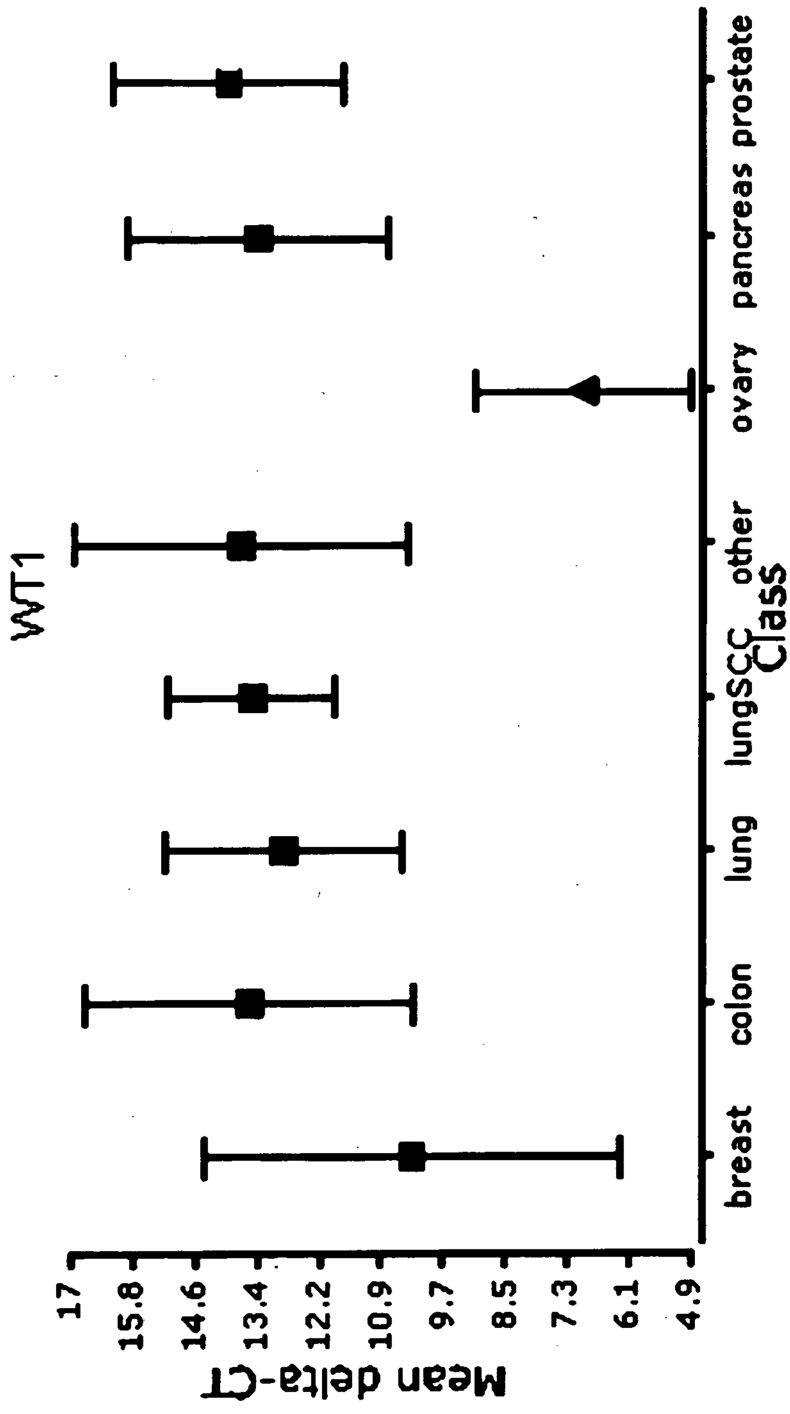
WT1
Mean delta-CT
17
15.8
14.6
13.4
12.2
10.9
9.7
8.5
7.3
6.1
4.9
breast
colon
lung
lungSCC
other
ovary
pancreas
prostate
Class

Figure 10

Figure 10(contd.)

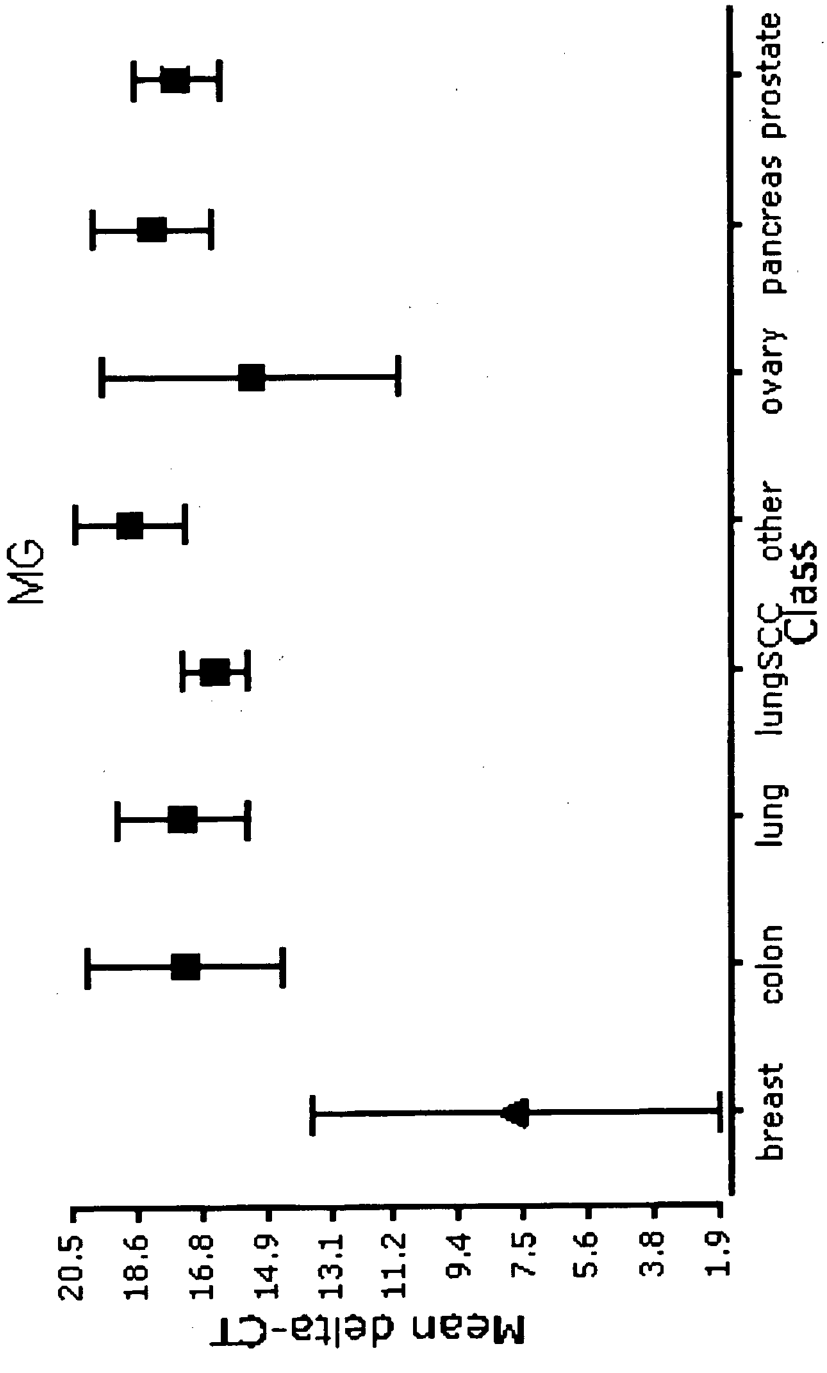

Figure 10(contd.)

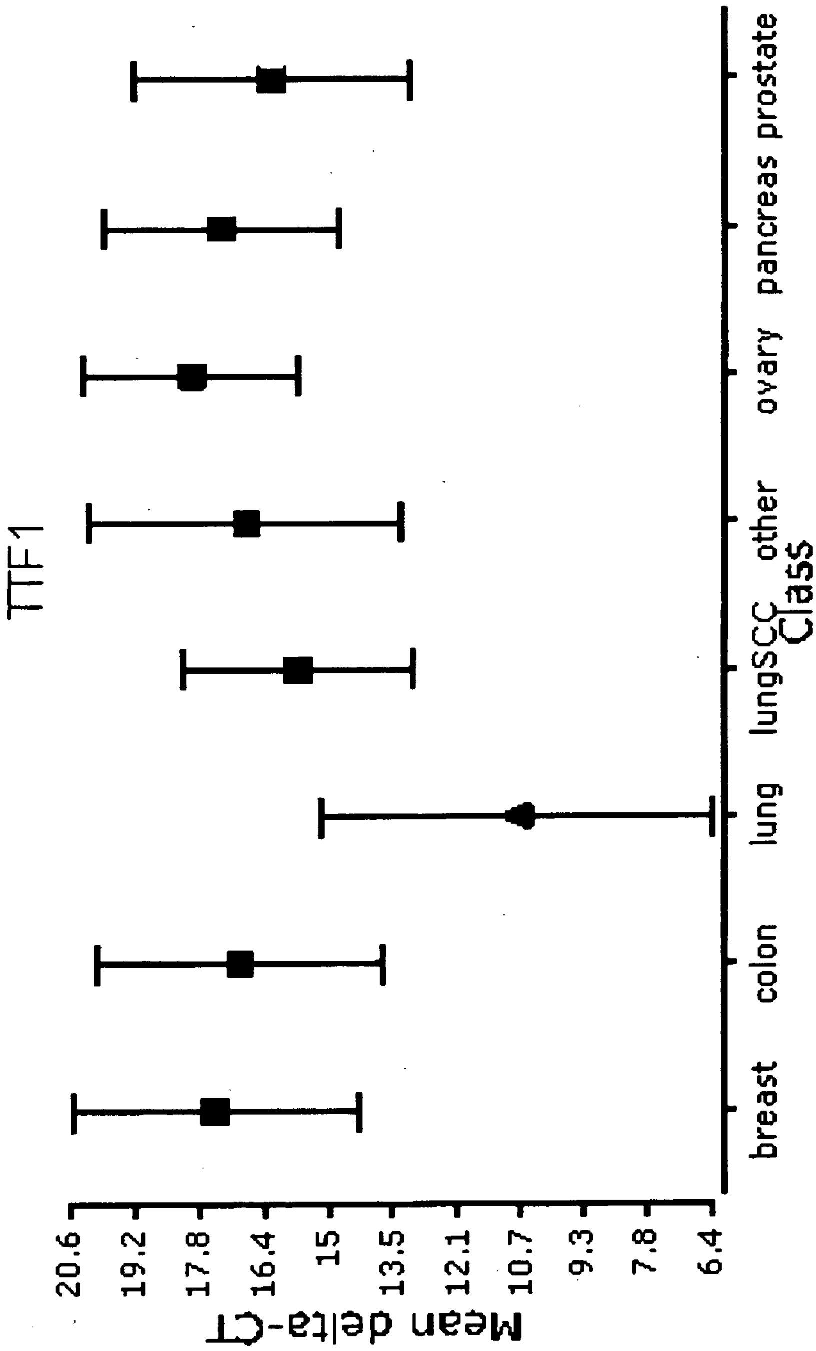

Figure 10(contd.)

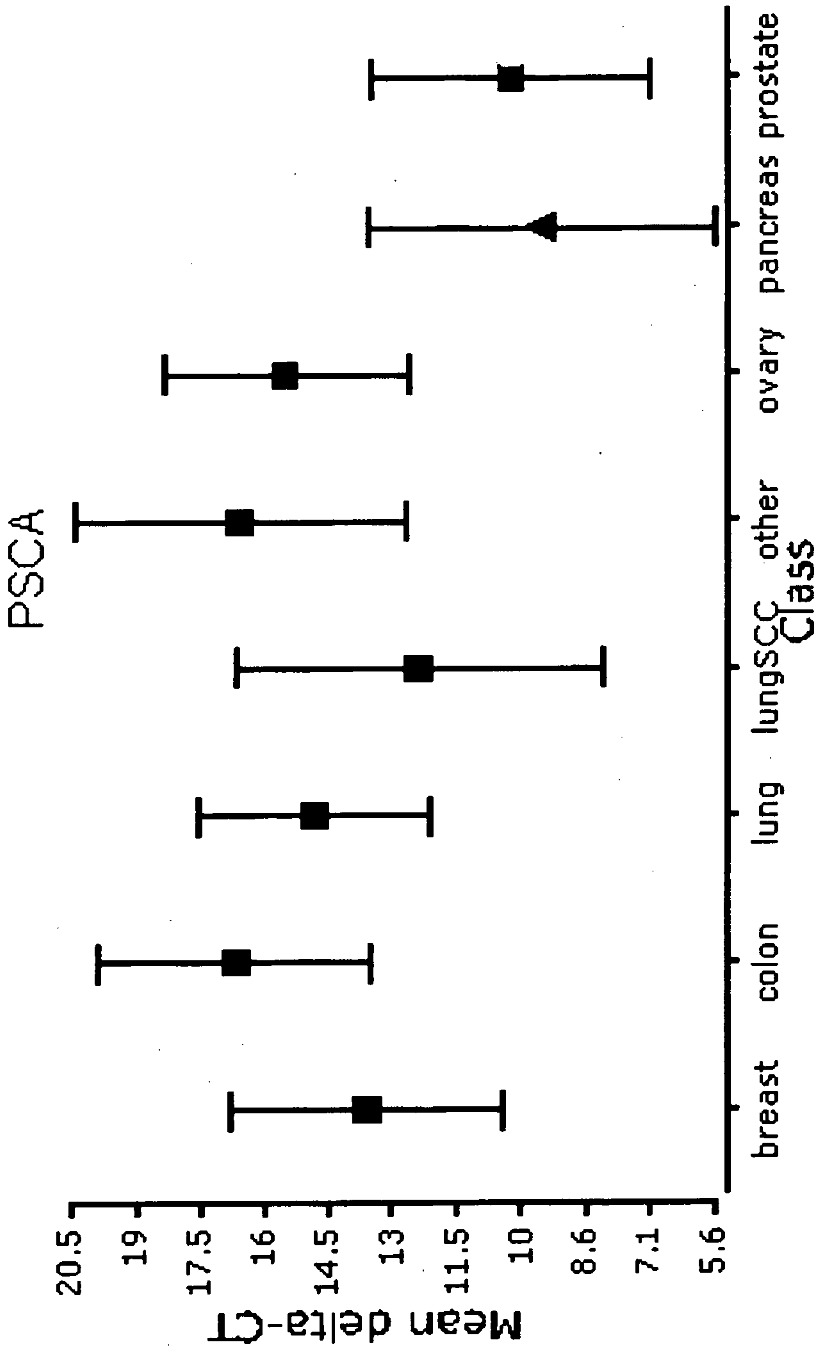

Figure 10(contd.)

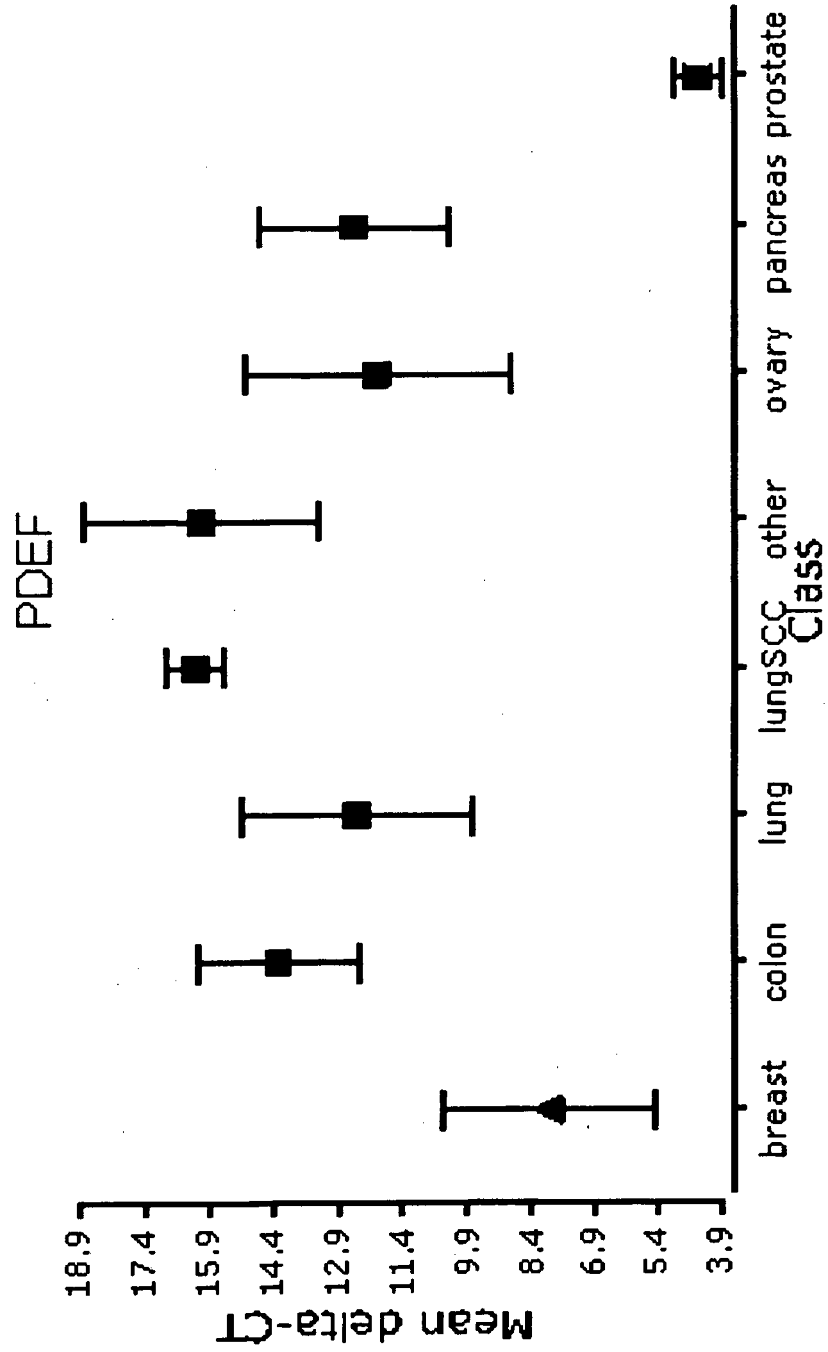

Figure 10(contd.)

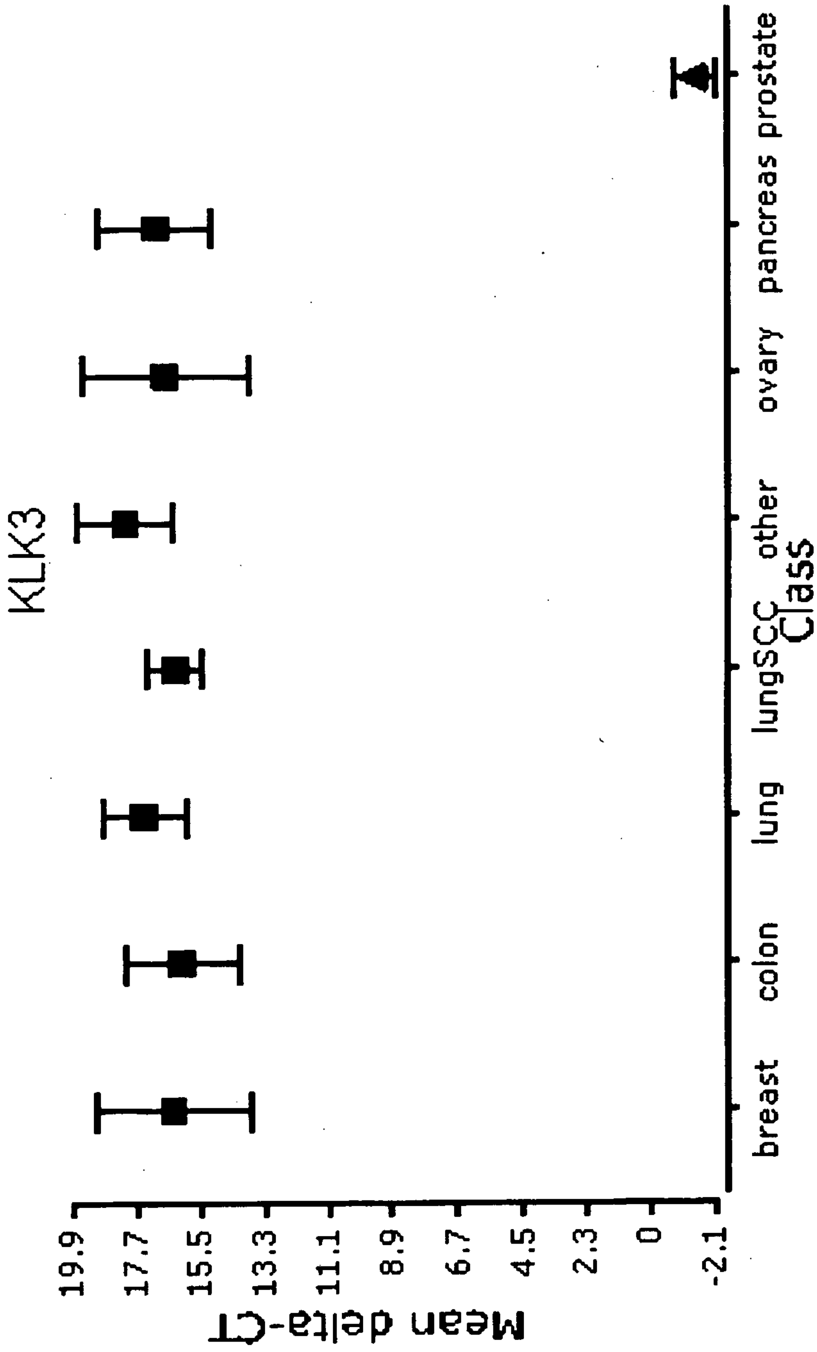

Figure 10(contd.)

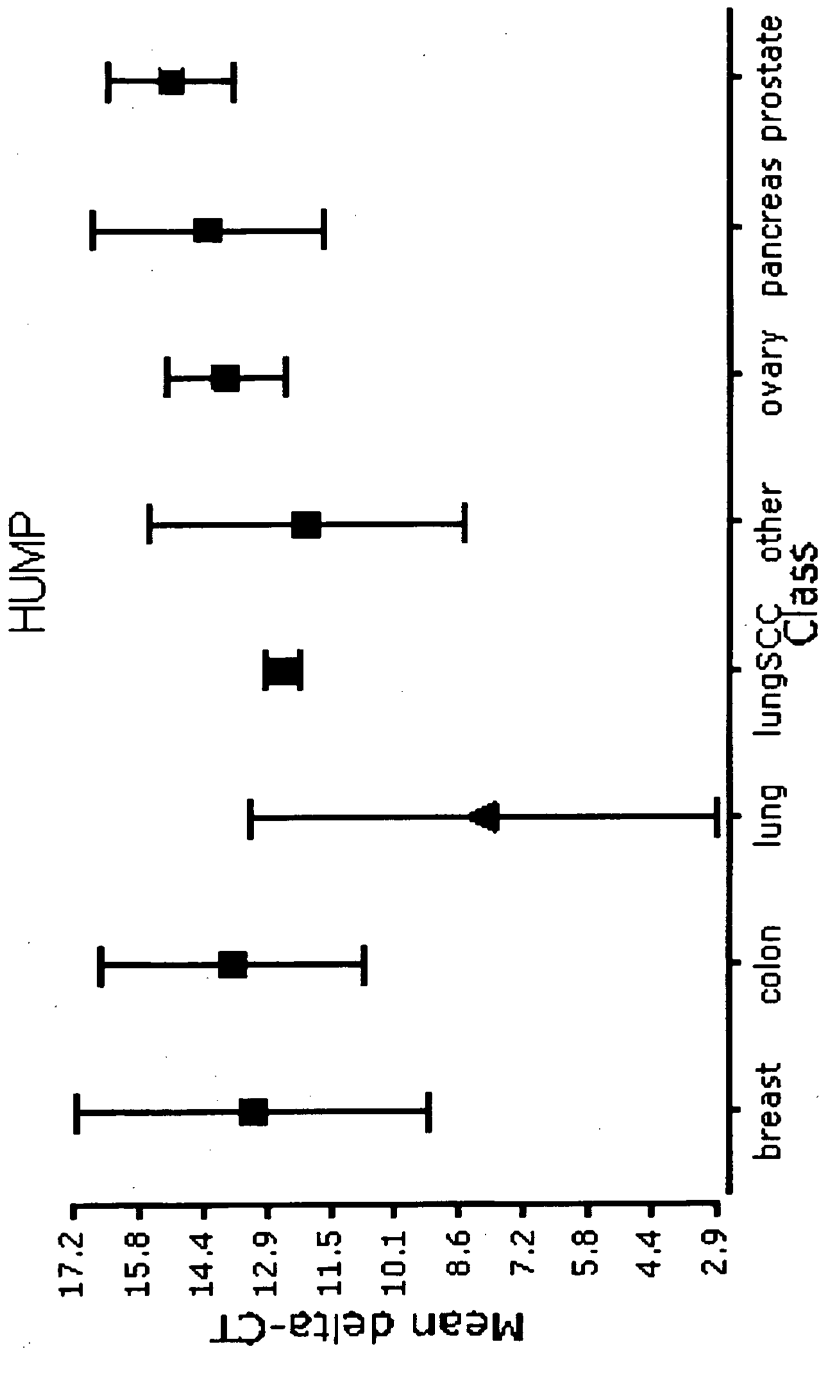

Figure 10(contd.)

CDH17

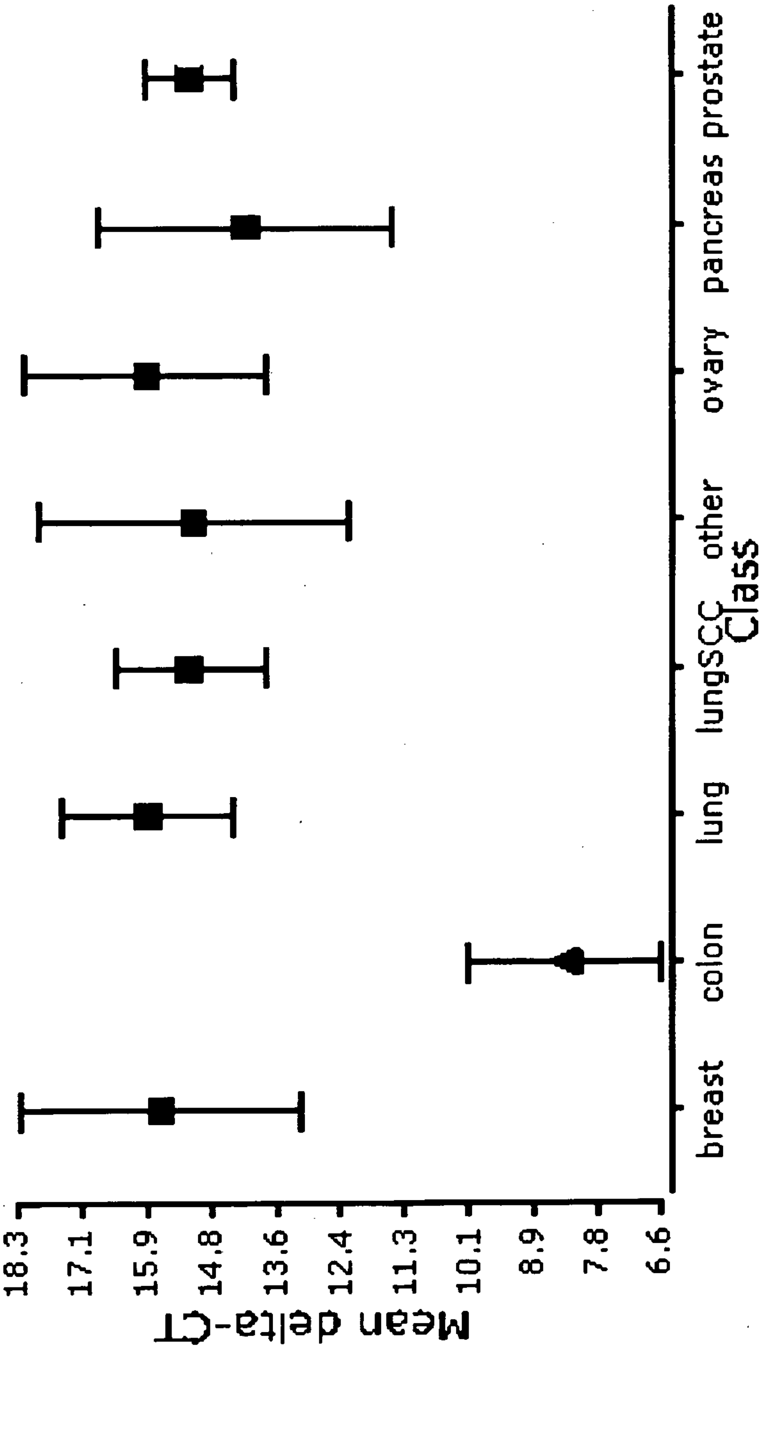

Figure 10(contd.)

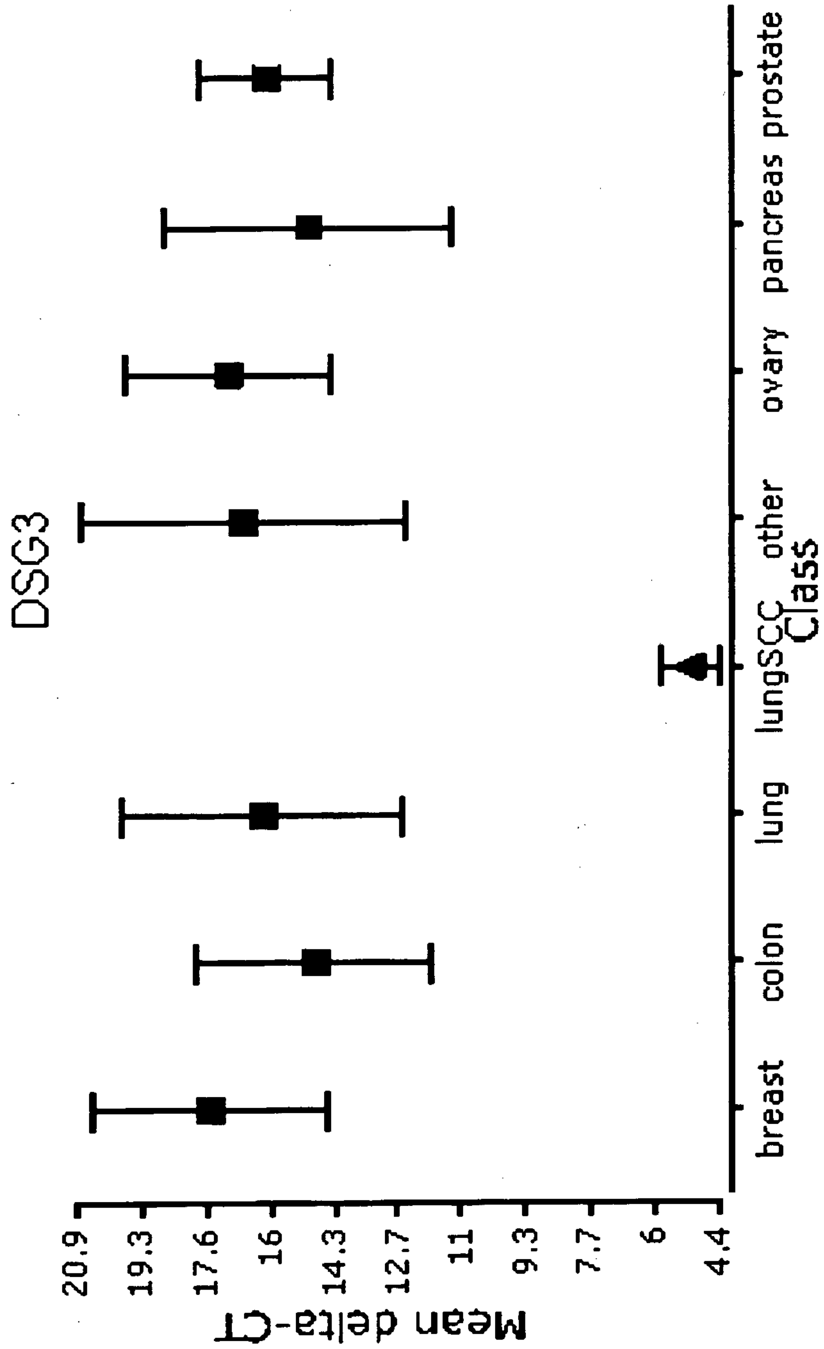

Figure 10(contd.)

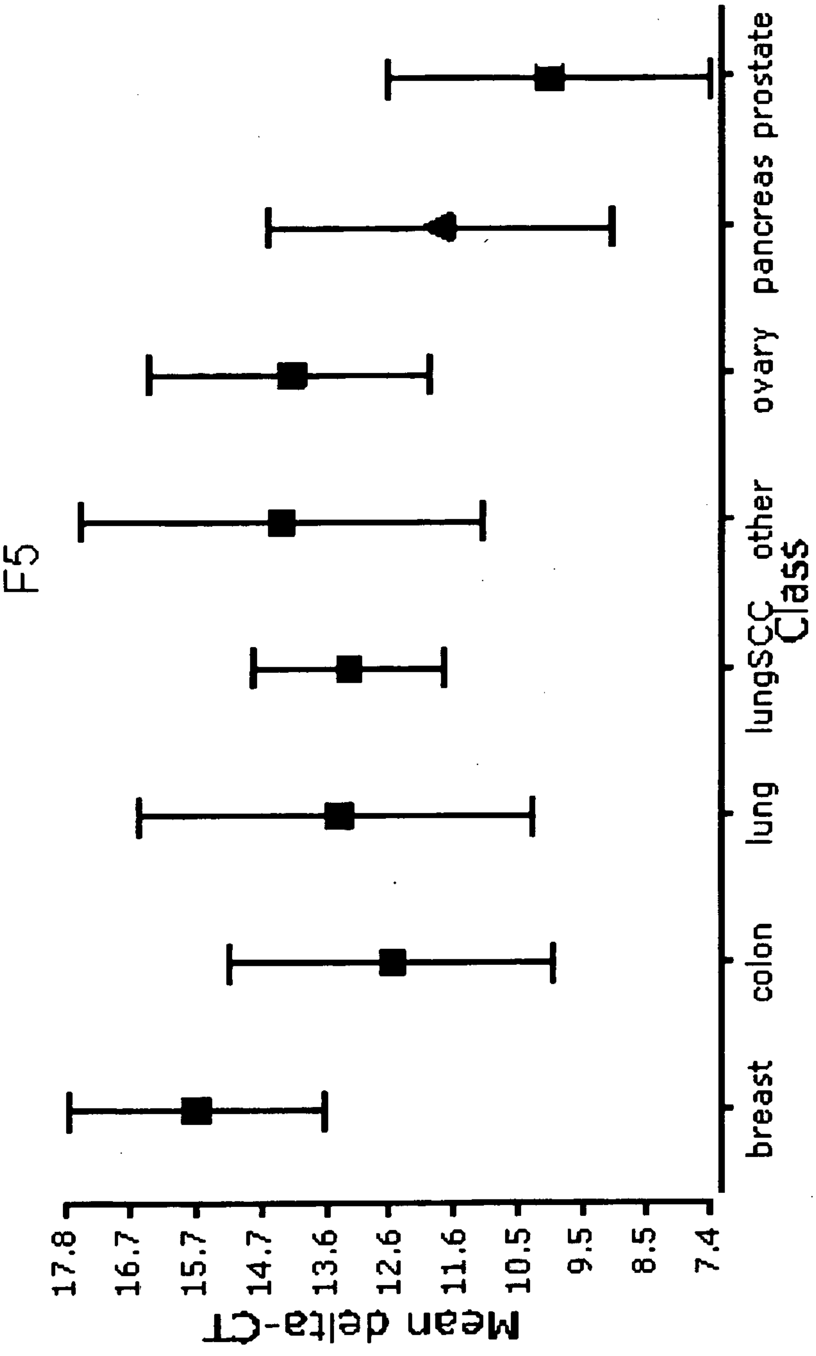

Figure 11(A)

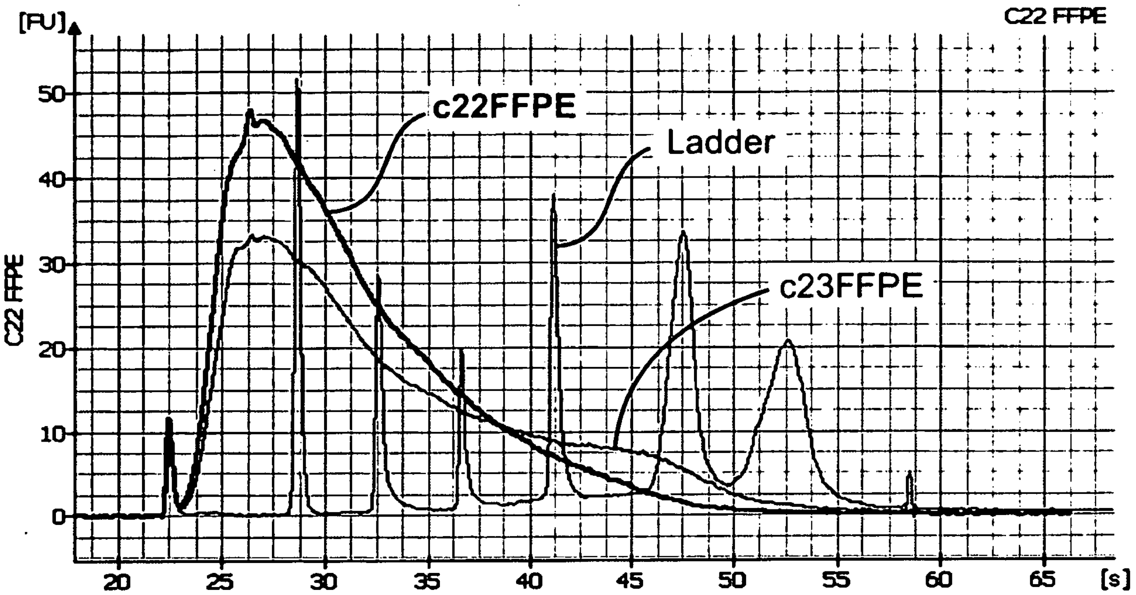


Figure 11(B)

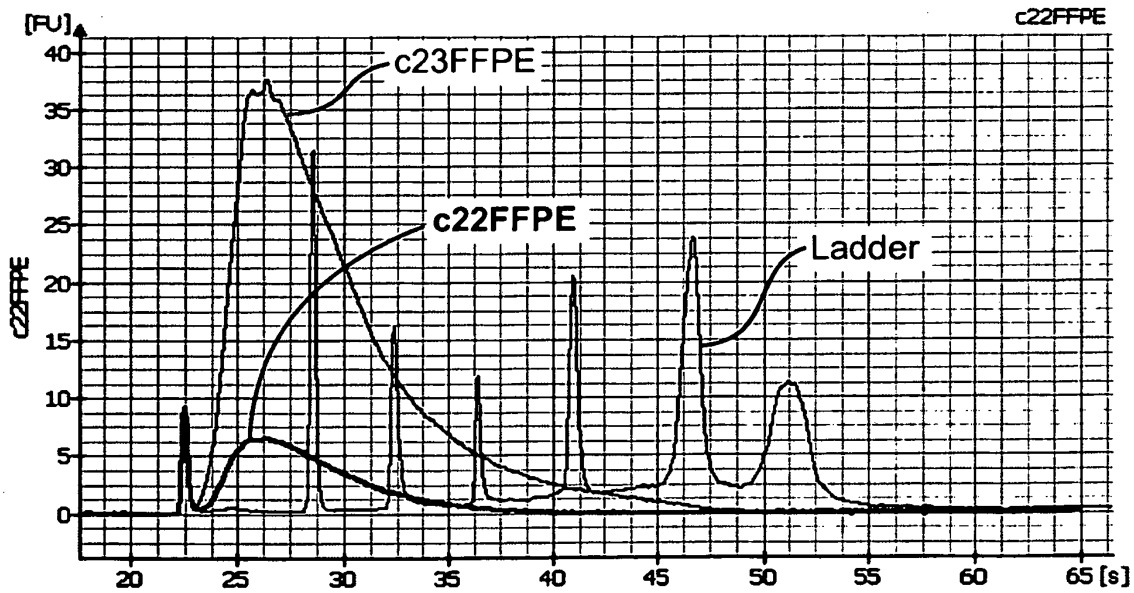

Figure 11(C)
β-actin

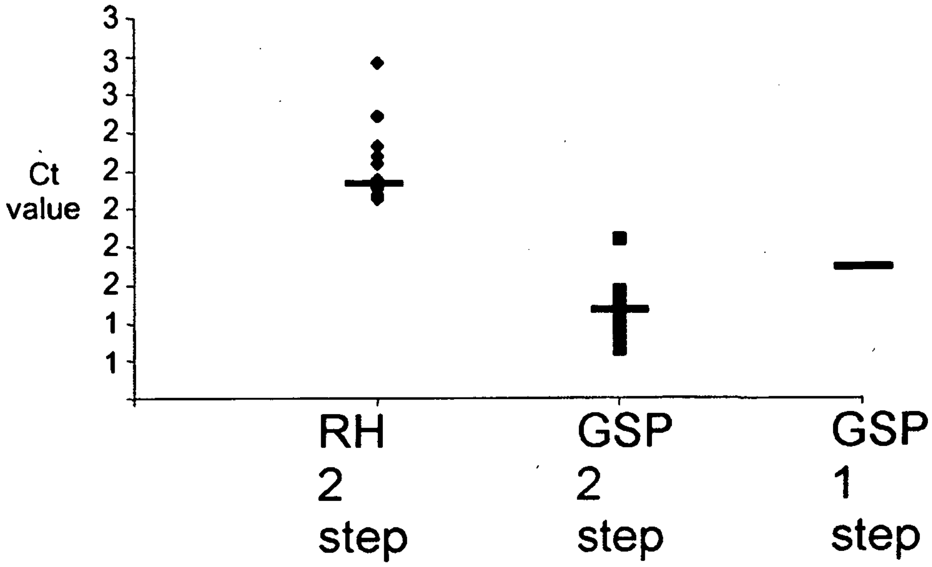


Figure 11(D)
HUMSPB

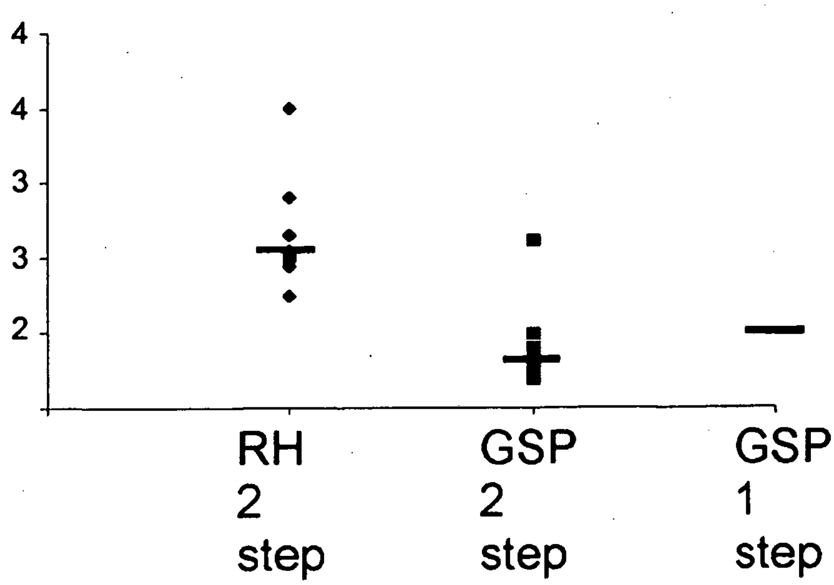

Figure 12

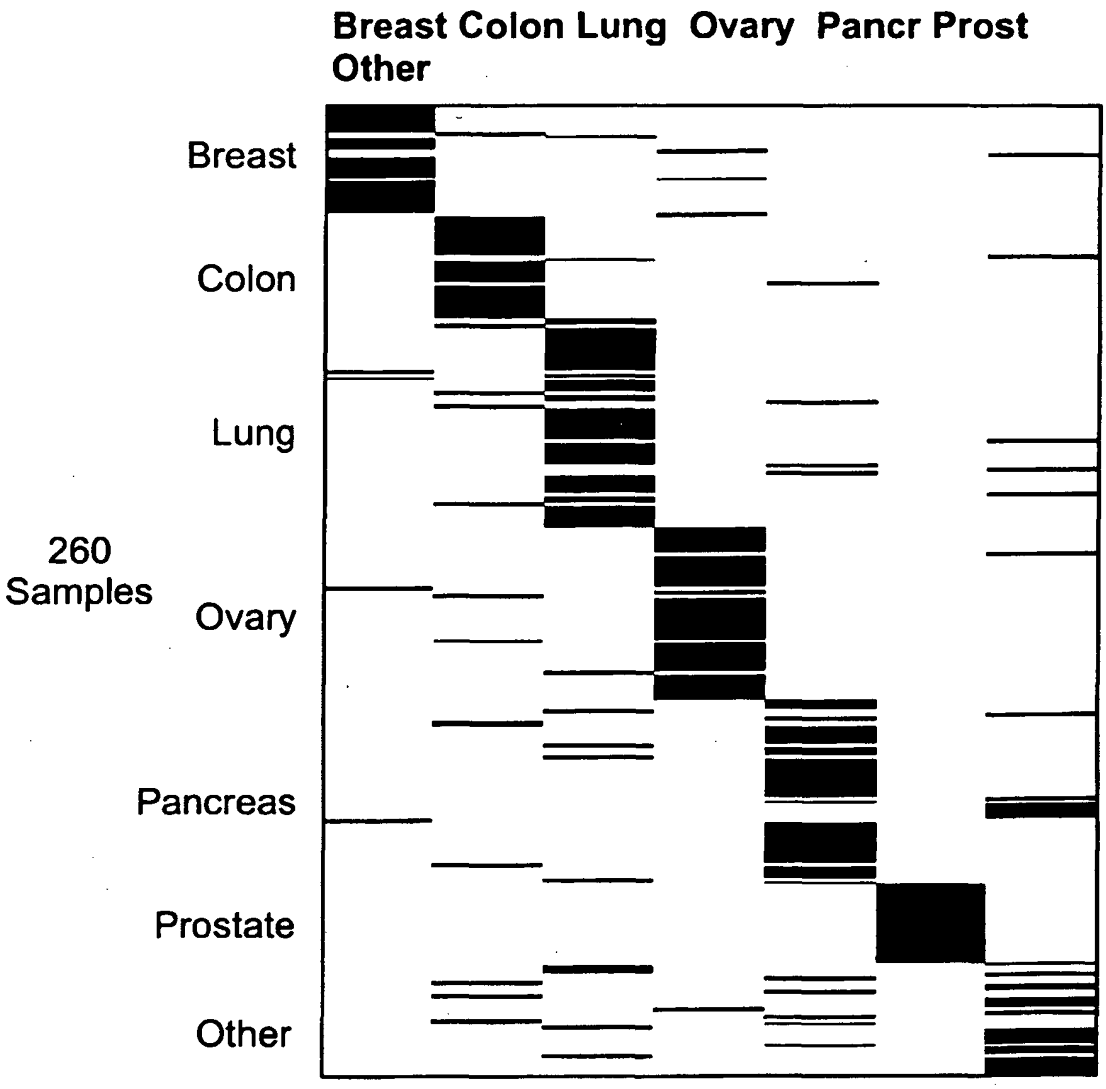
Breast Colon Lung Ovary Pancr Prost
Other
Breast
Colon
Lung
260
Samples
Ovary
Pancreas
Prostate
Other

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 20060094035 A
- WO 20040076955 A
- WO 20040005563 A
- WO 2004031412 A
- WO 1998040403 A
- WO 20030232350 A
- WO 2004063355 A
- WO 2004018999 A
- WO 6339148 A
- WO 20040770 A
- WO 20030235820 A
- WO 2005005601 A
- WO 20020055627 A
- WO 2000055320 A
- WO 20050059008 A
- WO 20010029020 A
- WO 20030219760 A
- US 20040219575 A
- WO 1998056953 A
- WO 2002073204 A
- WO 20030138793 A
- WO 2004030615 A
- WO 2002101357 A
- WO 20040146862 A
- WO 20040219572 A
- WO 2000006589 A
- WO 2001073032 A
- WO 20030124128 A
- WO 5668267 A
- WO 5350840 A
- WO 6232073 A
- WO 6225051 A
- WO 20050037010 A
- US 20030215835 A
- US 5786148 A
- US 6261766 B
- US 5242974 A
- US 5700637 A
- US 20030194733 A
- US 5350840 A
- US 20030198970 A
- US 5384261 A
- US 6004755 A
- US 20030215803 A
- US 5405783 A
- US 6136182 A
- US 5412087 A
- US 6218114 B
- US 20030219760 A
- US 5424186 A
- US 6218122 B
- US 20030219767 A
- US 5429807 A
- US 6225051 B
- US 20030232350 A
- US 5436327 A
- US 6232073 B
- US 20030235820 A
- US 5445934 A
- US 20040005563 A
- US 5472672 A
- US 6271002 B
- US 20040009154 A
- US 5527681 A
- US 6339148 B
- US 20040009489 A
- US 5529756 A
- US 20010029020 A
- US 20040018969 A
- US 5532128 A
- US 20020055627 A
- US 20040029114 A
- US 5545531 A
- US 20020068288 A
- US 20040076955 A
- US 5554501 A
- US 20020168647 A
- US 20040126808 A
- US 5556752 A
- US 20030044859 A
- US 20040146862 A
- US 5561071 A
- US 20030087818 A
- US 20040219572 A
- US 5571639 A
- US 20030104448 A
- US 5593839 A
- US 20030124128 A
- US 20050037010 A
- US 5599695 A
- US 20030124579 A
- US 20050059008 A
- US 5624711 A
- US 20030138793 A
- US 20060094035 A
- US 5658734 A
- US 20030190656 A
- WO 2002046467 A

- WO 2004077060 A
- WO 2001031342 A
- US 60718501 B
- US 60725680 B

**Non-patent literature cited in the description**


- **ABRAHAMSEN et al.** Towards quantitative mRNA analysis in paraffin-embedded tissues using real-time reverse transcriptase-polymerase chain reaction. *J Mol Diag,* 2003, vol. 5, 34-41
- **AL-MULLA et al.** BRCA1 gene expression in breast cancer: a correlative study between real-time RT-PCR and immunohistochemistry. *J Histochem Cytochem,* 2005, vol. 53, 621-629
- **ARGANI et al.** Discovery of new Markers of cancer through serial analysis of gene expression: prostate stem cell antigen is overexpressed in pancreatic adenocarcinoma. *Cancer Res,* 2001, vol. 61, 4320-4324
- **AUTIERO et al.** Intragenic amplification and formation of extrachromosomal small circular DNA molecules from the PIP gene on chromosome 7 in primary breast carcinomas. *Int J Cancer,* 2002, vol. 99, 370-377
- **BACKUS et al.** Identification and characterization of optimal gene expression Markers for detection of breast cancer metastasis. *J Mol Diagn,* 2005, vol. 7, 327-336
- **BENTOV.** The WT1 Wilms' tumor suppressor gene: a novel target for insulin-like growth factor-I action. *Endocrinol,* 2003, vol. 144, 4276-4279
- **BERA et al.** NGEP, a gene encoding a membrane protein detected only in prostate cancer and normal prostate. *Proc Natl Acad Sci USA,* 2004, vol. 101, 3059-3064
- **BIBIKOVA et al.** Quantitative gene expression profiling in formalin-fixed, paraffin-embedded tissues using universal bead arrays. *Am j Pathol,* 2004, vol. 165, 1799-1807
- **BLOOM et al.** Multi-platform, multi-site, microarray-based human tumor classification. *Am J Pathol,* 2004, vol. 164, 9-16
- **BORCHERS et al.** Heart-type fatty acid binding protein - involvement in growth inhibition and differentiation. *Prostaglandins Leukot Essent Fatty Acids,* 1997, vol. 57, 77-84
- **BORGONO et al.** Human tissue kallikreins: physiologic roles and applications in cancer. *Mol Cancer Res,* 2004, vol. 2, 257-280
- **BROOKES.** *The essence of SNPs Gene,* 1999, vol. 23, 177-186
- **BROWN et al.** Immunohistochemical identification of tumor Markers in metastatic adenocarcinoma. A diagnostic adjunct in the determination of primary site. *Am J Clin Pathol,* 1997, vol. 107, 12-19
- **BUCKHAULTS et al.** Identifying tumor origin using a gene expression-based classification map. *Cancer Res,* 2003, vol. 63, 4144-4149
- **CHAN et al.** Human liver fatty acid binding protein cDNA and amino acid sequence. Functional and evolutionary implications. *J Biol Chem,* 1985, vol. 260, 2629-2632
- *Somat Cell Mol Genet,* 1986, vol. 12, 303-306
- **CHEUNG et al.** Detection of the PAX8-PPAR gamma fusion oncogene in both follicular thyroid carcinomas and adenomas. *J Clin Endocrinol Metab,* 2003, vol. 88, 354-357
- **CLARK et al.** The potential role for prolactin-inducible protein (PIP) as a Marker of human breast cancer micrometastasis. *Br J Cancer,* 1999, vol. 81, 1002-1008
- **CRONIN et al.** Measurement of gene expression in archival paraffin-embedded tissue. *Am J Pathol,* 2004, vol. 164, 35-42
- **CUNHA et al.** Tissue-specificity of prostate specific antigens: Comparative analysis of transcript levels in prostate and non-prostatic tissues. *Cancer Lett,* 2006, vol. 236, 229-238
- **DENNIS et al.** Identification from public data of molecular Markers of adenocarcinoma characteristic of the site of origin. *Can Res,* 2002, vol. 62, 5999-6005
- **DENNIS et al.** Hunting the primary: novel strategies for defining the origin of tumors. *J Pathol,* 2005, vol. 205, 236-247
- **DENNIS et al.** Markers of adenocarcinoma characteristic of the site of origin: development of a diagnostic algorithm. *Clin Can Res,* 2005, vol. 11, 3766-3772
- **DEYOUNG et al.** Immunohistologic evaluation of metastatic carcinomas of unknown origin: an algorithmic approach. *Semin Diagn Pathol,* 2000, vol. 17, 184-193
- **DI PALMA et al.** The paired domain-containing factor Pax8 and the homeodomain-containing factor TTF-1 directly interact and synergistically activate transcription. *Biol Chem,* 2003, vol. 278, 3395-3402
- **DWIGHT et al.** Involvement of the PAX8 peroxisome proliferator-activated receptor gamma rearrangement in follicular thyroid tumors. *J Clin Endocrinol Metab,* 2003, vol. 88, 4440-4445
- **FELDMAN et al.** PDEF expression in human breast cancer is correlated with invasive potential and altered gene expression. *Cancer Res,* 2003, vol. 63, 4626-4631
- **FLEMING et al.** Mammaglobin, a breast-specific gene, and its utility as a Marker for breast cancer. *Ann N Y Acad Sci,* 2000, vol. 923, 78-89

- **FUKUSHIMA et al.** Characterization of gene expression in mucinous cystic neoplasms of the pancreas using oligonucleotide microarrays. *Oncogene,* 2004, vol. 23, 9042-9051
- **GHOSH et al.** Management of patients with metastatic cancer of unknown primary. *Curr Probl Surg,* 2005, vol. 42, 12-66
- **GIORDANO et al.** Organ-specific molecular classification of primary lung, colon, and ovarian adenocarcinomas using gene expression profiles. *Am J Pathol.,* 2001, vol. 159, 1231-1238
- **GLASSER et al.** cDNA, deduced polypeptide structure and chromosomal assignment of human pulmonary surfactant proteolipid. *SPL(pVal) J Biol Chem,* 1988, vol. 263, 9-12
- **GODFREY et al.** Quantitative mRNA expression analysis from formalin-fixed, paraffin-embedded tissues using 5' nuclease quantitative reverse transcription-polymerase chain reaction. *J Mol Diag,* 2000, vol. 2, 84-91
- **GOLDSTEIN et al.** WT1 immunoreactivity in uterine papillary serous carcinomas is different from ovarian serous carcinomas. *Am J Clin Pathol,* 2002, vol. 117, 541-545
- **GRADI et al.** The human steroidogenic acute regulatory (StAR) gene is expressed in the urogenital system and encodes a mitochondrial polypeptide. *Biochim Biophys Acta,* 1995, vol. 1258, 228-233
- **GRECO et al.** Carcinoma of unknown primary site: sequential treatment with paclitaxel/carboplatin/etoposide and gemcitabine/irinotecan: A Minnie Pearl cancer research network phase II trial. *The Oncologist,* 2004, vol. 9, 644-652
- **HAAS et al.** Combined application of RT-PCR and immunohistochemistry on paraffin embedded sentinel lymph nodes of prostate cancer patients. *Pathol Res Pract,* 2005, vol. 200, 763-770
- **HWANG et al.** Wilms tumor gene product: sensitive and contextually specific Marker of serous carcinomas of ovarian surface epithelial origin. *Appl Immunohistochem Mol Morphol,* 2004, vol. 12, 122-126
- **ISHIKAWA et al.** Experimental trial for diagnosis of pancreatic ductal carcinoma based on gene expression profiles of pancreatic ductal cells. *Cancer Sci,* 2005, vol. 96, 387-393
- **ITALIANO et al.** Epidermal growth factor receptor (EGFR) status in primary colorectal tumors correlates with EGFR expression in related metastatic sites: biological and clinical implications. *Ann Oncol,* 2005, vol. 16, 1503-1507
- **JONES et al.** Comprehensive analysis of matrix metalloproteinase and tissue inhibitor expression in pancreatic cancer: increased expression of matrix metalloproteinase-7 predicts poor survival. *Clin Cancer Res,* 2004, vol. 10, 2832-2845
- **JONES et al.** Thyroid transcription factor 1 expression in small cell carcinoma of the urinary bladder: an immunohistochemical profile of 44 cases. *Hum Pathol,* 2005, vol. 36, 718-723
- **KHOOR et al.** Expression of surfactant protein B precursor and surfactant protein B mRNA in adenocarcinoma of the lung. *Mod Pathol,* 1997, vol. 10, 62-67
- **KIM.** Comparison of oligonucleotide-microarray and serial analysis of gene expression (SAGE) in transcript profiling analysis of megakaryocytes derived from CD34+ cells. *Exp Mol Med,* 2003, vol. 35, 460-466
- **KIM et al.** Steroidogenic acute regulatory protein expression in the normal human brain and intracranial tumors. *Brain Res,* 2003, vol. 978, 245-249
- **LAM et al.** Prostate stem cell antigen is overexpressed in prostate cancer metastases. *Clin Can Res,* 2005, vol. 11, 2591-2596
- **LEMBERSKY et al.** Metastases of unknown primary site. *Med Clin North Am.,* 1996, vol. 80, 153-171
- **LEWIS et al.** Unlocking the archive-gene expression in paraffin-embedded tissue. *J Pathol,* 2001, vol. 195, 66-71
- **LIPSHUTZ et al.** High density synthetic oligonucleotide arrays. *Nature Genetics,* 1999, vol. 21, 20-24
- **LOWE et al.** Human liver fatty acid binding protein. Isolation of a full length cDNA and comparative sequence analyses of orthologous and paralogous proteins. *J Biol Chem,* 1985, vol. 260, 3413-3417
- **MA et al.** Molecular classification of human cancers using a 92-gene real-time quantitative polymerase chain reaction assay. *Arch Pathol Lab med,* 2006, vol. 130, 465-473
- **MAGKLARA et al.** Characterization of androgen receptor and nuclear receptor co-regulator expression in human breast cancer cell lines exhibiting differential regulation of kallikreins 2 and 3. *Int J Cancer,* 2002, vol. 100, 507-514
- **MARKOWITZ.** *Portfolio Selection J Finance,* 1952, vol. 7, 77-91
- **MARQUES et al.** Expression of PAX8-PPAR gamma 1 rearrangements in both follicular thyroid carcinomas and adenomas. *J Clin Endocrinol Metab,* 2002, vol. 87, 3947-3952
- **MASUDA et al.** Analysis of chemical modification of RNA from formalin-fixed samples and optimization of molecular biology applications for such samples. *Nucl Acids Res,* 1999, vol. 27, 4436-4443
- **MCCARTHY et al.** Novel Markers of pancreatic adenocarcinoma in fine-needle aspiration: mesothelin and prostate stem cell antigen labeling increases accuracy in cytologically borderline cases. *Appl Immunohistochem Mol Morphol,* 2003, vol. 11, 238-243
- *BioTechniques,* 2004, vol. 36, 1-4

- **MONIAUX et al.** Multiple roles of mucins in pancreatic cancer, a lethal and challenging malignancy. *Br J Cancer,* 2004, vol. 91, 1633-1638
- **MURPHY et al.** Isolation and sequencing of a cDNA clone for a prolactin-inducible protein (PIP). Regulation of PIP gene expression in the human breast cancer cell line. *T-47D J Biol Chem,* 1987, vol. 262, 15236-15241
- **MYAL et al.** The prolactin-inducible protein (PIPGCDFP-15) gene: cloning, structure and regulation. *J Mol Cell Endocrinol,* 1991, vol. 80, 165-175
- **NAKAMURA et al.** Expression of thyroid transcription factor-1 in normal and neoplastic lung tissues. *Mod Pathol,* 2002, vol. 15, 1058-1067
- **NOONAN et al.** Characterization of the homeodomain gene EMX2: sequence conservation, expression analysis, and a search for mutations in endometrial cancers. *Genomics,* 2001, vol. 76, 37-44
- **OETTGEN et al.** PDEF, a novel prostate epithelium-specific Ets transcription factor, interacts with the androgen receptor and activates prostate-specific antigen gene expression. *J Biol Chem,* 2000, vol. 275, 1216-1225
- **OJI et al.** Overexpression of the Wilms' tumor gene WT1 in head and neck squamous cell carcinoma. *Cancer Sci,* 2003, vol. 94, 523-529
- **PAVLIDIS et al.** Diagnostic and therapeutic management of cancer of an unknown primary. *Eur J Can,* 2003, vol. 39, 990-2005
- **PILOT-MATHIAS et al.** Structure and organization of the gene encoding human pulmonary surfactant proteolipid. *SP-B DNA,* 1989, vol. 8, 75-86
- **PILOZZI et al.** CDX1 expression is reduced in colorectal carcinoma and is associated with promoter hypermethylation. *J Pathol,* 2004, vol. 204, 289-295
- **POLEEV et al.** PAX8, a human paired box gene: isolation and expression in developing thyroid, kidney and Wilms' tumors. *Development,* 1992, vol. 116, 611-623
- **PRASAD et al.** Gene expression profiles in pancreatic intraepithelial neoplasia reflect the effects of Hedgehog signaling on pancreatic ductal epithelial cells. *Cancer Res,* 2005, vol. 65, 1619-1626
- **RAMASWAMY.** Translating cancer genomics into clinical oncology. *N Engl J Med,* 2004, vol. 350, 1814-1816
- **RAMASWAMY et al.** Multiclass cancer diagnosis using tumor gene expression signatures. *Proc Natl Acad Sci USA,* 2001, vol. 98, 15149-15154
- **RAUSCHER.** The WT1 Wilms tumor gene product: a developmentally regulated transcription factor in the kidney that functions as a tumor suppressor. *FASEB J,* 1993, vol. 7, 896-903
- **REINHOLZ et al.** Evaluation of a panel of tumor Markers for molecular detection of circulating cancer cells in women with suspected breast cancer. *Clin Cancer Res,* 2005, vol. 11, 3722
- **SCHLAG et al.** Cancer of unknown primary site. *Ann Chir Gynaecol,* 1994, vol. 83, 8-12
- **SENOO et al.** A second p53-related protein, p73L, with high homology to p73. *Biochem Biophys Res Comm,* 1998, vol. 248, 603-607
- **SPECHT et al.** Quantitative gene expression analysis in microdissected archival formalin-fixed and paraffin-embedded tumor tissue. *Amer J Pathol,* 2001, vol. 158, 419-429
- **SU et al.** Molecular classification of human carcinomas by use of gene expression signatures. *Cancer Res,* 2001, vol. 61, 7388-7393
- **TAKAHASHI et al.** Cloning and characterization of multiple human genes and cDNAs encoding highly related type II keratin 6 isoforms. *J Biol Chem,* 1995, vol. 270, 18581-18592
- **TAKAMURA et al.** Reduced expression of liver-intestine cadherin is associated with progression and lymph node metastasis of human colorectal carcinoma. *Cancer Lett,* 2004, vol. 212, 253-259
- **TOTHILL et al.** An expression-based site of origin diagnostic method designed for clinical application to cancer of unknown origin. *Can Res,* 2005, vol. 65, 4031-4040
- **VAN RUISSEN et al.** Evaluation of the similarity of gene expression data estimated with. *SAGE and Affymetrix GeneChips BMC Genomics,* 2005, vol. 6, 91
- **VARADHACHARY et al.** Diagnostic strategies for unknown primary cancer. *Cancer,* 2004, vol. 100, 1776-1785
- **VENABLES et al.** Modern Applied Statistics with S. Springer, 2002
- **WALLACE et al.** Accurate Molecular detection of non-small cell lung cancer metastases in mediastinal lymph nodes sampled by endoscopic ultrasound-guided needle aspiration. *Cest,* 2005, vol. 127, 430-437
- **WAN et al.** Desmosomal proteins, including desmoglein 3, serve as novel negative Markers for epidermal stem cell-containing population of keratinocytes. *J Cell Sci,* 2003, vol. 116, 4239-4248
- **WATSON et al.** Mammaglobin, a mammary-specific member of the uteroglobin gene family, is overexpressed in human breast cancer. *Cancer Res,* 1996, vol. 56, 860-865
- **WATSON et al.** Structure and transcriptional regulation of the human mammaglobin gene, a breast cancer associated member of the uteroglobin gene family localized to chromosome 11q13. *Oncogene,* 1998, vol. 16, 817-824

- **WEIGELT et al.** Gene expression profiles of primary breast tumors maintained in distant metastases. *Proc Natl Acad Sci USA,* 2003, vol. 100, 15901-15905
- **ZAPATA-BENAVIDES et al.** Downregulation of Wilms' tumor 1 protein inhibits breast cancer proliferation. *Biochem Biophys Res Commun,* 2002, vol. 295, 784-790